# EUROPEAN PATENT SPECIFICATION

(11) **EP 1 838 674 B1**
(45) Date of publication and mention of the grant of the patent: **30.03.2011**
(21) Application number: 06718419.2
(22) Date of filing: 13.01.2006
(51) Int. Cl.: C07D 213/02, C07D 401/02, A61K 31/44

(54) **HETEROARYL SULFONAMIDES AND CCR2**
HETEROARYLSULFONAMIDE UND CCR2
SULFONAMIDES D'HETEROARYLE ET CCR2

(30) Priority: 14.01.2005 US 644103 P; 06.12.2005 US 742821 P; 16.12.2005 US 750985 P
(43) Date of publication of application: 03.10.2007
(73) Proprietor: ChemoCentryx, Inc., Mountain View California 94043 (US)
(72) Inventor: UNGASHE, Solomon, Fremont, California 94536 (US); WRIGHT, John, Jessen, Sandringam, VIC 3191 (AU); ZENG, Yibin, San Mateo, CA 94403 (US); BADAK, Arindrajit, Mountain View, CA 94041 (US); CHARVAT, Trevor, T., San Jose, CA 95132 (US); DAIRAGHI, Daniel, Palo Alto, CA 94306 (US); HANSEN, Derek, San Francisco, CA 94017 (US); JIN, Jeff, Fremont, CA 94555 (US); MOORE, Jimmie, Redwood City, CA 94065 (US); PENNELL, Andrew M.K., San Francisco, CA 94131 (US); PUNNA, Sreenivas, Sunnyvale, CA 94086 (US); WEI, Zheng, Union City, CA 94587 (US)
(74) Representative: Hayes, Adrian Chetwynd
(86) International application number: PCT/US2006/001341
(87) International publication number: WO 2006/076644

(56) References cited:
- EP-A- 0 472 053
- EP-A- 0 937 711
- EP-A- 1 334 719
- WO-A-01/00611
- WO-A-01/72744
- WO-A-03/032994
- WO-A-03/051870
- WO-A-2004/056774
- WO-A-2004/058265
- WO-A-2004/105794
- WO-A1-03/099773
- JP-A- 6 135 934
- JP-A- 61 113 060
- JP-A- 2000 159 665
- JP-A- 2001 089 412
- US-A- 3 901 855
- US-A1- 2003 229 081
- US-B1- 6 489 452
- YOSHIMATSU K ET AL: "Mechanism of action of E7010, an orally active sulfonamide antitumor agent: Inhibition of mitosis by binding to the colchicine site of tubulin" CANCER RESEARCH, AMERICAN ASSOCIATION FOR CANCER RESEARCH, BALTIMORE, MD., US, vol. 57, no. 15, 1 January 1997 (1997-01-01), pages 3208-3213, XP002460383 ISSN: 0008-5472
- HIROSHI YOSHINO, NORIHIRO UEDA, JUN NIIJIMA ET AL.: "Novel sulfonamides as potential systemically active antitumor agents" J. MED. CHEM., vol. 35, 1992, pages 2496-2497, XP002556905
- SEIICHI YOGI, KOZO HOKAMA, SADAMORI TAKAYOSHI, OTOHIKO TSUGE: "Synthesis of imido-substituted 3,8-diphenyl-1,2-diazacycloocta-2,4,6,8-te traenes and their thermolysis" BULL. CHEM. SOC. JPN., vol. 60, 1987, pages 731-735, XP002556906
- MASAKI KUSE, NOBUHIRO KONDO, YUKI OHYABU, MINORU ISOBE: "Novel synthetic route of aryl-aminopyrazine" TETRAHEDRON, vol. 60, 2004, pages 835-840, XP002556907
- M. SPRECHER, D. B. SPRINSON: "Methylated purines and pyrimidines. II. Synthesis and properties of 2,6-diamino-5-(methylamino)-4-pyrimidinol" BIOCHEMISTRY, vol. 4, no. 4, 1965, pages 655-661, XP002556908
- HELLMUT BREDERECK, ULRICH GOTSMANN: "Synthesen in der Purinreihe. XVI. Über die Darstellung von 5-Alkyl- bzw. 5-Arylsulfonylamino-4-amino-uracilen, 4-Amino-5-alkylamino-uracilen und 4-Amino-5-[pyridinio-methylenamino]-uracil -chloriden" CHEMISCHE BERICHTE, vol. 95, 1962, pages 1902-1909, XP002556909
- B. R. BAKER, ROBERT E. SCHAUB, JOSEPH P. JOSEPH: "Puromycin. Synthetic studies. II. The position of glycosidation on the 6-dimethylaminopurine moiety" J. ORG. CHEM., vol. 19, 1954, pages 638-645, XP002556910
- FRANCIS G. MCDONALD, RUDOLPH C. ELLINGSON: "Pyrazine chemistry. II. Derivatives of 3-hydroxypyrazinoic acid" J. AM. CHEM. SOC., vol. 69, 1947, pages 1034-1037, XP002556911
- RUDOLPH C. ELLINGSON, ROBERT L. HENRY: "Pyrazine chemistry. III. Derivatives of 3-amino-5,6-dimethylpyrazinoic acid" J. AM. CHEM. SOC., vol. 70, 1948, pages 1257-1261, XP002556912
- DATABASE CROSSFIRE BEILSTEIN BEILSTEIN INSTITUT ZUR FOERDERUNG DER WISSENSCHAFTEN, FRANKFURT AM MAIN, DE; XP002464251 Database accession no. BRN = 7929845 & P. K. DUBEY, R. VINOD KUMAR,: INDIAN J. CHEM. SECT. B, vol. 39, no. 10, 2000, pages 746-751,
- DATABASE CROSSFIRE BEILSTEIN BEILSTEIN INSTITUT ZUR FOERDERUNG DER WISSENSCHAFTEN, FRANKFURT AM MAIN, DE; XP002464252 Database accession no. BRN = 7313089 & H. I. EL-SUBBAGH, M. A. EL-SHERBENY, M. N. NASR, F. E. GODA, F. A. BADRIA: BOLL. CHIM. FARM., vol. 134, no. 2, 1995, pages 80-84,
- DATABASE CROSSFIRE BEILSTEIN BEILSTEIN INSTITUT ZUR FOERDERUNG DER WISSENSCHAFTEN, FRANKFURT AM MAIN, DE; XP002464255 Database accession no. BRN = 6875780 & P. PECORARI, G. VAMPA, A. ALBASINI, A. RINALDI, M. MELEGARI, A. PROVVISIONATO: IL FARMACO, vol. 38, no. 5, 1983, pages 352-359,

## Description

### RELATED APPLICATIONS

This application claims priority to U.S. provisional applications Serial No. 60/644,103 filed January, 14, 2005; Serial No. 60/742,821, filed December 6, 2005 (entitled "Heteroaryl Sulfonamides and CCR2", and filed as attorney reference number 10709/82); and Serial No. 60/750,985, filed December 16, 2005 (entitled "Heteroaryl Sulfonamides and CCR2" and filed as attorney reference number 10709/82A).

### FEDERALLY SPONSORED RESEARCH OR DEVELOPMENT

The present invention described herein was supported at least in part by NIH (U19-AI056690). The government has certain rights in the invention.

### BACKGROUND

The present invention provides compounds, pharmaceutical compositions containing one or more of those compounds or their pharmaceutically acceptable salts, which are effective in inhibiting the binding or function of various chemokines to chemokine receptors. As antagonists or modulators of chemokine receptors, the compounds and compositions have utility in treating various immune disorder conditions and diseases.

Chemokines, also known as chemotactic cytokines, are a group of small molecular-weight proteins that are released by a wide variety of cells and have a variety of biological activities. Chemokines attract various types of cells of the immune system, such as macrophages, T cells, eosinophils, basophils and neutrophils, and cause them to migrate from the blood to various lymphoid and none-lymphoid tissues. They mediate infiltration of inflammatory cells to sites of inflammation, and are responsible for the initiation and perpetuation of many inflammation diseases (reviewed in Schall, Cytokine, 3:165-183 (1991), Schall et al., Curr. Opin. Immunol., 6:865 873 (1994)).

In addition to stimulating chemotaxis, chemokines can induce other changes in responsive cells, including changes in cell shape, granule exocytosis, integrin up-regulation, formation of bioactive lipids (e.g., leukotrienes), respiratory burst associated with leukocyte activation, cell proliferation, resistance to induction of apoptosis and angiogenesis. Thus, chemokines are early triggers of the inflammatory response, causing inflammatory mediator release, chemotaxis and extravasation to sites of infection or inflammation. They are also stimulators of a multitude of cellular processes that bear important physiological functions as well as pathological consequences.

Chemokines exert their effects by activating chemokine receptors expressed by responsive cells. Chemokine receptors are a class of G-protein coupled receptors, also known as seven-transmembrane receptors, found on the surface of a wide variety of cell types such as leukocytes, endothelial cells, smooth muscle cells and tumor cells.

Chemokine receptor CCR2 is found on the surface of monocytes, macrophages, B cells, activated T cells, dendritic cells, endothelial cells and tumor cells. It is a receptor for a number of chemokine ligands, including MCP-1, MCP-2, MCP-3 and MCP-4. Among them, MCP-1 appears to interact only with CCR2, and not any other chemokine receptors identified so far.

CCR2 mediates migration of monocytes, antigen-presenting cells (also called dendritic cells) and lymphocytes to various tissues under inflammatory conditions. CCR2 has been implicated in the pathogenesis of a number of diseases, including atherosclerosis, restenosis, multiple sclerosis, pulmonary fibrosis, inflammatory bowel disease, rheumatoid arthritis, renal fibrosis, psoriasis, transplantation rejection, graft-versus-host disease, obesity, diabetes and cancer.

CCR2-mediated monocyte recruitment is one of the earliest steps that lead to the development of atherosclerosis. CCR2 is expressed by monocytes and is essential to migration of these cells to the artery well, where its ligand MCP-1 is highly expressed. In experimental models of atherosclerosis, arterial plague formation depends on the integrity of CCR2 and MCP-1, since deletion of either genes results in decreased atherosclerotic lesion formation in mice that otherwise develop severe disease (Gu et al., Mol. Ce// 2:275-81 (1998); Boring et al., Nature 394:894-7 (1998); Boring et al., J. Clin. Invest. 100:2552-61 (1997)).

In addition to many inflammation diseases, neuropathic pain is a condition in which CCR2 signaling may play a pathogenic role. It has been shown that the absence of CCR2 reduces inflammatory and neuropathic pain in mouse pain models, suggesting that recruitment and activation of macrophage and microglia to neural tissues play an important role in the pain states (Abbadie et al., Proc. Natl. Acad Sci. USA. 100:13 (2003)). Small molecule antagonists of CCR2 described in this patent may useful in the treatment of chronic pain.

CCR2 has also been implicated in restenosis, the reclosure of the artery after balloon angioplasty. Studies in animal models have shown that restenosis is initiated, at least in part, by infiltration of monocytes to the site of artery injury. Deficiency of CCR2 or blockade of MCP-1 activity dramatically inhibits cell proliferation and expansion of the artery wall's inner lining (Furukawa et al., Circ. Res. 84:306-14 (1999); Egashira et al., Circ. Res. 90:1167-72 (2002); (Roque et al., Arterioscler. Thromb. Vasc. Biol. 22:554-9 (2002); Horvath et al., Circ. Res. 90:488-94 (2002); Egashira et al., FASEB J 14:1974-8 (2000)).

CCR2-mediated migration of monocytes is believed to be pathogenic in human multiple sclerosis (MS), an inflammatory demyelinating disease of the central nervous system (CNS). CCR2 and MCP-1 expression is present in the cerebrospinal fluid (CSF) in MS patients. In a mouse model of human MS, namely the experimental autoimmune encephalomyelitis (EAE), deficiency in CCR2 or MCP-1 prevents the development of EAE (Izikson et al., Clin. Immunol. 103:125-31 (2002); Huang et al., J. Exp. Med. 193:713-26 (2001); Fife et al., J Exp Med 192:899-905 (2000); Karpus et al., J. Leukoc. Biol. 62:681-7 (1997)).

CCR2 is required for infiltration of monocytes and macrophages to the lung. In the lung of chronic obstructive pulmonary disease (COPD) patients, an increased number of CD8+ lymphocytes, macrophages, eosinophils, granulocytes are present. Accumulation of inflammatory cells is associated with a remodeling response that leads to lung airway destruction. In a mouse model of pulmonary fibrosis, deficiency of CCR2 results in a marked reduction in inflammation and tissue fibrosis (Zhu et al., Immunol. 168:2953-62 (2002)).

CCR2 also appears to play a key role in idiopathic pulmonary fibrosis (IPF), another manifestation of severe lung inflammatory disorders. IPF is the scarring of the lung, characterized by the loss of lung elasticity and loss of alveolar surface area, leading to impairment of gas exchange and severe degradation in lung function. Inflammatory cell accumulation is one of the key features of IPF. In experimental models of IPF, CCR2 deficiency results in significant protection of lung fibrosis (Moore et al., J Immunol 167:4368-77 (2001); Gharaee-Kermani et al., Cytokine 24:266-76 (2003)).

CCR2 may be a mediator of idiopathic pneumonia syndrome (IPS) as well, a major complication after allogeneic bone marrow transplantation. Patients with IPS have elevated levels of MCP-1 in the bronchoalveolar lavage (BAL) fluid. In an experimental model of IPS, expression of MCP-1 and CCR2 mRNA increases significantly in the lung, and transplantation of CCR2-deficient donor cells results in a significant reduction in IPS severity compared with transplantation of wild-type cells. Moreover, neutralization of MCP-1 is efficacious in reducing lung injury (Hildebrandt, Duffner et al., Blood 103:2417-26 (2004)).

CCR2 appears to play a role in migration of T cells to the intestine, and may have a pathogenic role in Inflammatory bowel disease (IBD). Inflammatory bowel disease, consisting of ulcerative colitis and Crohn's disease, is associated with accumulation of inflammatory cells and destruction of the intestinal mucosal tissues. In the IL-10 knockout mice, which spontaneously develop ulcerative colitis, MCP-1 and CCR2 are among the chemokines and chemokine receptors to be significantly up-regulated as the disease progresses (Scheerens et al., Eur. J. Immunol. 31:1465-74 (2001)). In human IBD patients, the level of MCP-1 significantly increase in gut tissues (van Deventer, Aliment. Pharmacol. Ther. 11 Supp) 3:116-20; discussion 120-1 (1997); Mazzucchelli et al., J. Pathol. 178:201-6 (1996); Banks et al., J. Pathol. 199:28-35 (2003)).

Chemokines and chemokine receptors are expressed by intrinsic renal cells and infiltrating cells during renal inflammation (Segerer et al., J. Am. Soc. Nephrol. 11:152-76 (2000); Morii et al., J. Diabetes Complications 17:11-5 (2003); Lloyd et al. J. Exp. Med. 185:1371-80 (1997); Gonzalez-Cuadrado et al. Clin. Exp. Immunol. 106:518-22 (1996); Eddy & Giachelli, Kidney Int. 47:1546-57 (1995); Diamond et al., Am. J. Physiol. 266:F926-33 (1994)). In humans, CCR2 and ligand MCP-1 are among the proteins expressed in renal fibrosis, and are correlated with the extent of macrophage infiltration into the interstitium (Yang et al., Zhonghua Yi Xue Za Zhi 81:73-7 (2001); Stephan et al., J. Urol. 167:1497-502 (2002); Amann et al., Diabetes Care 26:2421-5. (2003); Dai et al., Chin. Med. J. (Engl) 114:864-8 (2001)). In animal models of renal fibrosis, blockade of CCR2 or MCP-1 leads to a marked reduction in severity of renal inflammation (Kitagawa et al., Am. J. Pathol. 165:237-46 (2004); Wada et al., Am. J. Pathol. 165:237-46 (2004); Shimizu et al., J. Am. Soc. Nephrol. 14:1496-505 (2003)).

Rheumatoid arthritis is a chronic disease of the joints characterized by synovial inflammation that leads to the destruction of cartilage and bone. Although the underlying causes of the disease are unknown, it is believed that macrophages and Th-1 type T cells play a key role in the initiation and perpetuation of the chronic inflammatory process (Vervoordeldonk et al., Curr. Rheumatol. Rep. 4:208-17 (2002)).

MCP-1 is among the several chemokines, including MiP-1α and IL-8, identified in rheumatoid synovium (Villiger et al., J. Immunol. 149:722-7 (1992); Scaife et al., Rheumatology (Oxford) 43:1346-52 (2004); Shadidi et al., Scand. J. Immunol. 57:192-8 (2003); Taylor et al., Arthritis Rheum. 43:38-47 (2000); Tucci et al., Biomed Sci. Instrum. 34:169-74 (1997)). Chemokine receptors CCR1, CCR2, CCR3 and CCR5 are upregulated in the joints from arthritic mice (Plater-Zyberk et al., Immunol. Lett. 57:117-20 (1997). Blockade of MCP-1 activity using a CCR2 antagonist or an antibody against MCP-1 have been shown efficacious in reducing joint inflammation in experimental models of rheumatoid arthritis (Gong et al., J. Exp. Med. 186:131-7 (1997); Ogata et al., J. Pathol. 182:106-14 (1997)).

CCR2-mediated infiltration of macrophages in the fat tissues may also contribute to the complications arising from obesity, a condition resulting from excessive storage of fat in the body. Obesity predisposes the affected individuals to many disorders, such as noninsulin-dependent diabetes, hypertension, stroke, and coronary artery disease. In obesity, adipose tissues have altered metabolic and endocrine functions that lead to an increased release of fatty acids, hormones, and proinflammatory molecules. Adipose tissue macrophages are believed to be a key source of proinflammatory cytokines including TNF-alpha, iNOS and IL-6 (Weisberg et al., J. Clin. Invest. 112:1796-808 (2003)). Recruitment of macrophages to the adipose tissue is likely mediated by MCP-1 produced by adipocytes (Christiansen et al., Int. J. Obes. Relat. Metab. Disord. (2004); Sartipy et al., Proc. Natl. Acad. Sci. U.S.A. 100:7265-70 (2003)).

Chemokine and chemokine receptors are the key regulators of immune cell trafficking. MCP-1 is a potent chemoattractant of monocytes and T cells; its expression is induced under inflammatory conditions including proinflammatory cytokine stimulations and hypoxia. The interaction between MCP-1 and CCR2 mediates migration of monocytes, macrophage as well as activated T cells and play a key role in the pathogenesis of many inflammatory diseases. Inhibition of CCR2 functions using small molecule antagonists described in this invention represents a new approach for the treatments of inflammatory disorders.

Psoriasis is a chronic inflammatory disease characterized by hyperproliferation of keratinocytes and pronounced leukocyte infiltration. It is known that keratinocytes from psoriasis lesion express abundant CCR2 ligand MCP-1, particularly when stimulated by proinflammatory cytokines such as TNF-α (Vestergaard et al., Acta. Derm. Venereol. 84(5):353-8 (2004); Gillitzer et al., J. Invest. Dermatol, 101 (2): 127-31 (1993); Deleuran et al., J. Dermatol. Sci. 13(3):228-36 (1996)). Since MCP-1 can attract migration of both macrophages and dendritic cells expressing CCR2 to the skin, this receptor and ligand pair is believed to be important in regulating the interaction between proliferating keratinocytes and dermal macrophage during the development of psoriasis. A small molecule antagonist may thus be useful in the treatment of psoriasis.

In addition to inflammatory diseases, CCR2 has also been implicated in cancers (Broek et al., Br J Cancer. 88(6):855-62 (2003)). Tumor cells stimulate the formation of stroma that secretes various mediators pivotal for tumor growth, including growth factors, cytokines, and proteases. It is known that the level of MCP-1 is associated significantly with tumor-associated macrophage accumulation, and prognostic analysis reveals that high expression of MCP-1 is a significant indicator of early relapse in breast cancer (Ueno et al., Clin. Cancer Res. 6(8):3282-9 (2001)). A small molecule antagonist of CCR2 may thus be able to reduce the release of growth-stimulating cytokines by blocking accumulation of macrophages at sites of tumor formation.

T lymphocyte (T cell) infiltration into the small intestine and colon has been linked to the pathogenesis of Coeliac diseases, food allergies, rheumatoid arthritis, human inflammatory bowel diseases (IBD) which include Crohn's disease and ulcerative colitis. Blocking trafficking of relevant T cell populations to the intestine can lead to an effective approach to treat human IBD. More recently, chemokine receptor 9 (CCR9) has been noted to be expressed on gut-homing T cells in peripheral blood, elevated in patients with small bowel inflammation such as Crohn's disease and celiac disease. The only CCR9 ligand identified to date, TECK (thymus-expressed chemokine) is expressed in the small intestine and the ligand receptor pair is now thought to play a pivotal role in the development of IBD. In particular, this pair mediates the migration of disease causing T cells to the intestine. See for example, Zaballos, et al., J. Immunol., 162(10):5671 5675 (1999); Kunkel, et al., J. Exp. Med. 192(5):761-768 (2000); Papadakis, et al., J. Immunol., 165(9):5069 5076 (2000); Papadakis, et al., Gastroenterology, 121 (2):246 254 (2001); Campbell, et al., J. Exp. Med., 195(1):135 141 (2002); Wurbel, et al., Blood, 98(9):2626-2632 (2001); and Uehara, et al., J. Immunol, 168(6):2811-2819 (2002).

### BRIEF SUMMARY

The present invention is directed to compounds and pharmaceutically acceptable salts thereof, compositions, and methods useful in modulating chemokine activity. The compounds and salts thereof, compositions, and methods described herein are useful in treating or preventing chemokine-mediated conditions or diseases, including certain inflammatory and immunoregulatory disorders and diseases.

The compounds of the present invention have been shown to modulate one or more of CCR2, CCR3, CCR4, CCR5, CCR6, CCR7, CCR8, CCR9, CCR10, CXCR3, CXCR4, CXCR5, and CX3CR1. In particular, various compounds of the present invention modulate CCR2 and CCR9 as shown in the examples.

In one embodiment, the present compound may be represented by formula (I) or salts thereof:

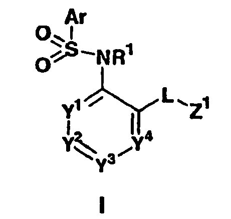

where Ar, R¹, Y¹, Y², Y³, Y⁴, L and Z¹ are as defined below.

In another aspect, the present invention provides compositions useful in modulating CCR2 chemokine activity. In one embodiment, a composition according to the present invention comprises a compound according to the invention and a pharmaceutically acceptable carrier or excipient.

In yet another aspect, the present invention provides an in vitro method of modulating CCR2 function in a cell, comprising contacting the cell with a CCR2 modulating amount of a compound according to the invention.

In still another aspect, the present invention provides a compound according to the invention for use in treating a CCR2-mediated condition or disease.

In addition to the compounds provided herein, the present invention further provides pharmaceutical compositions containing one or more of these compounds, as well as these compounds for use in therapeutic methods, primarily to treat diseases associated with CCR2 signaling activity.

### BRIEF SUMMARY OF THE DRAWINGS

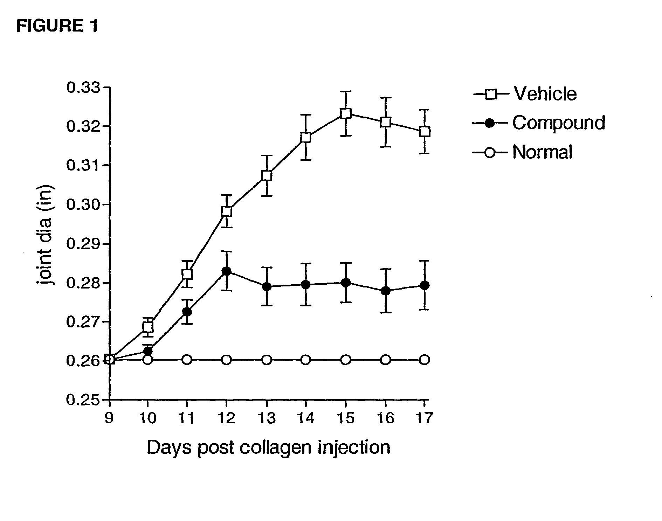

Figure 1 is a graph depicting ankle joint diameter as a function of time in an inflamed ankle treated with a compound in accordance with the present invention.

### DETAILED DESCRIPTION

### General

The present invention is directed to compounds and salts thereof, compositions and methods useful in the modulation of chemokine receptor function, particularly CCR2 function. Modulation of chemokine receptor activity, as used herein in its various forms, is intended to encompass antagonism, agonism, partial antagonism, inverse agonism and/or partial agonism of the activity associated with a particular chemokine receptor; preferably the CCR2 receptor. Accordingly, the compounds of the present invention are compounds which modulate at least one function or characteristic of mammalian CCR2, for example, a human CCR2 protein. The ability of a compound to modulate the function of CCR2, can be demonstrated in a binding assay (e.g., ligand binding or agonist binding), a migration assay, a signaling assay (e.g., activation of a mammalian G protein, induction of rapid and transient increase in the concentration of cytosolic free calcium), and/or cellular response assay (e.g., stimulation of chemotaxis, exocytosis or inflammatory mediator release by leukocytes).

### Abbreviations and Definitions

When describing the compounds, compositions, methods and processes of this invention, the following terms have the following meanings, unless otherwise indicated.

"Alkyl" by itself or as part of another substituent refers to a hydrocarbon group which may be linear, cyclic, or branched or a combination thereof having the number of carbon atoms designated (i.e., C₁₋₈ means one to eight carbon atoms). Examples of alkyl groups include methyl, ethyl, n-propyl, isopropyl, n-butyl, t-butyl, isobutyl, sec-butyl, cyclohexyl, cyclopentyl, (cyclohexyl)methyl, cyclopropylmethyl, bicyclo[2.2.1]heptane and bicyclo[2.2.2]octane. Alkyl groups can be substituted or unsubstituted, unless otherwise indicated. Examples of substituted alkyl include haloalkyl, thioalkyl and aminoalkyl.

"Alkoxy" refers to -O-alkyl. Examples of an alkoxy group include methoxy, ethoxy and n-propoxy.

"Alkenyl" refers to an unsaturated hydrocarbon group which may be linear, cyclic or branched or a combination thereof. Alkenyl groups with 2-8 carbon atoms are preferred. The alkenyl group may contain 1, 2 or 3 carbon-carbon double bonds. Examples of alkenyl groups include ethenyl, n-propenyl, isopropenyl, n-but-2-enyl, n-hex-3-enyl, cyclohexenyl and cyclopentenyl. Alkenyl groups can be substituted or unsubstituted, unless otherwise indicated.

"Alkynyl" refers to an unsaturated hydrocarbon group which may be linear, cyclic or branched or a combination thereof. Alkynyl groups with 2-8 carbon atoms are preferred. The alkynyl group may contain 1, 2 or 3 carbon-carbon triple bonds. Examples of alkynyl groups include ethynyl, n-propynyl, n-but-2-ynyl and n-hex-3-ynyl. Alkynyl groups can be substituted or unsubstituted, unless otherwise indicated.

"Aryl" refers to a polyunsaturated, aromatic hydrocarbon group having a single ring (monocyclic) or multiple rings (bicyclic), which can be fused together or linked covalently. Aryl groups with 6-10 carbon atoms are preferred, where this number of carbon atoms can be designated by C₆₋₁₀, for example. Examples of aryl groups include phenyl and naphthalene-1-yl, naphthalene-2-yl and biphenyl. Aryl groups can be substituted or unsubstituted, unless otherwise indicated.

"Halo" or "halogen", by itself or as part of a substituent refers to a chlorine, bromine, iodine, or fluorine atom.

"Haloalkyl", as a substituted alkyl group, refers to a monohaloalkyl or polyhaloalkyl group, most typically substituted with from 1-3 halogen atoms. Examples include 1-chloroethyl, 3-bromopropyl and trifluoromethyl.

"Heterocyclyl" refers to a saturated or unsaturated non-aromatic ring containing at least one heteroatom (typically 1 to 5 heteroatoms) selected from nitrogen, oxygen or sulfur. The heterocyclyl ring may be monocyclic or bicyclic. Preferably, these groups contain 0-5 nitrogen atoms, 0-2 sulfur atoms and 0-2 oxygen atoms. More preferably, these groups contain 0-3 nitrogen atoms, 0-1 sulfur atoms and 0-1 oxygen atoms. Examples of heterocycle groups include pyrrolidine, piperidine, imidazolidine, pyrazolidine, butyrolactam, valerolactam, imidazolidinone, hydantoin, dioxolane, phthalimide, piperidine, 1,4-dioxane, morpholine, thiomorpholine, thiomorpholine-S-oxide, thiomorpholine-S,S-dioxide, piperazine, pyran, pyridone, 3-pyrroline, thiopyran, pyrone, tetrahydrofuran, tetrahydrothiophene and quinuclidine. Preferred heterocyclic groups are monocyclic, though they may be fused or linked covalently to an aryl or heteroaryl ring system.

In one preferred embodiment, heterocyclic groups may be represented by formula (AA) below: 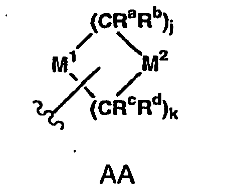 where formula (AA) is attached via a free valence on either M¹ or M²; M¹ represents O, NR^{e}, or S(O)ₗ; M² represents CR^{f}R^{g}, O, S(O)ₗ, or NR^{e}; l is 0, 1 or 2; j is 1, 2 or 3 and k is 1, 2 or 3, with the proviso that j + k is 3, 4, or 5; and R^{a}, R^{b}, R^{c}, R^{d}, R^{e}, R^{f}, and R^{g} are independently selected from the group consisting of hydrogen, halogen, unsubstituted or substituted C₁₋₈ alkyl, unsubstituted or substituted C₂₋₈ alkenyl, unsubstituted or substituted C₂₋₈ alkynyl, -COR^{h}, -CO₂R^{h}, -CONR^{h}Rⁱ, -NR^{h}CORⁱ, -SO₂R^{h}, -SO₂NR^{h}Rⁱ, -NSO₂R^{h}Rⁱ-NR^{h}Rⁱ, -OR^{h}, -Q¹COR^{h}, -Q¹CO₂R^{h}, -Q¹CONR^{h}Rⁱ, -Q¹NR^{h}CORⁱ, -Q¹SO₂R²⁸, -Q¹SO₂NR^{h}Rⁱ, -Q¹NSO₂R^{h}Rⁱ, -Q¹NR^{h}Rⁱ, -Q¹OR^{h}, wherein Q¹ is a member selected from the group consisting of C₁₋₄alkylene, C₂₋₄alkenylene and C₂₋₄alkynylene, and R^{h} and Rⁱ are independently selected from the group consisting of hydrogen and C₁₋₈ alkyl, and wherein the aliphatic portions of each of the R^{a}, R^{b}, R^{c}, R^{d}, R^{e}, R^{f}, R^{g}, R^{h}and Rⁱ substituents are optionally substituted with from one to three members selected from the group consisting of halogen, -OH, -ORⁿ, -OC(O)NHRⁿ, -OC(O)NRⁿR^{o}, -SH, -SRⁿ, -S(O)Rⁿ, -S(O)₂Rⁿ, -SO₂NH₂, -S(O)₂NHRⁿ, -S(O)₂NRⁿR^{o}, -NHS(O)₂Rⁿ, -NRⁿS(O)₂R^{o}, -C(O)NH₂, -C(O)NHRⁿ, -C(O)NRⁿR^{o}, -C(O)Rⁿ, -NHC(O)R^{o}, -NRⁿC(O)R^{o}, -NHC(O)NH₂, -NRⁿC(O)NH₂, -NRⁿC(O)NHR^{o}, -NHC(O)NHRⁿ, -NRⁿC(O)NR^{o}R^{p}, -NHC(O)NRⁿR^{o}, -CO₂H, -CO₂Rⁿ, -NHCO₂Rⁿ, -NRⁿCO₂R^{o}, -CN, -NO₂, -NH₂, -NHRⁿ, -NRⁿR^{o}, -NRⁿS(O)NH₂ and -NRⁿS(O)₂NHR^{o}, wherein Rⁿ, R^{o} and R^{p} are independently an unsubstituted C₁₋₈ alkyl. Additionally, any two of R^{a}, R^{b}, R^{c}, R^{d}, R^{e}, R^{f} and R^{g} may be combined to form a bridged or spirocyclic ring system.

In one preferred embodiment, the number of R^{a} + R^{b}+ R^{c} + R^{d} groups that are other than hydrogen is 0, 1 or 2. In a more preferred embodiment, R^{a}, R^{b}, R^{e}, R^{d}, R^{e}, R^{f}, and R^{g} are independently selected from the group consisting of hydrogen, halogen, unsubstituted or substituted C₁₋₈ alkyl, -COR^{h}, -CO₂R^{h}, -CONR^{h}R^{h}, -NR^{h}COR^{h}, -SO₂R^{h}, -SO₂NR^{h}Rⁱ, -NSO₂R^{h}Rⁱ, -NR^{h}Rⁱ, and -OR^{h}, wherein R^{h} and Rⁱ are independently selected from the group consisting of hydrogen and unsubstituted C₁₋₈ alkyl and wherein the aliphatic portions of each of the R^{a}, R^{b}, R^{c}, R^{d}, R^{e}, R^{f} and R^{g} substituents are optionally substituted with from one to three members selected from the group consisting of halogen, -OH, -ORⁿ, -OC(O)NHRⁿ, -OC(O)NRⁿR^{o}, -SH, -SRⁿ, -S(O)R^{o}, -S(O)₂Rⁿ, -SO₂NH₂, -S(O)₂NHRⁿ, -S(O)₂NRⁿR^{o}, -NHS(O)₂Rⁿ, -NRⁿS(O)₂R^{o}, -C(O)NH₂, -C(O)NHRⁿ, -C(O)NRⁿR^{o}, -C(O)Rⁿ, -NHC(O)Rⁿ, -NRⁿC(O)R^{o}, -NHC(O)NH₂, -NRⁿC(O)NH₂, -NRⁿC(O)NHR^{o}, -NHC(O)NHRⁿ, -NRⁿC(O)NR^{o}R^{p}, -NHC(O)NRⁿR^{o}, -CO₂H, -CO₂Rⁿ, -NHCO₂Rⁿ, -NRⁿCO₂R^{o}, -CN, -NO₂, -NH₂, -NHRⁿ, -NRⁿR^{o}, -NRⁿS(O)NH₂ and -NRⁿS(O)₂NHR^{o}, wherein Rⁿ, R^{o} and R^{p} are independently an unsubstituted C₁₋₈ alkyl.

In a more preferred embodiment, R^{a}, R^{b}, R^{c}, R^{d}, R^{e}, R^{f}, and R^{g} are independently hydrogen or C₁₋₄ alkyl. In another preferred embodiment, at least three of R^{a}, R^{b}, R^{c}, R^{d}, R^{e}, R^{f}, and R^{g} are hydrogen.

"Heteroaryl" refers to an aromatic group containing at least one heteroatom, where the heteroaryl group may be monocyclic or bicyclic. Examples include pyridyl, pyridazinyl, pyrazinyl, pyrimidinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiazolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, oxadiazolyl, thiadiazolyl, pyrrolyl, thiazolyl, furyl or thienyl. Preferred heteroaryl groups are those having at least one aryl ring nitrogen atom, such as quinolinyl, quinoxalinyl, purinyl, benzimidazolyl, benzopyrazolyl, benzotriazolyl, benzothiazolyl, indolyl, quinolyl and isoquinolyl. Preferred 6-ring heteroaryl systems include pyridyl, pyridazinyl, pyrazinyl, pyrimidinyl, triazinyl and the like. Preferred 5-ring heteroaryl systems include isothiazolyl, pyrazolyl, imidazolyl, thienyl, furyl, triazolyl, tetrazolyl, oxazolyl, isoxazolyl, oxadiazolyl, thiadiazolyl, pyrrolyl and thiazolyl.

Heterocyclyl and heteroaryl can be attached at any available ring carbon or heteroatom. Each heterocyclyl and heteroaryl may have one or more rings. When multiple rings are present, they can be fused together or linked covalently. Each heterocyclyl and heteroaryl must contain at least one heteroatom (typically 1 to 5 heteroatoms) selected from nitrogen, oxygen or sulfur. Preferably, these groups contain 0-5 nitrogen atoms, 0-2 sulfur atoms and 0-2 oxygen atoms. More preferably, these groups contain 0-3 nitrogen atoms, 0-1 sulfur atoms and 0-1 oxygen atoms. Heterocyclyl and heteroaryl groups can be substituted or unsubstituted, unless otherwise indicated. For substituted groups, the substitution may be on a carbon or heteroatom. For example, when the substitution is oxo (=O or-O⁻), the resulting group may have either a carbonyl (-C(O)-) or a N-oxide (-N⁺-O⁻).

Suitable substituents for substituted alkyl, substituted alkenyl, and substituted alkynyl include halogen, -CN, -CO₂R, -C(O)R', -C(O)NR'R", oxo (=O or -O⁻), -OR', -OC(O)R', -OC(O)NR'R" -NO₂, -NR'C(O)R'^{'}, -NR"'C(O)NR'R", -NR'R", -NR'CO₂R", -NR'S(O)R", -NR'S(O)₂R"', -NR"'S(O)NR'R", -NR"'S(O)₂NR'R", -SR', -S(O)R', -S(O)₂R', -S(O)₂NR'R", -NR'-C(NHR")=NR"', -SiR'R"R"',-N₃, substituted or unsubstituted C₆₋₁₀ aryl, substituted or unsubstituted 5- to 10-membered heteroaryl, and substituted or unsubstituted 3- to 10-membered heterocyclyl. The number of possible substituents range from zero to (2m'+1), where m' is the total number of carbon atoms in such radical.

Suitable substituents for substituted aryl, substituted heteroaryl and substituted heterocyclyl include halogen, -CN, -CO₂R', -C(O)R', -C(O)NR'R", oxo (=O or -O⁻), -OR', -OC(O)R', -OC(O)NR'R", -NO₂, -NRC(O)R', -NR"'C(O)NR'R", -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)₂NR'R", -NR'-C(NHR")=NR"', -SiR'R"R"',-N₃, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10 membered heterocyclyl. The number of possible substituents range from zero to the total number of open valences on the aromatic ring system.

As used above, R', R" and R"' each independently refer to a variety of groups including hydrogen, substituted or unsubstituted C₁₋₈ alkyl, substituted or unsubstituted C₂₋₈ alkenyl, substituted or unsubstituted C₂₋₈ alkynyl, substituted or unsubstituted aryl, substituted or unsubstituted heteroaryl, substituted or unsubstituted heterocyclyl, substituted or unsubstituted arylalkyl, substituted or unsubstituted aryloxyalkyl. When R' and R" are attached to the same nitrogen atom, they can be combined with the nitrogen atom to form a 3-, 4-, 5-, 6-, or 7-membered ring (for example, -NR'R" includes 1-pyrrolidinyl and 4-morpholinyl). Furthermore, R' and R", R" and R"', or R' and R'" may together with the atom(s) to which they are attached, form a substituted or unsubstituted 5- ,6- or 7-membered ring.

Two of the substituents on adjacent atoms of an aryl or heteroaryl ring may optionally be replaced with a substituent of the formula -T-C(O)-(CH₂)_{q}-U-, wherein T and U are independently -NR""-, -O-, -CH₂- or a single bond, and q is an integer of from 0 to 2. Alternatively, two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR""-, - S-, -S(O)-, -S(O)₂-, -S(O)₂NR""- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR""-, -S-, -S(O)-, -S(O)₂-, or -S(O)₂NR'-. R"" in is selected from hydrogen or unsubstituted C₁₋₈ alkyl.

"Heteroatom" is meant to include oxygen (O), nitrogen (N), sulfur (S) and silicon (Si).

"Pharmaceutically acceptable" carrier, diluent, or excipient is a carrier, diluent, or excipient compatible with the other ingredients of the formulation and not deleterious to the recipient thereof.

"Pharmaceutically-acceptable salt" refers to a salt which is acceptable for administration to a patient, such as a mammal (e.g., salts having acceptable mammalian safety for a given dosage regime). Such salts can be derived from pharmaceutically-acceptable inorganic or organic bases and from pharmaceutically-acceptable inorganic or organic acids, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Salts derived from pharmaceutically-acceptable inorganic bases include for example, aluminum, ammonium, calcium, copper, ferric, ferrous, lithium, magnesium, manganic, manganous, potassium, sodium and zinc and the like. Salts derived from pharmaceutically-acceptable organic bases include for example, salts of primary, secondary, tertiary and quaternary amines, including substituted amines, cyclic amines and naturally-occurring amines, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine and tromethamine. When compounds of the present invention contain relatively basic functionalities, acid addition salts can be obtained by contacting the neutral form of such compounds with a sufficient amount of the desired acid, either neat or in a suitable inert solvent. Salts derived from pharmaceutically-acceptable acids include for example, acetic, ascorbic, benzenesulfonic, benzoic, camphosulfonic, citric, ethanesulfonic, fumaric, gluconic, glucoronic, glutamic, hippuric, hydrobromic, hydrochloric, isethionic, lactic, lactobionic, maleic, malic, mandelic, methanesulfonic, mucic, naphthalenesulfonic, nicotinic, nitric, pamoic, pantothenic, phosphoric, succinic, sulfuric, tartaric and p-toluenesulfonic.

Also included are salts of amino acids such as for example, arginate and salts of organic acids like for example glucuronic or galactunoric acids (see, for example, Berge, S.M. et al, "Pharmaceutical Salts", J. Pharmaceutical Science, 1977, 661-19). Certain specific compounds of the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound, for the purposes of the present invention.

"Salt thereof" refers to a compound formed when the hydrogen of an acid is replaced by a cation, such as for example a metal cation or an organic cation . Preferably, the salt is a pharmaceutically-acceptable salt, although this is not required for salts of intermediate compounds which are not intended for administration to a patient.

In addition to salt forms, the present invention provides compounds which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

"Therapeutically effective amount" refers to an amount sufficient to effect treatment when administered to a patient in need of treatment.

"Treating" or "treatment" as used herein refers to the treating or treatment of a disease or medical condition (such as a viral, bacterial or fungal infection or other infectious diseases, as well as autoimmune or inflammatory conditions) in a patient, such as a mammal (particularly a human or a companion animal) which includes:
ameliorating the disease or medical condition, i.e., eliminating or causing regression of the disease or medical condition in a patient;
suppressing the disease or medical condition, i.e., slowing or arresting the development of the disease or medical condition in a patient; or
alleviating the symptoms of the disease or medical condition in a patient.

Certain compounds of the present invention can exist in unsolvated forms as well as solvated forms, including hydrated forms. In general, both solvated forms and unsolvated forms are intended to be encompassed within the scope of the present invention. Certain compounds of the present invention may exist in multiple crystalline or amorphous forms (i.e., as polymorphs). In general, all physical forms are equivalent for the uses contemplated by the present invention and are intended to be within the scope of the present invention.

It will be apparent to one skilled in the art that certain compounds of the present invention may exist in tautomeric forms, all such tautomeric forms of the compounds being within the scope of the invention. Certain compounds of the present invention possess asymmetric carbon atoms (optical centers) or double bonds; the racemates, diastereomers, geometric isomers and individual isomers (e.g., separate enantiomers) are all intended to be encompassed within the scope of the present invention. The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

### Compounds that Modulate CCR2 Activity

The present invention provides compounds that modulate CCR2 activity. Chemokine receptors are integral membrane proteins which interact with an extracellular ligand, such as a chemokine, and mediate a cellular response to the ligand, e.g., chemotaxis, increased intracellular calcium ion concentration, etc. Therefore, modulation of a chemokine receptor function, e.g., interference with a chemokine receptor ligand interaction, will modulate a chemokine receptor mediated response, and treat or prevent a chemokine receptor mediated condition or disease. Modulation of a chemokine receptor function includes both inducement and inhibition of the function. The type of modulation accomplished will depend on the characteristics of the compound, i.e, antagonist or full, partial or inverse agonist.
Without intending to be bound by any particular theory, it is believed that the compounds provided herein interfere with the interaction between a chemokine receptor and one or more cognate ligands. In particular, it is believed that the compounds interfere with the interaction between CCR2 and a CCR2 ligand, such as MCP-1. Compounds contemplated by the invention include, but are not limited to, the exemplary compounds provided herein and salts thereof.
For example, compounds of this invention act as potent CCR2 antagonists, and this antagonistic activity has been further confirmed in animal testing for inflammation, one of the hallmark disease states for CCR2. Accordingly, the compounds provided herein are useful in pharmaceutical compositions, methods for the treatment of CCR2-mediated diseases, and as controls in assays for the identification of competitive CCR2 antagonists.

### Compounds

In one embodiment, the compounds of the present invention are represented by formula (I), or salts thereof: 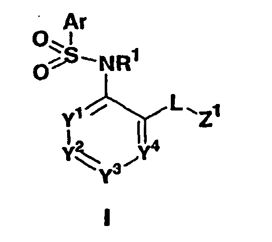 Ar is unsubstituted phenyl or phenyl substituted with one or more of halogen, substituted or unsubstituted C₁₋₈ alkyl, substituted or unsubstituted C₂₋₈ alkenyl, substituted or unsubstituted C₂₋₈ alkynyl, -CN, -C(O)R¹⁸, -CO₂R¹⁸, -C(O)NR¹⁸R¹⁹, -OC(O)R¹⁹, -OC(O)NR¹⁸R¹⁹, -NO₂, -NR¹⁸C(O)R¹⁹, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, substituted or unsubstituted C₆₋₁₀ aryl, substituted or unsubstituted 5- to 10-membered heteroaryl, and substituted or unsubstituted 3-to 10-membered heterocyclyl;
R¹⁸, R¹⁹ and R²⁰ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted aryl, substituted or unsubstituted heteroaryl, and substituted or unsubstituted heterocyclyl;
R¹ is selected from the group consisting of hydrogen, unsubstituted C₁ alkyl, and unsubstituted C₃₋₈ alkyl;
Y¹ is -CR^{2a}-;
Y² is -CR^{2b}-;
Y³ is -CR^{2c}-;
R^{2a}, R^{2b}, and R^{2c} are each independently selected from the group consisting of hydrogen, halogen and unsubstituted C₁₋₈ alkyl;
Y⁴ is -N-;
L is -C(O)-;
Z¹ is substituted or unsubstituted phenyl;
wherein
substituents for substituted alkyl, substituted alkenyl, and substituted alkynyl are selected from halogen, -CN, -CO₂R', -C(O)R', -C(O)NR'R", oxo (=O or -O⁻), -OR', -OC(O)R^{'}, -OC(O)NR^{'}R^{"}, -NO₂, -NR'C(O)R", -NR'"C(O)NR'R", -NR'R", -NR'CO₂R", -NR'S(O)R", -NR'S(O)₂R"', -NR'"S(O)NR'R", -NR'"S(0)₂NR'R", -SR', -S(O)R', -S(O)₂R', -S(O)₂NR'R", -NR'-C(NHR")=NR''', -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻), -OR', -OC(O)R', -OC(O)NR'R", -NO₂, -NR'C(O)R'', -NR'''C(O)NR'R'', -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)₂NR'R", -NR'-C(NHR")=NR"', -SiR'R"R"', -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R"' are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5-, 6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NR""-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NMR"'-, -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula - (CH₂)ₛ-X-(CH₂)ₜ-, where s and t are independently integers of from 0 to 3, and X is -O-, -NR""-, -S-, -S(O) -, -S(O)₂-, or -S(O)₂NR'-, wherein R"" is selected from hydrogen or unsubstituted C₁₋₈ alkyl.

In a further embodiment, the compounds are represented by formula (II), or salts thereof:

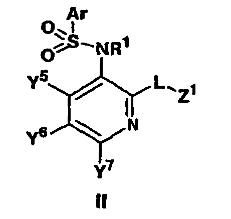

Formula II is an example of Formula I.

Ar, R¹, L and Z¹ are as defined above.

Y⁵, Y⁶ and Y⁷ are each independently selected from the group consisting of hydrogen, halogen, and unsubstituted C₁₋₈ alkyl.

In another embodiment, the compounds are represented by formula (III), or salts thereof: 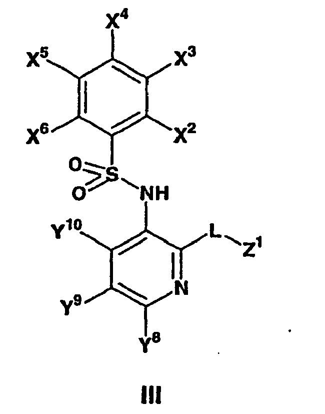 L is -C(O)-;
Z¹ is selected from the group consisting of substituted or unsubstituted 5- to 10-membered heteroalkyl;

X², X³, X⁴, X⁵, and X⁶ are each independently selected from the group consisting of hydrogen, halogen, substituted or unsubstituted C₁₋₈ alkyl, substituted or unsubstituted C₂₋₈ alkenyl, substituted or unsubstituted C₂₋₆ alkynyl, -CN, -NO₂, -C(O)R¹⁸, -CO₂R¹⁸, -C(O)NR¹⁸R¹⁹, -OR¹⁸, -OC(O)R¹⁹, -OC(O)NR¹⁸R¹⁹, -NO₂, -NR¹⁸C(O)R¹⁹, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, substituted or unsubstituted C₆₋₁₀ aryl, substituted or unsubstituted 5- to 10-membered heteroaryl, and substituted or unsubstituted 3- to 10-membered heterocyclyl;
R¹⁸, R¹⁹ and R²⁰ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl;
Y⁸, Y⁹ and Y¹⁰ are each independently selected from the group consisting of hydrogen, halogen, -CN, -NO₂, -OR²¹, -CO₂R²¹, -OC(O)R²¹, -OC(O)NR²¹R²², -C(O)NR²¹R²², -C(O)R²¹, -SR²¹, -S(O)R²¹, -S(O)₂R²¹, NR²¹R²², -NR²¹C(O)R²², -NR²¹C(O)₂R²², -NR²¹S(O)₂R²², -NR²¹C(O)NR²²R²³, substituted or unsubstituted C₁₋₈ alkyl and substituted or unsubstituted 3- to 10-membered heterocyclyl,
R²¹, R²² and R²³ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl;
wherein
substituents for substituted alkyl, substituted alkenyl, and substituted alkynyl are selected from halogen, -CN, -CO₂R', -C(O)R', -C(O)NR'R", oxo (=O or -O⁻), -R', -OC(O)R', -OC(O)NR'R", -NO₂, -NR'C(O)R", -NR'" C(O)NR'R", -NR'R", -NR'CO₂R", -NR'S(O)R", -NR'S(O)₂R"', -NR"'S(O)NR'R", -NR'"S(O)₂NR'R", -SR', -S(O)R', -S(O)₂R', -S(O)₂NR'R", -NR'-C(NHR")=NR"', -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻), -OR', -OC(O)R', -OC(O)NR'R", -NO₂ -NR'C(O)R", -NR"'C(O)NR'R", -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)₂NR'R", -NR'-C(NHR")=NR''', -SiR'R"R'", -N₃, C₁₋₈ alkyl, C₂₋₈alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R"' are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5-, 6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NR"'-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR"'-, -S-, -S(O)-, -S(O)₂-, -8(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR""-, -S-, -S(O)-, -S(O)₂-, or -S(O)₂NR'-, wherein R"" is selected from hydrogen or unsubstituted C₁₋₈ alkyl.
In another embodiment, the invention provides a compound of formula (CLI) or a salt thereof: 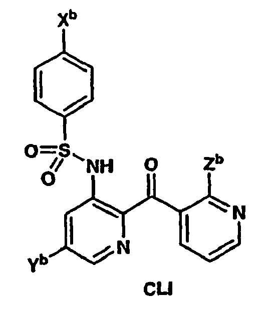 where
X^{b} is selected from the group consisting of -OR^{24a}, and substituted or unsubstituted C₁₋₈ alkyl;
R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and Y^{b} is selected from the group consisting of hydrogen, halogen, -CN, -C(O)R^{27a}, -CO₂R^{27a}, -C(O)NR^{27a}R^{28a}, -OR^{27a}, -OC(O)R^{27a}, -OC(O)NR^{27a}R^{28a,} -SR^{27a}, -S(O)R^{27a}, -S(O)₂R^{27a}, -S(O)₂NR^{27a}R^{28a}, -NO₂, -NR^{27a}R^{28a}, -NR^{27a}C(O)R^{28a}, -NR^{27a}C(O)₂R^{28a}, -NR^{27a}S(O)₂R^{28a}, -NR^{27a}C(O)NR^{28a}R^{29a}, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R^{27a}, R^{28a} and R^{29a} are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10- membered heterocyclyl; and R^{27a} and R^{28a}, R^{28a} and R^{29a} or R^{27a} and R^{29a} may, together with the atoms to which they are attached, form a substituted or unsubstituted 5-, 6-, or 7-membered ring;
Z^{b} is selected from the group consisting of substituted or unsubstituted C₁₋₈ alkyl;
wherein
substituents for substituted alkyl, substituted alkenyl, and substituted alkynyl are selected from halogen, -CN, -CO₂R', -C(O)R', -C(O)NR'R", oxo (=O or -O⁻), -OR', -OC(O)R', -OC(O)NR'R", -NO₂, -NR'C(O)R", -NR'"C(O)NR'R", -NR'R", -NR'CO₂R", -NR'S(O)R", -NR'S(O)₂R"', -NR'"S(O)NR'R", -NR'"S(O)₂NR'R", -SR', -S(O)R', -S(O)₂R', -S(O)₂NR'R", -NR'-C(NHR")=NR'", -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR'''S(O)NR'R", -NR"'S(O)₂NR'R", -SR', -S(O)R', -S(O)2R', -S(O)₂NR'R", -NR'-C(NHR")=NR'", -SiR'R"R"', -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein R', R" and R'" are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5- , 6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NMR"" -, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR"', -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NMR""-, -S-, -S(O)-, -S(O)₂-, or -S(O)₂NR'-, wherein R"" is selected from hydrogen or unsubstituted C₁₋₈ alkyl.
In another embodiment, the invention provides a compound of formula (CLII) or a salt thereof: 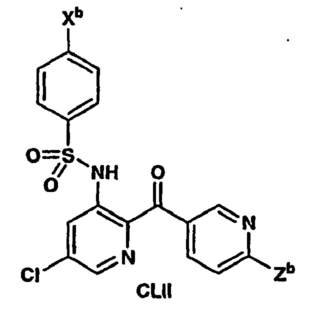 where
X^{b} is selected from the group consisting of -OR^{24a} , and substituted or unsubstituted C₁₋₈ alkyl;
R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
Z^{b} is -OR^{30a};
R^{30a} is selected from the group consisting of hydrogen and substituted or unsubstituted C₁₋₈ alkyl;
wherein
substituents for substituted alkyl, substituted alkenyl, and substituted alkynyl are selected from halogen, -CN, -CO₂R', -C(O)R', -C(O)NR'R", oxo (=O or -O), -OR', -OC(O)R', -OC(O)NR'R", -NO₂, -NR'C(O)R", -NR'"C(O)NR'R", -NR'R", -NR'(O)₂R", -NR'S(O)R", -NR'S(O)₂R"', -NR'"S(O)NR'R", -NR"'S(O)₂NR'R", -SR', -S(O)R', -S(O)₂R', -S(O)₂NR'R", -NR'-C(NHR")=NR'", -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O), -OR', -OC(O)R', -OC(O)NR'R", -NO₂, -NR'C(O)R", -NR'"C(O)NR'R", -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)₂NR'R", -NR'-C(NH")=NR'", -SiR'R"R"', -N₃, C₁₋₈alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R'" are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R"' may together with the atom(s) to which they are attached, form a 5- , 6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NMR"" -, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NMR"'-, -S-, -S(O)-, -S(O)₂-, -S(O)₂NR""- or a single bond, and r is an integers of from 1 to 3; one of the single bonds of the new ring so formed may optionally be replaced with a double bond; two of the substituents on adjacent atoms of the aryl or heteroaryl ring may optionally be replaced with a substituent of the formula-(CH₂)ₛ-X-(CH₂)ₜ -, where s and t are independently integers of from 0 to 3, and X is -O-, -NR""-, -S-, -S(O)-, -S(O)₂-, or -S(O)₂NR'-, wherein R"" is selected from hydrogen or unsubstituted C₁₋₈ alkyl.
In another embodiment, the invention provides a compound of formula (CLIII) or a salt thereof: 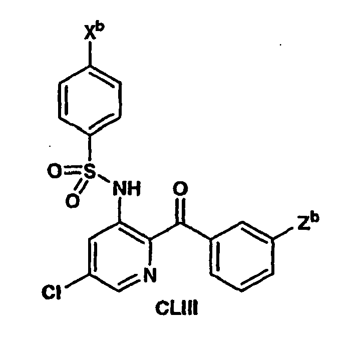 where
X^{b} is selected from the group consisting of -OR^{24a}, and substituted or unsubstituted C₁₋₈ alkyl;
R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
Z^{b} is halogen;
wherein
substituents for substituted alkyl, substituted alkenyl, and substituted alkynyl are selected from halogen, -CN, -CO₂R', -C(O)R', -C(O)NR'R", oxo (=O or -O), -OR', -OC(O)R', -OC(O)NR'R", -NO₂, -NR'C(O)R", -NR'"C(O)NR'R", -NR'R", -NR'CO₂R", -NR'S(O)R", -NR'S(O)₂R"', -NR'"S(O)NR'R", -NR'"S(O)₂NR'R", -SR', -S(O)R', -S(O)₂R', -S(O)₂NR'R", -NR'-C(NHR")=NR'", -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻), -OR', -OC(O)R', -OC(O)NR'R", -NO₂ -NR'C(O)R", -NR'"C(O)NR'R", -NR'R", -NR'CO₂R", -NR'S(O)R", -NR'S(O)₂R", -NR'"S(O)NR'R", -NR'"S(O)₂NR'R", -SR', -S(O)R', -S(O)₂R', -S(O)₂NR'R", -NR'-C(NHR")=NR"', -SiR'R"R'", -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R'" are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R"' may together with the atom(s) to which they are attached, form a 5-, 6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NMR"" -, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR"'-, -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NMR""-, -S-, -S(O)-, -S(O)₂-, or -S(O)₂NR'-, wherein R"" is selected from hydrogen or unsubstituted C₁₋₈ alkyl.

### Known Compound

The compound of the formula (I) where Ar is para-isopropoxy phenyl, R¹ is hydrogen, Y¹, Y² and Y³ are each -CH-, Y⁴ is N, L is C=O, and Z¹ is phenyl, (N-(2-benzoyl-pyridin-3-yl)-4-isopropoxy-benzenesulfonamide) is known, but not as a CCR2 antagonist.

### Preferred Compounds

In several preferred embodiments, the compounds may be represented by the following formulae, or salts thereof:

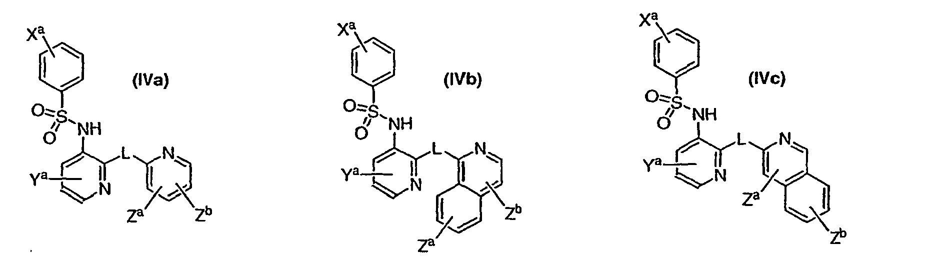

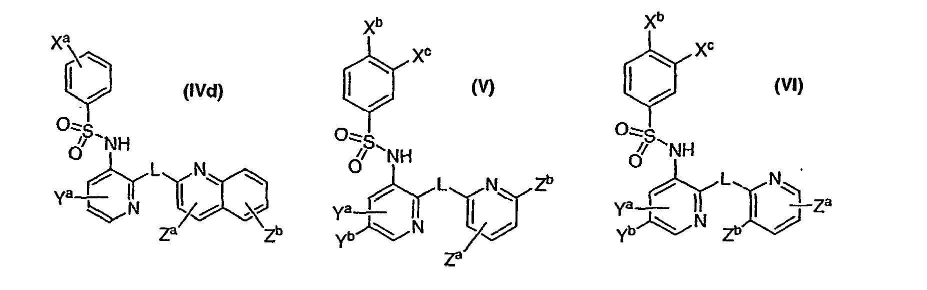

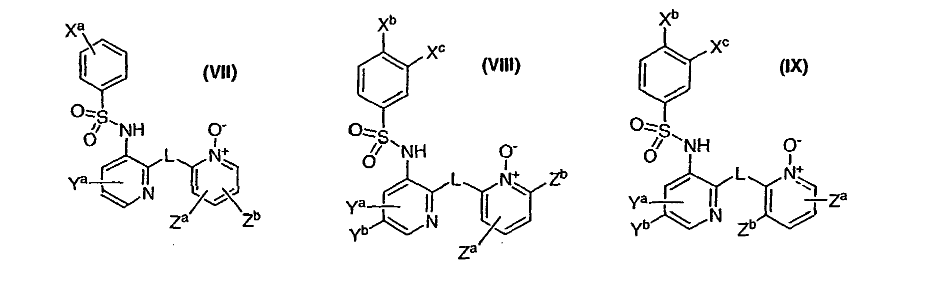

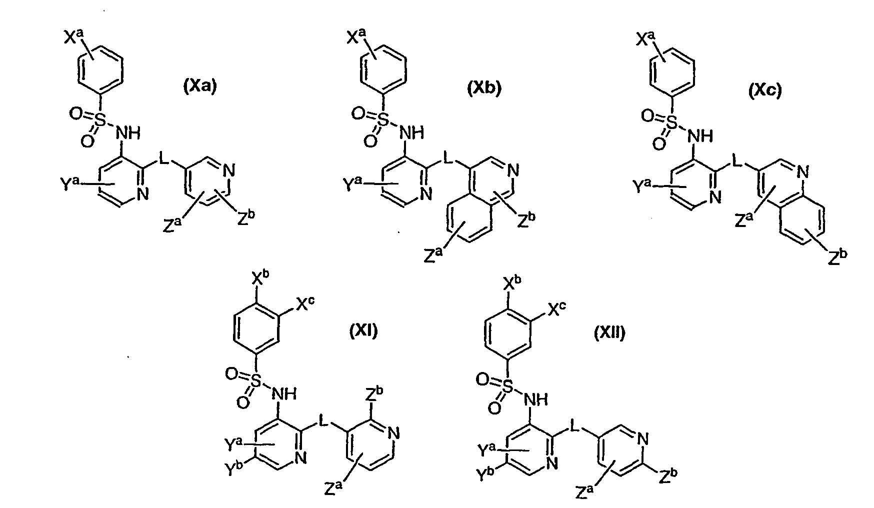

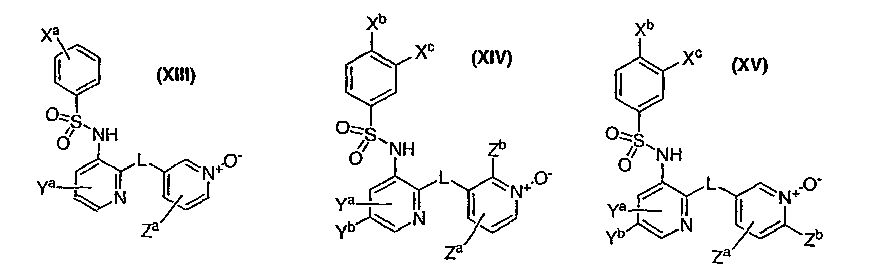

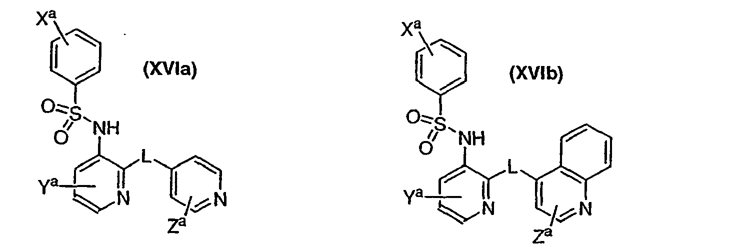

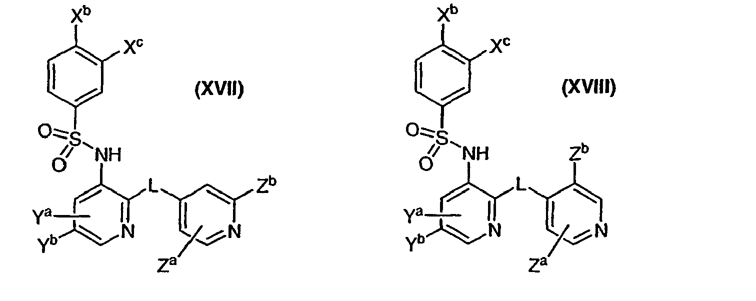

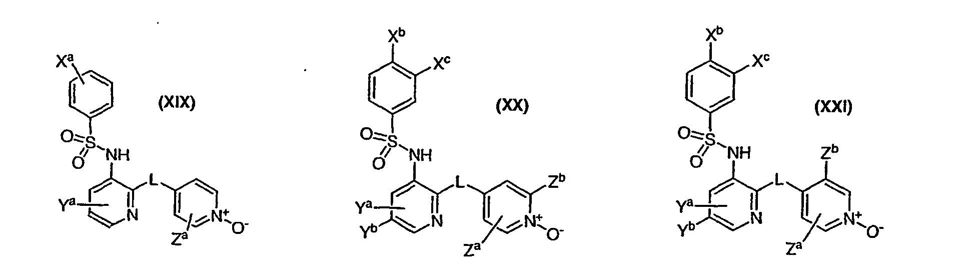

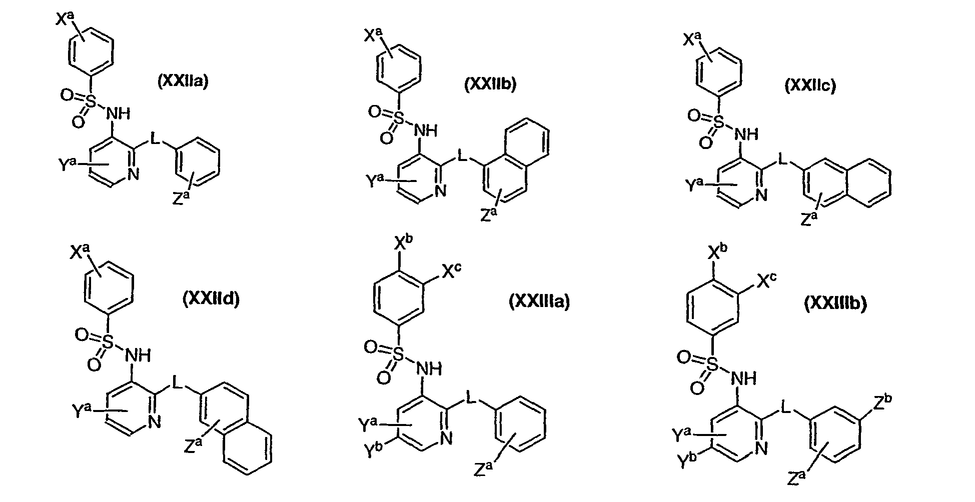

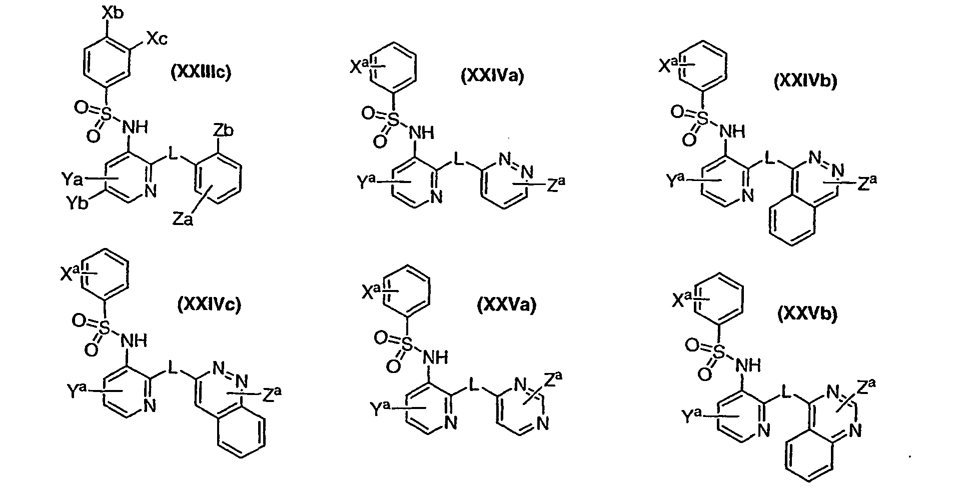

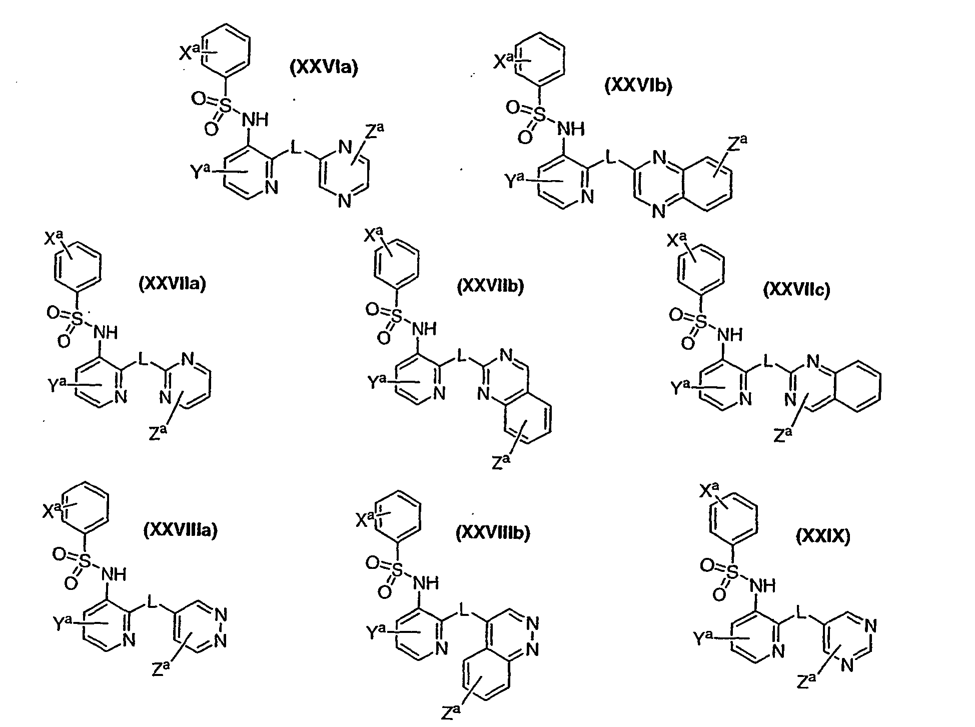

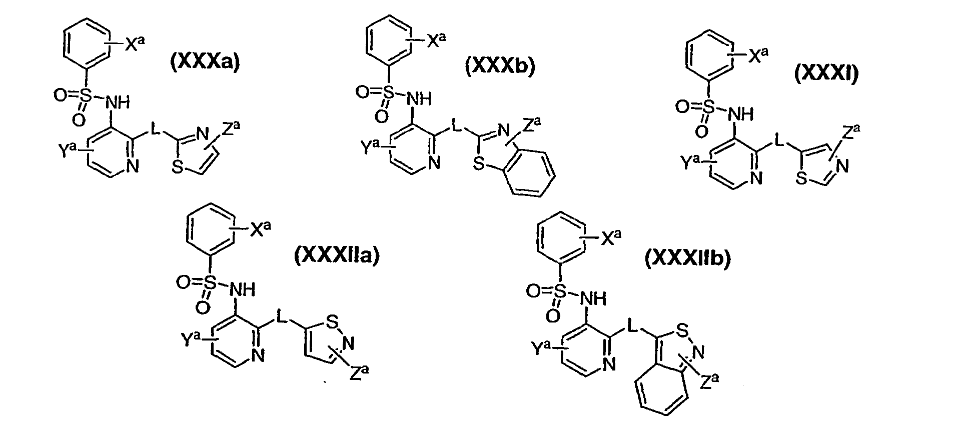

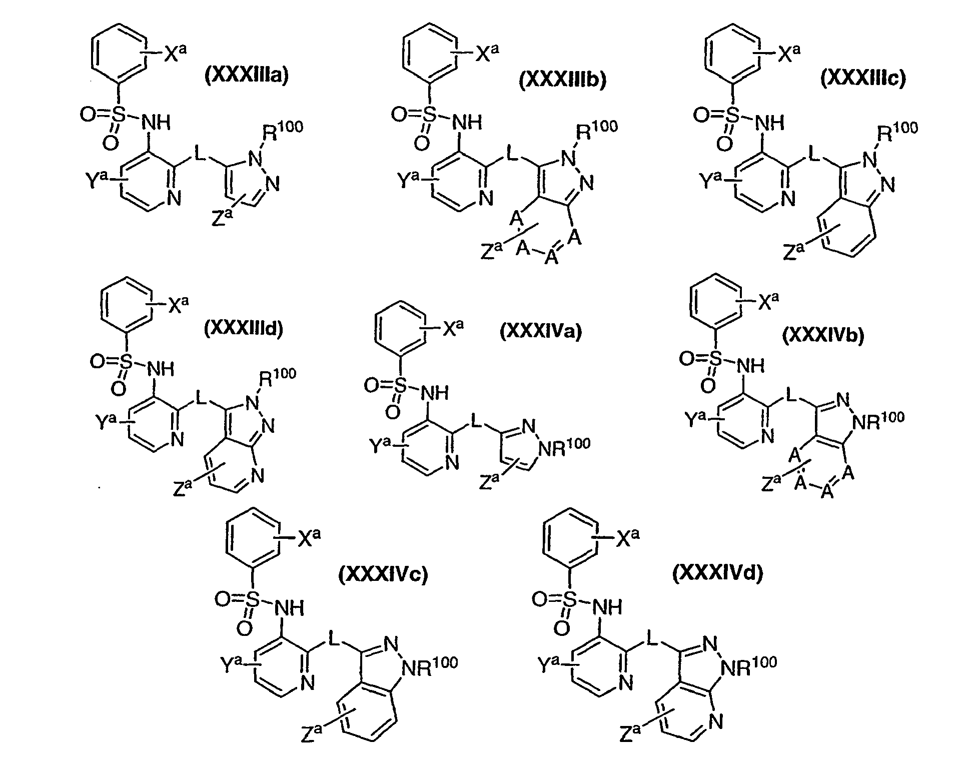

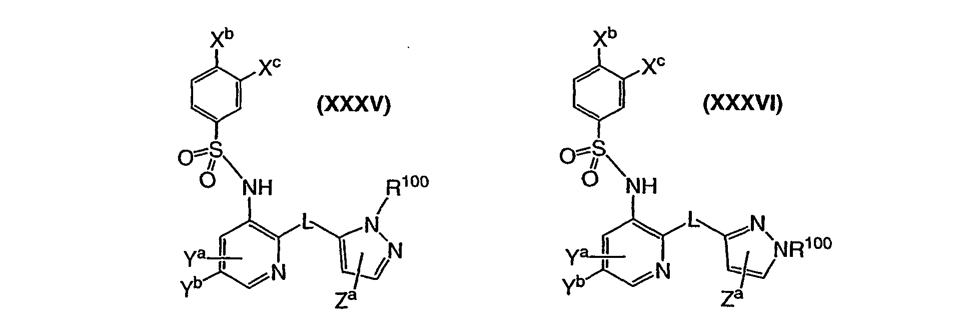

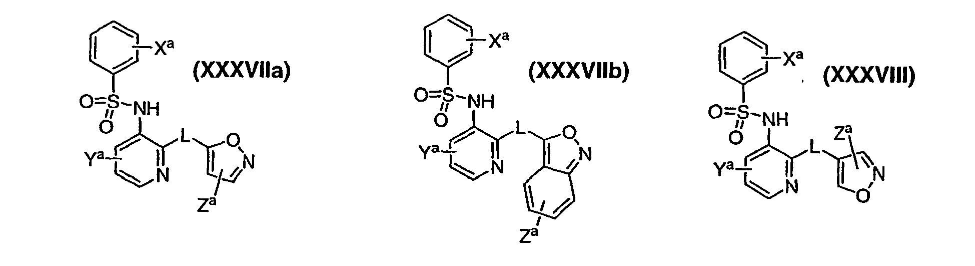

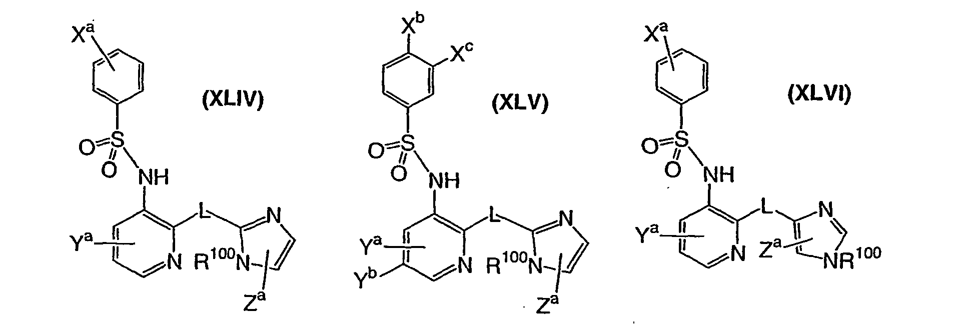

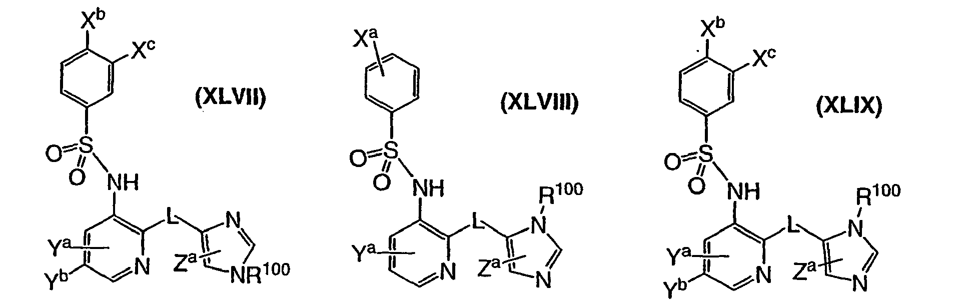

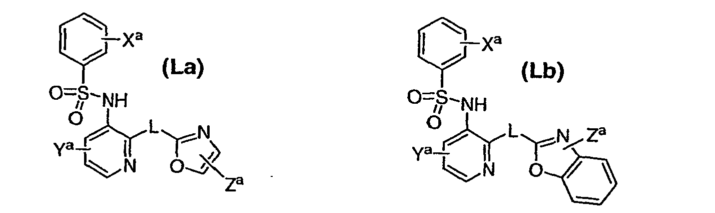

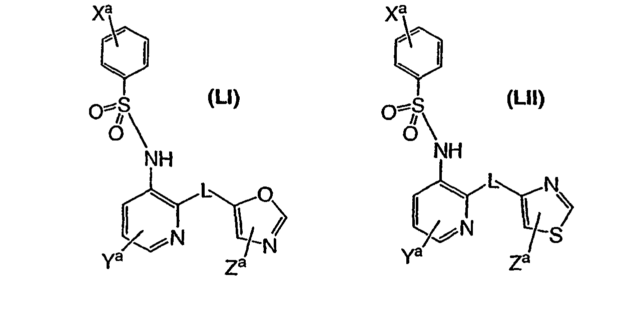

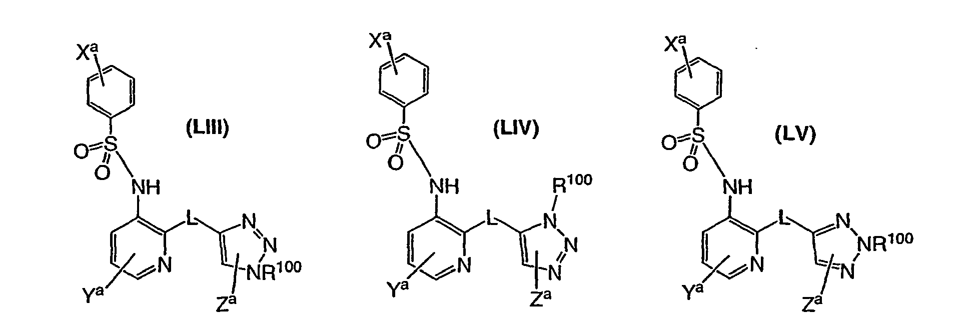

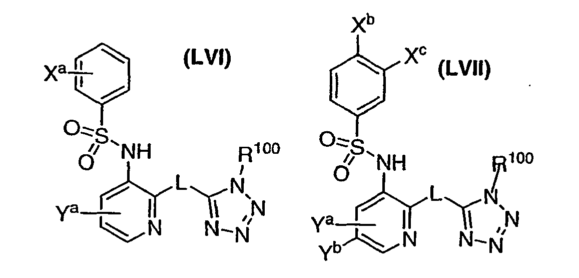

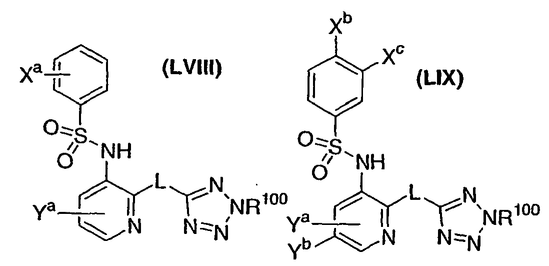

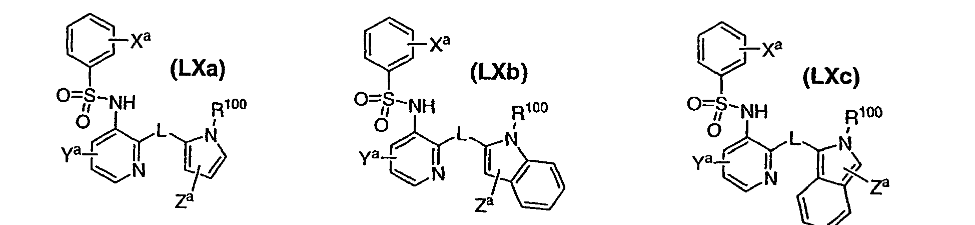

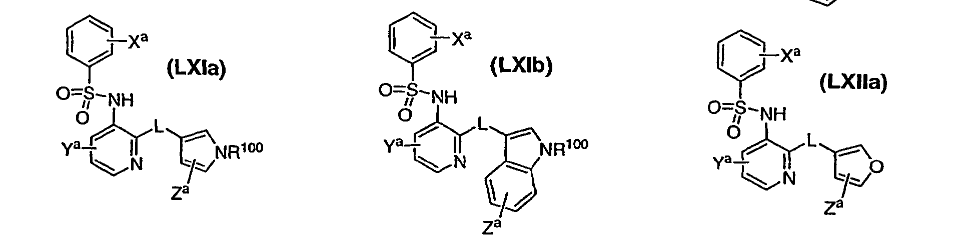

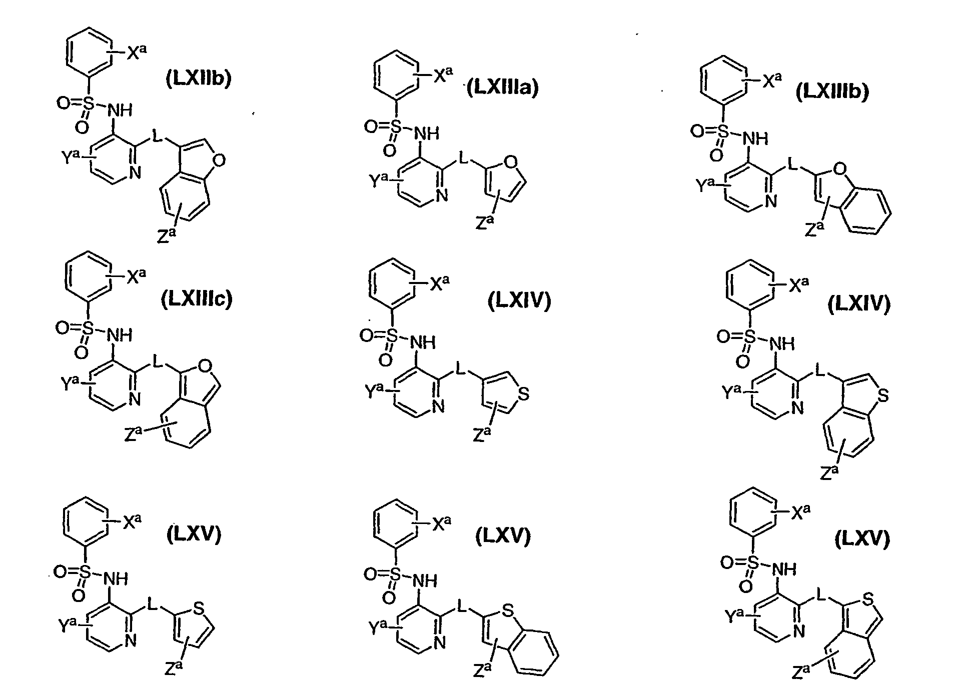

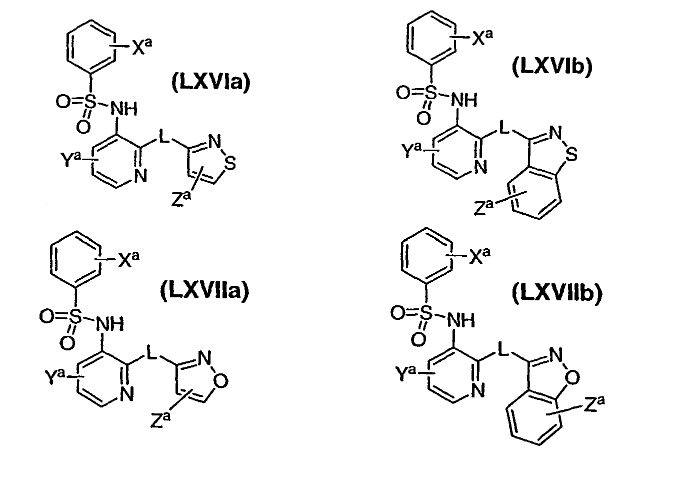

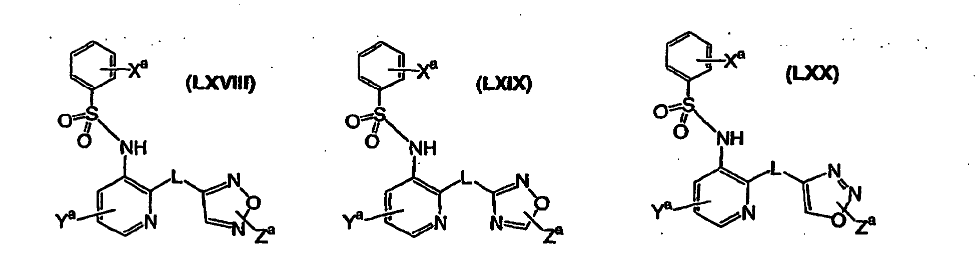

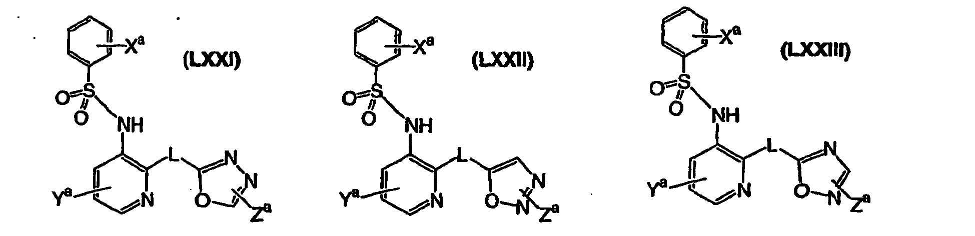

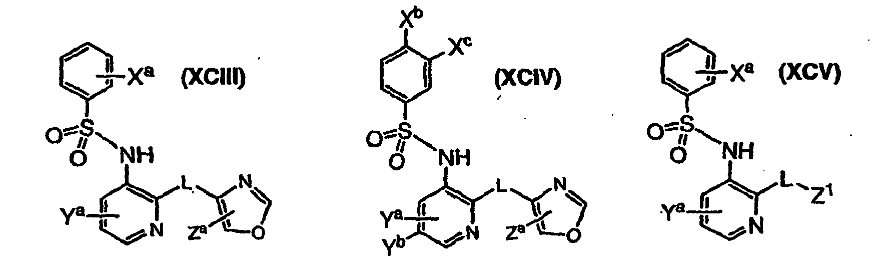

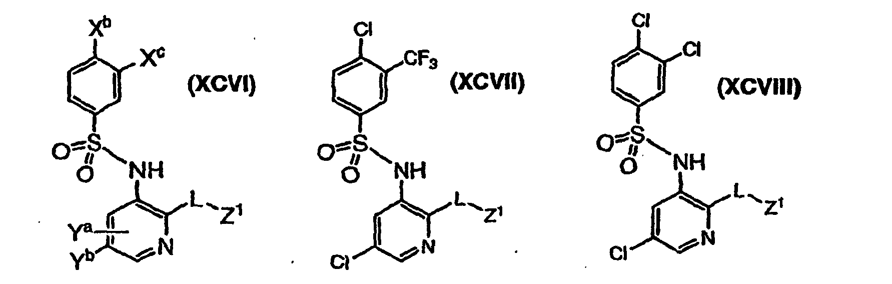

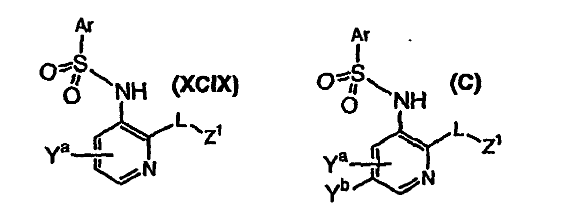

In the following descriptions and embodiments, references to specific substituents only correspond to formula numbers in which those specific substituents are present or appear.

In each of the formulae (IV to C), Ar, L and Z¹ are as defined above.

A is selected from the group consisting of -CH-, -CZ¹⁶-, -N-, and - N⁺(O)⁻-;
each occurrence of Z¹⁶ is independently selected from the group consisting of halogen, substituted or unsubstituted C₁₋₈ alkyl, substituted or unsubstituted C₂₋₈ alkenyl, substituted or unsubstituted C₂₋₈ alkynyl, - CN, oxo (=O or -O), -NO₂, -OR⁴², -OC(O)R⁴², -CO₂R⁴², -C(O)R⁴², -C(O)NR⁴²R⁴³, -OC(O)NR⁴²R⁴³, -NR⁴²C(O)R⁴³, -NR⁴²C(O)NR⁴³R⁴⁴, -NR⁴²R⁴³, -NR⁴²CO₂R⁴³, -SR⁴², -S(O)R⁴², -S(O)₂R⁴², -S(O)₂NR⁴²R⁴³, -NR⁴²S(O)₂R⁴³, substituted or unsubstituted C₆₋₁₀ aryl, substituted or unsubstituted 5- to 10-membered heteroaryl, substituted or unsubstituted 3- to 10-membered heterocyclyl, substituted or unsubstituted (C₁₋₄ alkyl)-(C₆₋₁₀ aryl), and substituted or unsubstituted (C₁₋₄ alkyl)-(5- to 10-membered heteroaryl);
R⁴², R⁴³ and R⁴⁴ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5- to 10-membered heteroaryl;

X^{a} represents 0 to 5 substituents each independently selected from the group consisting of halogen, -CN, -C(O)R²⁴, -CO₂R²⁴, -C(O)NR²⁴R²⁵, -OR²⁴, -OC(O)R²⁴, -OC(O)NR²⁴R²⁵, -SR²⁴, -S(O)R²⁴, -S(O)₂R²⁴, -S(O)₂NR²⁴R²⁵, -NO₂, -NR²⁴R²⁵, -NR²⁴C(O)R²⁵, -NR²⁴C(O)₂R²⁵, -NR²⁴S(O)₂R²⁵, -NR²⁴C(O)NR²-R²⁶, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R²⁴, R²⁵ and R²⁶ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
R²⁴ and R²⁵, R²⁵ and R²⁶ or R²⁴ and R²⁶ may, together with the atoms to which they are attached, form a substituted or unsubstituted 5-, 6-, or 7-membered ring.

X^{b} and X^{c} are each independently selected from the group consisting of hydrogen, halogen, -CN, -C(O)R^{24a}, -CO₂R^{24a}, -C(O)NR^{24a}R^{25a}, -OR^{24a}, -OC(O)R^{24a}, -OC(O)NR^{24a}R^{25a}, -SR^{24a}, -S(O)R^{24a}, -S(O)₂R^{24a}, -S(O)₂NR^{24a}R^{25a}, -N₂, -NR^{24a}R^{25a}, -NR^{24a}C(O)^{R25a}, -NR^{24a}C(O)₂R^{25a}, -NR^{24a}S(O)₂R^{25a}, -NR^{24a}C(O)NR^{25a}R^{2sa}, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R^{24a}, R^{25a} and R^{26a} are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
R^{24a} and R^{25a}, R^{25a} and R^{26a} or R^{24a} and R^{26a} may, together with the atoms to which they are attached, form a substituted or unsubstituted 5-, 6-, or 7-membered ring.

Y^{a} represents 0 to 3 substituents each independently selected from the group consisting of halogen, -CN, -C(O)R²⁷, -CO₂R²⁷, -C(O)NR²⁷R²⁸, -OR²⁷, -OC(O)R²⁷, -OC(O)NR²⁷R²⁸, -SR²⁷, -S(O)R²⁷, -S(O)₂R²⁷, -S(O)₂NR²⁷R²⁸, -NO₂, -NR²⁷R²⁸, -NR²⁷C(O)R²⁸, -NR²⁷C(O)₂R²⁸, -NR²⁷S(O)₂R²⁸, -NR²⁷C(O)NR²⁸R²⁹, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R²⁷, R²⁸ and R²⁹ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
R²⁷ and R²⁸, R²⁸ and R²⁹ or R²⁷ and R²⁹ may, together with the atoms to which they are attached, form a substituted or unsubstituted 5-, 6-, or 7-membered ring.

Y^{b} is independently selected from the group consisting of hydrogen, halogen, -CN, -C(O)R^{27a}, -CO₂R^{27a}, -C(O)NR^{27a}R^{28a}, -OR^{27a}, -OC(O)R^{27a}, -OC(O)NR^{27a}R^{28a}, -SR^{27a}, -S(O)R^{27a}, -S(O)₂R^{27a}, -S(O)₂NR^{27a}R^{28a}, -NO₂, -NR^{27a}R^{28a}, -NR^{27a}C(O)R^{28a}, -NR^{27a}C(O)₂R^{28a}, -NR^{27a}S(O)R^{28a}, -NR^{27a}C(O)NR^{28a}R^{29a}, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R^{27a}, R^{28a} and R^{29a} are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
R^{27a} and R^{28a}, R^{28a} and R^{29a} or R^{27a} and R^{29a} may, together with the atoms to which they are attached, form a substituted or unsubstituted 5-, 6-, or 7-membered ring.

Z^{a} represents 0 to 5 substituents each independently selected from the group consisting of halogen, -CN, -C(O)R³⁰, -CO₂R³⁰, -C(O)NR³⁰R³¹, -OR³⁰, -OC(O)R³⁰, -OC(O)NR³⁰R³¹, -SR³⁰, -S(O)R³⁰, -S(O)₂R³⁰, -S(O)₂NR³⁰R³¹, -NO₂, -NR³⁰R³¹, -NR³⁰C(O)R³¹, -NR³⁰C(O)₂R³¹, -NR³⁰S(O)₂R³¹, -NR³⁰C(O)NR³¹R³², oxo (=O or -O-), substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, substituted or unsubstituted 5- to 10-membered heteroaryl, substituted or unsubstituted (C₁₋₄ alkyl)-(C₆₋₁₀ aryl), and substituted or unsubstituted (C₁₋₄ alkyl)-(5- to 10-membered heteroaryl);
R³⁰, R³¹ and R³² are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
R³⁰ and R³¹, R³¹ and R³² or R³⁰ and R³² may, together with the atoms to which they are attached, form a substituted or unsubstituted 5-, 6-, or 7-membered ring.

Z^{b} is selected from the group consisting of hydrogen, halogen, -CN, -C(O)R^{30a}, -CO₂R^{30a}, -C(O)NR^{30a}R^{31a}, -OR^{30a}, -OC(O)_{R}^{30a}, -OC(O)NR^{30a}R^{31a}, -SR^{30a}, -S(O)R^{30a}, -S(O)₂R^{30a}, -S(O)₂NR^{30a}R^{31a}, -NO₂, -NR^{30a}R^{31a}, -NR^{30a}C(O)R^{31a}, -NR^{30a}C(O)₂R^{31a}, -NR^{30a}S(O)₂R^{31a}, -NR^{30a}C(O)NR^{31a}R^{32a}, oxo (=O or -O⁻), substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, substituted or unsubstituted 5- to 10-membered heteroaryl, substituted or unsubstituted (C₁₋₄ alkyl)-(C₆₋₁₀ aryl), and substituted or unsubstituted (C₁₋₄ alkyl)-(5- to 10-membered heteroaryl);
R^{30a}, R^{31a} and R^{32a} are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
R^{30a} and R^{31a}, R^{31a} and R^{32a} or R^{30a} and R^{32a} may, together with the atoms to which they are attached, form a substituted or unsubstituted 5-, 6-, or 7-membered ring.

R¹⁰⁰ is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, -S(O)R³³, or-S(O)₂R³³; -C(O)R³³, -C(Q)₂R³³;
R³³ is independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl.

R¹⁰¹ and R¹⁰² are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R¹⁰¹ and R¹⁰² may, together with the nitrogen, form a substituted or unsubstituted 4-, 5-, 6-, or 7-membered heterocyclyl.

r is 0, 1, or 2.

### Preferred Embodiments

In one embodiment, any of formulae (I-CIII) is other than N-(2-benzoyl-pyridin-3-yl)-4-isopropoxy-benzenesulfonamide.

In one embodiment, any of formulae (I-CIII), is other than formula CC: 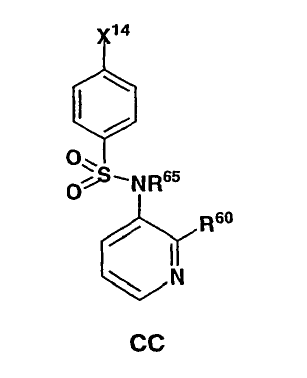 where X¹⁴ is selected from the group consisting of -Cl, -NO₂, -OCH₃, -CH₃, -NHC(O)CH₃, and -CH₂CH₂-(phenyl);
R⁶⁵ is selected from the group consisting of hydrogen, substituted or unsubstituted C₁₋₄ alkyl, and substituted or unsubstituted -SO₂(phenyl); and
R⁶⁰ is selected from the group consisting of -NR⁶¹CH₂CH₂OR⁶², - NR⁶¹CH₂CH₂NR⁶³R⁶⁴, -NR⁶¹CH₂CH₂SR⁶², 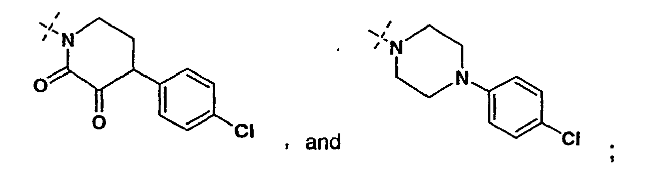 where R⁶¹ is selected from the group consisting of hydrogen and substituted or unsubstituted phenyl;
R⁶² is selected from the group consisting of substituted or unsubstituted phenyl, and substituted or unsubstituted C₁₋₄ alkyl; and
R⁶³ and R⁶⁴ are each independently selected from the group consisting of hydrogen, substituted or unsubstituted C₁₋₈ alkyl, substituted or unsubstituted phenyl, substituted or unsubstituted - SO₂(phenyl), -C(O)CH₃, -C(O)C(O)OH, and -C(O)₂C(CH₃)₃.

### Preferred Ar and X Substituents

In one embodiment of formula (III), at least one of X², X³, X⁴, X⁵ and X⁶ is other than hydrogen; or at least two of X², X³, X⁴, X⁵ and X⁶ is other than hydrogen.

In one embodiment of formula (III), X³ is other than hydrogen.

In one embodiment of formula (III), X⁴ is other than hydrogen.

In one embodiment of formula (III), X⁴ is other than isopropoxy.

In one embodiment of formula (III), X⁴ is other than methyl.

In one embodiment of formula (III), X³ and X⁴ are other than hydrogen.

In one embodiment of formula (III), when X⁴ is isopropoxy, at least one of X², X³, X⁵ and X⁶ is other than hydrogen.

In one embodiment of formula (III), X², X³, X⁴, X⁵ and X⁶ are each independently selected from the group consisting of hydrogen, halogen, -CN, -NO₂, -OR¹⁸, -C(O)R¹⁸, -SO₂R¹⁸, -NR¹⁸R¹⁹, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In one embodiment of formula (III), X², X³, X⁴, X⁵ and X⁶ are each independently selected from the group consisting of hydrogen, halogen, -CN, -NO₂, -OR¹⁸, -C(O)R¹⁸, -SO₂R¹⁸, -NR¹⁸R¹⁹, unsubstituted C₂₋₈ alkyl, substituted C₁₋₈ alkyl, unsubstituted or substituted phenyl, unsubstituted or substituted 5- or 6-membered heteroaryl, and unsubstituted or substituted 5- or 6-membered heterocyclyl.

In one embodiment of formula (III), X², X³, X⁴, X⁵ and X⁶ are each independently selected from the group consisting of hydrogen, halogen, -CN, -NO₂, -OR¹⁸ (but not isopropoxy), -C(O)R¹⁸, -SO₂R¹⁸, -NR¹⁸R¹⁹, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In one embodiment of formula (III), X², X³, X⁴, X⁵ and X⁶ are each independently selected from the group consisting of hydrogen, halogen, -CN, -NO₂, -OR¹⁸ (but not isopropoxy), -C(O)R¹⁸, -SO₂R¹⁸, -NR¹⁸R¹⁹, unsubstituted C₂₋₈ alkyl, substituted C₁₋₈ alkyl, unsubstituted or substituted phenyl, unsubstituted or substituted 5- or 6-membered heteroaryl, and unsubstituted or substituted 5- or 6-membered heterocyclyl.

In another embodiment of formula (III), X², X³, X⁴, X⁵ and X⁶ are each independently selected from the group consisting of: hydrogen, halogen, -CN, -NO₂, -OR¹⁸, -C(O)R¹⁸, -SO₂R¹⁸, -NR¹⁸R¹⁹, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl; with the proviso that at least two of X², X³, X⁴, X⁵ and X⁶ are other than hydrogen; or with the proviso that at least one of X², X³, X⁴, X⁵ and X⁶ is other than hydrogen.

In another embodiment of formula (III), X², X³, X⁴, X⁵ and X⁶ are-each independently selected from the group consisting of: hydrogen, halogen, -CN, -NO₂, -OR¹⁸, -C(O)R¹⁸, -SO₂R¹⁸, -NR¹⁸R¹⁹, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl; with the proviso that at least two of X³, X⁴ and X⁵ are other than hydrogen; or with the proviso at least one of X³, X⁴ and X⁵ is other than hydrogen.

In a further embodiment of formula (III), X², X³, X⁴, X⁵ and X⁶ are each independently selected from the group consisting of: hydrogen, halogen, -CN, -NO₂, -OR¹⁸, -C(O)R¹⁸, -SO₂R¹⁸, and -NR¹⁸R¹⁹; with the proviso that at least three of X², X³, X⁴, X⁵ and X⁶ are other than hydrogen; or with the proviso that at least two of X², X³, X⁴, X⁵ and X⁶ is other than hydrogen; or with the proviso that at least one of X², X³, X⁴, X⁵ and X⁶ is other than hydrogen.

In a further embodiment of formula (III), X², X³, X⁴, X⁵ and X⁶ are each independently selected from the group consisting of: hydrogen, halogen, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl; with the proviso that at least three of X², X³, X⁴, X⁵ and X⁶ are other than hydrogen; or with the proviso that at least two of X², X³, X⁴, X⁵ and X⁶ is other than hydrogen; or with the proviso that at least one of X², X³, X⁴, X⁵ and X⁶ is other than hydrogen.

In one embodiment of formula (III), X⁴ is selected from the group consisting of hydrogen, substituted or unsubstituted C₃₋₈ alkyl, substituted or unsubstituted C₂₋₈ alkenyl, substituted or unsubstituted C₂₋₈ alkynyl, -CN, - C(O)R¹⁸, -CO₂R¹⁸, -C(O)NR¹⁸R¹⁹, -OC(O)R¹⁹, -OC(O)NR¹⁸R¹⁹, -NO₂, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, substituted or unsubstituted C₆₋₁₀ aryl, substituted or unsubstituted 5- to 10-membered heteroaryl, and substituted or unsubstituted 3-to 10-membered heterocyclyl.

In one embodiment of formula (III), X⁴ is selected from the group consisting of halogen and -CH₃, and X³ is selected from the group consisting of halogen, -CH₃, -CF₃, -OCH₃, and -OCF₃.

In one embodiment of formula (III), when X², X³, X⁵ and X⁶ are hydrogen, X⁴ is other than -Cl, -NO₂, -OCH₃, -CH₃, -NHC(O)CH₃, or -CH₂CH₂-(phenyl).

In one embodiment of formulae (IV - C), X^{a} represents 1 to 5 substituents each independently selected from the group consisting of hydrogen, substituted or unsubstituted C₃₋₈ alkyl, substituted or unsubstituted C₂₋₈ alkenyl, substituted or unsubstituted C₂₋₈ alkynyl, -CN, -C(O)R¹⁸, -CO₂R¹⁸, -C(O)NR¹⁸R¹⁹, -OC(O)R¹⁹, -OC(O)NR¹⁸R¹⁹, -NO₂, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, substituted or unsubstituted C₆₋₁₀ aryl, substituted or unsubstituted 5- to 10-membered heteroaryl, and substituted or unsubstituted 3- to 10-membered heterocyclyl.

In one embodiment of formulae (IV - C), X^{a} represents 1 to 5 substituents each independently selected from the group consisting of halogen, -CN, -C(O)R²⁴, -CO₂R²⁴, -C(O)NR²⁴R²⁵, -OR²⁴, -OC(O)R²⁴, -OC(O)NR²⁴R²⁵, -SR²⁴, -S(O)R²⁴, -S(O)₂R²⁴, -S(O)₂NR²⁴R²⁵, -NO₂, -NR²⁴R²⁵, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl.

In one embodiment of formulae (IV - C), X^{a} represents 1 to 5 substituents each independently selected from the group consisting of halogen, -CN, -C(O)R²⁴, -CO₂R²⁴, -C(O)NR²⁴R²⁵, -OR²⁴, -OC(O)R²⁴, -OC(O)NR²⁴R²⁵, -SR²⁴, -S(O)R²⁴, -S(O)₂R²⁴, -S(O)₂NR²⁴R²⁵, -NO₂, substituted or unsubstituted C₁-8 alkyl, substituted or unsubstituted C₂₋₈ alkenyl, substituted or unsubstituted Z₂₋₃ alkynyl, substituted or unsubstituted 3- to 10-membered heterocyclyl, substituted or unsubstituted C₆₋₁₀ aryl, and substituted or unsubstituted 5- to 10-membered heteroaryl.

In one embodiment of formulae (IV - C), when X^{a} is para alkyl, L is C(O), and Z¹ is phenyl, at least one other X^{a} substituent is present.

In one embodiment of formulae (IV - C), when X^{a} is para isopropoxy, L is C(O), and Z¹ is phenyl, at least one other X^{a} substituent is present.

In one embodiment of formulae (IV - C), X^{a} represents 1 to 3 substituents each independently selected from the group consisting of halogen, -CN, -NO₂, -OR²⁴, -C(O)R²⁴, -SO₂R²⁴, -NR²⁴R²⁵, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In one embodiment of formulae (IV - C), X^{a} represents 1 to 3 substituents each independently selected from the group consisting of halogen, -CN, -NO₂, -OR²⁴ (but not isopropoxy), -C(O)R²⁴, -SO₂R²⁴, -NR²⁴R²⁵, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In one embodiment of formulae (IV - C), X^{a} represents two substituents each independently selected from the group consisting of halogen, -CN, -NO₂, -OR²⁴, -C(O)R²⁴, -SO₂R²⁴, -NR²⁴R²⁵, unsubstituted or substituted C₁₋₆ alkyl, unsubstituted or substituted phenyl, unsubstituted or substituted 5- or 6-membered heteroaryl, and unsubstituted or substituted 5- or 6-membered heterocyclyl.

In one embodiment of formulae (IV - C), X^{a} represents two substituents each independently selected from the group consisting of halogen, methyl, -OCH₃, -OCF₃, and -CF₃.

In one embodiment of formulae (IV - C), X^{a} represents 1 to 2 substituents each independently selected from the group consisting of halogen, -CN, and -CF3.

In one embodiment of formulae (IV - C), at least one of X^{b} and X^{c} is other than hydrogen.

In one embodiment of formulae (IV - C), X^{b} and X^{c} are each independently selected from the group consisting of hydrogen, halogen, -CN, -C(O)R^{24a}, -O₂R^{24a}, -C(O)NR^{24a} R^{25a}, -OR^{24a}, -OC(O)R^{24a}, -OC(O)NR^{24a}R^{25a}, -SR^{24a}, -S(O)R^{24a}, -S(O)₂R^{24a}, -S(O)₂NR^{24a}R^{25a}, -NO₂, -NR^{24a}R^{25a}, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl.

In one embodiment of formulae (IV - C), at least one of X^{b} and X^{c} is selected from the group consisting of halogen, -CN, -NO₂, -OR²⁴, -C(O)R²⁴, -SO₂R²⁴, -NR²⁴R²⁵, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In one embodiment of formulae (IV - C), at least one of X^{b} and X^{c} is selected from the group consisting of halogen, -CN, -NO₂, -OR²⁴ (but not isopropoxy), -C(O)R²⁴, -SO₂R²⁴, -NR²⁴R²⁵, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In another embodiment of formulae (IV - C), X^{b} and X^{c} are both other than hydrogen.

In another embodiment of formulae (IV - C), when X^{b} is isopropoxy, X^{c} is other than hydrogen.

In another embodiment of formulae (IV - C), when X^{b} is methyl, X^{c} is other than hydrogen.

In another embodiment of formulae (IV - C), X^{b} and X^{c} are both selected from the group consisting of halogen, -CN, -NO₂, -OR²⁴, -C(O)R²⁴, -SO₂R²⁴, -NR²⁴R²⁵, unsubstituted or substituted C₁₋₈ alkyl, unsubstituted or substituted phenyl, unsubstituted or substituted 5- or 6-membered heteroaryl, and unsubstituted or substituted 5- or 6-membered heterocyclyl.

In another embodiment of formulae (IV - C), X^{b} and X^{c}, or any two occurrences of X^{a} that are located adjacently to each other, can be joined to form a substituted 5- or 6-membered substituted or unsubstituted heterocyclyl or substituted or unsubstituted heteroaryl.

In one embodiment of formulae (IV - C), at least one of X^{b} and X^{c} is selected from the group consisting of halogen, -CN, and -CF₃.

In one embodiment of formulae (IV - C), both X^{b} and X^{c} are selected from the group consisting of halogen, -CN, and -CF₃.

In an additional embodiment of formulae (IV - C), one of X^{b}, and X^{c} is halogen and one of X^{b} and X^{c} is selected from the group consisting of halogen, -CN, and -CF₃.

In an additional embodiment of formulae (IV - C), one of X^{b}, and X^{c} is halogen and one of X^{b} and X^{c} is selected from the group consisting of halogen, -CN, -CH₃, and -CF₃.

In an additional embodiment of formulae (IV - C), one of X^{b}, and X^{c} is hydrogen and one of X^{b} and X^{c} is selected from the group consisting of halogen, -CN, and -CF₃.

In an additional embodiment of formulae (IV - C), one of X^{b}, and X^{c} is hydrogen and one of X^{b} and X^{c} is selected from the group consisting of halogen, -CN, -CH₃, and -CF₃.

In an additional embodiment of formulae (IV - C), X^{b} is selected from the group consisting of halogen and -CH₃, and X^{c} is selected from the group consisting of halogen, -CN, -CH₃, -OCH₃, - OCF₃ and -CF₃.

In an additional embodiment of formulae (IV - C), when X^{c} is hydrogen, X^{b} represents 1 to 5 substituents each independently selected from the group consisting of hydrogen, substituted or unsubstituted C₃₋₈ alkyl, substituted or unsubstituted C₂₋₈ alkenyl, substituted or unsubstituted C₂₋₈ alkynyl, -CN, -C(O)R¹⁸, -CO₂R¹⁸, -C(O)NR¹⁸R¹⁹, -OC(O)R¹⁹, -OC(O)NR¹⁸R¹⁹, - NO₂, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, substituted or unsubstituted C₆₋₁₀ aryl, substituted or unsubstituted 5- to 10-membered heteroaryl, and substituted or unsubstituted 3- to 10-membered heterocyclyl.

In an additional embodiment of formulae (IV - C), when X^{c} is hydrogen, X^{b} is other than -Cl, -NO₂, -OCH₃, -CH₃, -NHC(O)CH₃, or -CH₂CH₂-(phenyl).

In an additional embodiment of formulae (IV - C), X^{a} represents 2 substituents, one of which is halogen and the other of which is selected from the group consisting of halogen, -CN, and -CF₃.

In an additional embodiment of formulae (IV - C), X^{a} represents 2 substituents, where one occurrence of X^{a} is halogen and one occurrence of X^{a} is located para to the sulfonamido bond.

In an additional embodiment of formulae (IV - C), at least one of one occurrence of X^{a} or alternatively one of X^{b} or X^{c} is -CN.

In another embodiment of formulae (IV - C), at least one of one occurrence of X^{a} or alternatively one of X^{b} or X^{c} is halogen.

In an additional embodiment of formulae (IV - C), at least one of one occurrence of X^{a} or alternatively one of X^{b} or X^{c} is -CF₃.

In an additional embodiment of formulae (IV - C), at least one of one occurrence of X^{a}, or one of X^{b} and X^{c}, is selected from the group consisting of:

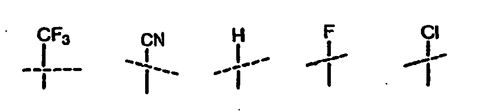

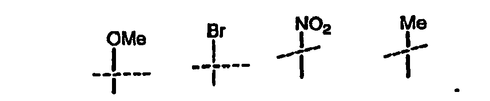

In one embodiment of the formulae (I, II, XCV-C), Ar is selected from the group consisting of substituted or unsubstituted phenyl, with the proviso that Ar is other than para-isopropoxy phenyl.

In one embodiment of the formulae (I, II, XCV-C), Ar is selected from the group consisting of:

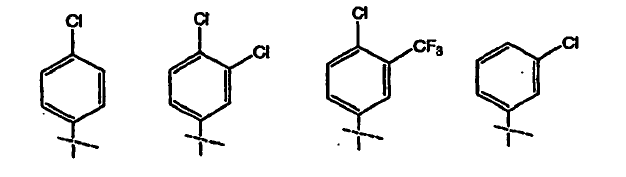

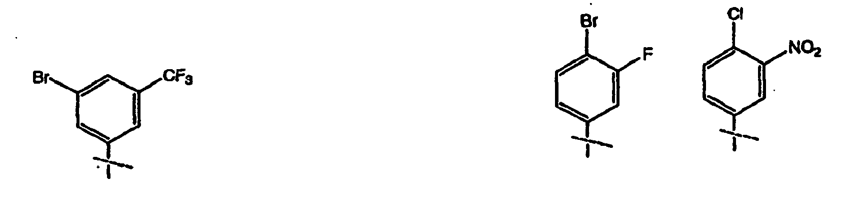

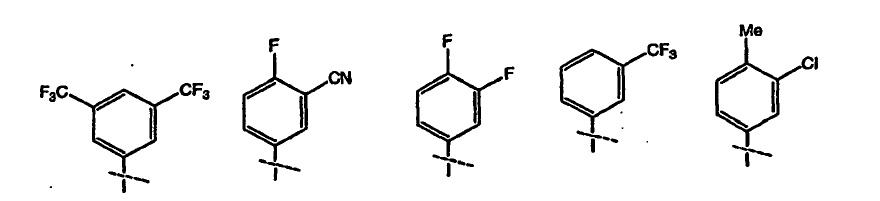

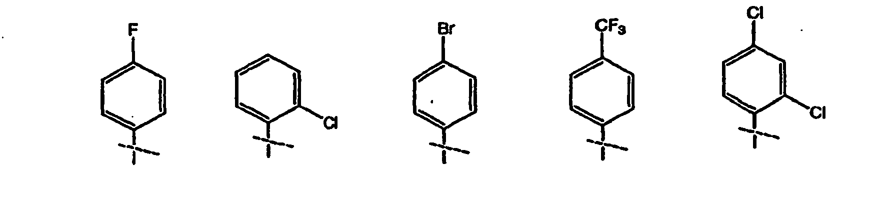

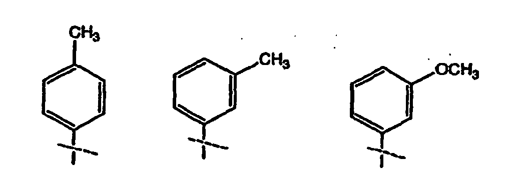

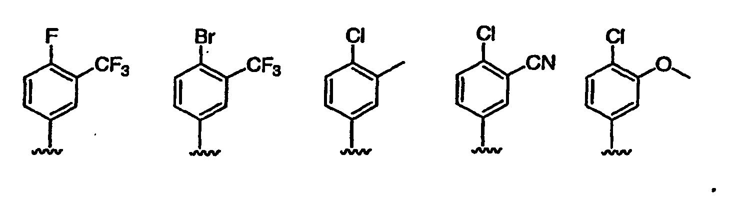

### Preferred R¹ Substituents

In one embodiment of formula (I and II), R¹ is hydrogen.

In formula (I and II), R¹ is selected from the group consisting of hydrogen, unsubstituted C₁ alkyl and unsubstituted C₃₋₈ alkyl,

In one embodiment of formula (I and II), R¹ is selected from the group consisting of unsubstituted C₁ alkyl and unsubstituted C₃₋₈ alkyl

In one embodiment of formula (I and II), R¹ is selected from the group consisting of substituted or unsubstituted C₅₋₈ alkyl, substituted or unsubstituted C₂₋₆ alkenyl, substituted or unsubstituted C₂₋₆ alkynyl, and substituted or unsubstituted 3- to 10-membered heterocyclyl;

In one embodiment of formula (I and II), R¹ is other than hydrogen, and substituted or unsubstituted C₁₋₄ alkyl.

### Preferred Y and R² Substituents

In one embodiment of formula (I), Y¹ , Y² and Y³ are -CR^{2a}-, -CR^{2b}-, and -CR^{2c}- respectively, where R^{2a}, R^{2b}, and R^{2c} are each hydrogen.

In one embodiment of formula (I), Y¹, Y² and Y³ are -CR^{2a}-_{.} -CR^{ab}-, and -CR^{2c}- respectively, where 1 to 3 of R²⁸, R^{2b}, and R^{2c} are other than hydrogen.

In one embodiment of formula (I), Y¹, Y² and Y³ are -CR^{2a}-, -CR^{2b}-, and -CR^{2c}- respectively and R^{2a}, R^{2b}, and R^{2c} are each independently selected from the group consisting of halogen, and unsubstituted C₁₋₈ alkyl, where 1 to 3 of R^{2a}, R^{2b}, and R^{2c} are other than hydrogen.

In one embodiment of formula (I), Y¹, Y² and Y³ are -CR^{2a}-, -CR^{2b} , and -CR^{2c}- respectively and R^{2a}, R^{2b}, and R^{2c} are each independently selected from the group consisting of hydrogen, fluorine, chlorine, and bromine, where 1 to 2 of R^{2a}, R^{2b}, and R^{2c} are other than hydrogen.

In one embodiment of formula (II), Y⁵, Y⁶, and Y⁷ are each independently selected from the group consisting of hydrogen, halogen, and unsubstituted C₁₋₈ alkyl, where 1 to 2 of Y⁵, Y⁶, and Y⁷ are other than hydrogen.

In one embodiment of formula (II), Y⁵, Y⁶, and Y⁷ are each independently selected from the group consisting of hydrogen, fluorine, chlorine, and bromine, where 1 to 2 of Y⁵, Y⁶, and Y⁷ are other than hydrogen.

In another embodiment of formula (II), at least one of Y⁵, Y⁶ and Y⁷ is other than hydrogen; preferably Y⁶ is halogen.

In another embodiment of formula (II), Y⁵ and Y⁷ are hydrogen and Y⁶ is halogen.

In another embodiment of formula (II), Y⁵ and Y⁷ are hydrogen and Y⁶ is chloro.

In one embodiment of formula (III), Y⁸, Y⁹, and Y¹⁰ are each independently selected from the group consisting of hydrogen, halogen, -CO₂R³, -OR³, and substituted or unsubstituted C₁₋₈ alkyl, where 1 to 2 of Y⁸, Y⁹, and Y¹⁰ are other than hydrogen.

In one embodiment of formula (III), Y⁸, Y⁹, and Y¹⁰ are each independently selected from the group consisting of hydrogen, fluorine, chlorine, and bromine, where 1 to 2 of Y⁸, Y⁹, and Y¹⁰ are other than hydrogen.

In one embodiment of formula (III), at least one of Y⁸, Y⁹ and Y¹⁰ is other than hydrogen; preferably Y⁹ is other than hydrogen.

In another embodiment of formula (III), at least two of Y⁸, Y⁹ and Y¹⁰ are other than hydrogen.

In a further embodiment of formula (III), Y⁸ and Y¹⁰ are hydrogen and Y⁹ is halogen.

In a further embodiment of formula (III), Y⁸ and Y¹⁰ are hydrogen and Y⁹ is chloro.

In one embodiment of formulae (IV - C), Y^{b} or at least one occurrence of Y^{a} is other than hydrogen.

In one embodiment of formulae (IV - C), Y^{a} represents zero substituents and Y^{b} is hydrogen.

In one embodiment of formulae (IV - C), Y^{a} represents zero substituents and Y^{b} is other than hydrogen.

In one embodiment of formulae (IV - C), Y^{b} and each occurrence of Y^{a} are selected from the group consisting of halogen, -C(O)R²⁷, -OR²⁷, and substituted or unsubstituted C₁₋₈ alkyl.

In one embodiment of formulae (IV - C), Y^{b} and each occurrence of Y^{a} are selected from the group consisting of halogen, fluorine, chlorine and bromine.

In one embodiment of formulae (IV - C), Y^{b} and each occurrence of Y^{a} are selected from the group consisting of fluorine, chlorine and bromine.

In one embodiment of formulae (IV - C), Y^{b} and each occurrence of Y^{a} are selected from the group consisting of chlorine and hydrogen, where one of Y^{a} and Y^{b} is hydrogen and one of Y^{a} and Y^{b} is chlorine.

### Preferred L Substituents

In formula (I-C), L is -C(O)-.

### Preferred Z¹ Groups and Substituents

In one embodiment of formulae (I - C), Z¹ is substituted or unsubstituted phenyl.

In one embodiment of formulae (I - C), Z¹ is substituted phenyl.

In one embodiment of formulae (I - C), Z¹ is substituted or unsubstituted heteroaryl.

In one embodiment of formulae (I - C), Z¹ is substituted heteroaryl.

In one embodiment of formulae (I - C), Z¹ is unsubstituted heteroaryl.

In one embodiment of formulae (I - C), Z¹ is the following formula (CII): 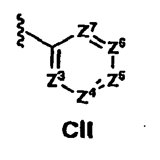 where:
Z³, Z⁴, Z⁵, Z⁶ and Z⁷ are each independently selected from the group consisting of -CZ^{7a}-, -N-, and -N⁺(O)⁻-;
Z^{7a} is selected from the group consisting of halogen, -CN, -C(O)R^{34a}, -CO₂R^{34a}, -C(O)NR^{34a}R^{36a}, -OR^{34a}, -OC(O)R^{34a}, -OC(O)NR^{34a}R^{36a}, -SR^{34a}, -S(O)R^{34a}, -S(O)₂R^{34a}, -S(O)₂NR^{34a}R^{36a}, -NO₂, - NR^{34a}R^{35a}, -NR^{34a}C(O)R^{35a}, -NR^{34a}C(O)₂R^{35a}, -NR^{34a}S(O)₂R^{35a},-NR^{34a}C(O)NR^{35a}R^{36a}, oxo (=O or -O⁻), substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, substituted or unsubstituted 5- to 10-membered heteroaryl, substituted or unsubstituted (C₁₋₄ alkyl)-(C₆₋₁₀ aryl), and substituted or unsubstituted (C₁₋₄ alkyl)-(5- to 10-membered heteroaryl);
R^{34a}, R^{35a}, and R^{36a} are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5- to 10-membered heteroaryl.

In one embodiment of formula ( CII), Z³, Z⁴, Z⁵, Z⁶ and Z⁷ are -CH-.

In one embodiment of formula ( CII), at least one of Z³, Z⁴, Z⁵, Z⁶ and Z⁷ is -N-, or -N⁺(O)⁻-.

In one embodiment of formula (CII), at least one of Z³, Z⁴, Z², Z⁶ and Z⁷ is -N⁺(O)⁻-.

In one embodiment of formula (CII), at least one of Z³, Z⁴, Z⁵, Z⁶ and Z⁷ is -N-.

In one embodiment of formula (CII), Ar is substituted or unsubstituted phenyl and one of Z³, Z⁴, Z⁵, Z⁶ and Z⁷ is -N-, or -N⁺(O)⁻-.

In one embodiment of formula (CII), Ar is substituted or unsubstituted phenyl, L is -C(O)- and one of Z³, Z⁴, Z⁵ and Z⁶ is -N-, or -N⁺(O)⁻-.

In one embodiment of formulae (CI and CII), Ar is substituted phenyl, L is -C(O)- and one of Z³, Z⁴, Z⁵ and Z⁸ is -N-, or -N⁺(O)⁻-.

In one embodiment of formulae (I - C), Z¹ is one of the following formulae (CIII and CIV): 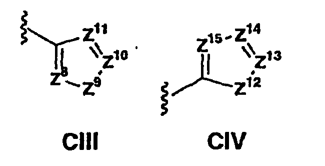 where:
Z⁸, Z¹⁰, Z¹¹, Z¹³, Z¹⁴ and Z¹⁵ are each independently selected from the group consisting of of -CR³⁷-, -N-, and -N⁺(O)⁻-;
R³⁷ represents 0 to 4 substituents selected from the group consisting of halogen, -CN, -C(O)R³⁸, -CO₂R^{3a}, -C(O)NR³⁸R³⁹, -OR³⁸, -OC(O)R³⁸, -OC(O)NR³⁸R³⁹, -SR³⁸, -S(O)R³⁹, -S(O)₂R³⁸, -S(O)₂NR³⁸R³⁹, -N^{O2}, -NR³⁸R³⁹, -NR³⁸C(O)R³⁹, -NR³⁸C(O)₂R³⁹, -NR³⁸S(O)₂R³⁹, -NR³⁸C(O)NR³⁹R⁴⁰, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, substituted or unsubstituted 5- to 10-membered heteroaryl, substituted or unsubstituted (C₁₋₄ alkyl)-(C₆-₁₀ aryl), and substituted or unsubstituted (C₁₋₄ alkyl)-(5- to 10-membered heteroaryl);
R³⁸, R³⁹, and R⁴⁰ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl;
Z⁹ and Z¹² are each independently selected from the group consisting of - O-, -S-, -NR⁴¹-, and -N⁺(O)⁻-;
R⁴¹ is selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5- to 10-membered heteroaryl.

In one embodiment of formulae (CIII and CIV), one of Z⁸, Z¹⁰ and 2¹¹ is -N- or -N⁻-O⁺ or one of Z¹³, Z¹⁴ and Z¹⁵ is -N- or -N⁻-O⁺.

In one embodiment of formulae (CIII and CIV), at least one of Z⁸, Z₁₀ and Z¹¹ is -N- or -N⁻-O⁺ or one of Z¹³, Z¹⁴ and Z¹⁵ is -N- or -N⁻-O⁺.

In one embodiment of formulae (I - C), Z¹ is selected from the formulae (CV - CXXI): 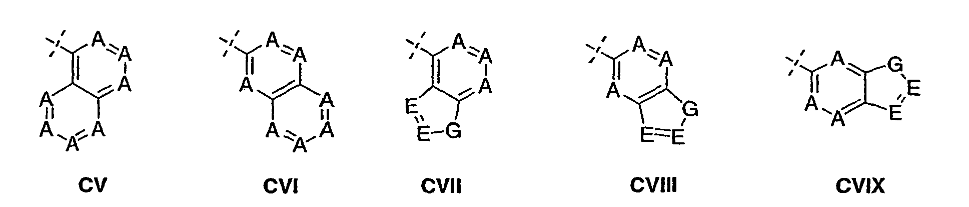 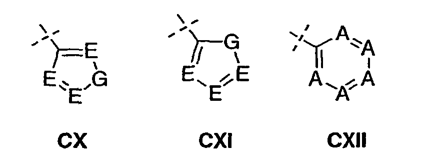 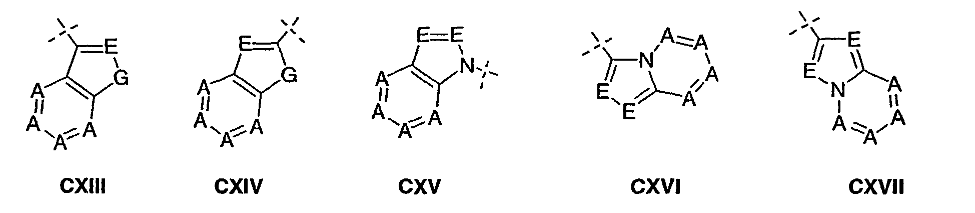 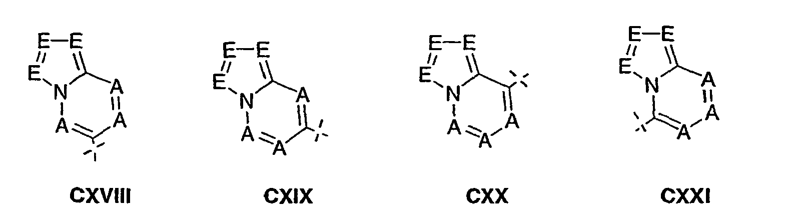 where,
A is selected from the group consisting of -CH-, -CZ¹⁶-, -N-, and -N⁺(O)⁻-;
each occurrence of Z¹⁶ is independently selected from the group consisting of halogen, substituted or unsubstituted C₁₋₈ alkyl, substituted or unsubstituted C₂₋₈ alkenyl, substituted or unsubstituted C₂₋₈ alkynyl, - CN, oxo (=O or -O⁻), -NO₂, -OR⁴², -OC(O)R⁴², -CO₂R⁴², -C(O)R⁴², -C(O)NR⁴²R⁴³, -OC(O)NR⁴²R⁴³, -NR⁴²C(O)R⁴³, -NR⁴²C(O)NR⁴³R⁴⁴, -NR⁴²R⁴³, -NR⁴²CO₂R⁴³, -SR⁴², -S(O)R⁴², -S(O)₂R⁴², -S(O)₂NR⁴²R⁴³, -NR⁴²S(O)₂R⁴³, substituted or unsubstituted C₆₋₁₀ aryl, substituted or unsubstituted 5- to 10-membered heteroaryl, substituted or unsubstituted 3- to 10-membered heterocyclyl, substituted or unsubstituted (C₁₋₄ alkyl)-(C₆₋₁₀ aryl), and substituted or unsubstituted (C₁₋₄ alkyl)-(5- to 10-membered heteroaryl);
R⁴², R⁴³and R⁴⁴ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆-₁₀ aryl, and substituted or unsubstituted 5- to 10-membered heteroaryl;
E is selected from the group consisting of -CH-,-CZ¹⁷-, -N-, and -N⁺(O)⁻-; each occurrence of Z¹⁷ is independently selected from the group consisting of halogen, substituted or unsubstituted C₁₋₈ alkyl, substituted or unsubstituted C₂₋₈ alkenyl, substituted or unsubstituted C₂₋₈ alkynyl, - CN, oxo (=O or-O⁻), -NO₂, -OR⁴⁴, -OC(O)R⁴⁴, -CO₂R⁴⁴, -C(O)R⁴⁴, -C(O)NR⁴⁴R⁴⁵, -OC(O)NR⁴⁴R⁴⁵, -NR⁴⁴C(O)R⁴⁵, -NR⁴⁴C(O)NR⁴⁵R⁴⁶, -NR⁴⁴R⁴⁵, -NR⁴⁴CO₂R⁴⁵, -SR⁴⁴, -S(O)R⁴⁴, -S(O)₂R⁴⁴, -S(O)₂NR⁴⁴R⁴⁵, -NR⁴⁴S(O)₂R⁴⁵, substituted or unsubstituted C₆₋₁₀ aryl, substituted or unsubstituted 5- to 10-membered heteroaryl, substituted or unsubstituted 3- to 10-membered heterocyclyl, substituted or unsubstituted (C₁₋₄ alkyl)-(C₆₋₁₀ aryl), and substituted or unsubstituted (C₁₋₄ alkyl)-(5- to 10-membered heteroaryl);
R⁴⁴, R⁴⁵ and R⁴⁶ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5- to 10-membered heteroaryl;
G is selected from the group consisting of -O-, -S-, and -NR⁴⁷-;
R⁴⁷ is selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5- to 10-membered heteroaryl.

In one embodiment of formulae (I- C), Z¹ is substituted or unsubstituted 5- to 10-membered heteroaryl.

In one embodiment of formulae (I- C), Z¹ is substituted 5- or 6-membered heteroaryl.

In one embodiment of formulae (I- C), Z¹ is unsubstituted 5- or 6-membered heteroaryl.

In one embodiment of formulae (I- C), Z¹ is substituted 9- or 10-membered heteroaryl.

In one embodiment of formulae (I- C), Z¹ is unsubstituted 9- or 10-membered heteroaryl.

In one embodiment of formulae (I- C), Z¹ is substituted 5- to 10-membered heteroaryl, wherein one substituent is located ortho- to one of the heteroatoms in the ring.

In one embodiment of formulae (I - C), Z¹ is selected from the group consisting of substituted or unsubstituted 5- or 6-membered heteroaryl with 1 to 2 nitrogen atoms and with 0 or 2 substituents independently selected from the group consisting of halogen, unsubstituted or substituted C₁₋₈ alkyl, oxo (=O or - O⁻), -CN, -NO₂, -OR⁴⁸,-C(O)R⁴⁸, -C(O)NR⁴⁸R⁴⁹, -NR⁴⁸C(O)R⁴⁹, -NR⁴⁸R⁴⁹, -SR⁴⁸, - S(O)R⁴⁸, -S(O)₂R⁴⁸, -SO₂NR⁴⁸R⁴⁹, -NR⁴⁸SO₂R⁴⁹, unsubstituted or substituted 5-to 6-membered heteroaryl, unsubstituted or substituted 3- to 10-membered heterocyclyl, substituted or unsubstituted (C₁₋₄ alkyl)-(C₆₋₁₀ aryl),and substituted or unsubstituted (C₁₋₄ alkyl)-(5- to 10-membered heteroaryl);
R⁴⁸ and R⁴⁹ are each independently selected from the group consisting of hydrogen, substituted or unsubstituted C₁₋₈ alkyl, substituted or unsubstituted 3- to 10-membered heterocyclyl, substituted or unsubstituted C₂₋₈ alkenyl, substituted or unsubstituted C₂₋₈ alkynyl, substituted or unsubstituted C₆₋₁₀ aryl, and substituted or unsubstituted 5-to 10-membered heteroaryl.

In one embodiment of formulae (I - C), Z¹ is selected from the group consisting of substituted or unsubstituted 5- or 6-membered heteroaryl with 1 to 2 nitrogen atoms and with 0 or 2 substituents each independently selected from the group consisting of halogen, substituted or unsubstituted C₁₋₈ alkyl, substituted or unsubstituted C₂₋₈ alkenyl, substituted or unsubstituted C₂₋₈ alkynyl, -CN, oxo (=O or-O⁻), -NO₂, -OR⁴⁴, -OC(O)R⁴⁴, -CO₂R⁴⁴, -C(O)R⁴⁴, -C(O)NR⁴⁴R⁴⁵, -OC(O)NR⁴⁴R⁴⁵, -NR⁴⁴C(O)R⁴⁵, -NR⁴⁴C(O)NR⁴⁵R⁴⁶, -NR⁴⁴R⁴⁵, -NR⁴⁴CO₂R⁴⁵, - SR⁴⁴, -S(O)R⁴⁴, -S(O)₂R⁴⁴, -S(O)₂NR⁴⁴R⁴⁵, -NR⁴⁴S(O)₂R⁴⁵, substituted or unsubstituted C₆₋₁₀ aryl, substituted or unsubstituted 5- to 10-membered heteroaryl and substituted or unsubstituted 3- to 10-membered heterocyclyl;
R⁴⁴, R⁴⁵ and R⁴⁶ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl;

In one embodiment of formulae (I- C), Z¹ is selected from the group consisting of substituted or unsubstituted 5- or 6-membered heteroaryl with 1 to 2 nitrogen atoms and with 0 or 2 substituents independently selected from the group consisting of substituted or unsubstituted C₂₋₈ alkenyl, substituted or unsubstituted C₂₋₈ alkynyl, -CO₂R^{44a}, -OC(O)NR^{44a}R^{45a}, -NR^{44a}C(O)NR^{45a}R^{46a}, -NR^{44a}CO₂R^{45a} and substituted or unsubstituted C₆₋₁₀ aryl;
R^{44a}, R^{45a} and R^{46a} are each independently selected from the group consisting of hydrogen, substituted or unsubstituted C₁₋₈ alkyl, substituted or unsubstituted 3- to 10-membered heterocyclyl, substituted or unsubstituted C₂₋₈ alkenyl, substituted or unsubstituted C₂₋₈ alkynyl, substituted or unsubstituted C₆₋₁₀ aryl, and substituted or unsubstituted 5-to 10-membered heteroaryl;

In one embodiment of formulae (I - C), Z¹ is selected from the group consisting of substituted or unsubstituted 9- or 10-membered heteroaryl with 1 to 2 nitrogen atoms and with 0 or 2 substituents independently selected from the group consisting of halogen, unsubstituted or substituted C₁₋₈ alkyl, oxo (=O or-O⁻), -CN, -NO₂, -OR⁴⁸,-C(O)R⁴⁸, -C(O)NR⁴⁸R⁴⁹, -NR⁴⁸C(O)R⁴⁹, -NR⁴⁸R⁴⁹, -SR⁴⁸,-S(O)R⁴⁸, -S(O)₂R⁴⁸, -SO₂NR⁴⁸R⁴⁹, -NR⁴⁸SO₂R⁴⁹, unsubstituted or substituted 5-to 6-membered heteroaryl, unsubstituted or substituted 3- to 10-membered heterocyclyl, and substituted or unsubstituted (C₁₋₄ alkyl)-(5- to 10-membered heteroaryl);
R⁴⁸ and R⁴⁹ are each independently selected from the group consisting of hydrogen, substituted or unsubstituted C₁₋₈ alkyl, substituted or unsubstituted 3- to 10-membered heterocyclyl, substituted or unsubstituted C₂₋₈ alkenyl, substituted or unsubstituted Z₂₋₈ alkynyl, substituted or unsubstituted C₆₋₁₀ aryl, and substituted or unsubstituted 5-to 10-membered heteroaryl.

In one embodiment of formulae (I - C), Z¹ is selected from the group consisting of substituted or unsubstituted 9- or 10-membered heteroaryl with 1 to 2 nitrogen atoms and with 0 or 2 substituents each independently selected from the group consisting of halogen, substituted or unsubstituted C₁₋₈ alkyl, substituted or unsubstituted C₂₋₈ alkenyl, substituted or unsubstituted C₂₋₈ alkynyl, -CN, oxo (=O or-O⁻), -NO₂, -OR⁴⁴, -OC(O)R⁴⁴, -CO₂R⁴⁴, -C(O)R⁴⁴, -C(O)NR⁴⁴R⁴⁵, -OC(O)NR⁴⁴R⁴⁵, -NR⁴⁴C(O)R⁴⁵, -NR⁴⁴C(O)NR⁴⁵R⁴⁶, -NR⁴⁴R⁴⁵, -NR⁴⁴CO₂R⁴⁵, -SR⁴⁴, -S(O)R⁴⁴, -S(O)₂R⁴⁴, -S(O)₂NR⁴⁴R⁴⁵, -NR⁴⁴S(O)₂R⁴⁵, substituted or unsubstituted C₆₋₁₀ aryl, substituted or unsubstituted 5- to 10-membered heteroaryl and substituted or unsubstituted 3- to 10-membered heterocyclyl;
R⁴⁴, R⁴⁵ and R⁴⁶ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl;

In one embodiment of formulae (I- C), Z¹ is selected from the group consisting of substituted or unsubstituted 9- or 10-membered heteroaryl with 1 to 2 nitrogen atoms and with 0 or 2 substituents independently selected from the group consisting of substituted or unsubstituted C₂₋₈ alkenyl, substituted or unsubstituted C₂₋₈ alkynyl, -CO₂R^{44a}, -OC(O)NR^{44a}R^{45a}, -NR^{44a}C(O)NR^{45a}R^{46a}, -NR^{44a}CO₂R^{45a} and substituted or unsubstituted C₆₋₁₀ aryl;
R^{44a}, R^{45a} and R^{46a} are each independently selected from the group consisting of hydrogen, substituted or unsubstituted C₁₋₈ alkyl, substituted or unsubstituted 3- to 10-membered heterocyclyl, substituted or unsubstituted C₂₋₈ alkenyl, substituted or unsubstituted C₂₋₈ alkynyl, substituted or unsubstituted C₆₋₁₀aryl, and substituted or unsubstituted 5-to 10-membered heteroaryl;

In one embodiment of formulae (I - C), Z¹ is selected from the group consisting of substituted or unsubstituted pyridyl, substituted or unsubstituted pyrimidinyl, substituted or unsubstituted pyridazinyl, and substituted or unsubstituted pyrazinyl, where the nitrogen atoms may also be -N⁺(O)⁻-.

In one embodiment of formulae (I - C), Z¹ is substituted or unsubstituted 5- to 10-membered heteroaryl, where the heteroaryl group contains only one nitrogen.

In one embodiment of formulae (I - C), Z¹ is substituted or unsubstituted 5- or 6-membered heteroaryl, where the heteroaryl group contains only one nitrogen.

In one embodiment of formulae (I-C), Z¹ is substituted or unsubstituted 9- or 10-membered heteroaryl, where the heteroaryl group contains only one nitrogen.

In one embodiment of formulae (I - C), Z¹ is selected from the group consisting of isothiazolyl, pyrazolyl, imidazolyl, thienyl, furyl, triazolyl, tetrazolyl, oxazolyl, isoxazolyl, oxadiazolyl, thiadiazolyl, pyrrolyl, thiazolyl, where each of these groups may be substituted or unsubstituted.

In one embodiments of formulae (I - C), Z¹ is substituted pyrazolyl with 0 to 3 substituents, substituted imidazolyl with 0 to 3 substituents, substituted tetrazolyl with 0 to 3 substituents, or substituted oxazolyl with 0 to 3 substituents.

In one embodiment of formulae (I - C), Z¹ is selected from the group consisting of pyridyl, pyridazinyl, pyrazinyl, pyrimidinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiazolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, oxadiazolyl, thiadiazolyl, pyrrolyl, thiazolyl, furyl and thienyl, where each of these groups may be substituted or unsubstituted.

In one embodiment of formulae (I - C), Z¹ is selected from the group consisting of pyridyl, pyridazinyl, pyrazinyl, pyrimidinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzoxazolyl, benzisoxazolyl, isobenzofuryl, isoindolyl, indolizinyl, benzotriazinyl, thienopyridinyl, thienopyrimidinyl, pyrazolopyridyl, imidazopyridyl, oxazolopyridyl, isoxaxolopyridyl, thiazolopyridyl, isothiazolopyridyl, benzothiazolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, oxadiazolyl, thiadiazolyl, pyrrolyl, thiazolyl, furyl and thienyl, where each of these groups may be substituted or unsubstituted.

In one embodiment of formulae (I - C), Z¹ is selected from the group consisting of quinolinyl, quinoxalinyl, quinazolinyl, cinnolinyl, phthalazinyl, benzotriazinyl, purinyl, benzimidazolyl, benzopyrazolyl, benzotriazolyl, benzoxazolyl, benzoxazolyl, benzisoxazolyl, isobenzofuryl, isoindolyl, indolizinyl, benzotriazinyl, pyrazolopyridyl, imidazopyridyl, oxazolopyridyl, isoxaxolopyridyl, thiazolopyridyl, isothiazolopyridyl, pyridopyridazinyl, pyridopyrimidinyl, pyridopyrazinyl, benzothiazolyl, benzofuranyl, benzothienyl, indolyl, quinolyl, isoquinolyl, indazolyl, and pteridinyl where each of these groups may be substituted or unsubstituted.

In one embodiment of formulae (I - C), Z¹ is selected from the group consisting of pyrazolopyridyl, imidazopyridyl, oxazolopyridyl, isoxaxolopyridyl, thiazolopyridyl, isothiazolopyridyl, pyridopyridazinyl, pyridopyrimidinyl, and pyridopyrazinyl where each of these groups may be substituted or unsubstituted.

In one embodiment of formulae (I - C), Z¹ is selected from the group consisting of pyrazolopyridyl, isoxaxolopyridyl, and isothiazolopyridyl, where each of these groups may be substituted or unsubstituted.

In one embodiment of formulae (I - C), when Z¹ is substituted Z¹ may be substituted with chlorine, fluorine, unsubstituted or substituted C₁₋₈ alkyl, oxo (=O or -O⁻), -CN, -NO₂, -OMe, -C(O)Me, -CONH₂, -CONHMe, -CONMe₂, - NHC(O)Me, -NH₂, -NHMe, -NMe₂, -SMe, -S(O)Me, -S(O)₂Me, -NHSO₂Me morpholinyl, -CH₂OH, -CH₂OMe,-CH₂NH₂, -CH₂NHMe, or -CH₂NMe₂.

In one embodiment of formulae (I-C), when Z¹ is substituted, Z¹ may be substituted with substituted or unsubstituted (C₁₋₄ alkyl)-(C₆₋₁₀aryl), substituted or unsubstituted (C₁₋₄ alkyl)-(5- to 10-membered heteroaryl).

In one embodiment of formulae (I - C), when Z¹ is substituted Z¹ may be substituted with chlorine, fluorine, methyl, oxo (=O or -O⁻), -CN, -OMe.

In one embodiment of formulae (I - C), Z¹ is substituted or unsubstituted 2-pyridyl.

In one embodiment of formulae (I - C), Z¹ is substituted or unsubstituted 2-pyridyl-N-oxide.

In one embodiment of formulae (I - C), Z¹ is substituted or unsubstituted 3-pyridyl.

In one embodiment of formulae (I - C), Z¹ is substituted or unsubstituted 3-pyridyi-N-oxide.

In one embodiment of formulae (I - C), Z¹ is substituted or unsubstituted 4-pyridyl.

In one embodiment of formulae (I - C), Z¹ is substituted or unsubstituted 4-pyrldyl-N-oxide.

In one embodiment of formulae (I - C), Z¹ is substituted or - unsubstituted pyrazolyl.

In one embodiment of formulae (I - C), Z¹ is substituted or unsubstituted imidazolyl.

In one embodiment of formulae (I - C), Z¹ is substituted or unsubstituted tetrazolyl.

In one embodiment of formulae (I - C), Z¹ is substituted or unsubstituted oxazolyl.

In one embodiment of formulae (I - C), Z¹ is substituted or unsubstituted pyridopyridazinyl.

In one embodiment of formulae (I - C), Z¹ is substituted or unsubstituted phthalazinyl.

In one embodiment of formulae (I - C), Z¹ is substituted or unsubstituted pyrazolopyridyl.

In one embodiment of formulae (I - C), Z¹ is not substituted.

In one embodiment of formula (I - C), Z¹ is substituted by -CH₃ or oxo (=O or -O⁻).

In one embodiment of formula (I - C), Z¹ is substituted by -CH₃ and oxo (=O or -O⁻).

In one embodiment of formulae (I - C), Z¹ is selected from the group consisting of:

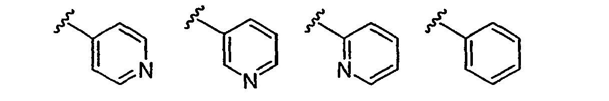

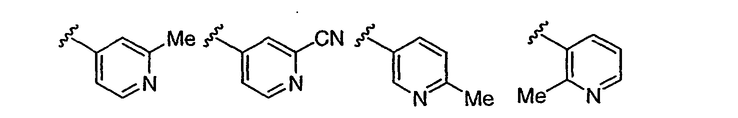

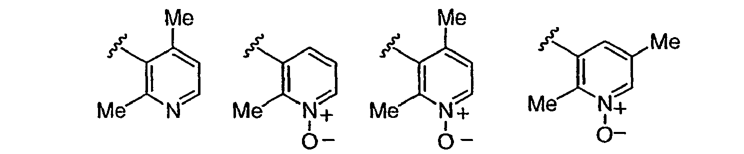

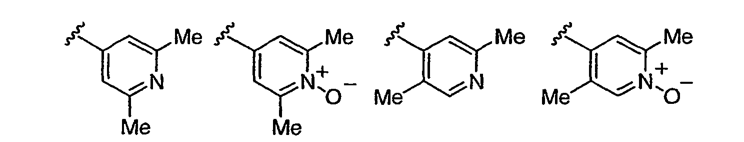

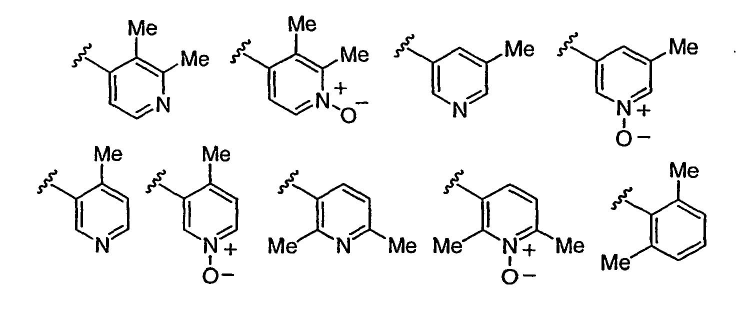

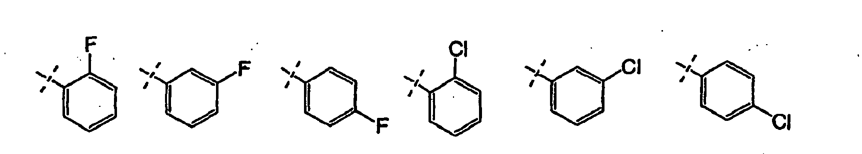

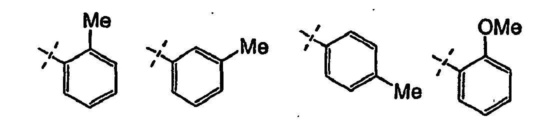

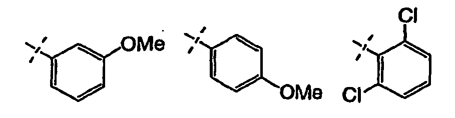

In one embodiment of formulae (I - C), Z¹ is selected from the group consisting of:

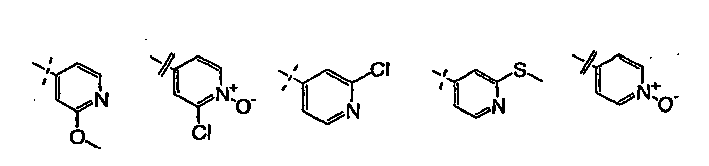

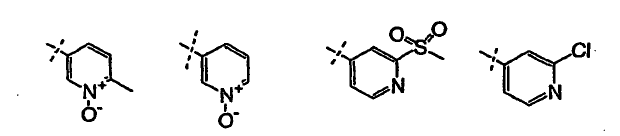

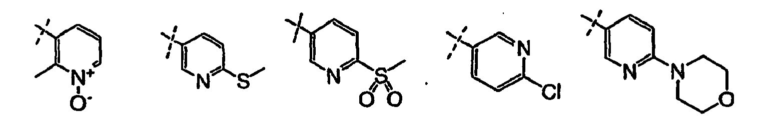

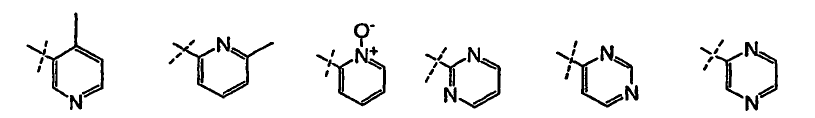

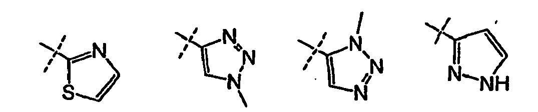

In one embodiment of formulae (I - C), Z¹ is selected from the group consisting of:

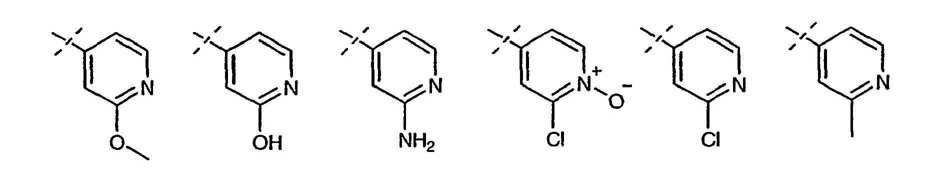

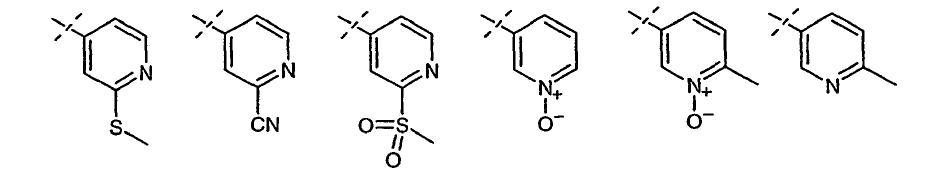

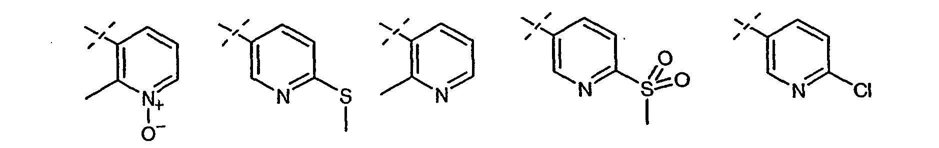

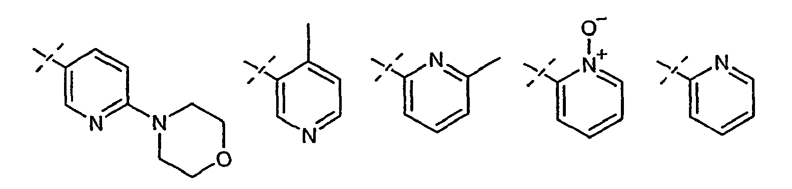

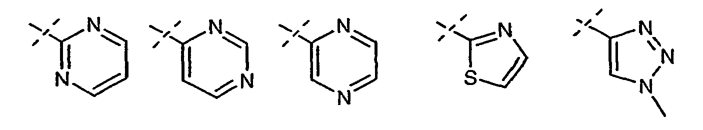

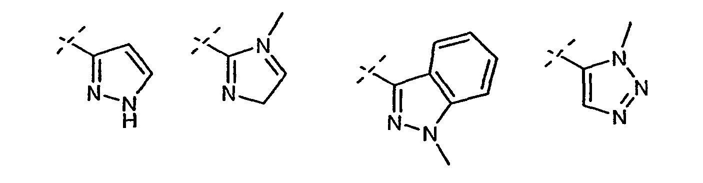

In one embodiment of formulae (I - C), Z¹ is selected from the group consisting of:

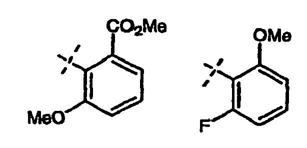

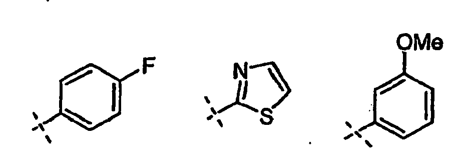

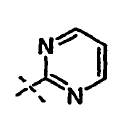

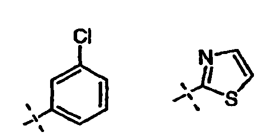

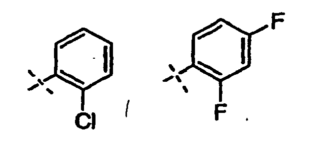

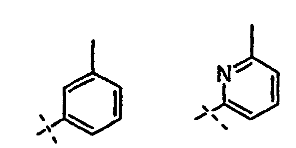

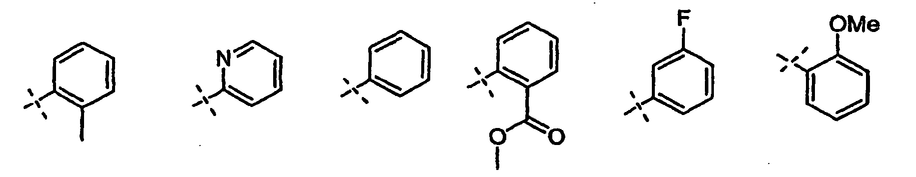

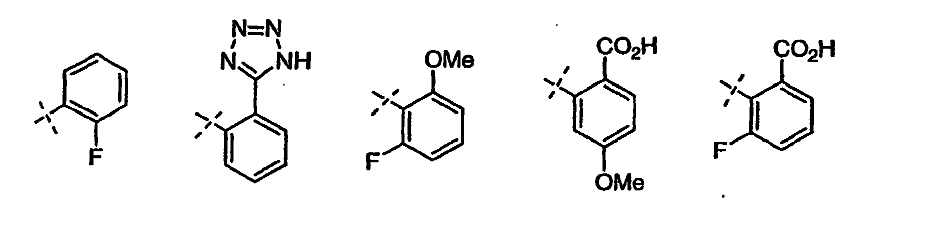

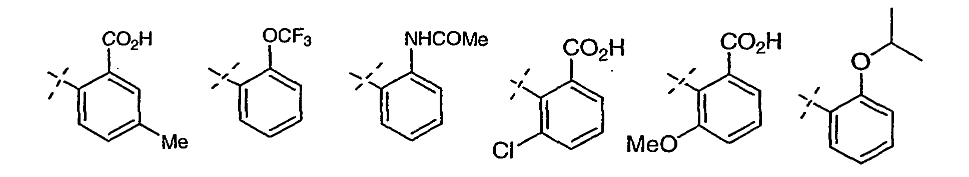

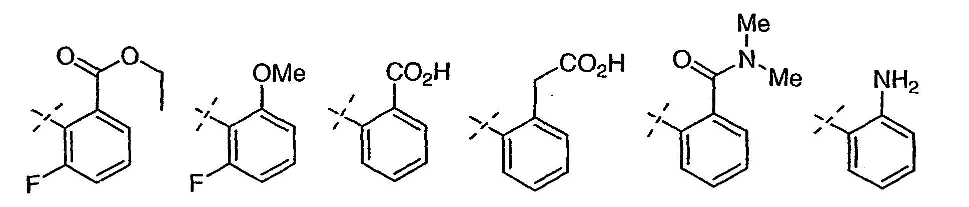

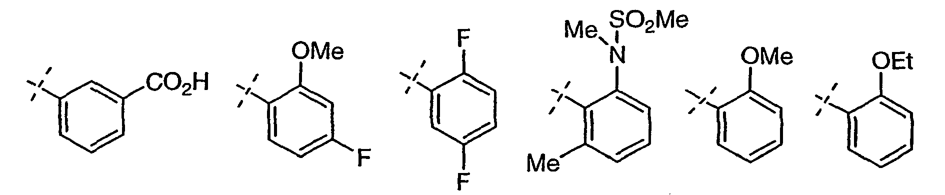

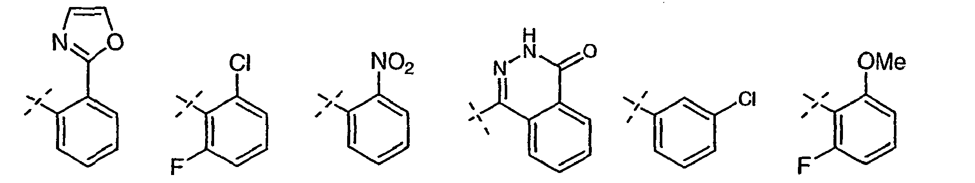

In one embodiment of formulae (I - C), Z¹ may have one or more substituents selected from the group consisting of:

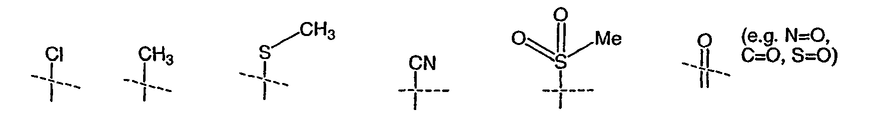

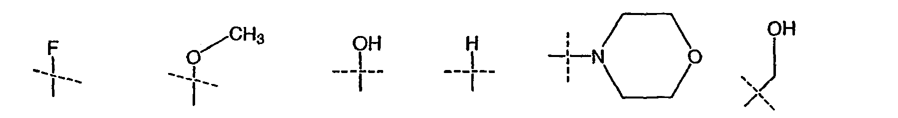

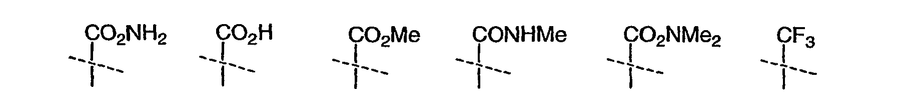

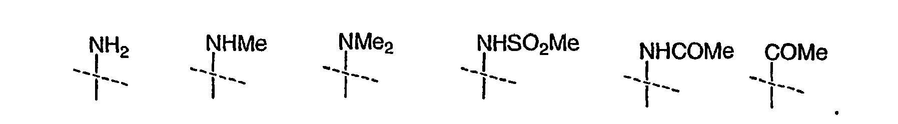

In one embodiment of formula (I - C), Z¹ has 0 to 3 substituents, each of which are independently selected from the group consisting of -CH₃ and oxo (=O or -O-).

In one embodiment of formulae (I- C), Z^{a} and / or Z^{b} is located ortho- to one of the heteroatoms in the ring.

In one embodiment of formulae (I - C), Z^{a} and Z^{b} are each independently selected from the group consisting of halogen, unsubstituted or substituted C₁₋₈ alkyl, oxo (=O or -O⁻), -CN, -NO₂, -OR⁵⁰,-C(O)R⁵⁰, -CONR⁵⁰R⁵¹,-NR⁵⁰C(O)R⁵¹, -NR^{5o}R⁵¹, -SR⁵⁰, -S(O)R⁵⁰, -S(O)₂R⁵⁰, -SO₂NR⁵⁰R⁵¹ -NR⁵⁰SO₂R⁵¹, unsubstituted or substituted 5- to 6-membered heteroaryl, unsubstituted or substituted 4- to 7-membered heterocyclyl, substituted or unsubstituted (C₁₋₄ alkyl)-(C₆₋₁₀ aryl), and substituted or unsubstituted (C₁₋₄ alkyl)-(5- to 10-membered heteroaryl);
R⁵⁰ and R⁵¹ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl.

In one embodiment of formulae (I - C), Z^{a} and Z^{b} are each independently selected from the group consisting of halogen, substituted or unsubstituted C₁₋₈ alkyl, substituted or unsubstituted C₂₋₈ alkenyl, substituted or unsubstituted C₂₋₈ alkynyl, -CN, oxo (=O or -O-), -NO₂, -OR^{50a}, -OC(O)R^{50a}, -CO₂R^{50a}, -C(O)R^{50a}, -C(O)NR^{50a}R^{51a}, -OC(O)NR^{50a}R^{51a}, -NR^{50a}C(O)R^{51a}, -NR^{5oa}C(O)NR^{51a}R^{51b}, -NR^{50a}R^{51a}, -NR^{50a}CO₂R^{51a}, -SR^{5oa}, -S(O)R^{5oa}, -S(O)₂R^{50a}, -S(O)₂NR^{50a}R^{51a}, -NR^{5oa}S(O)₂R^{51a}, substituted or unsubstituted C₆₋₁₀ aryl, substituted or unsubstituted 5- to 10-membered heteroaryl, substituted or unsubstituted 3- to 10-membered heterocyclyl, substituted or unsubstituted (C₁₋₄ alkyl)-(C₆₋₁₀ aryl), and substituted or unsubstituted (C₁₋₄ alkyl)-(5- to 10-membered heteroaryl);
R^{50a}, R^{51a} and R^{51b} are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl.

In one embodiment of formulae (I - C), Z^{a} and Z^{b} are each independently selected from the group consisting of substituted or unsubstituted C₂₋₈alkenyl, substituted or unsubstituted C₂₋₈ alkynyl, -CO₂R^{50a}, -OC(O)NR^{50a}R^{51a}, -NR^{50a}C(O)NR^{51a}R^{51b}, -NR^{50a}CO₂R^{51a}, substituted or unsubstituted C₆₋₁₀ aryl and substituted or unsubstituted 3- to 10-membered heterocyclyl;
R^{50a}, R^{51a} and R^{51b} are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl.

In one embodiment of formulae (I - C), Z^{a} and Z^{b} are each independently selected from the group consisting of substituted C₁₋₈ alkyl, substituted C₂₋₈ alkenyl or substituted C₂₋₈ alkynyl, the substituted C₁₋₈ alkyl, substituted C₂₋₈ alkenyl or substituted C₂₋₈ alkynyl may have 1 to 3 substituents each independently selected from the group consisting of halogen, oxo (=O or - O⁻), -OR⁵², -CO₂R⁵², -C(O)R⁵², -CONR⁵²R⁵³, -NR⁵²C(O)R⁵³, -NR⁵²R⁵³, -SR⁵²,-S(O)R⁵², -S(O)₂R⁵², -SO₂NR⁵²R⁵³, -NR⁵²SO₂R⁵³, unsubstituted or substituted phenyl, unsubstituted or substituted 5- to 6-membered heteroaryl, unsubstituted or substituted 4- to 7-membered heterocyclyl, substituted or unsubstituted (C₁₋₄ alkyl)-(C₆₋₁₀ aryl), and substituted or unsubstituted (C₁₋₄ alkyl)-(5- to 10-membered heteroaryl);
R⁵² and R⁵³ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl.

In one embodiment of formulae (I - C), Z^{a} and Z^{b} are each independently selected from the group consisting of substituted phenyl, substituted 5- or 6-membered heteroaryl or substituted 4- to 7-membered heterocyclyl, the substituted phenyl, substituted 5- or 6-membered heteroaryl and substituted 4- to 7-membered heterocyclyl may have 1 to 3 substituents independently selected from the group consisting of halogen, -OR⁵⁶, -CN, -NO₂, oxo (=O or -O⁻), -OC(O)R⁵⁶, -CO₂R⁵⁶, -C(O)R⁵⁶, -CONR⁵⁶R⁵⁷, -NR⁵⁶C(O)R⁵⁷, -NR⁵⁶R⁵⁷, -SR⁵⁶, -S(O)R⁵⁶, -S(O)₂R⁵⁶, -NR⁵⁶SO₂R⁵⁷, unsubstituted 4- to 7-membered heterocyclyl and unsubstituted C₁₋₈ alkyl, with the proviso that if the substituent on the Z¹ group is heterocyclic, then the substituents on this heterocycle do not include another heterocycle;
R⁵⁶ and R⁵⁷ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl.

In one embodiment of formulae (I - C), Z^{a} and Z^{b} are each independently selected from the group consisting of chlorine, fluorine, unsubstituted or substituted C₁₋₈ alkyl (particularly methyl and trifluoromethyl), oxo (=O or -O⁻), -CN, -NO₂, -OMe, -C(O)Me, -CONH₂, -CONHMe, -CONMe₂, - NHC(O)Me, -NH₂, -NHMe, -NMe₂, -SMe, -S(O)Me, -S(O)₂Me, -NHSO₂Me, morpholinyl, -CH₂OH, -CH₂OMe,-CH₂NH₂, -CH₂NHMe, and -CH₂NMe₂.

In one embodiment of formulae (I - C), Z^{a} and Z^{b} are selected from the group consisting of chlorine, fluorine, methyl, oxo (=O or -O-), -CN, and - OMe.

In one embodiment of formulae (I - C), Z^{a} and Z^{b} are selected from the group consisting of chlorine, fluorine, methyl, isopropyl, oxo (=O or -O-), -CN, and -OMe.

In one embodiment of formulae (I - C), one of Z^{a} and Z^{b} is hydrogen and one of Z^{a} and Z^{b} is selected from the group consisting of chlorine, fluorine, methyl, isopropyl, oxo (=O or -O-), -CN, and -OMe.

In one embodiment of formulae (I - C), Z^{a} and Z^{b} are selected from the group consisting of chlorine, fluorine, methyl, isopropyl, oxo (=O or -O-), -CN, -OMe, -S(O)Me, -SO₂Me, -CO₂H, and -CO₂Me.

In one embodiment of any of formulae (I - C) Z^{a} and Z^{b} are selected from the group consisting of:

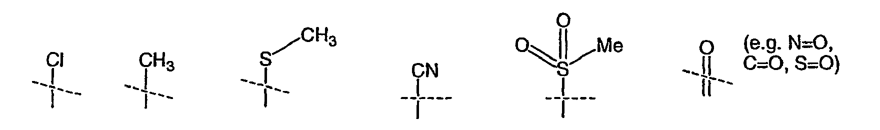

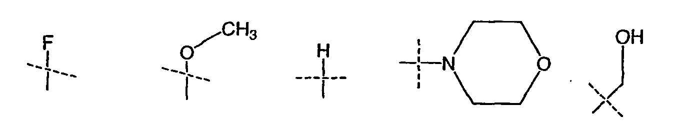

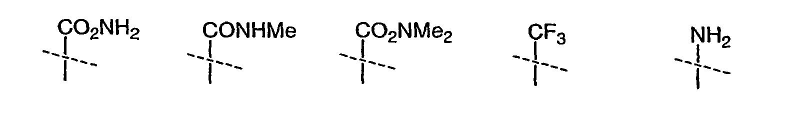

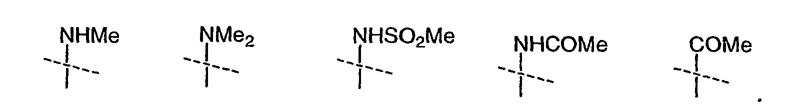

In a further embodiment, the compounds are represented by formula (CLI), or salts thereof: 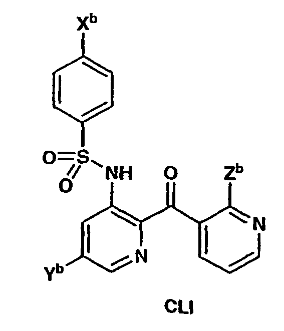 X^{b} is selected from the group consisting of -OR^{24a}, and substituted or unsubstituted C₁₋₈ alkyl; R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10- membered heteroaryl, and substituted or unsubstituted 3- to 10-membered heterocyclyl;
Y^{b} is selected from the group consisting of hydrogen, halogen, -CN, -C(O)R^{27a}, -CO₂R^{27a}, -C(O)NR^{27a}R^{28a}, -OR^{27a}, -OC(O)R^{27a}, -OC(O)NR^{27a}R^{28a}, -SR^{27a}, -S(O)R^{27a}, -S(O)₂R^{27a}, -S(O)₂NR^{27a}R^{28a}, -NO₂, -NR^{27a}R^{28a}, -NR^{27a}C(O)R^{28a}, -NR²⁷aC(O)₂R^{28a}, -NR^{27a}S(O)₂R^{28a}, -NR^{27a}C(O)NR^{28a}R^{29a}, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10- membered heteroaryl; R^{27a}, R^{28a} and R^{29a} are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and R^{27a} and R^{28a}, R²⁸a and R²⁹a or R^{27a} and R^{29a} may, together with the atoms to which they are attached, form a substituted or unsubstituted 5-, 6-, or 7-membered ring;
Z^{b} is selected from the group consisting of substituted or unsubstituted C₁₋₈ alkyl.
In one embodiment if formula (CLI), Y^{b} is halogen.
In one embodiment if formula (CLI), Y^{b} is Cl.
In one embodiment of formula (CLI), X^{b} is selected from the group consisting of -OR^{24a}, and substituted or unsubstituted C₁₋₈ alkyl; and Z^{b} is selected from the group consisting of substituted or unsubstituted C₁₋₈ alkyl.
In a further embodiment, the compounds are represented by formula (CLII), or salts thereof: 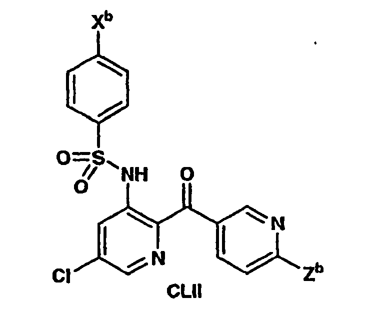 X^{b} is selected from the group consisting of -OR^{24a}, and substituted or unsubstituted C₁₋₈ alkyl; R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl;
Z^{b} is -OR^{30a}; R^{30a} is selected from the group consisting of hydrogen and substituted or unsubstituted C₁₋₈ alkyl.
In one embodiment of formula (CLII), X^{b} is selected from the group consisting of -OR^{24a}, and substituted or unsubstituted C₁₋₈ alkyl; and Z^{b} is -OR^{30a,} where R^{30a} is selected from the group consisting of hydrogen and substituted or unsubstituted C₁₋₈ alkyl.
In a further embodiment, the compounds are represented by formula (CLIII), or salts thereof: 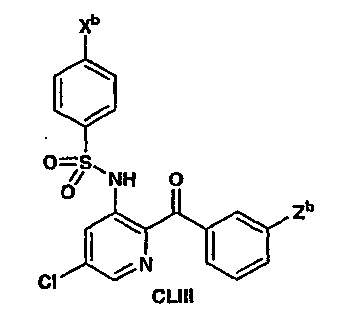 X^{b} is selected from the group consisting of -OR^{24a}, and substituted or unsubstituted C₁₋₈ alkyl; R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl;
Z^{b} is halogen.
In one embodiment of formula (CLIII), X^{b} is selected from the group consisting of -OR^{24a} and substituted or unsubstituted C₁₋₈ alkyl; and Z^{b} is halogen. Preferably Z^{b} is fluorine.
In one embodiment of formulae (CLI-CLIII), X^{b} is -OR^{24a}.
In one embodiment of formulae (CLI-CLIII), X^{b} is OR^{24a}; and R^{24a} is substituted or unsubstituted C₁₋₈ alkyl.
In one embodiment of formulae (CLI-CLIII), X^{b} is -OR^{29a}; and R^{29a} is substituted C₁₋₈ alkyl.
In one embodiment of formulae (CLI-CLIII), x^{b} is isopropoxy.
In one embodiment of formulae (CLI-CLIII), X^{b} is substituted or unsubstituted C₁₋₈ alkyl.
In one embodiment of formulae (CLI-CLIII), X^{b} is substituted C₁₋₈ alkyl or unsubstituted C₂₋₈ alkyl.
In one embodiment of formulae (CLI-CLIII), X^{b} is substituted C₁₋₈ alkyl.
In one embodiment of formulae (CLI-CLIII), X^{b} is unsubstituted C₂₋₈ alkyl.
In one embodiment of formulae (CLI-CLIII), X^{b} is tert-butyl.
In one embodiment of formula (CLI), Z^{b} is unsubstituted C₁₋₈ alkyl.
In one embodiment of formula (CLI), Z^{b} is methyl.
In one embodiment of formula (CLII), Z^{b} is -OR^{30a}, where R^{30a} is unsubstituted C₁₋₈ alkyl.

In one embodiment of formula (CLII), Z^{b} is -OR^{30a}, where R^{30a} is substituted C₁₋₈ alkyl.

In one embodiment of formula (CLII), Z^{b} is hydroxy.

In one embodiment of formula (CLII), Z^{b} is methoxy.

In one embodiment of formula (CLIII), Z^{b} is fluorine.

In one embodiment of formula (CLIII), Z^{b} is chlorine.

In one embodiment of formulae (CLIII), Z^{b} is bromine.

In one embodiment of formula (CLIII), Z^{b} is iodine.

### Compositions that Modulate CCR2

In another aspect, the present invention provides compositions that modulate CCR2 activity. Generally, the compositions for modulating chemokine receptor activity in humans and animals will comprise a pharmaceutically acceptable excipient or diluent and a compound according to the invention

The term "composition" as used herein is intended to encompass a product comprising the specified ingredients in the specified amounts, as well as any product which results, directly or indirectly, from combination of the specified ingredients in the specified amounts. By "pharmaceutically acceptable" it is meant the carrier, diluent or excipient must be compatible with the other ingredients of the formulation and not deleterious to the recipient thereof.

The pharmaceutical compositions for the administration of the compounds of this invention may conveniently be presented in unit dosage form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases.

The pharmaceutical compositions containing the active ingredient may be in a form suitable for oral use, for example, as tablets, troches, lozenges, aqueous or oily suspensions, dispersible powders or granules, emulsions and self emulsifications as described in U.S. Patent Application 20020012680, hard or soft capsules, or syrups or elixirs. Compositions intended for oral use may be prepared according to any method known to the art for the manufacture of pharmaceutical compositions. Such compositions may contain one or more agents selected from sweetening agents, flavoring agents, coloring agents and preserving agents in order to provide pharmaceutically elegant and palatable preparations. Tablets contain the active ingredient in admixture with other nontoxic pharmaceutically acceptable excipients which are suitable for the manufacture of tablets. These excipients may be, for example, inert diluents such as cellulose, silicon dioxide, aluminum oxide, calcium carbonate, sodium carbonate, glucose, mannitol, sorbitol, lactose, calcium phosphate or sodium phosphate; granulating and disintegrating agents, for example, corn starch, or alginic acid; binding agents, for example PVP, cellulose, PEG, starch, gelatin or acacia, and lubricating agents, for example magnesium stearate, stearic acid or talc. The tablets may be uncoated or they may be coated enterically or otherwise by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Pat. Nos. 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil. Additionally, emulsions can be prepared with a non-water miscible ingredient such as oils and stabilized with surfactants such as mono-diglycerides of PEG esters.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil in water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. Oral solutions can be prepared in combination with, for example, cyclodextrin, PEG and surfactants.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non toxic parenterally acceptable diluent or solvent, for example as a solution in 1,3-butane diol. Among the acceptable vehicles and solvents that may be employed are water, Ringer's solution and isotonic sodium chloride solution. In addition, sterile, axed oils are conventionally employed as a solvent or suspending medium. For this purpose any bland fixed oil may be employed including synthetic mono- or diglycerides. In addition, fatty acids such as oleic acid find use in the preparation of injectables.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols. Additionally, the compounds can be administered via ocular delivery by means of solutions or ointments. Still further, transdermal delivery of the subject compounds can be accomplished by means of, for example, iontophoretic patches.
For topical use, creams, ointments, jellies, solutions or suspensions containing the compounds of the present invention are employed. As used herein, topical application is also meant to include the use of mouth washes and gargles.
The pharmaceutical compositions and methods of the present invention may further comprise other therapeutically active compounds as noted herein, such as those applied in the treatment of the above mentioned pathological conditions.
In yet another aspect, the present invention provides a compound according to the invention for use in treating a CCR2-mediated condition or disease.
The "subject" is defined herein to include animals such as mammals, including, but not limited to, for example, primates (e.g., humans), cows, sheep, goats, horses, dogs, cats, rabbits, rats and mice. In preferred embodiments, the subject is a human.
As used herein, the phrase "CCR2-mediated condition or disease" and related phrases and terms refer to a condition or disease characterized by inappropriate, i.e., less than or greater than normal, CCR2 functional activity. Inappropriate CCR2 functional activity might arise as the result of CCR2 expression in cells which normally do not express CCR2, increased CCR2 expression (leading to, e.g., inflammatory and immunoregulatory disorders and diseases) or decreased CCR2 expression. Inappropriate CCR2 functional activity might also arise as the result of MCP-1 secretion by cells which normally do not secrete MCP-1, increased MCP-1 expression (leading to, e.g., inflammatory and immunoregulatory disorders and diseases) or decreased MCP-1 expression. A CCR2-mediated condition or disease may be completely or partially mediated by inappropriate CCR2 functional activity. However, a CCR2-mediated condition or disease is one in which modulation of CCR2 results in some effect on the underlying condition or disease (e.g., a CCR2 antagonist results in some improvement in patient well being in at least some patients). Furthermore, MCP-2, 3 and 4 are also CCR2 ligands.

Depending on the disease to be treated and the subject's condition, the compounds and compositions of the present invention may be administered by oral, parenteral (e.g., intramuscular, intraperitoneal, intravenous, ICV, intracisternal injection or infusion, subcutaneous injection, or implant), inhalation, nasal, vaginal, rectal, sublingual, or topical routes of administration and may be formulated, alone or together, in suitable dosage unit formulations containing conventional non toxic pharmaceutically acceptable carriers, adjuvants and vehicles appropriate for each rouse of administration. The present invention also contemplates administration of the compounds and compositions of the present invention In a depot formulation.

In the treatment or prevention of conditions which require chemokine receptor modulation an appropriate dosage level will generally be 0.001 to 100 mg per kg patient body weight per day which can be administered in single or multiple doses. Preferably, the dosage level will be 0.01 to 25 mg/kg per day; more preferably 0.05 to 10 mg/kg per day. A suitable dosage level may be 0.01 to 25 mg/kg per day, 0.05 to 10 mg/kg per day, or 0.1 to 5 mg/kg per day. Within this range the dosage may be 0.005 to 0.05, 0.05 to 0.5, 0.5 to 5.0, or 5.0 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.
It will be understood, however, that the specific dose level and frequency of dosage for any particular patient may be varied and will depend upon a variety of factors including the activity of the specific compound employed, the metabolic stability and length of action of that compound, the age, body weight, hereditary characteristics, general health, sex, diet, mode and time of administration, rate of excretion, drug combination, the severity of the particular condition, and the host undergoing therapy.
In still other embodiments, the methods are directed to the treatment of allergic diseases, wherein a compound or composition of the invention is administered either alone or in combination with a second therapeutic agent, wherein said second therapeutic agent is an antihistamine. When used in combination, the practitioner can administer a combination of the compound or composition of the present invention and a second therapeutic agent. Also, the compound or composition and the second therapeutic agent can be administered sequentially, in any order.
In one embodiment, the present invention provides a composition consisting of a pharmaceutically acceptable carrier and a compound of the invention.
In one embodiment, the present invention provides a compound according to the invention for use in treating a CCR2-mediated condition or disease.
In one embodiment, the present invention provides a compound according to the invention for use in treating a CCR2-mediated condition or disease, where the CCR2-mediated condition or disease is atherosclerosis.
In one embodiment, the present invention provides a compound according to the invention for use in treating a CCR2-mediated condition or disease, where the CCR2-mediated condition or disease is restenosis.
In one embodiment, the present invention provides a compound according to the invention for use in treating a CCR2-mediated condition or disease, where the CCR2-mediated condition or disease is multiple sclerosis.
In one embodiment, the present invention provides a compound according to the invention for use in treating a CCR2-mediated condition or disease, where the CCR2-mediated condition or disease is selected from the group consisting of inflammatory bowel disease, renal fibrosis, rheumatoid arthritis, obesity and noninsulin-dependent diabetes.
In one embodiment, the present invention provides a compound according to the invention for use in treating a CCR2-mediated condition or disease, where the CCR2-mediated condition or disease is selected from the group consisting of chronic obstructive pulmonary disease, idiopathic pulmonary fibrosis and idiopathic pneumonia syndrome.
In one embodiment, the present invention provides a compound according to the invention for use in treating a CCR2-mediated condition or disease, where the condition or disease is treated by administering the compound by the oral, parenteral, rectal, transdermal, sublingual, nasal or topical route.
In one embodiment, the present invention provides a compound according to the invention for use in treating a CCR2-mediated condition or disease, where the compound is administered in combination with an anti-inflammatory or analgesic agent.
In one embodiment, the present invention provides an in vitro method of modulating CCR2 function in a cell, where the CCR2 function in the cell is modulated by contacting the cell with a CCR2 modulating amount of the compound of the present invention.
In one embodiment, the present invention provides a compound according to the invention for use in treating a CCR2-mediated condition or disease, where the disease is selected from the group consisting of pulmonary fibrosis, transplantation rejection, graft-versus-host disease and cancer.
In yet other embodiments, the methods are directed to the treatment of psoriasis wherein a compound or composition of the invention is used alone or in combination with a second therapeutic agent such as a corticosteroid, a lubricant, a keratolytic agent, a vitamin D₃ derivative, PUVA and anthralin.
In other embodiments, the methods are directed to the treatment of atopic dermatitis using a compound or composition of the invention either alone or in combination with a second therapeutic agent such as a lubricant and a corticosteroid.
In further embodiments, the methods are directed to the treatment of asthma using a compound or composition of the invention either alone or in combination with a second therapeutic agent such as a β2-agonist and a corticosteroid.
The compounds and compositions of the present invention can be combined with other compounds and compositions having related utilities to prevent and treat the condition or disease of interest, such as inflammatory conditions and diseases, including inflammatory bowel disease, allergic diseases, psoriasis, atopic dermatitis and asthma, and those pathologies noted above. Selection of the appropriate agents for use in combination therapies can be made by one of ordinary skill in the art. The combination of therapeutic agents may act synergistically to effect the treatment or prevention of the various disorders. Using this approach, one may be able to achieve therapeutic efficacy with lower dosages of each agent, thus reducing the potential for adverse side effects.

The weight ratio of the compound of the present invention to the second active ingredient may be varied and will depend upon the effective dose of each ingredient. Generally, an effective dose of each will be used. Thus, for example, when a compound of the present invention is combined with an NSAID the weight ratio of the compound of the present invention to the NSAID will generally range from 1000:1 to 1:1000, preferably 200:1 to
1:200. Combinations of a compound of the present invention and other active ingredients will generally also be within the aforementioned range, but in each case, an effective dose of each active ingredient should be used.

### Preparation of CCR 9 modulators

The following examples are offered to illustrate, but not to limit, the claimed invention.

Additionally, those skilled in the art will recognize that the molecules claimed in this patent may be synthesized using a variety of standard organic chemistry transformations.

Certain general reaction types employed widely to synthesize target compounds in this invention are summarized in the examples. Specifically, generic procedures for sulfonamide formation, pyridine N-oxide formation and 2-aminophenyl-arylmethanone synthesis via Friedel-Crafts type approaches are given, but numerous other standard chemistries are described within and were employed routinely.

While not intended to be exhaustive, representative synthetic organic transformations which can be used to prepare compounds of the invention are included below.

These representative transformations include, for example, standard functional group manipulations; reductions such as nitro to amino; oxidations of functional groups including alcohols and pyridines; aryl substitutions via *IPSO* or other mechanisms for the introduction of a variety of groups including nitrile, methyl and halogen; protecting group introductions and removals; Grignard formation and reaction with an electrophile; metal-mediated cross couplings including but not limited to Buckwald, Suzuki and Sonigashira reactions; halogenations and other electrophilic aromatic substitution reactions; diazonium salt formations and reactions of these species; etherifications; cyclative condensations, dehydrations, oxidations and reductions leading to heteroaryl groups; aryl metallations and transmetallations and reaction of the ensuing aryl-metal species with an electrophile such as an acid chloride or Weinreb amide; amidations; esterifications; nuclephilic substitution reactions; alkylations; acylations; sulfonamide formation; chlorosulfonylations; and ester and related hydrolyses.

Certain molecules claimed in this patent can exist in different enantiomeric and diastereomeric forms and all such variants of these compounds are within the scope of the invention.

In the descriptions of the syntheses that follow, some precursors were obtained from commercial sources. These commercial sources include Aldrich Chemical Co., Acros Organics, Ryan Scientific Incorporated, Oakwood Products Incorporated, Lancaster Chemicals, Sigma Chemical Co., Lancaster Chemical Co., TCI-America, Alfa Aesar, Davos Chemicals, and GFS Chemicals.

Compounds of the invention, including those listed in the table of activities, can be made by the methods and approaches described in the following experimental section, and by the use of standard organic chemistry transformations that are well known to those skilled in the art.

### Examples

Reagents and solvents used below can be obtained from commercial sources such as Aldrich Chemical Co. (Milwaukee, Wisconsin, USA). ¹H-NMR were recorded on a Varian Mercury 400 MHz NMR spectrometer. Significant peaks are tabulated in the order: multiplicity (br, broad; s, singlet; d, doublet; t, triplet; q, quartet; m, multiplet) and number of protons. Mass spectrometry results are reported as the ratio of mass over charge, followed by the relative abundance of each ion (in parenthesis). In tables, a single m/e value is reported for the M+H (or, as noted, M-H) ion containing the most common atomic isotopes. Isotope patterns correspond to the expected formula in all cases. Electrospray ionization (ESI) mass spectrometry analysis was conducted on a Hewlett-Packard MSD electrospray mass spectrometer using the HP1100 HPLC for sample delivery. Normally the analyte was dissolved in methanol at 0.1 mg/mL and 1 microliter was infused with the delivery solvent into the mass spectrometer, which scanned from 100 to 1500 daltons. All compounds could be analyzed in the positive ESI mode, using acetonitrile / water with 1 % formic acid as the delivery solvent. The compounds provided below could also be analyzed in the negative ESI mode, using 2mM NH₄OAc in acetonitrile / water as delivery system.

This compound was prepared according to the following literature procedure: Zhou et al.; Bioorganic & Med. Chem., 9, 2061-2071 (2001).

### Example 1: 2-Bromo-5-chloro-3-nitro-pyridine

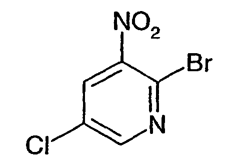

This compound was prepared according to the following literature procedure: Zhou et al.; Bioorganic & Med. Chem., 9, 2061-2071 (2001).

### Example 2: 2-Bromo-5-chloro-pyridin-3-ylamine

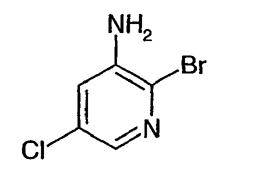

2-Bromo-5-chloro-3-nitro-pyridine (11.87 g, 50 mmol) was dissolved in 100 mL ether. Tin(II) chloride dihydrate (56.4g, 0.5 mol) was dissolved in 100 mL of concentrated hydrochloric acid and added drop wise over 15 minutes to the stirring ethereal solution of the nitro compound. The exothermic reaction brought the ether to boiling and it was allowed to evaporate off. After the addition was complete the reaction mixture was placed on a 50 °C oil bath and stirred for 30 minutes to boil of the remaining ether. The flask was then cooled on in an ice bath. The precipitate formed was collected and by filtration and dissolved in 100 mL of water. The pH was adjusted to 9-10 by the addition of concentrated ammonium hydroxide solution and the product was extracted with ethyl acetate (2 x 100 mL). The organic layer was washed with diluted ammonium hydroxide, water and brine and dried over Na₂SO₄ and the solvent was evaporated to afford 7.4 g of tan crystalline solid. MS m/z : 208.9 (M+H).

### Example 3: General procedure A: Synthesis of 5-chloro-3-nitro-2-aryloxypyridines and 5-chloro-3-nitro-2-arylsulfanyl-pyridines

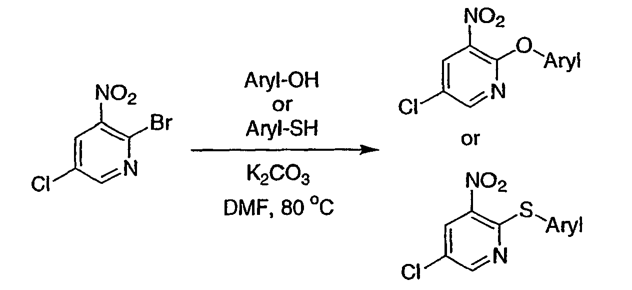

A mixture of the appropriate hydroxyaryl or thioaryl (1.3 equiv), 2-bromo-5-chloro-3-nitro-pyridine (1 equiv) and K₂CO₃ (1.5 equiv) in DMF was heated at 80°C overnight. The resulting mixture was cooled to room temperature, and diluted with water and CH₂Cl₂. The biphasic mixture was separated and the aqueous portion was extracted with CH₂Cl₂. The combined extracts were washed with saturated aqueous NaHCO₃, brine and dried (Na₂SO₄). It was then filtered and filtrate was concentrated under reduced pressure and the product was purified by flash column chromatography on silica gel to provide desired product.

### Example 4: 5-Chloro-3-nitro-2-phenoxy-pyridine

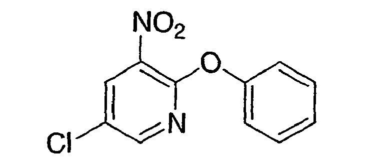

This compound was prepared according to the general procedure described above using 2-bromo-5-chloro-3-nitro-pyridine (500 mg, 2.11 mmol), phenol (258 mg, 2.75 mmol), K₂CO₃ (437 mg, 3.16 mmol) and DMF (2 mL). MS m/z : 250.4 (M+H).

### Example 5: 5-Chloro-3-nitro-2-(pyridin-4-ylsulfanyl)-pyridine

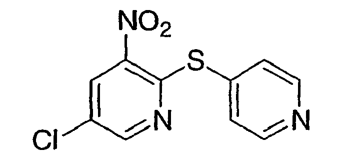

The title compound was prepared according to the above procedure using 2-bromo-5-chloro-3-nitro-pyridine (500 mg, 2.11 mmol), 4-mercaptopyridinel (306 mg, 2.75 mmol), K₂CO₃ (437 mg, 3.16 mmol) and DMF (2 mL). MS m/z: 267.5 (M+H).

### Example 6: 5-Chloro-2-(2-fluoro-phenoxy)-3-nitro-pyridine

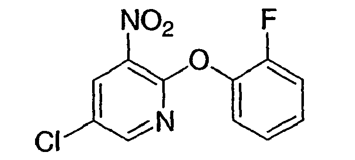

This compound was prepared according to the above procedure using 2-bromo-5-chloro-3-nitro-pyridine (750 mg, 3.17 mmol), 2-fluorophenol (463 mmol, 4.13 mmol), K₂CO₃ (437 mg, 4.75 mmol) and DMF (5 mL). MS m/z : 269.0 (M+H).

### Example 7: 5-Chloro-2-(4-fluoro-phenoxy)-3-nitro-pyridine

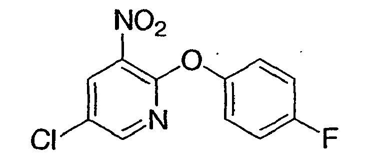

The title compound was prepared according to the above procedure using 2-bromo-5-chloro-3-nitro-pyridine (750 mg, 3.17 mmol), 2-fluorophenol (463 mmol, 4.13 mmol), K₂CO₃ (437 mg, 4.75 mmol) and DMF (5 mL). MS m/z : 269.0 (M+H).

### Example 8: 5-Chloro-3-nitro-pyridine-2-carboxylic acid

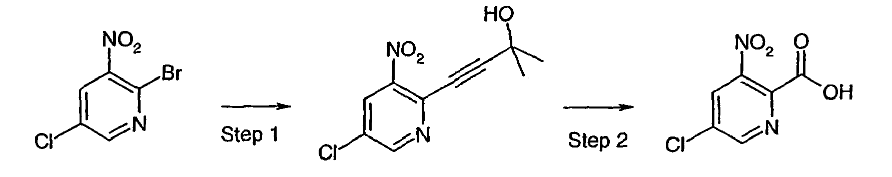

Step 1: To a mixture of 2-bromo-5-chloro-3-nitro-pyridine (2.5 g, 10.6 mmol), Cul (141 mg, 0.74 mmol), Pd(PPh₃)₄, (367 mg, 0.32 mmol) and 2-Methyl-3-butyn-2-ol (1.5 mL, 15.8 mmol) was added 1-methyl-2-pyrorolidone (20 mL) followed by Et₃N (6 mL, 22 mmol). After stirring for 4 h 30 min at room temperature, the reaction mixture was poured into water (20 mL) and extracted with EtOAc (3 x 30 mL). The combined organic extracts were washed with brine (20 mL), dried (Na₂SO₄), filtered and concentrated under reduced pressure, and the residue was purified by flash column chromatography over silica gel (EtOAc/Hexanes, 2:98, then 5:95) to provide 4-(5-chloro-3-nitro-pyridin-2-yl)-2-methyl-but-3-yn-2-ol. MS m/z : 241.0 (M+H).

Step 2: A suspension of the alkyne from step 1 (1.96 g, 8.2 mmol) in water (20 mL) was heated at 75-80 °C. KMnO₄ (4.27 g, 27.1 mmol) was added portion wise over 30 min. After complete addition, heating was continued for 45 min. The reaction mixture was then cooled to room temperature and the pH adjusted to 9 by the addition of 1.0 M aqueous NaOH and filtered through filter paper. The filter cake was thoroughly washed with 0.3 M aqueous NaOH. The filtrate was extracted with EtOAc and discarded. The aqueous part was acidified to pH 3-4 with 1 M HCl and saturated with solid NaCl. This solution was extracted with EtOAc (4 x 30 mL). The combined extract was dried (Na₂SO₄) and concentrated under reduced pressure to provide the crude acid (500 mg). MS m/z: 200.8 (M-H).

### Example 9: (5-Chloro-3-nitro-pyridin-2-yl)-phenyl-methanone

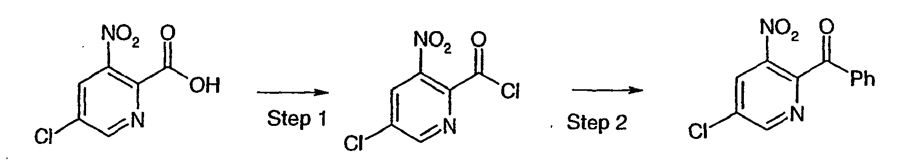

Step 1: To a stirred solution of the carboxylic acid (250 mg, 1.3 mmol) in CH₂Cl₂, at room temperature, was added oxalyl chloride (216 µL, 2.5 mmol) followed by a drop of DMF. After stirring for 1-2 h, the reaction mixture the solvent was removed under reduced pressure to provide the chloride which was used in the next step without further purification.

Step 2: The above acid chloride was dissolved in benzene (30 mL) and AlCl₃ (246 mg, 1.85 mmol) was added. The resulting mixture was heated at 80 °C for 3.5 h. The reaction mixture was quenched with aqueous NaHCO₃. The organic phases then separated and the aqueous portion was extracted with EtOAc and the combined organic extracts washed with brine, dried (Na₂SO₄) and filtered. The filtrate was concentrated under reduced pressure and residue was purified by flash chromatography to on silica gel to provide the title compound. Mass spectrum m/z : 262.5 (M+H).

### Example 10: (3-Amino-5-chloro-pyridin-2-yl)-phenyl-methanone

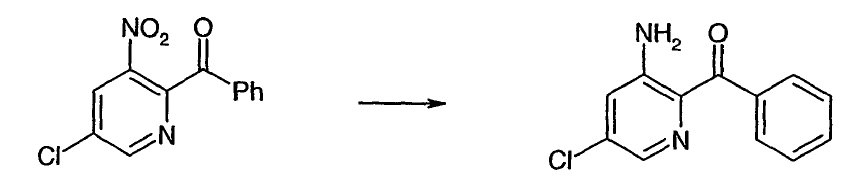

A mixture of (5-chloro-3-nitro-pyridin-2-yl)-phenyl-methanone (159 mg, 0.61) and SnCl₂ (771 mg, 3.42mmol) in EtOH (10 mL) was heated at 80 °C in an oil bath for several days. The progress of the reaction was followed by LCMS and upon completion the solvent was removed and treated with aqueous NaOH to adjust the pH 10-11. This cloudy mixture was extracted with EtOAc and washed with water and brine. The combined extracts were dried over anhydrous Na₂SO₄, filtered and concentrated under reduced pressure and the residue was purified by flash column chromatography to provide the aniline. Mass spectrum m/z: 232.6 (M+H).

### Example 11: General Procedure B: Reduction of nitropyridines

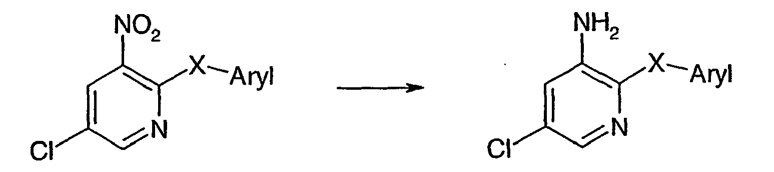

A mixture of the nitropyridine (1 equiv) and SnCl₂ (3-5 equiv) in EtOH was heated at 80 °C in an oil bath for several hour. The progress of the reaction was followed by LCMS and upon completion the solvent was removed and treated with aqueous NaOH. This cloudy mixture was extracted with EtOAc and washed with water and brine. The combined extracts were dried over anhydrous Na₂SO₄, filtered and concentrated under reduced pressure and the residue was purified by flash column chromatography to provide the appropriate aminopyridine.

### Example 12: 5-Chloro-2-phenoxypyridin-3-ylamine

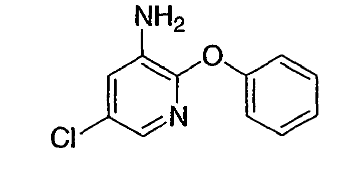

This compound was prepared according to general procedure B using the corresponding nitropyridine (500 mg, 2.11 mmol), SnCl₂ (1.77 mmol, 5.4 mmol), and EtOH (10 mL). MS m/z : 220.5 (M+H).

### Example 13: 5-Chloro-2-(2-fluoro-phenoxy)-pyridin-3-ylamine

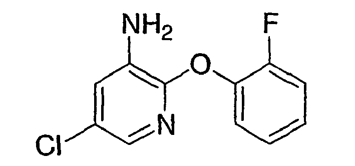

The title compound was prepared according to general procedure B using the appropriate nitropyridine (450 mg, 1.67 mmol), SnCl₂ (1.50 mmol, 6.68 mmol), and EtOH (10 mL). MS m/z : 239.0 (M+H).

### Example 14: 5-Chloro-2-(4-fluoro-phenoxy)-pyridin-3-ylamine

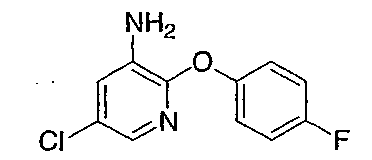

The title compound was prepared according to general procedure B using the corresponding nitro compound (450 mg, 1.67 mmol), SnCl₂ (1.50 mmol, 6.68 mmol) and EtOH (10 mL). Mass spectrum m/z : 239.0 (M+H).

### Example 15: 5-Chloro-2-(pyridin-4-ylsulfanyl)-pyridin-3-ylamine

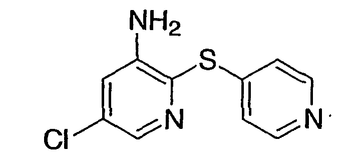

The title compound was prepared according to general procedure B using the corresponding nitro compound (296 mg, 1.11 mmol), SnCl₂ (1.00 gm mmol, 4.40 mmol), EtOH (10 mL) Reaction mixture was extracted with a mixture of EtOAc and THF (1:1). MS m/z : 237.3 (M+H).

### Example 16: (3-Amino-5-chloro-pyridin-2-yl)-pyridin-3-yl-methanone

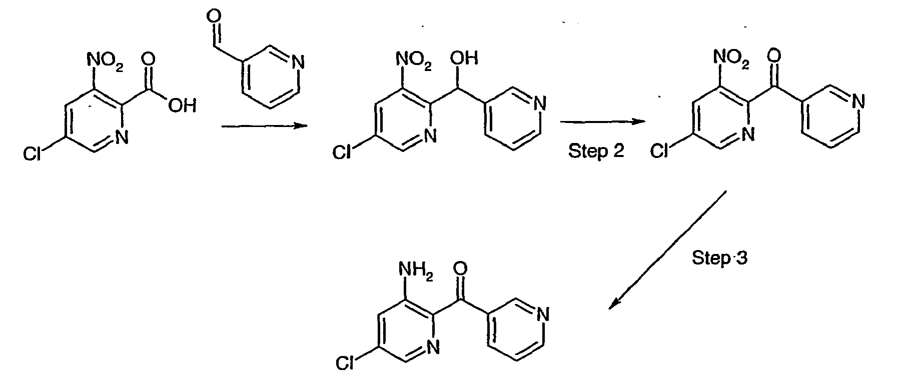

Step 1: A mixture of pyridine-3-carboxaldehyde (0.5 mL) and 5-Chloro-3-nitro-pyridine-2-carboxylic acid (200 mg) was heated at 190 °C for 4 minutes in a sealed tube using a microwave apparatus. The reaction mixture was diluted with CH₂Cl₂ and concentrated under reduced pressure. This residue was washed with aqueous NaHCO₃ and extracted with EtOAc. The combined organic extracts washed with brine, dried (Na₂SO₄) and filtered. The residue which contained the desired alcohol was utilized in the following oxidation without further purification. MS m/z: 266.0 (M+H).

Step 2: A mixture of the alcohol obtained from the above reaction and PCC (426 mg) was stirred at room temperature for 2 h. It was then treated with small amount of silica gel and filtered. The filtrate was concentrated under reduced pressure and the residue was purified by flash column chromatography on silica gel to provide the ketone. MS m/z : 264.0 (M+H).

Step 3: A mixture of the (5-Chloro-3-nitro-pyridin-2-yl)-pyridin-3-yl-methanone from Step 2 above (30 mg, 0.11 mmol) and SnCl₂ (200 mg, 0.88 mmol) in EtOH (3 mL) was heated at 80 °C in an oil bath for 3 days. The progress of the reaction was followed by LCMS and upon completion the solvent was removed and treated with aqueous NaOH to adjust the pH to 10-11. This cloudy mixture was extracted with EtOAc and washed with water and brine. The combined extracts were dried over anhydrous Na₂SO₄, filtered and concentrated under reduced pressure and the residue separated by preparative HPLC (20 → 80% gradient of ACN-water) and pure product fractions were treated with NaHCO₃ and extracted with EtOAc to provide (3-amino-5-chloro-pyridin-2-yl)-pyridin-3-yl-methanone. MS m/z: 234.1 (M+H).

### Comparative Example 1: 4-Chloro-N-[5-chloro-2-(pyridin-4-ylsulfanyl)-pyridin-3-yl]-3-trifluoromethyl-benzenesulfonamide

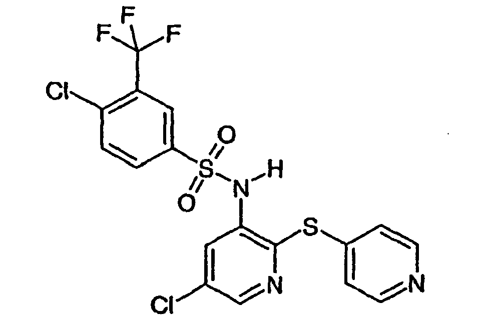

To a solution of 5-chloro-2-(pyridin-4-ylsulfanyl)-pyridin-3-ylamine (100 mg, 0.42 mmol) in anhydrous pyridine (0.5 mL) was added drop wise a solution of 4-chloro-3-trifluoromethyt-benzenesulfonyl chloride (117 mg, 0.42 mmol) in pyridine (0.25 mL). The resulting mixture was stirred at room temperature for 2 days. The reaction mixture was concentrated under reduced pressure and diluted with EtOAc and brine. The aqueous portion was separated and extracted with EtOAc. The combined extracts were dried (Na₂SO₄) filtered and concentrated under reduced pressure. The residue was treated with 0.3 M aqueous NaOH and washed with CH₂Cl₂. The aqueous portion was acidified with cone HCl to pH 3 and extracted with EtOAc. The combined extracts were dried (Na₂SO₄) filtered and concentrated under reduced pressure. The residue was separated by preparative HPLC (20 → 80% gradient of ACN-water) and pure product fractions were lyophilized to provide pure product as a solid. Mass spectrum m/z: 480.0 (M+H).

### Example 17: N-(2-Benzoyl-5-chloro-pyridin-3yl)-4-chloro-3-trifluoromethylbenzene sulfonamide

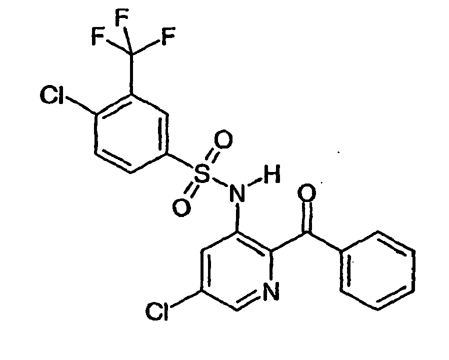

A solution of (3-amino-5-chloro-pyridin-2-yl)-phenyl-methanone (40 mg, 0.17 mmol), 4-chloro-3-trifluoromethyl-benzenesulfonyl chloride (72 mg, 0.26 mmol), and DMAP (11 mg, 0.09 mmol) in anhydrous pyridine (0.5 mL) was heated at 60 °C for 4 days. The product was separated by preparative HPLC and pure product fractions were lyophilized to provide pure product as a solid. ¹H NMR (400 MHz, CDCl₃) δ10.56 (s, 1 H), 8.39 (s, 1 H), 8.17 (s, 1 H), 8.07 (s, 1 H), 7.87 (d, J = 8.0 Hz, 1 H), 7.54 (d, J = 7.6 Hz, 2 H), 7.61-7.58 (m, 1 H), 7.50 (d, J = 8.4 Hz, 1 H), 7.45-7.42 (m, 2 H); MS m/z: 475.0 (M+H).

### Example 18: 4-Chloro-N[5-chloro-2-(pyridine-3-carbonyl)-pyridin-3-yl]-3-trifluoromethyl-benzenesulfonamide

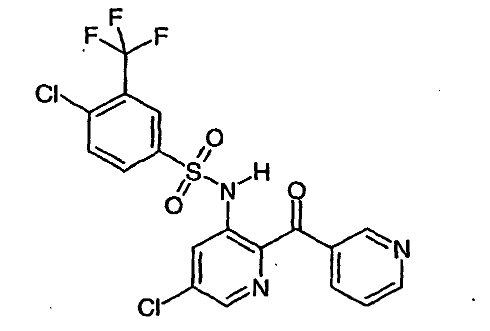

A solution of (3-amino-5-chloro-pyridin-2-yl)-pyridin-3-yl-methanone (26 mg, 0.11 mmol), 4-chloro-3-trifluoromethyl-benzenesulfonyl chloride (28 mg, 0.10 mmol), DMAP (7.3 mg, 0.06 mmol) in anhydrous pyridine (0.5 mL) was heated at 60 °C for 2 days. The reaction mixture was separated by preparative HPLC and pure product fractions were lyophilized to provide pure product as a solid. ¹H NMR (400 MHz, CDCl₃) δ 11.03 (s, 1 H), 9.15 (s, 1 H), 8.81-8.80 (m, 1 H), 8.37 (d, J = 2.0 Hz, 1 H), 8.29-8.26 (m, 1 H), 8.20-8.18 (m, 2 H), 7.98 (dd, J = 8.4,2.8 Hz, 1 H), 7.63 (d, J = 8.4 Hz, 1 H), 7.51-7.48 (m, 1 H). Mass spectrum m/z: 476.0 (M+H).

### Example 19: N-(2-Bromo-5-chloro-pyridin-3-yl)-4-chloro-3-trifluoromethyl-benzenesulfonamide

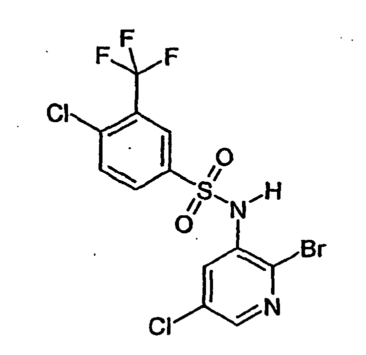

A mixture of 4-chloro-3-trifluaromethyl-benzenesulfonyl chloride (1.5 g, 5.3 mmol) and 2-bromo-5-chloro-pyridin-3-ylamine (500 mg, 2.4 mmol) dissolved in pyridine (20 mL) was heated at 60 °C for 16 h. The solvent was evaporated and the residue suspended in a 1:1 mixture of 2 M NaOH and methanol (20 mL) and heated at 70 °C for 30 min. The methanol was evaporated under reduced pressure and the residue diluted with 15 mL water. The solution was cooled on ice bath and the pH was adjusted to 3 by drop wise addition of concentrated HCl. The solid formed was collected by filtration and the product was purified by flash chromatography on silica gel column to afford the desired product. MS m/z : 450.8 (M+H).

### Example 20: N-(2-Bromo-5-chloro-pyridin-3-yl)-4-chloro-N-methoxymethyl-3-trifluoromethyl-benzenesulfonamide

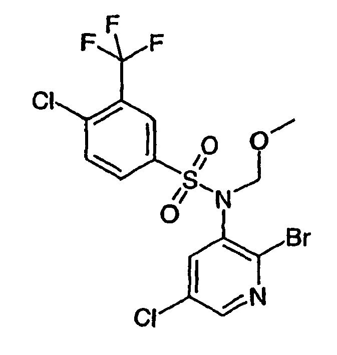

To a stirred solution of N-(2-bromo-5-chloro-pyridin-3-yl)-4-chloro-3-trifluoromethyl-benzenesulfonamide (100 mg, 0.22 mmol) and K₂CO₃ (91 mg, 0.66 mmol) in THF (5 mL) was added chloromethyl methyl ether (0.054 mL, 0.66 mmol) at room temperature. The resulting mixture was stirred for 3 h at room temperature then filtered. The filter cake was washed with THF and the combined filtrate was concentrated under reduced pressure. The resulting residue was purified by flash column chromatography to provide desired product. Mass spectrum m/z: 494.9 (M+H).

### Example 21: 4-Chloro-N-[5-chloro-2-(hydroxy-pyridin-4-yl-methyl)-pyridin-3-yl]-N-methoxymethyl-3-trifluoromethyl-benzenesulfonamide

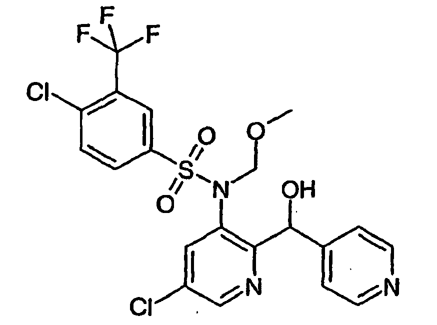

To a stirred solution N-(2-bromo-5-chloro-pyridin-3-yl)-4-chloro-N-methoxymethyl-3-trifluoromethyl-benzenesulfonamide (105 mg, 0.21 mmol) in anhydrous THF (3 mL) was added 2 M isopropylmagnesiumbromide (0.15 mL, 0.3 mmol) at -60 °C. The solution was then slowly warmed to room temperature over 1 h and stirred at room temperature for 20 minutes. At this point the reation mixture turns brownish and additional 0.05 mL of isopropylmagnesiumbromide solution was added and stirring continued for 10 minutes. It was then cooled to - 40 °C and pyridine-4-carboxaldehyde (0.05 mL, 0.52 mmol) was added. After 1.5 h the reaction mixture was quenched with brine and extracted with Et₂O. The combined extract was dried (Na₂SO₄), filtered and concentrated under reduced pressure. The residue was purified by flash column chromatography on silica gel to provide alcohol. Mass spectrum m/z : 522.0 (M+H).

### Example 22: 4-Chloro-N-[5-chloro-2-(hydroxy-pyridin-4-yl-methyl)-pyridin-3-yl]-3-trifluoromethyl-benzonesulfonamide

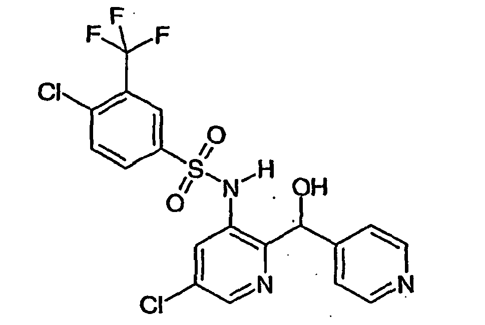

4-Chloro-N-[5-chloro-2-(hydroxy-pyridin-4-yl-methyl)-pyridin-3-yl]-N-methoxymethyl-3-trifluoromethyl-benzenesulfonamide (30 mg, 0.06 mmol) in 50% aqueous of MeOH (10 mL) was treated with 5-10 drops of conc. HCl and heated at 80 °C for 3 days. The resulting mixture was cooled to room temperature, concentrated under reduced pressure and neutralized with aqueous NaHCO₃ to pH 7. It was then extracted with EtOAc to provide crude 4-chloro-N-[5-chloro-2-(hydroxy-pyridin-4-yl-methyl)-pyridin-3-yl]-3-trifluoromethyl-benzenesulfonamide which was purified by preparative HPLC. ¹H NMR (400 MHz, CDCl₃) δ 8.34 (br, 2 H), 8.29 (d, *J*= 2.0 Hz, 1 H), 7.97 (s, 1 H), 7.91 (s, 1 H), 7.44 (d, *J* = 8.4 Hz, 1 H), 7.38 (d, *J* = 8.4 Hz, 1 H), 7.11 (d, *J* = 5.6 Hz, 2 H), 5.98 (s, 1 H); MS m/z : 478.0 (M+H).

### Example 23: 4-Chloro-N-[5-chloro-2-(pyridine-4-carbonyl)-pyridin-3-yl]-3-trifluoromethyl-benzenesulfonamide

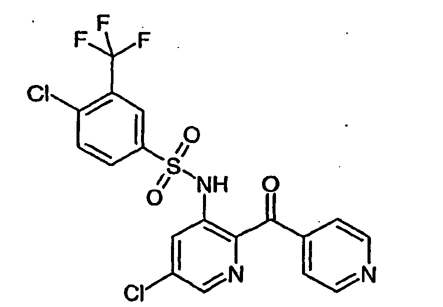

A mixture of 4-chloro-N-[5-chloro-2-(hydroxy-pyridin-4-yl-methyl)-pyridin-3-yl]-3-trifluoromethyl-benzenesulfonamide (15 mg, 0.03 mmol) and PCC (15 mg) was stirred at room temperature for 2 h. It was then treated with small amount of silica gel and filtered. The filtrate was concentrated under reduced pressure to provide the crude ketone. ¹H NMR (400 MHz, CDCl₃) δ 10.90 (br s, 1 H), 8.78 (dd, *J* = 4.4, 1.6 Hz, 2 H), 8.35 (d, *J* = 2.0 Hz, 1 H), 8.19-8.17 (m, 2 H), 7.98 (dd, *J*= 8.4, 2.0 Hz, 1 H), 7.65-7.61 (m, 3 H). MS m/z : 476.0 (M+H).

### Example 24: 4-Chloro-N-[5-chloro-2-(6-methyl-pyridine-3-carbonyl)-pyridin-3-yl]-3-trifluoromethyl-benzenesultonamide

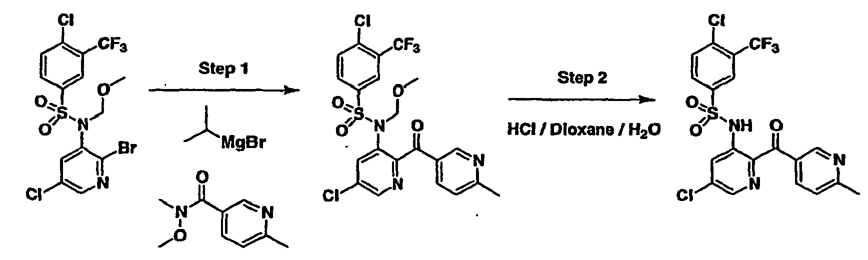

Step 1: To a solution of N-(2-bromo-5-chloro-pyridin-3-yl)-4-chloro-N-methoxymethyl-3-trifluoromethyl-benzenesulfonamide (90 mg, 0.2 mmol) in 2.0 mL of THF under nitrogen atmosphere at -78°C was added drop-wise 0.3 mL (1.5 mmol) of isopropylmagnesium chloride. The mixture was then stirred for 10 min at -78 °C followed by warming to room temperature and stirred at that temperature for 30 min. The mixture was cooled to 0 °C (ice water) and a solution of N-methoxy-6,N-dimethyl-nicotinamide (72mg, 0.4 mmol) in 1 mL of THF was added. The mixture was stirred at room temperature for 3 hours, quenched with 1 M HCl and neutralized with 1 M NaOH until pH was 9. The mixture was extracted with ethyl acetate, dried and concentrated. The residue was purified by flash column (silica gel, 50% ethyl acetate in hexane) to afford 36 mg of 4-chloro-N-[5-chloro-2-(6-methyl-pyridine-3-carbonyl)-pyridin-3-yl]-N-methoxymethyl-3-trifluoromethyl-benzenesulfonamideas an off white solid. MS: (M+H)/z = 534.0.

Step 2: A mixture of 4-Chloro-N-[5-chloro-2-(6-methyl-pytidine-3-carbonyl)-pyidin-3-yl]-N-methoxymethyl-3-trifluoromethyl-benzenesulfonamide (30 mg) in 3 mL of 4M HCl in dioxane and 1 mL of water was refluxed for 3 hours. After cooling to rt the mixture was diluted with water and sodium bicarbonate was added until pH was 9. The mixture was extracted with ethyl acetate, dried and concentrated. The residue was further purified via flash column (70% ethyl acetate in hexane) to afford 9.1 mg of title compound as an off white solid. ¹H NMR: (400 MHZ, CDCl3) δ 8.97 (m, 1H), 8.35 (m, 1H), 8.16-8.13 (m, 2H), 8.08-8.05 (m, 1H), 7.93-7.91 (m, 1H), 7.59-7.57 (m, 1H), 7.25-7.23 (1H), 2.63 (s, 3H). MS: (M+H) /z = 490.0.

### Example 25: 4-Chloro-N-[5-chloro-2-(2-methyl-pyridine-3-carbonyl)-pyridin-3-yl]-3-trifluoromethyl-benzenesulfonamide

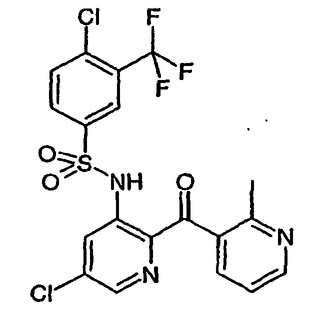

The title compound was prepared by procedure analogous to that described in Example 24 using N-(2-bromo-5-chloro-pyridin-3-yl)-4-chloro-N-methoxymethyl-3-trifluoromethyl-benzenesulfonamide and N-methoxy-2,N-dimethyl-nicotinamide. ¹H NMR: (400 MHZ, CDCl3) δ 8.61 (m, 1H), 8.26 (m, 1H), 8.20 (m, 1H), 8.17 (m, 1H), 8.04-8.01 (m, 1H), 7.67 (d, 1H), 7.50 (m, 1H), 7.18 (m, 1H), 2.41 (s, 3H). MS: (M+H) /z = 490.0.

### Example 26: 4-Chloro-N-[5-chloro-2-(2,4-dimethyl-pyridine-3-carbonyl)-pyridin-3-yl]-3-trifluoromethyl-benzenesulfonamide

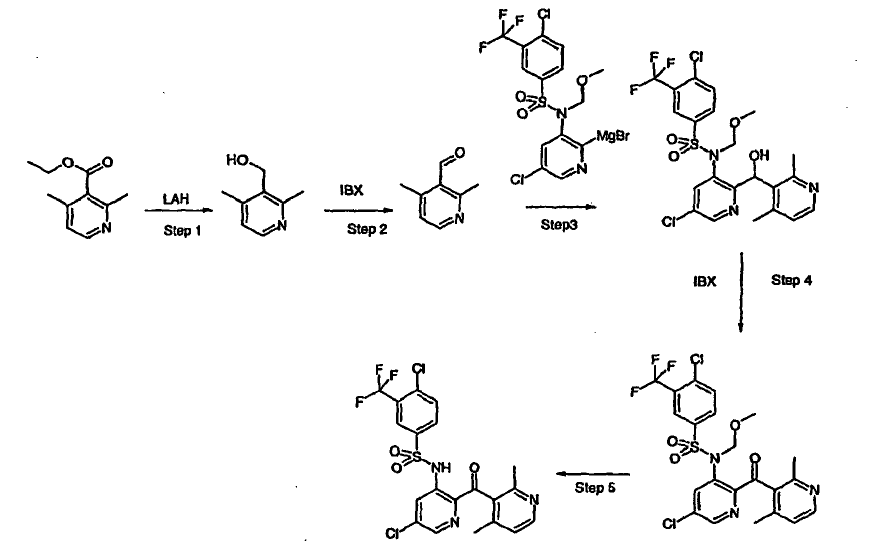

Step 1: A solution of lithium aluminum hydride in THF (1.0 M, 40 mL, 40 mmol) placed in a 100-mL round bottom flask equipped with a magnetic stirring bar was cooled in an ice bath and cautiously treated with a solution of 2,4-dimethyl-nicotinic acid ethyl ester (5.4 g, 30 mmol) in anhydrous THF (10 mL) over 5 min period. After the addition was complete the cold bath was removed and the mixture was allowed to stir at room temperature for 2 h. The reaction mixture was then cooled in an ice bath and the excess LAH was quenched by drop wise addition of water until gas evolution stopped. The reaction mixture was then basified with 2M NaOH to pH 9-10. Ether (100 mL) was added the mixture was washed with 1 M NaOH, water and brine. The organic layer was dried over MgSO₄ and the solvent was evaporated to yield (2,4-dimethyt-pyridin-3-yl)-methanol as a white solid. MS: (M+H) /z = 138.

Step 2: A magnetically stirred mixture of (2,4-dimethyl-pyridin-3-yl)-methanol (0.342 g, 2.5 mmol) and IBX (1.40 g, 5 mmol) in acetonitrile (5 mL) placed in a round bottom flask was heated at 80 °C in an oil bath for 1 h. The mixture was cooled to room temperature and filtered. The solid was washed with acetonitrile. The filtrate was concentrated and applied to a short silica gel column and chromatographed using hexane/ethyl acetate solvent mixture (30-60% ethyl acetate). Product containing fractions were combined and the solvent was evaporated to afford 0.283 g of 2,4-dimethyl-pyridine-3-carbaldehyde as a white solid. MS: (M+H) /z =136.

Step 3: N-(2-Bromo-5-chloro-pyridin-3-yl)-4-chloro-N-methoxymethyl-3-trifluoromethyl-benzenesulfonamide (0.494 g, 1 mmol) placed in a 10 mL round bottom flask under nitrogen atmosphere was dissolved in anhydrous THF (5 mL). The solution was cooled to -20 °C. The magentically stirring solution was treated with solution of 2.0 M isopropylmagnesium bromide in THF (1.05 mL, 2.1 mmol) drop wise and after the addition was complete the reaction mixture was allowed to warm to 0 °C and maintained at this temperature for 1 h. The resulting deep purple solution was cooled to -20 °C and treated with a solution of 2,4-dimethyl-pyridine-3-carbaldehyde (0.28 g, 2.1 mmol) in THF (2 mL). The mixture was allowed to warm to room temperature and stirred at room temperature over night. After diluting with saturated ammonium chloride solution (10 mL) the product was extracted with ethyl acetate (3 x 25 mL). The combined organic extract was concentrated and product purified by flash chromatography on silica gel column (10-30% ethyl acetate in hexanes). Product containing fractions were combined and the solvent evaporated to afford 0.33 g of 4-chloro-N-{5-chloro-2-[(2,4-dimethyl-pyridin-3-yl)-hydroxy-methyl]-pyridin-3-yl}-N-methoxymethyl-3-trifluoromethyl-benzenesulfonamide as an off white solid. MS: (M+H) /z = 550.

Step 4: The product obtained from Step 3 above was oxidized with IBX according to the procedure described in Step 2 and the product was purified by flash chromatography on silica gel column (30-50% ethyl acetate in hexanes) to afford 4-chloro-N-[5-chloro-2-(2,4-dimethyl-pyridine-3-carbonyl)-pyridin-3-yl]-N-methoxymethyl-3-trifluoromethyl-benzenesulfonamide as a light yellow solid. MS: (M+H) /z = 548.

Step 5: The product from Step 4 above (60 mg, 0.1 mmol) was dissolved in 4 M HCl in dioxane (4 mL). Water (1 mL) was added and the mixture was refluxed for 2 h. The solvent was evaporated and the residue was dissolved 20% aqueous acetonitrile and lyophylization yielded 4-chloro-N-[5-chloro-2-(2,4-dimethyl-pyridine-3-carbonyl)-pyridin-3-yl]-3-trifluoromethyl-benzenesulfonamide as the hydrochloride salt. ¹H NMR: (400 MHZ, DMSO-d₆) δ 8.55 (d, *J*= 6 Hz, 1H), 8.45 (d, *J*= 2 Hz, 1H), 8.32 (d, *J*= 2Hz, 1H), 8.18 - 8.15 (m, 1H), 8.05 (d, *J* = 2 Hz, 1H), 7.95 (d, *J* = 8.4 Hz, 1H), 7.53 (m, 1H), 2.20 (s, 3H), 2.03 (s, 3H). MS: (M+H)/z = 504.

### Example 27: Preparation of 2-methyl-pyridine-4-carbaldehyde

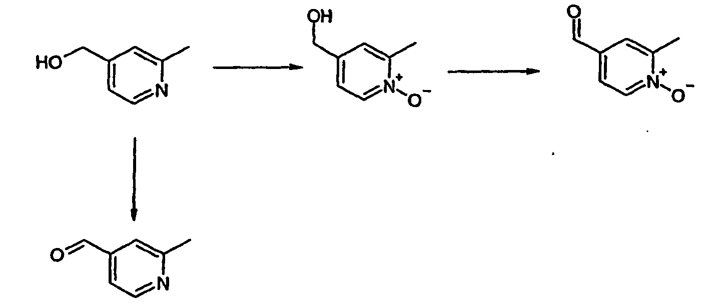

(2-Methyl-pyridin-4-yl)-methanol (1.3 g, 10.6 mmol) (prepared according to the literature procedure, see Ragan, J.A. *et al. Synthesis,* **2002**, *4*, 483-486) and MnO₂ (5 .0 g, 57.5 mmol) in CHCl₃ (50 mL) was refluxed for 18 h. The reaction mixture was cooled to room temperature and filtered. The filter cake was washed with CHCl₃ (2 x 100 mL). The combined filtrates were concentrated under reduced pressure and the resulting residue was purified by flash column chromatography on silica gel (EtOAc/Hexanes, 3:7) to provide desired aldehyde. Mass spectrum m/z : 122.1 (M+H).

### Example 28: Preparation of (2-methyl-1-oxy-pyridin-4-yl)-methanol

A mixture of (2-methyl-pyridin-4-yl)-methanol (1.1 g, 8.9 mmol) and *m-*CPBA (2.5 g, 10.7 mmol, 75% pure) in CH₂Cl₂ (50 mL) was stirred at room temperature for 18 h. The reaction mixture was concentrated to 10 mL under reduced pressure and the resulting residue was purified by flash column chromatography on silica gel (MeOH/CH₂Cl₂, 1:99, then 1:19, then, 2:23) to provide desired the title compound. Mass spectrum m/z : 140.1 (M+H).

### Example 29: Preparation of 2-methyl-1-oxy-piridine-4-carbaldehyde

A mixture of alcohol (1.3 g, 5.04 mmol) and MnO₂ (3 .0 g, 34.5 mmol) in CHCl₃ (50 mL) was refluxed for 18 h. The reaction mixture was cooled to room temperature and filtered. The filter cake was washed with CHCl₃ (2 x 100 mL). The combined filtrates were concentrated under reduced pressure to provide desired aldehyde. Mass spectrum m/z : 138.0 (M+H).

### Example 30: 4-Chloro-N-{5-chloro-2-[hydroxy-(2-methyl-1-oxy-pyridin-4-yl)-methyl]-pyridin-3-yl}-N-methoxymethyl-3-trifluoromethyl-benzenesulfonamide

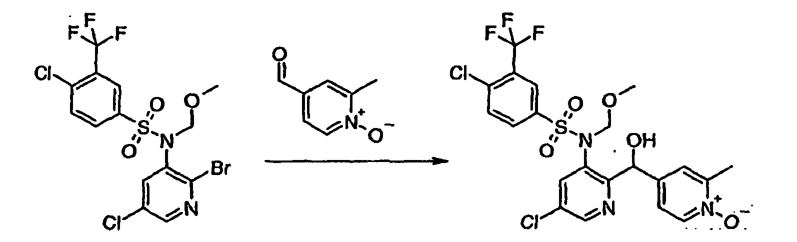

To a stirred solution of N-(2-bromo-5-chloro-pyridin-3-yl)-4-chloro-N-methoxymethyl-3-trifluoromethyl-benzenesulfonamide (494 mg, 1.0 mmol) in anhydrous THF (10 mL) was added 2 M isopropylmagnesiumchloride in THF (1.20 mL, 2.4 mmol) at -40 °C. After 5 minutes dry ice-acetone bath was replaced with a ice water bath and stirred at 0 °C for 1 h. Solid 2-methyl-1-oxy-pyridine-4-carbaldehyde (382 mg, 2.7 mmol) was added in one portion and the progress of the reaction was followed by LCMS. The reaction mixture was warmed to room temperature and stirred for 6 h and stirred overnight. It was then quenched with saturated aqueous NH₄Cl (2 mL), and extracted with EtOAc. The combined extracts were dried (Na₂SO₄), filtered and concentrated under reduced pressure to provide the desired product which was utilized in the following step without further purification. MS m/z : 552.0 (M+H).

### Example 31: 4-Chloro-N-[5-chloro-2-(2-methyl-1-oxy-pyridine-4-carbonyl)-pyridin-3-yl]-N-methoxymethyl-3-trifluoromethyl-benzenesulfonamide

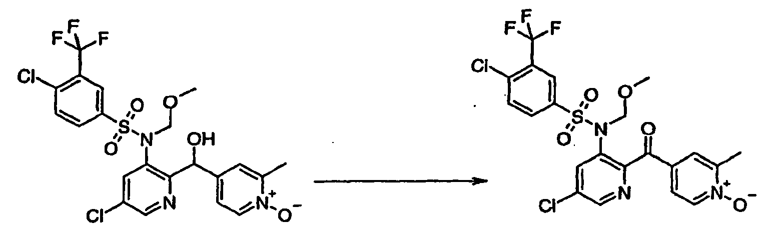

To a stirred solution of the alcohol from Example 35 (∼500 mg) in anhydrous CHCl₃ (50 mL) was added MnO₂ (2.00 g, 23 mmol). The resulting mixture was heated at reflux for 2 days. The reaction mixture was cooled to room temperature and filtered through a filter paper and the filter cake was washed with EtOAc. The filtrate was partitioned between EtOAc and brine. The organic portion was separated and aqueous part was extracted with EtOAc. The combined extracts were dried (Na₂SO₄), filtered and concentrated under reduced pressure. The residue was purified by flash column chromatography on silica gel to provide the desired ketone (EtOAc/Hexanes, 2:3 then MeOH/CH₂Cl₂, 3:97). Mass spectrum m/z: 550.0 (M+H).

### Example 32: 4-Chloro-N-[5-chloro-2-(2-methyl-1-oxy-pyridine-4-carbonyl)-pyridin-3-yl]-3-trifluoromethyl-benzenesulfonamide

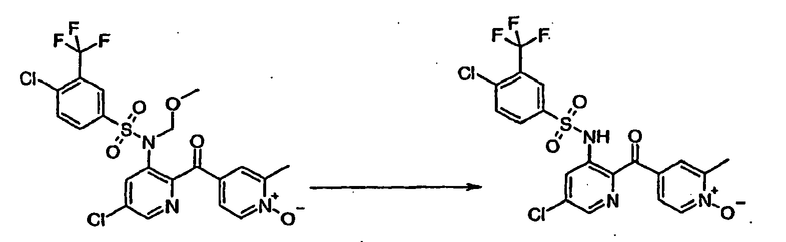

A mixture of mom-protected sulfonamide (150 mg, 0.27 mmol) in 4 M HCl in dioxane (10 mL), and water (4 mL) was heated at 100 °C for 3 h. The reaction mixture was concentrated to dryness under reduced pressure and it was treated with aqueous NaHCO₃ to adjust pH to 5-6 and extracted with EtOAc. The combined extracts were dried (Na₂SO₄), filtered and concentrated under reduced pressure. The residue was purified by flash column chromatography on silica gel (MeOH: CH₂Cl₂: 1:99, then 2:98) to provide the desired product. ¹H NMR (400 MHz, CDCl₃) δ 10.86 (s, 1 H), 8.39 (s, 1 H), 8.24 (d, *J*= 6.8 Hz, 1 H), 8.18-8.14 (m, 2 H), 7.95 (d, *J* = 9.2 Hz, 1 H), 7.90 (s, 1 H), 7.82 (d, *J* = 6.8 Hz, 1 H), 7.61 (d, *J*= 8.8 Hz, 1 H), 2.54 (s, 3 H); MS m/z : 506.0 (M+H).

### Example 33: Preparation of 4-Chloro-N-{5-chloro-2-[hydroxy-(2-methyl-pyridin-4-yl)-methyl]-pyridin-3-yl}-N-methoxymethyl-3-trifluoromethyl benzenesulfonamide

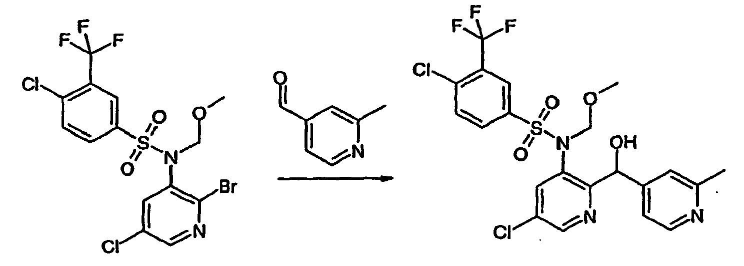

To a stirred solution of N-(2-bromo-5-chloro-pyridin-3-yl)-4-chloro-N-methoxymethyl-3-trifluoromethyl-benzenesulfonamide (494 mg, 1.0 mmol) in anhydrous THF (10 mL) was added 2 M isopropylmagnesiumchloride in THF (1.20 mL, 2.4 mmol) at -40 °C. After 5 minutes dry ice-acetone bath was replaced with a ice water bath and stirred at 0 °C for 1 h. Solid 2-methyl-pyridine-4-carbaldehyde (327 mg, 2.7 mmol) was added in one portion and the progress of the reaction was followed by LCMS. The reaction mixture was warmed to room temperature and stirred overnight. It was then quenched with saturated aqueous NH₄Cl (2 mL), and extracted with EtOAc. The combined extracts were dried (Na₂SO₄), filtered and concentrated under reduced pressure to provide the desired product which was utilized in the following step without further purification. MS m/z: 536.0 (M+H).

### Example 34: Preparation of 4-Chloro-N-[5-chloro-2-(2-methyl-pyridine-4-carbonyl)-pyridin-3-yl]-N-methoxymethyl-3-trifluoromethyl-benzenesulfonamide

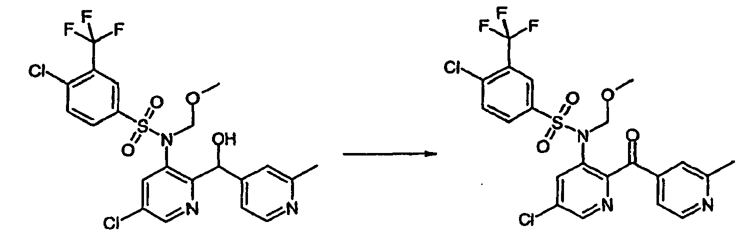

To a stirred solution of 4-chloro-N-[5-chloro-2-(2-methyl-pyridine-4-carbonyl)-pyridin-3-yl]-N-methoxymethyl-3-trifluoromethyl-benzenesulfonamide (∼500 mg) in anhydrous CHCl₃ (50 mL) was added MnO₂ (2.00 g, 23 mmol). The resulting mixture was heated at reflux for 4 days. The reaction mixture was cooled to room temperature and filtered through a filter paper and the filter cake was washed with EtOAc. The filtrate was partitioned between EtOAc and brine. The organic portion was separated and aqueous part was extracted with EtOAc. The combined extracts were dried (Na₂SO₄), filtered and concentrated under reduced pressure. The residue was purified by flash column chromatography on silica gel to provide the desired ketone (EtOAc/Hexanes, 2:3 then 4:1). MS m/z: 534.0 (M+H).

### Example 35: 4-Chloro-N-[5-chloro-2-(2-methyl-pyridine-4-carbonyl)-pyridin-3-yl]-3-trifluoromethyl-benzenesulfonamide

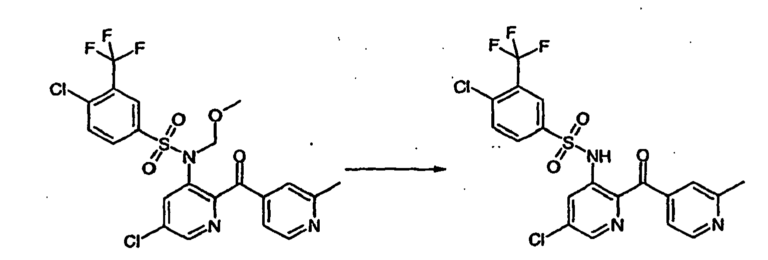

A mixture of 4-Chloro-N-[5-chloro-2-(2-methyl-pyridine-4-carbonyl)-pyridin-3-yl]-N-methoxymethyl-3-trifluoromethyl-benzenesulfonamide (150 mg, 0.27 mmol) from Example 39 dissolved in 4 M HCl in dioxane (10 mL), and water (4 mL) was heated at 100 °C for 3 h. The reaction mixture was concentrated to dryness under reduced pressure and it was treated with aqueous NaHCO₃ to adjust pH to 5-6 and extracted with EtOAc. The combined extracts were dried (Na₂SO₄), filtered and concentrated under reduced pressure. The residue was purified by flash column chromatography on silica gel (MeOH: CH₂Cl₂: 1:99, then 2:98) to provide the desired product. ¹H NMR (400 MHz, CDCl₃) δ 10.9 (s, 1 H), 8.64 (d, *J* = 5.2 Hz, 1 H), 8.36 (s, 1 H), 8.18-8.16 (m, 2 H), 7.96 (d, *J* = 8.4 Hz, 1 H), 7.62 (d, *J*= 8.4 Hz, 1 H), 7.41-7.38 (m, 2 H), 2.64 (s, 3 H); MS m/z : 490.0 (M+H).

### Example 35: 4-Chtoro-N-[5-chloro-2-(4-chloro-benzoyl)-pyridin-3-yl]-3-trifluoromethyl-benzenesulfonamide

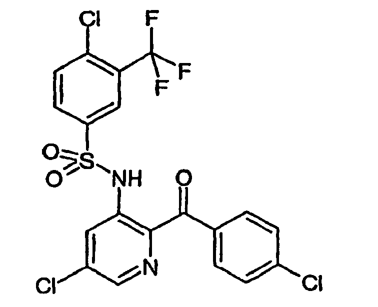

The title compound was prepared by procedure analogous to that described in Example 24 using N-(2-bromo-5-chloro-pyridin-3-yl)-4-chloro-N-methoxymethyl-3-trifluoromethyl-benzenesulfonamide and 4-chloro-N-methoxy-N-methyl-banzamide ¹H NMR (400 MHz, CDCl₃) δ10.61 (s, 1H), 8.37 (m, 1H), 8.16 (m, 1H), 8.09 (m, 1H), 7.84 (m, 1H), 7.77 -7.75 (m, 2H), 7.53 (m, 1H), 7.42-7.40 (m, 2H), 7.23 (m, 1H) MS: (M+H) /z = 509.0.

### Example 37: N-(2-Bromo-5-chloro-pyridin-3-yl)-4-tert-butyl-N-methoxymethyl-benzenesulfonamide

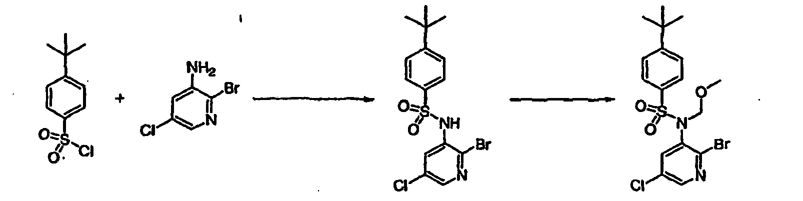

A 200 mL round-bottom flask was charged with 2-bromo-5-chloro-pyridin-3-ylamine (10.4 g, 50.0 mmol), 4-tert-butyl-benzenesulfonyl chloride (20.0 g, 85.0 mmol), and pyridine (38 mL). The resultant solution was heated to 70 °C and stirred overnight. The following day, the pyridine was removed by removed *in vacuo* and THF (30 mL) and 4.0 N NaOH (100 mL) were added and the reaction was stirred at 60 °C overnight. The organics were subsequently removed *in vacuo* and the residues were diluted with water (400 mL). The small quantity of insoluble solid was removed by filtration and the pH was adjusted to 6-7 with concentrated HCl. The resultant aqueous solution was extracted with EtOAc, washed with brine, dried over MgSO₄, and concentrated under reduced pressure to afford the diarylsulfonamide (13.4 g) in 66 % yield. To a solution of the crude sulfonamide (12.0 g, 35.0 mmol) and K₂CO₃ (24.0 g, 170 mmol) in anhydrous THF (80 mL) was added chloromethyl methyl ether (4.0 mL, 52.7 mmol). The resultant heterogeneous solution was stirred for 60 min at ambient temperature and the solids were subsequently removed via filtration. The filtrate was then removed *in vacuo* and the residue was dissolved in EtOAc. The organics were washed with saturated Na₂CO₃, dried over MgSO₄, and evaporated *in vacuo* to generate a brownish oil. The oil was triturated with hexanes and the resultant solid filtered to produce the desired product as a light yellowish solid (11.5 g, 86% yield).

### Example 38: 4-tert-Butyl-N-[5-chloro-2-(2-fluoro-benzoyl)-pyridin-3-yl]-benzenesulfonamide

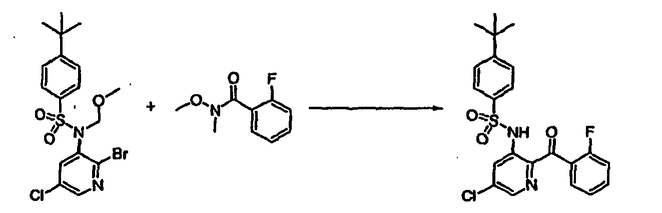

N-(2-Bromo-5-chloro-pyridin-3-yl)-4-tert-butyl-N-methoxymethyl-benzenesulfonamide (140 mg, 300 mmol) was placed in a dry 2-neck 10 mL round-bottom flask. The flask was evacuated and purged with nitrogen, followed by the addition of THF (1 mL). The homogeneous mixture was lowered to -5 °C and *i*PrMgCl (0.33 mL, 2.0 M) was added dropwise. Upon completion of the addition, the reaction was stirred 90 minutes, followed by the slow addition of 2-fluoro-N-methoxy-N-methyl-benzamide (140 mg, 750 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl (in dioxane) (1 mL, 4.0 mmol) and H₂O (0.33 mL), and then stirred at 80 °C for two hours. The resultant solution was diluted with EtOAc, washed with water, saturated sodium bicarbonate, and brine; dried with MgSO₄, and evaporated employing reduced pressure. The crude sulfonamide was finally purified via preparatory TLC (20% EtOAc/hexanes) and recrystallization from MeCN/H₂O to afford 98 mg of the title compound: ¹H NMR (400 MHz, CDCl₃) δ 10.6 (s, 1H), 8.3 (d, 1H), 8.2 (d, 1H), 7.74 (d, 2H), 7.6 (d, 1H), 7.5 (d, 1H), 7.43 (d, 2H), 7.42-7.37 (m, 2H), 1.26 (s, 9H); MS (ES) M+H expect 447.1, found 447.5.

### Example 39: 4-tert-Butyl-N-[5-chloro-2-(4-fluoro-benzoyl)-pyridin-3-yl]-benzenesulfonamide

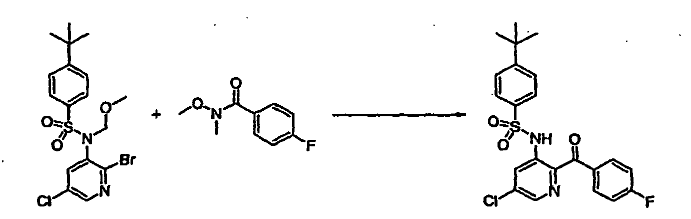

N-(2-Bromo-5-chloro-pyridin-3-yl)-4-tert-butyl-N-methoxymethyl-benzenesulfonamide (140 mg, 300 mmol) was placed in a dry 2-neck 10 mL round-bottom flask. The flask was evacuated and purged with nitrogen, followed by the addition of THF (1 mL). The homogeneous mixture was lowered to -5 °C and *i*PrMgCl (0.33 mL, 2.0 M) was added dropwise. Upon completion of the addition, the reaction was stirred 90 minutes, followed by the slow addition of 4-fluoro-N-methoxy-N-methyl-benzamide (137 mg, 750 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl in dioxane (1 mL, 4.0 mmol) and H₂O (0.33 mL), and then stirred at 80 °C for two hours. The resultant solution was diluted with EtOAc, washed with water, saturated sodium bicarbonate, and brine; dried with MgSO₄, and evaporated employing reduced pressure. The crude residue was subsequently purified via reverse phase HPLC (Column: Varian Dynamax 250 X 21.4 mm Microsorb100-8 C18, 0.1% TFA/H₂O (Eluent'A): 0.1% TFA/MeCN (Eluent B)):¹H NMR (400 MHz, CDCl₃) δ 10.63 (bs, 1H), 8.29 (d, 1H), 8.20 (d, 1H), 7.85-7.91 (m, 2H), 7.74 (d, 2H), 7.42 (d, 2H), 7.09 (t, 2H), 1.25 (s, 9H); MS (ES) M+Na expect 469.1, found 469.4.

### Example 40: 4-tert-Butyl-N-[5-chloro-2-(3-methoxy-benzoyl)-pyridin-3-yl]-benzenesulfonamide

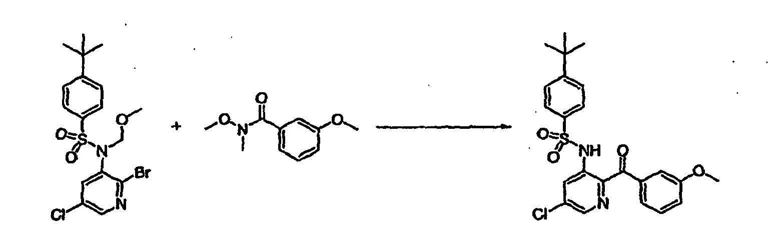

N-(2-Bromo-5-chloro-pyridin-3-yl)-4-tert-butyl-N-methoxymethyl-benzenesulfonamide (140 mg, 300 mmol) was placed in a dry 2-neck 10 mL round-bottom flask. The flask was evacuated and purged with nitrogen, followed by the addition of THF (1 mL). The homogeneous mixture was lowered to -5 °C and *i*PrMgCl (0.33 mL, 2.0 M) was added dropwise. Upon completion of the addition, the reaction was stirred 90 minutes, followed by the slow addition of 3,N-dimethoxy-N-methyl-benzamide (146 mg, 750 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl in dioxane (1 mL, 4.0 mmol) and H₂O (0.33 mL), and then stirred at 80 °C for two hours. The resultant solution was diluted with EtOAc, washed with water, saturated sodium bicarbonate, and brine; dried with MgSO₄, and evaporated employing reduced pressure. The crude residue was subsequently purified via reverse phase HPLC (Column: Varian Dynamax 250 X 21.4 mm Microsorb100-8 C18, 0.1% TFA/H₂O (Eluent A): 0.1% TFA/MeCN (Eluent B)):¹H NMR (400 MHz, CDCl₃) δ 10.61 (s, 1H), 8.29 (d, 1H), 8.20 (d, 1H), 7.74 (d, 2H), 7.58 (s, 1H), 7.50 (d, 1H), 7.42 (d, 2H), 7.37 (d, 1H), 7.29 (t, 1H), 2.40 (s, 3H), 1,25 (s, 9H); MS (ES) M+H expect 459.1, found 459.5.

### Example 41: 4-tert-Butyl-N-5-chloro-2-(3-methanesulfonyl-benzoyl)-pyridin-3-yl]-benzenesulfonamide

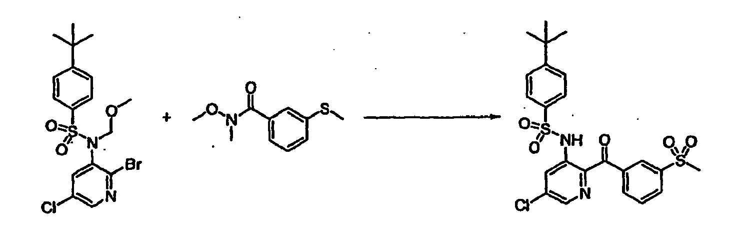

N-(2-Bromo-5-chloro-pyridin-3-yl)-4-tert-butyl-N-methoxymethyl-benzenesulfonamide (500 mg, 1.12 mmol) was placed in a dry 2-neck 50 mL round-bottom flask. The flask was evacuated and purged with nitrogen, followed by the addition of THF (5.6 mL). The homogeneous mixture was lowered to -5 °C and *i*PrMgCl (1.28 mL, 2.0 M) was added dropwise. Upon completion of the addition, the reaction was stirred 90 minutes, followed by the slow addition of N-methoxy-N-methyl-3-methylsulfanyl-benzamide (471 mg, 2.23 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl in dioxane (4.76 mL, 19.0 mmol) and H₂O (1.59 mL), and then stirred at 80 °C overnight. The following day, the organics were removed *in vacuo* and the residue was diluted with EtOAc. The resultant organics were washed with water, saturated sodium bicarbonate, and brine; dried with MgSO₄, and evaporated employing reduced pressure. The crude residue was subsequently purified via automated flash chromatography to afford the desired arylthioether. To a stirring solution of the thioether (100 mg, 0.211 mmol) in methylene chloride (0.8 mL) was added *m*CPBA (77% maximum) (141 mg, 0.632 mmol), during which the exothermic reaction caused slight boiling of the methylene chloride. Upon stirring 30 min, the reaction was quenched with aqueous sodium metabisulfite and then stirred a further 15 min. The solution was diluted with EtOAc and the combined organics were washed with sodium metabisulfite and saturated sodium bicarbonate. The organic layer was then dried with MgSO₄ and concentrated *in vacuo* to generate the desired arylmethylsulfone: ¹H NMR (400 MHz, CDCl₃) 810.86 (s, 1H), 8.43 (dd, 1H), 8.27 (d, 1H), 8.22 (d, 1H), 8.11 (dd, 2H), 7.80 (d, 2H), 7.65 (t, 1H), 7.48 (d, 2H), 3.18 (s, 3H), 1.27 (s, 9H); MS (ES) M+H expect 507.1, found 507.5.

### Example 42: N-[2-(3-Amino-benzoyl)-5-chloro-pyridin-3-yl]-4-tert-butyl-benzenesulfonamide

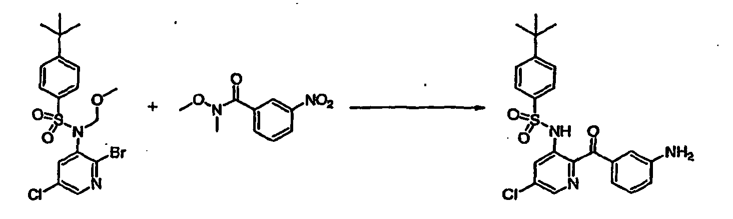

N-(2-Bromo-5-chloro-pyridin-3-yl)-4-tert-butyl-N-methoxymethyl-benzenesulfonamide (3.84 g, 8.57 mmol) was placed in a dry 2-neck 250 mL round-bottom flask. The flask was evacuated and purged with nitrogen, followed by the addition of THF (43 mL). The homogeneous mixture was lowered to -5 °C and *i*PrMgCl (9.86 mL, 2.0 M) was added dropwise. Upon completion of the addition, the reaction was stirred 90 minutes, followed by the slow addition of N-methoxy-N-methyl-3-nitro-benzamide (4.50 g, 21.43 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl in dioxane (36 mL, 146 mmol) and H₂O (12 mL), and then stirred at 80 °C overnight. The following day, the organics were removed *in vacuo* and the residue was diluted with EtOAc. The resultant organics were washed with water, saturated sodium bicarbonate, and brine; dried with MgSO₄, and evaporated employing reduced pressure. The crude residue was subsequently purified via automated flash chromatography to afford the desired nitroarene. To a rapidly stirring solution of iron (274 mg, 5.07 mmol) in acetic acid (5 mL) at 80 °C was added dropwise a homogeneous solution of the nitroarene (685 mg, 1.45 mmol) in AcOH/CH₂Cl₂ (8 mL/2mL). The reaction was stirred 60 min, cooled to ambient temperature, diluted with EtOAc (12 mL), and filtered through celite. The filtrate was washed thoroughly with EtOAc and concentrated *in vacuo.* The resultant residue was dissolved in EtOAc, washed two times with saturated NaHCO₃, dried with sodium sulfate, and concentrated at reduced pressure to afford the crude aniline: ¹H NMR (400 MHz, CDCl₃) δ 10.54 (s, 1H), 8.28 (d, 1H), 8.19 (d, 1H), 7.72 (d, 2H), 7.40 (d, 2H), 7.16 (t, 1H), 7.08 (ddd, 1H), 7.04 (ddd, 1H), 6.85 (ddd, 1H), 3.78 (bs, 2H), 1.25 (s, 9H); MS (ES) M+H expect 4444.1, found 444.1.

### Example 43: 4-tert-Butyl-N-[5-chloro-2-(3-methanesulfonylamino-benzoyl)-pyndin-3-yl]-benzenesulfonamide

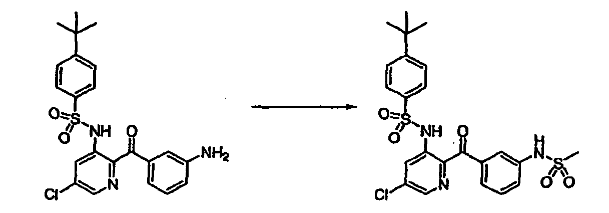

To a stirring solution of N-[2-(3-amino-benzoyl)-5-chloro-pyridin-3-yl]-4-tert-butyl-benzenesulfonamide (60 mg, 0.135 mmol) in pyridine (0.2 mL) was added methanesulfonyl chloride (0.012 mL, 0.163 mmol). The homogeneous reaction was stirred overnight to generate a mixture of mono- and bis-sulfonamide. The resultant solution was partitioned with EtOAc/10% HCl, and the organics were washed with 10% HCl and saturated sodium bicarbonate. The organic layer was then dried with sodium, sulfate and concentrated *in vacuo* to produce the crude sulfonamide mixture that co-eluted via both reverse-phase and normal-phase chromatography. A 5 mL flask was charged with the resultant sulfonamide mixture, tetrabutylammonium fluoride (0.405 mL, 1.0 M), and THF (1.3 mL). The reaction was stirred 90 min and then partitioned with EtOAc/10% HCl. The organics were washed with 10% HCl and saturated sodium bicarbonate, dried with sodium sulfate, and concentrated *in vacuo* to produce the desired mono-sulfonamide which was further purified through automated normal-phase chromatography: ¹H NMR (400 MHz, CDCl₃) δ 10.71 (bs, 1H), 8.27 (d, 1H), 8.20 (d, 1H), 7.79 (d, 2H), 7.69-7.71 (m, 1H), 7.60-7.62 (m, 1H), 7.42-7.49 (m, 4H), 6.48 (bs, 1H), 3.06 (s, 3H), 1.28 (s, 9H); MS (ES) M+H expect 522.1, found 522.1.

### Example 44: N-{3-[3-(4-tert-Butyl-benzenesulfonylamino)-5-chloro-pyridine-2-carbonyl]-phenyl}-acetamide

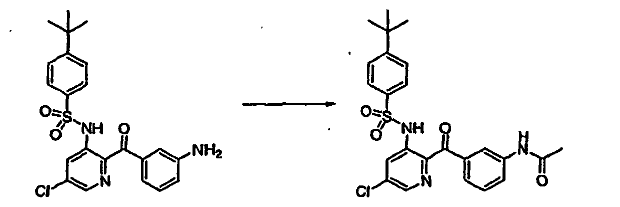

To a stirring solution of N-[2-(3-amino-benzoyl)-5-chloro-pyridin-3-yl]-4-tert-butyl-benzenesulfonamide (60 mg, 0.135 mmol), pyridine (0.022 mL, 0.271 mmol), and methylene chloride (0.68 mL) at 0 °C was added acetyl chloride (0.011 mL, 0.149 mmol). The homogeneous reaction was stirred 90 min, diluted with EtOAc, and then quenched with 10% HCl. The organics were washed with 10% HCl and saturated sodium bicarbonate, dried with sodium sulfate, and concentrated *in vacuo* to produce the target aryl acetamide: ¹H NMR (400 MHz, CDCl₃) δ 10.71 (bs, 1H), 8.28 (d, 1H), 8.18 (d, 1H), 7.88-7.91 (m, 1H), 7.79 (d, 1H), 7.74 (d, 2H), 7.50 (d, 1H), 7.43 (d, 2H), 7.36 (t, 1H), 7.22 (bs, 1H), 2.18 (s, 3H), 1.25 (s, 9H); MS (ES) M+H expect 486.1, found 486.5.

### Example 45: 4-tert-Buyl-N-[5-chloro-2-(6-hydroxy-pyridine-3-carbonyl)-pyridin-3-yl]-benzenesulfonamide

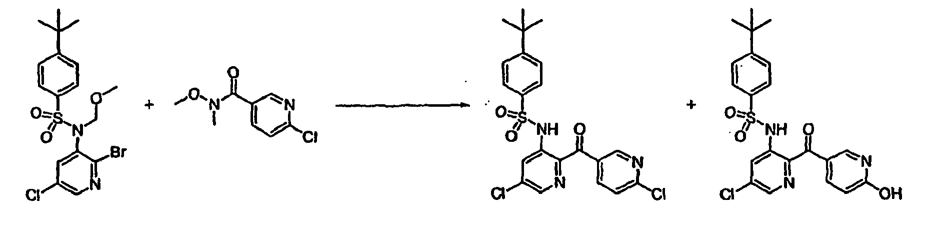

N-(2-Bromo-5-chloro-pyridin-3-yl)-4-tert-butyl-N-methoxymethyl-benzenesulfonamide (2.00 g, 4.46 mmol) was placed in a dry 2-neck 50 mL round-bottom flask. The flask was evacuated and purged with nitrogen, followed by the addition of THF (15 mL). The homogeneous mixture was lowered to -5 °C and *i*PrMgCl (5.13 mL, 2.0 M) was added dropwise. Upon completion of the addition, the reaction was stirred 90 minutes, followed by the slow addition of 6-chloro-N-methoxy-N-methyl-nicotinamide (1.44 g, 7.14 mmol). The reaction was stirred overnight, during which the ice-bath warmed to room temperature. The resultant solution was quenched with 10% HCl and diluted with EtOAc. The organics were washed with 10% HCl and saturated sodium bicarbonate, dried with sodium sulfate, concentrated *in vacuo,* and purified via automated silica gel chromatography to afford the diaryl ketone (1.049 g) in 46% yield. A microwave flask was charged with the diaryl ketone (40 mg, 0.078 mmol), HCl in dioxane (0.335 mL, 4.0 M), and water (0.12 mL). The reaction vessel was subjected to a microwave reaction at 90 °C for 10 min. The reaction was incomplete, thus the vessel was reacted a further 10 min at 120 °C, during which the starting materials were consumed. The crude mixture was diluted with EtOAc and neutralized to pH 7-8 with saturated sodium bicarbonate. The organics were washed with saturated sodium bicarbonate, dried with sodium sulfate, concentrated *in vacuo,* and purified via automated column chromatography to generate a mixture of the desired 4-tert-butyl-N-[5-chloro-2-(6-hydroxy-pyridine-3-carbonyl)-pyridin-3-yl]-benzenesulfonamide and 4-tert-butyl-N-[5-chloro-2-(6-chloro-pyridine-3-carbonyl)-pyridin-3-yl]-benzenesulfonamide: ¹H NMR (400 MHz, CDCl₃) δ 10.98 (bs, 1H), 8.70 (d, 1H), 8.29 (d, 1H), 8.16-8.20 (m, 2H), 7.76 (d, 2H), 7.45 (d, 2H), 6.59 (d, 1H), 1.27 (s, 9H); MS (ES) M+H expect 446.1, found 446.3.

### Example 46: 4-tert-Butyl-N-[5-chloro-2-(6-methoxy-pyridine-3-carbonyl)-pyridin-3-yl]-benzenesulfonamide

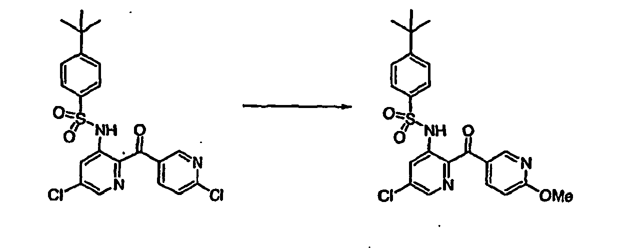

To a stirring solution of 4-tert-butyl-N-[5-chloro-2-(6-chloro-pyridine-3-carbonyl)-pyridin-3-yl]-benzenesulfonamide (30 mg, 0.065 mmol) in MeOH (0.65 mL) was added NaOMe (18 mg, 0.324 mmol). The resultant solution was heated to 80 °C and stirred 4h. The reaction was quenched with 10% HCl (to pH 6-7), diluted with EtOAc, and the pH adjusted to 7-8 with saturated sodium bicarbonate. The organics were washed with saturated sodium bicarbonate, dried with sodium sulfate, concentrated *in vacuo,* and purified via automated flash chromatography to produce the target methoxy pyridine: ¹H NMR (400 MHz, CDCl₃) δ 10.71 (bs, 1H), 8.72 (d, 1H), 8.26 (d, 1H), 8.08-8.20 (m, 2H), 7.72 (d, 2H), 7.37 (d, 2H), 6.75 (d, 1H), 4.00 (s, 3H), 1.23 (s, 9H); MS (ES) M+H expect 460.1, found 460.2.

### Example 47: N-2-6-Amino-pyridine-3-carbonyl-5-chloro-pyridin-3-yl]-4-tert-butyl-benzenesulfonamide

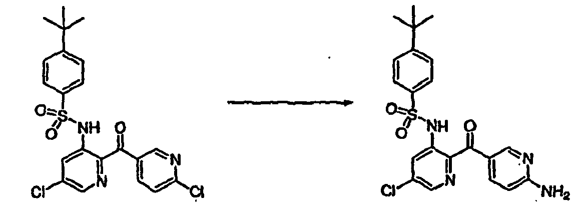

A sealed tube was charged with 4-tert-butyl-N-[5-chloro-2-(6-chloropyridine-3-carbonyl)-pyfidin-3-yl]-benzenesulfonamide (320 mg, 0.631 mmol), concentrated ammonium hydroxide (3.2 mL), and THF (3.2 mL). The tube was sealed and stirred for 3 days at 100 °C. The solvents were subsequently removed *in vacuo* and the residue purified by automated flash chromatography to provide the desired aminopyridine: ¹H NMR (400 MHz, CDCl₃) δ 10.72 (bs, 1H), 8.69 (d, 1H), 8.28 (dd, 1H), 8.16 (dd, 1H), 8.02 (dd, 1H), 7.71 (d, 2H), 7.40 (d, 2H), 6.46 (d, 1H), 5.00 (bs, 2H), 1.24 (s, 9H); MS (ES) M+H expect 445.1, found 445.1.

### Example 48: 4-tert-Bytyl-N-5-chloro-2-(2-methyl-pyridine-3-carbonyl)-pyridin-3-yl]-benzenesulfonamide

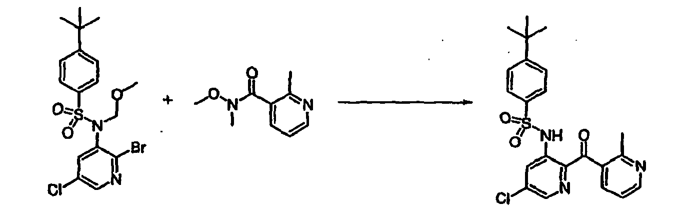

N-(2-Bromo-5-chloro-pyridin-3-yl)-4-tert-butyl-N-methoxymethyl-benzenesulfonamide (140 mg, 300 mmol) was placed in a dry 2-neck 10 mL round-bottom flask. The flask was evacuated and purged with nitrogen, followed by the addition of THF (1 mL). The homogeneous mixture was lowered to -5 °C and *i*PrMgCl (0.33 mL, 2.0 M) was added dropwise. Upon completion of the addition, the reaction was stirred 90 minutes, followed by the slow addition of N-methoxy-2,N-dimethyl-nicotinamide (135 mg, 750 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl in dioxane (1 mL, 4.0 mmol) and H₂O (0.33 mL), and then stirred at 80 °C for two hours. The resultant solution was diluted with EtOAc, washed with water, saturated sodium bicarbonate, and brine; dried with MgSO₄, and evaporated employing reduced pressure. The crude residue was subsequently purified via automated flash chromatography to afford the desired sulfonamide: ¹H NMR (400 MHz, CDCl₃) δ 10.9 (s, 1H), 8.58 (d, 1H), 8.22 (d, 2H), 7.81 (d, 2H), 7.53-7.46 (m, 3H), 7.20-7.14 (m, 1H), 2.30 (s, 3H), 1.31 (s, 9H); MS (ES) M+H expect 444.1, found 444.5.

### Example 49: (3-Amino-5-chloropyridin-2-yl)(3-fluorophenyl)methanone

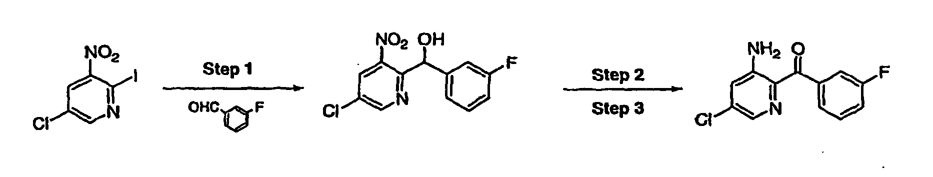

Step 1: A freshly oven dried 250 mL round-bottom flask was charged with 2-iodo-3-nitro-5-chloro-pyridine (1.41g, 5.0 mmol). The resultant solution was cooled to -78 °C under N₂ and PhMgCl (2 M, 3 mL, 6.0 mmol) added and then stirred at the same temperature for 30 min. 3-Fluorobenzaldehyde (1.24 g, 10 mmol) was added and the resulting mixture stirred at -78 °C for two hours and then at room temperature for 24 h. The reaction was quenched with NH₄Cl (sat), extracted with EtOAc, washed with brine, dried over MgSO₄, and concentrated under reduced pressure. The residue was further purified through automated normal-phase chromatography to afford 5-chloro-3-nitropyridin-2-yl)(3-fluorophenyl)methanol (560 mg, 40%) which was used directly for the next step: ¹H NMR (400 MHz, CDCl₃) δ 8.85 (d, 1H), 8.37 (d, 1H), 7.23 (m, 1H), 7.06 (dd, 1H), 6.95 (dd, 1H), 6.43 (d, 2H), 4.99 (d, 1H); MS (ES) (M⁺-OH) expect 265.0, found 265.1.

Step 2: A 50 mL round-bottom flask was charged with the above alcohol (500 mg, 1.78 mmol) and dichloromethane (10 mL). To the resultant solution was added Dess-Martin periodinane (900 mg, 2.13 mmol) and stirred at the room temperature for 30 min. The reaction was quenched with NaS₂O₃, filtered through celite, and extracted with EtOAc, washed with brine, dried over MgSO₄, and concentrated under reduced pressure to afford the crude nitro ketone (500 mg, quantitative) which was used directly for the next step: ¹H NMR (400 MHz, CDCl₃) δ 8.87 (d, 1H), 8.50 (s, 1H), 7.53 (m, 2H), 7.44 (m, 1H), 7.33 (m, 1H); MS (ES) (M⁺+H) expect 281.0, found 281.1.

Step 3: A 50 mL round-bottom flask was charged with the iron powder (336 mg, 6.0 mmol) in acetic acid (10 mL) and heated to 80 °C (oil bath) under N₂. To this mixture was added slowly the nitroketone (500 mg, 1.78 mmol) in acetic acid (5 mL) via dropping funnel and stirred at 80 °C for another 30 min after the addition. After cooling the reaction mixture, it was diluted with EtOAc, filtered through celite and the solvent evaporated *in vacuo.* The residue was dissolved in EtOAc and washed with saturated NaHCO₃, brine, dried over MgSO₄, and concentrated under reduced pressure. The residue was purified through automated normal-phase chromatography to afford 3-amino-5-chloropyridin-2-yl)(3-fluorophenyl)methanone (430 mg, 97%) which was used directly for the next step: ¹H NMR (400 MHz, CDCl₃) δ 7.93 (d, 1H), 7.70-7.62 (m, 1H), 7.40 (m, 1H), 7.20 (m, 1H), 7.09 (d, 1H), 6.26 (br s, 2H); MS (ES) (M⁺+H) expect 251.0, found 251.0.

### Example 50: 6-isopropoxypyridine-3-sultonyl chloride

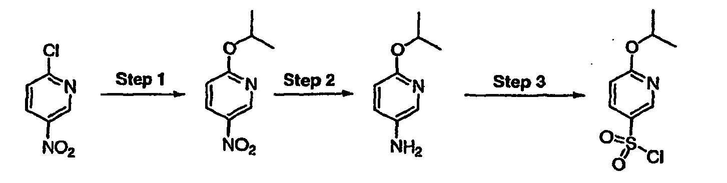

Step 1: A freshly oven dried 500 mL round-bottom flask was charged with 2-chloro-5-nitropyridine (5.0 g, 31.6 mmol) and anhydrous iPrOH (50 mL). The resultant solution was cooled in an ice bath and iPrONa (50 mmol) in iPrOH (250 mL) added slowly, followed by stirring at the same temperature for 30 min and then at room temperature for 14 h. The reaction was quenched with H₂O (5 mL), filtered through celite, and concentrated under reduced pressure. The residue was diluted with EtOAc and washed with brine, dried over MgSO₄, and concentrated under reduced pressure. The residue was purified through automated normal-phase chromatography to afford 2-isopropoxyl-5-nitropyridine (4.6 g, 79%) which was used directly for the next step: ¹H NMR (400 MHz, CDCl₃) δ 9.02 (d, 1H), 8.30 (dt, 1H), 6.75 (d, 1H), 5.40 (sep, 1H), 1.38 (d, 6H); MS (ES) (M⁺+H) expect 183.1, found 183.1.

Step 2: A 100 mL round-bottom flask was charged with the iron powder (5.6 g, 10 mmol) in acetic acid (30 mL) and heated to 80°C (oil bath) under N₂. To this mixture was added slowly the above 2-isopropoxyl-5-nitropyridine (4.6 g, 25.2 mmol) in acetic acid (30 mL) via dropping funnel and the reaction stirred at 80 °C for another 30 min after the addition. After cooling the reaction mixture, it was diluted with EtOAc, filtered through celite, and the solvent evaporated *in vacuo.* The residue was dissolved in EtOAc and washed with NaHCO₃ (sat.), brine, dried over MgSO₄, and concentrated under reduced pressure. The residue was purified through automated normal-phase chromatography to afford 2-isopropoxyl-5-aminopyridine (3.8 g, 99%) which was used directly for the next step: ¹H NMR (400 MHz, CDCl₃) δ 7.64 (d, 1H), 7.00
(dt, 1H), 6.52 (d, 1H), 5.10 (sep, 1H), 1.30 (d, 6H); MS (ES) (M⁺+H) expect 153.1, found 153.1.

Step 3: A 50 mL round-bottom flask was charged with 2-isopropoxyl-5-aminopyridine (1.6 g, 10.5 mmol), hydrochloric acid (*conc.*) (10 mL) and glacial acetic acid (8 mL) and cooled to -10 °C. To this mixture was added slowly a solution of NaNO₂ (828 mg, 12 mmol) in H₂O (2 mL) and the reaction stirred at -10 °C for another 30 min after the addition. In a second flask, sulfur dioxide was bubbled into a magnetically stirred acetic acid (16 mL) to saturation. Cuprous chloride (250 mg) was added and the introduction of sulfur dioxide was continued until the yellow-green suspension became blue-green. The mixture was then cooled to -10 °C. The diazonium salt mixture was added in portions (gas evolution) and the temperature was kept under -5 °C. The dark mixture was kept at -10 °C for 30 min and then at -5 °C for 1 h. The mixture was poured to ice water and extracted with ether, washed with NaHCO₃ (sat.) until the washings were neutral, then with cold water, dried over MgSO₄, and concentrated under reduced pressure to afford 6-isopropoxypyridine-3-sulfonyl chloride (1,5 g, 64%) which was used directly for the next step: ¹H NMR (400 MHz, CDCl₃) δ 8.80 (d, 1H), 8.06 (dt, 1H), 6.80 (d, 1H), 5.42 (sep, 1H), 1.38 (d, 6H); MS (ES) (M⁺+H) expect 235.0, found 235.1.

### Step 51: N-(2-Benzoyl)-5-chlorol-pyridin-3-yl)-4-tert-butyl-benzenesulfonamide

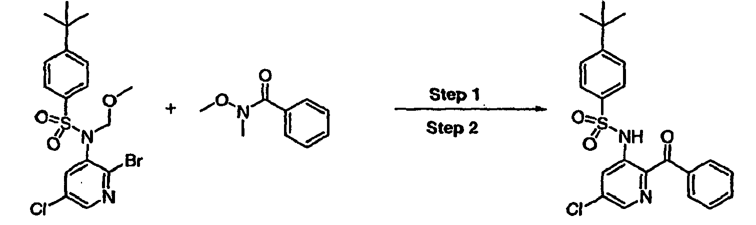

Step 1: N-(2-Bromo-5-chloro-pyridin-3-yl)-4-tert-butyl-N-methoxymethyl-benzenesulfonamide (450 mg, 1.0 mmol) was placed in a dry 100 mL round-bottom flask sealed with septa. The flask was evacuated and purged with nitrogen, followed by the addition of dry THF (30 mL). The homogeneous solution was cooled to 0 °C and *i*PrMgCl (1.5 mL, 2.0 M) was added dropwise. Upon completion of the addition, the mixture was stirred at 0 °C for 90 min, followed by the slow addition of N-mothoxy-N-methyl-benzamide (660 mg, 4.0 mmol). The reaction mixture was stirred at 0 °C for 2 h and then at room temperature overnight. The reaction was quenched with NH₄Cl (sat.) and extracted with EtOAc, washed with brine, dried over MgSO₄, and concentrated under reduced pressure. The residue was purified through automated normal-phase chromatography to afford MOM-protected sulfonamide (360 mg, 76%) which was used directly for the next step: ¹H NMR (400 MHz, CDCl₃) δ 8.55 (d, 1H), 7.65 (m, 3H), 7.50 (m, 3H), 7.37 (t, 2H), 7.24 (d, 2H), 5.20 (s, 2H), 3.45 (s, 3H), 1.19 (s, 9H); MS (ES) (M⁺+Na) expect 495.1, found 495.1.

Step 2: The above MOM-protected sulfonamide (340 mg, 0.72 mmol) was treated with 4.0 M HCl in dioxane (4 mL, 16 mmol) and H₂O (2 mL), and then stirred at 80 °C for 1.5 h. The mixture was diluted with EtOAc. The resultant organics were washed with NaHCO₃ (sat.), and brine; dried (MgSO₄), concentrated under reduced pressure, and purified through automated normal-phase chromatography to afford N-(2-benzoyl)-5-chloro-pyridin-3-yl)-4-*tert-*butyl-benzenesulfonamide: ¹H NMR (400 MHz, CDCl₃) δ 10.64 (s, 1H), 8.29 (d, 1H), 8.19 (d, 1H), 7.73 (t, 3H), 7.55 (t, 1H), 7.40 (t, 3H), 7.25 (d, 1H), 1.24 (s, 9H); MS (ES) (M⁺+H) expect 429.1, found 429.1.

### Example 52: N-(2-bromo-5-chloro-pyridin-3-yl)-4-isopropoxy-N-methoxymethyl-benzenesulfonamide

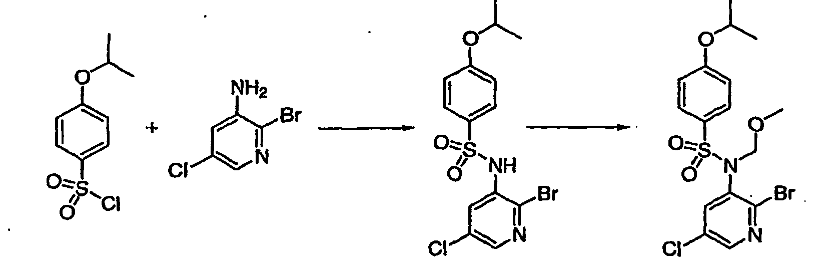

A 200 mL round-bottom flask was charged with 2-bromo-5-chloro-pyridin-3-ylamine (2.06 g, 9.94 mmol), 4-isopropoxysulfonyl chloride (3.5 g, 14.91 mmol), and pyridine (10 mL). The resultant solution was heated to 80 °C and stirred overnight. The following day, the pyridine was removed *in vacuo* and THF (20 mL) and 4.0 N NaOH (30 mL) were added and the reaction was stirred at 60 °C for 72 hours. The organics were subsequently removed *in vacuo* and the residues were diluted with water (100 mL). The small quantity of insoluble solid was removed by filtration and the pH was adjusted to 6-7 with concentrated HCl. The resultant precipitate was filtered and washed with water to afford the diarylsulfonamide (3.44 g) in 85 % yield. To a solution of the crude sulfonamide (3.41 g, 8.41 mmol) and K₂CO₃ (4.65 g, 33.64 mmol) in anhydrous THF (21 mL) was added chloromethyl methyl ether (1.3 mL, 16.81 mmol). The resultant heterogeneous solution was stirred overnight at ambient temperature and the solids were subsequently removed via filtration. The filtrate was then removed *in vacuo* to produce the desired product as a light yellowish solid (3.54 g, 94% yield).

### Example 53: N-[5-chloro-2-(2-methyl-pyridine-3-carbon)-pyridin-3-yl]-4-isopropoxy-benzenesulfonamide

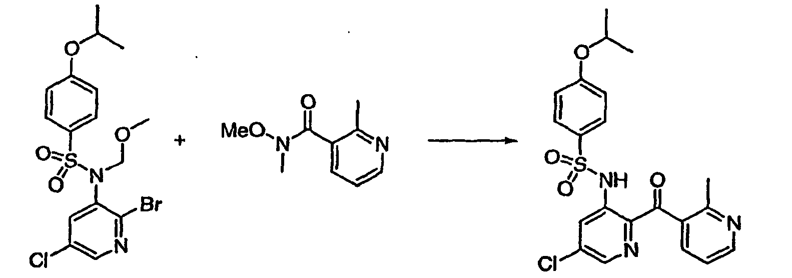

N-(2-bromo-5-chloro-pyridin-3-yl)-4-isopropoxy-N-methoxymethyl-benzenesulfonamide (592 mg, 1.32 mmol) was placed in a dry 25 mL round-bottom flask. The flask was evacuated and purged with nitrogen, followed by the addition of THF (5 mL). The homogeneous mixture was lowered to -5 °C and *i*PrMgCl (1.72 mL, 2.0 M) was added dropwise. Upon completion of the addition, the reaction was stirred 60 minutes, followed by the addition of N-methoxy-2,N-dimethyl-nicotinamide (379 mg, 2.11 mmol). The reaction was stirred overnight, during which the ice-bath warmed to room temperature. The following day, the reaction was quenched with a small quantity of MeOH and the solvents evaporated *in vacuo.* The residue was subsequently treated with 4.0 M HCl in dioxane (6 mL, 24.0 mmol) and H₂O (6.0 mL), and then stirred at 80 °C for two hours. The resultant solution was concentrated *in vacuo* and saturated sodium bicarbonate was added to adjust to neutral pH. The solution was extracted with ethyl acetate, dried over magnesium sulfate, filtered and concentrated *in vacuo.* The crude sulfonamide was finally purified via automated flash chromatography to afford the title compound: ¹H NMR (400 MHz, CDCl₃) δ 10.90 (s, 1H), 8.58 (d, 1H), 8.17 (m, 2H), 7.80 (d, 2H), 7.49 (d, 1H), 7.16 (m, 1H), 6.89 (d, 2H), 4.57 (sep, 1H), 2.34 (s, 3H), 1.33 (d, 6H); MS (ES) M+H expect 445.9, found 446.1.

### Example 54: 4-Chloro-3-methylsulfonylchloride

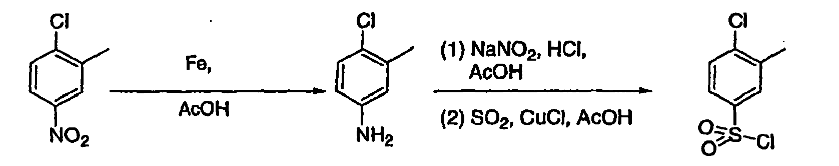

Iron powder (150 g, 2.68 mol) and AcOH (600 mL) were charged into a 2 L round bottom flask equipped with a mechanical stirrer and thermometer. The mixture as warmed to 80 °C. 4-Chloro-3-methylnitrobenzene (150 g, 0.87 mol) in AcOH (200 mL) was added slowly to the flask over 4 h, keeping the reaction temperature below 90 °C. Upon consumption of the reactants, the mixture was filtered through a pad of Celite and the filter cake was washed with methanol (200 mL). The filtrate was concentrated and the residue was poured into 1.5 L of ice water (1:1) and the resulting precipitate was filtered. The crude product was dissolved in 300 mL 6N HCl in water/dioxane (1:1) and stirred at 100 °C for 3 h. It was then cooled to room temperature and the precipitate was filtered and dried to provide 130 g of 4-chloro-3-methyl-phenylamine hydrochloride as a colorless powder.

4-Chloro-3-methylaniline hydrochloride (75 g, 0.54 mol) was dissolved in 200 mL concentrated hydrochloride acid (200 mL) and acetic acid (60 mL). The mixture was cooled to -5 °C and NaNO₂ (40.9 g, 0.59 mmol) was added. The mixture was stirred between -10 °C to -5 °C for 1 h. While the diazotization was in progress, glacial AcOH (600 mL) was placed in a 4000-mL beaker and stirred magnetically. Sulfur dioxide was introduced by a bubbler tube with a fritted end immersed below the surface of the AcOH until saturation was evident. Cuprous chloride (15 g) was added to the solution. The introduction of sulfur dioxide was continued until the yellow-green suspension becomes blue-green. The mixture was then placed in an ice bath and cooled to 10°C. The diazotization reaction mixture was subsequently added in portions over a 30 min period to the sulfur dioxide solution, ensuring the temperature of the solution did not exceed 30 °C. After all the diazonium salt mixture was added, the mixture was poured into ice water (2 L). The resulting precipitate was filtered and redissolved in hexane (500 mL). The mixture was filtered through a pad of silica gel (100 g) and the filter pad was washed with hexane (300 mL). The combined filtrate was concentrated to yield 50 g of 4-chloro-3-methylbenzenesulfonyl chloride as slightly yellow solid.

### Example 55: N-(2-Bromo-5-chloro-pyridin-3-yl)-N-methoxymethyl-3-trifluoromethyl-benzenesulfonamide

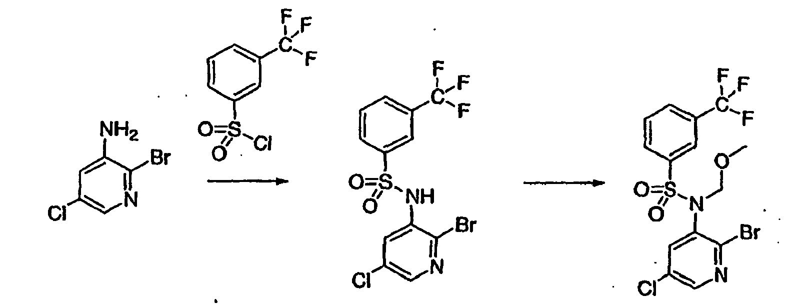

To a stirred solution of 2-bromo-5-chloro-pyridin-3-ylamine (2.07 g, 10.0 mmol) in anhydrous pyridine (20 mL) was added 3-(trifluoromethyl)benzenesulfonyl chloride (4.90 g, 20.0 mmol) and the progress of the reaction was followed by LCMS. The reaction mixture was stirred overnight (18 h), then concentrated to dryness. The residue was dissolved in THF (20 mL) and stirred 18 h with 1M TBAF (20 mL) in THF to cleave the bis-sulfonamide. The THF was subsequently evaporated and the residue was dissolved in ethyl acetate. The organic phase was then washed with water (2 x 100 mL), brine, and then dried (MgSO₄), filtered, and concentrated under reduced pressure. The residue was purified by chromatography on silica gel using ethyl acetate-hexane to provide *N-*(2-Bromo-5-chloro-pyridin-3-yl)-3-trifluoromethyl-benzenesulfonamide as a white crystalline solid. MS *m*/*z* 417.1 (M + H).

Methoxymethyl chloride (720 mg, 8.94 mmol) was added dropwise to a mixture of *N-*(2-bromo-5-chloro-pyridin-3-yl)-3-trifluoromethyl-benzenesulfonamide (2.51 g, 6.04 mmol) and potassium carbonate (5.1 g) in THF (60 mL) at room temperature. After 5 h, the potassium salts removed by vacuum filtration, and the filtrate was concentrated under reduced pressure. The residual light yellow solid was chromatographed on silica gel using ethyl acetate-hexane to provide *N-*(2-bromo-5-chloro-pyridin-3-yl)-*N-*methoxymethyl-3-trifluoromethyl-benzenesulfonamide as white crystals. MS *m*/*z* 461.2 (M + H).

### Example 56: N-(2-Bromo-5-chlora-pyridin-3-yl)-4-chloro-N-methoxymethyl-3-methyl-benzenesulfonamide

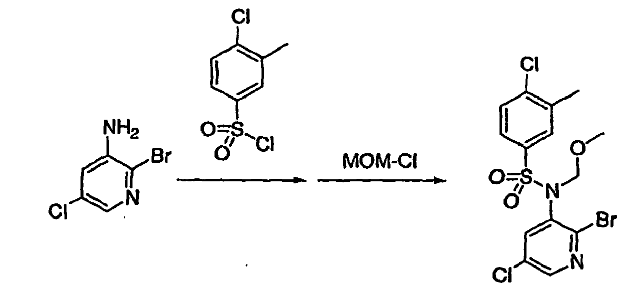

To a stirred solution of 2-bromo-5-chloro-pyridin-3-ylamine (4.12 g, 20 mmol) in anhydrous pyridine (100 mL) was added 3-methyl-4-chlorobenzenesulfonyl chloride (6.35 g, 26 mmol) and the progress of the reaction was followed by LCMS. The reaction mixture was stirred overnight (18 h), then concentrated to remove as much of the pyridine as possible. The residue was dissolved in THF and was stirred 18h with aqueous sodium hydroxide (20 mL) in THF to cleave the bis-sulfonamide. The reaction mixture was subsequently neutralized with aqueous HCl and extracted with EtOAc; the organic phase was washed with water (2 x 100 mL), brine, and then dried (MgSO₄), filtered, and concentrated under reduced pressure. The residue was purified by chromatography on silica gel using EtOAc-hexane to provide *N-*(2-bromo-5-chloro-pyridin-3-yl)-4-chloro-3-methyl-benzenesulfonamide as a white crystalline solid. MS *m*/*z*397.1 (M + H).

Methoxymethyl chloride (1.2 g, 14.3 mmol) was added dropwise to a mixture of *N-*(2-bromo-5-chloro-pyridin-3-yl)-4-chloro-3-methyl-benzenesulfonamide (5.0 g, 12.7 mmol) and potassium carbonate (3.2 g, 23.2 mmol) in THF (30 mL) at room temperature. After 5 h, the potassium salts were removed by vacuum filtration and the filtrate was concentrated under reduced pressure. The residual light yellow solid was chromatographed on silica gel using ethyl acetate-hexane to provide the *N-*(2-bromo-5-chloro-pyridin-3-yl)-4-chloro-*N-*methoxymethyl-3-methyl-benzenesulfonamide as white crystals. MS *m*/*z* 441.2 (M + H).

### Example 57: 4-Chloro-N-[5-chloro-2-(2-ethoxy-benzoyl)-pyridin-3-yl]-N-methoxymethyl-3-trifluoromethyl-benzenesulfonamide

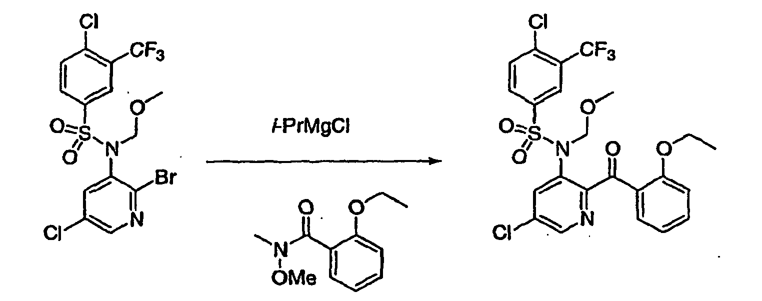

4-Chloro-*N-*[5-chloro-2-(2-ethoxy-benzoyl)-pyridin-3-yl]-*N-*methoxymethyl-3-trifluoromethyl-benzenesulfonamide was prepared from 2-ethoxy-*N-*methoxy-*N-*methyl-benzamide and *N-*(2-bromo-5-chloro-pyridin-3-yl)-4-chloro-*N-*methoxymethyl-3-trifluoromethyl-benzenesulfonamide according to previously described procedure in *example* 24. The product was purified by flash column chromatography on silica gel using ethyl acetate-hexane. MS *m*/*z*: 562.4 (M+H).

### Example 58: 4-Chloro-N-[5-chloro-2-(2-ethoxy-benzoyl)-pyridin-3-yl]-3-trifluoromethyl-benzenesulfonamide

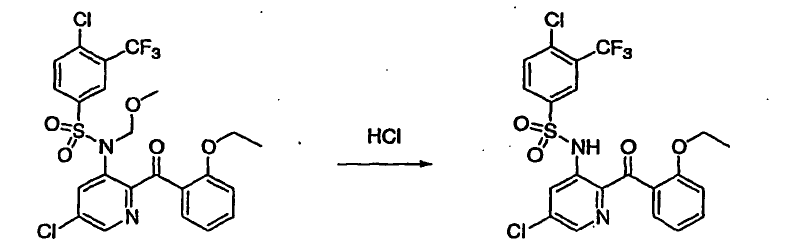

4-Chloro-*N-*[5-chloro-2-(2-ethoxy-benzoyl)-pyridin-3-yl]-*N-*methoxymethyl-3-trifluoromethyl-benzenesulfonamide was hydrolyzed using 4 M HCl in dioxane (12 mL) and water (4 mL) at 100 °C to provide 4-chloro-*N-*[5-chloro-2-(2-ethoxy-benzoyl)-pyridin-3-yl]-3-trifluoromethyl-benzenesulfonamide. Product was purified by HPLC. MS *m*/*z* 519.1 (M+H).

### Example 59: 4-Chloro-N-[5-chloro-2-(2-methoxy-6-methyl-benzoyl)-pyridin-3-yl]-N-methoxymethyl-3-methyl-benzenesulfonamide

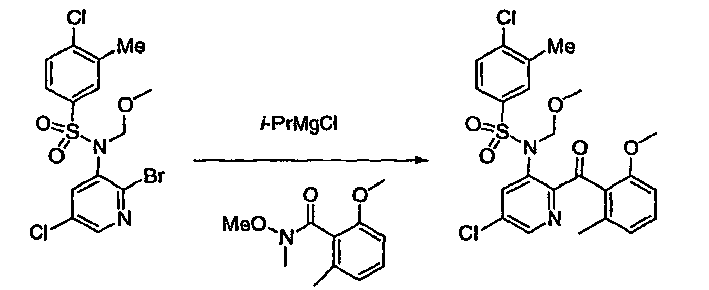

4-Chloro-*N-*[5-chloro-2-(2-methoxy-6-methyl-benzoyl)-pyridin-3-yl]-*N-*methoxymethyl-3-methyl-benzenesulfonamide was prepared from *N-*(2-Bromo-5-chloro-pyridin-3-yl)-4-chloro-*N-*methoxymethyl-3-methyl-benzenesulfonamide and 2,*N-*Dimethoxy-6,*N-*dimethyl-benzamide according to previously described procedure *example* 24. The product was purified by chromatography on silica gel using ethyl acetate-hexane. MS *m*/*z:* 509.4 (M+H).

### Example 60: 4-Chloro-N-[5-chloro-2-(2-methoxy-6-methyl-benzoyl)-pyridin-3-yl]-3-methyl-benzenesulfonamide

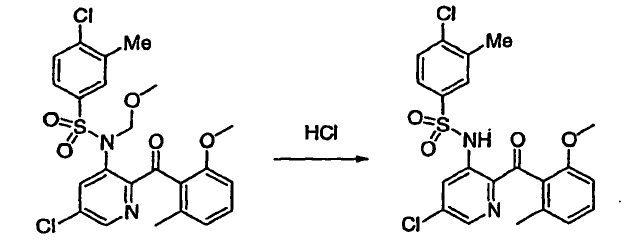

4-Chloro-*N-*[5-chloro-2-(2-methoxy-6-methyl-benzoyl)-pyridin-3-yl]-*N-*methoxymethyl-3-methyl-benzenesulfonamide was hydrolyzed using 4 M HCl in dioxane (12 mL), and water (4 mL) at 100 °C to provide 4-chloro-*N-*[5-chloro-2-(2-methoxy-6-methyl-benzoyl)-pyridin-3-yl]-3-methyl-benzenesulfonamide. The product was purified by HPLC. MS *m*/*z*: 465.4 (M+H).

### Example 61: N-[5-Chloro-2-(2-fluoro-6-methoxy-benzoyl)-pyridin-3-yl]-N-methoxymethyl-3-trifluoromethyl-benzenesulfonamide

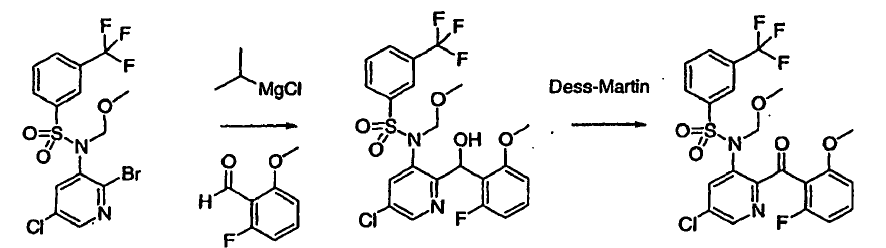

To a stirred solution of *N-*(2-Bromo-5-chloro-pyridin-3-yl)-*N-*methoxymethyl-3-trifluoromethyl-benzenesulfonamide (459 mg, 1.00 mmol) in anhydrous THF (5 mL) at -10 °C was added 2 M isopropylmagnesium chloride in THF (1.1 mL, 2.2 mmol) and stirred at the same temperature for 30 min. 2-Fluoro-6-methoxy-benzaldehyde (308 mg, 2.00 mmol) was subsequently added in one portion, and the reaction mixture was warmed to room temperature and stirred overnight (18 h). It was then quenched with saturated aqueous NH₄Cl (10 mL), and extracted with EtOAc (3 x 75 mL). The combined extracts were washed with aqueous NaHCO₃ and brine, dried (Na₂SO₄), filtered, and concentrated under reduced pressure. The residue was purified by chromatography on silica gel using ethyl acetate-hexane to provide *N-*{5-chloro-2-[(2-fluoro-6-methoxy-phenyl)-hydroxy-methyl]-pyridin-3-yl}-*N-*methoxymethyl-3-trifluoromethyl-benzenesulfonamide. MS *m*/*z* 535.4 (M + H).

A mixture of *N*-{5-chloro-2-[(2-fluoro-6-methoxy-phenyl)-hydroxymethyl]-pyridin-3-yl}-*N-*methoxymethyl-3-trifluoromethyl-benzenesulfonamide (0.38 g) and Dess-Martin reagent (0.90 g, 2.1 mmol) in CH₂Cl₂ (15 mL) at room temperature was stirred for 5 h. A mixture of 10% aqueous Na₂S₂O₃ (10 mL) and saturated aqueous NaHCO₃ (10 mL) was then added and the biphasic mixture vigorously stirred for 30 min. The organic phase was then separated and the aqueous portion was extracted with CH₂Cl₂. The combined organic extracts were washed with saturated aqueous NaHCO₃ and brine, dried (Na₂SO₄), and filtered. The filtrate was concentrated under reduced pressure and the residue purified by chromatography on silica gel using ethyl acetate-hexane to provide *N-*[5-chloro-2-(2-fluoro-6-methoxy-benzoyl)-pyridin-3-yl]-*N-*methoxymethyl-3-trifluoromethyl-benzenesulfonamide. MS *m*/*z* 555.1 (M + Na).

### Example 62: N-[5-Chloro-2-(2-fluoro-6-methoxy-benzoyl)-pyridin-3-yl]-3-trifluoromethyl-benzenesulfonamide

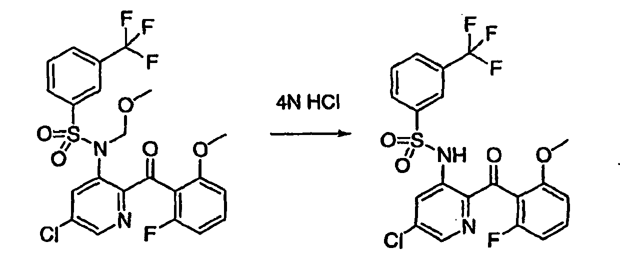

*N-*[5-Chloro-2-(2-fluoro-6-methoxy-benzoyl)-pyridin-3-yl]-*N-*methoxymethyl-3-trifluoromethyl-benzenesulfonamide (180 mg, 0.313mmol) was magnetically stirred in water (1.0 mL) and 4N HCl in dioxane (2.5 mL) and heated at 85 °C for 7 h. The reaction was subsequently concentrated and the residue was neutralized (pH 7) with aqueous sodium bicarbonate and extracted with EtOAc (3 x 80 mL). The extracts were dried (MgSO₄), filtered, and chromatographed on silica gel using EtOAc-hexane (gradient, 0:100 to 50:50) to provide *N-*[5-chloro-2-(2-fluoro-6-methoxy-benzoyl)-pyridin-3-yl]-3-trifluoromethyl-benzenesulfonamide: MS *m*/*z* 489.1 (M + H); 511.0 (M + Na).

### Example 63: 4-Chloro-N-{2-[(2-fluoro-6-methoxyehenyl)-hydroxy-methyl]-5-methyl-pyridin-3-yl}-N-methoxymethyl-3-trifluoromethyl-benzene sulfonamide.

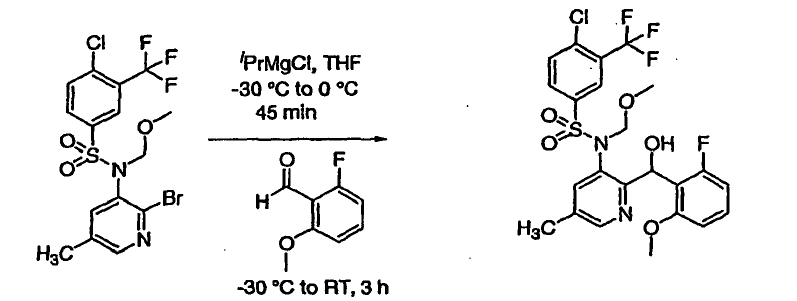

To a solution of *N-*(2-bromo-5-methyl-pyridin-3-yl)-4-chloro-*N-*methoxymethyl-3-trifluoromethyl-benzenesulfonamide (158 mg, 0.33 mmol) in THF (3 mL) under nitrogen atmosphere at -30°C was added isopropylmagnesium chloride (2 M solution in THF, 0.4 mL, 0.8 mmol) via dropwise addition. The mixture was then stirred for 30 min at 0 °C followed by the addition of a solution of 2-fluoro-6-methoxy-benzaldehyde (96.6 mg, 0.63 mmol) at -30 °C. The mixture was stirred at room temperature for 3 h, quenched with saturated aqueous NH₄Cl solution (5 mL), and extracted with EtOAc (2 x 25 mL). The combined organic extracts were washed with saturated aqueous NH₄Cl solution (25 mL) and brine (25 mL), dried (Na₂SO₄), and concentrated under reduced pressure. The obtained residue was purified by flash column chromatography on silica gel (30% EtOAc-hexanes) to obtain 4-chloro-*N-*{2-[(2-fluoro-6-methoxyphenyl)-hydroxy-methyl]-5-methyl-pyridin-3-yl}-*N-*methoxymethyl-3-trifluoromethyl-benzenesulfonamide (110 mg) as yellow syrup in 60% yield. ESMS *m*/*z*: 549 (M+H).

### Example 64: 4-Chloro-N-[2-(2-fluoro-6-methoxy-benzoyl)-5-methyl-pyridin-3-yl]-N-methoxymethyl-3-trifluoromethyl-benzenesulfonamide.

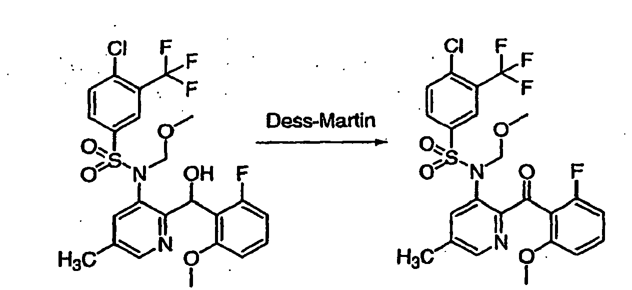

To a solution of 4-chloro-*N-*{2-[(2-fluoro-6-methoxyphenyl)-hydroxymethyl]-5-methyl-pyridin-3-yl}-*N-*methoxymethyl-3-trifluoromethyl-benzenesulfonamide (110 mg, 0.2 mmol) in CH₂Cl₂ (5 mL) was added Dess-Martin periodinane (170 mg, 0.4 mmol) and stirred for 3 h at room temperature. 10% aqueous Na₂S₂O₃ (5 mL) and saturated aqueous NaHCO₃ solution (5 mL) were added and stirred for 30 min. The aqueous layer was then extracted with EtOAc (2 x 25 mL). The combined organic extracts were subsequently washed with saturated aqueous NaHCO₃ solution (20 mL) and brine (20 mL), dried (Na₂SO₄), concentrated to obtain 4-chloro-*N-*[2-(2-fluoro-6-methoxy-benzoyl)-5-methyl-pyridin-3-yl]-*N-*methoxymethyl-3-trifluoro- methyl-benzenesulfonamide (85.4 mg) in 78% yield which was used without further purification. MS *m*/*z*: 515 (M+H-MeOH), 569 (M+Na).

### Example 65: 4-Chloro-N-[2-(2-fluoro-6-methoxy-benzoyl)-5-methyl-pyridin-3-yl]-3-trifluoromethyl-benzenesulfonamide.

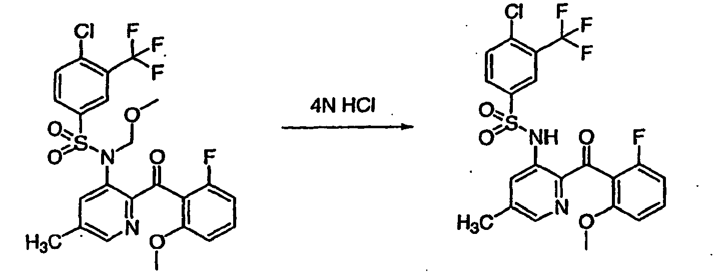

A mixture of 4-chloro-*N-*[2-(2-fluoro-6-methoxy-benzoyl)-5-methyl-pyridin-3-yl]-*N-*methoxymethyl-3-trifluoro-methyl-benzenesulfonamide (85 mg, 0.15 mmol) in 4 M HCl in dioxane (5 mL) and water (1 mL) was refluxed for 3 h. The reaction mixture was cooled to room temperature, evaporated to dryness, and neutralized with saturated aqueous NaHCO₃ solution to pH 7-8. The mixture was extracted with EtOAc (2 x 25 mL), dried (Na₂SO₄), filtered and concentrated. The obtained residue was purified by flash column chromatography on silica gel (70% EtOAc-hexanes) to afford 4-chloro-*N-*[2-(2-fluoro-6-methoxy-benzoyl)-5-methyl-pyridin-3-yl]-3-trifluoromethyl-benzenesulfonamide (55 mg) as an off white solid in 70% yield. MS *m*/*z* 503 (M+H).

### Example 66: 4-Chloro-N-{5-chloro-2-[(2-chloro-6-fluoro-phenyl)-hydroxy-methyl]-pyridin-3-yl}-N-methoxymethyl-3-trifluoromethyl-benzenesulfonamide.

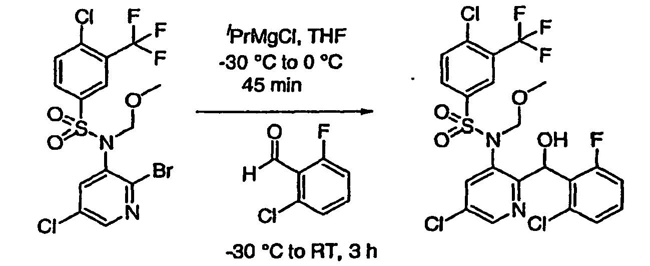

To a solution of *N-*(2-bromo-5-methyl-pyridin-3-yl)-4-chloro-*N-*methoxymethyl-3-trifluoromethyl-benzenesulfonamide (246 mg, 0.5 mmol) in THF (3 mL) under nitrogen atmosphere at -30 °C was added isopropylmagnesium chloride (2 M solution in THF, 0.6 mL, 1.2 mmol) *via* dropwise addition. The mixture was then stirred for 30 min at 0 °C followed by the addition of a solution of 2-chloro-6-fluoro-benzaldehyde (150.6 mg, 0.95 mmol) at -30 °C. The mixture was stirred at room temperature for 3 h, quenched with saturated aqueous NH₄Cl solution (5 mL) and extracted with EtOAc (2 x 25 mL). The combined organic layers were washed with saturated aqueous NH₄Cl solution (25 mL) and brine (25 mL), dried (Na₂SO₄), and concentrated under reduced pressure. The obtained residue was purified by flash column chromatography on silica gel (30% EtOAc-hexanes) to obtain 4-chloro-*N-*{5-chloro-2-[(2-chloro-6-fluoro-phenyl)-hydroxy-methyl]-pyridin-3-yl}-*N-*methoxymethyl-3-trifluoromethyl-benzenesulfonamide (144 mg) as yellow syrup in 51% yield. MS *m*/*z* 573 (M+H).

### Example 67: 4-Chloro-N-[5-chloro-2-(2-chloro-6-fluoro-benzoyl)-pyridin-3-yl]-N-methoxymethyl-3-trifluoromethyl-benzenesulfonamide.

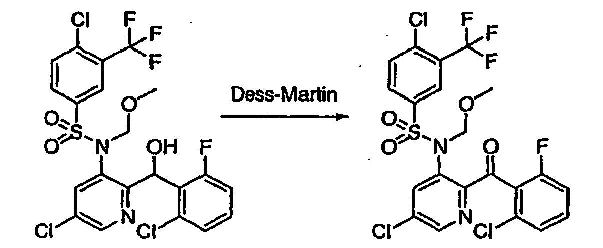

To a solution of 4-chloro-*N-*{5-chloro-2-[(2-chloro-6-fluoro-phenyl)-hydroxy-methyl]-pyridin-3-yl}-*N-*methoxymethyl-3-trifluoromethyl-benzenesulfonamide (144 mg, 0.252 mmol) in CH₂Cl₂ (5 mL) was added Dess-Martin periodinane (213.5 mg, 0.5 mmol) and stirred for 3 h at room temperature. 10% aqueous Na₂S₂O₃ (5 mL) and saturated aqueous NaHCO₃ solution (5 mL) were added to the reaction mixture and stirred for 30 min. The aqueous layer was then extracted with EtOAc (2 x 25 mL). The combined organic extracts were subsequently washed with saturated aqueous NaHCO₃ solution (20 mL) and brine (20 mL), dried (Na₂SO₄), and concentrated to obtain 4-chloro-*N-*[5-chloro-2-(2-chloro-6-fluoro-benzoyl)-pyridin-3-yl]-*N-*methoxymethyl-3-trifluoromethyl-benzenesulfonamide (107 mg) in 75% yield which was used without further purification. MS *m*/*z:* 539 (M-MeOH+H), 593 (M+Na).

### Example 68: 4-chloro-N-[5-chloro-2-(2-chloro-6-fluoro-benzoyl)-pyridin-3-yl]-3-trifluoromethyl-benzenesulfonamide.

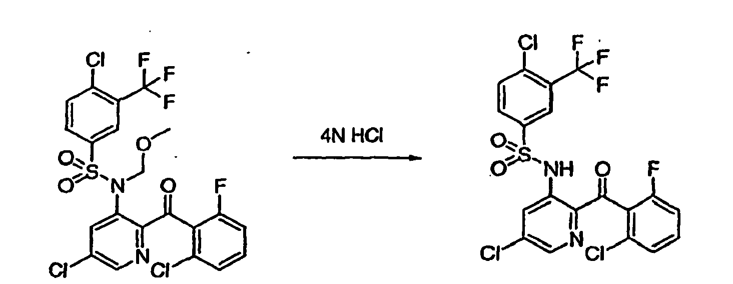

A mixture of 4-chloro-*N-*[5-chloro-2-(2-chloro-6-fluoro-benzoyl)-pyridin-3-yl]-*N-*methoxymethyl-3-trifluoromethyl-benzenesulfonamide (100 mg, 0.17 mmol) in 4 M HCl in dioxane (5 mL) and water (1 mL) was heated at reflux for 3 h. The reaction mixture was cooled to room temperature, evaporated to dryness, and neutralized with saturated aqueous NaHCO₃ solution to pH 7-8. The mixture was extracted with EtOAc (2 x 25 mL), dried (Na₂SO₄), filtered and concentrated. The residual solid was purified by flash column chromatography (70% EtOAc-hexanes) on silica gel to afford 4-chloro-*N-*[5-chloro-2-(2-chloro-6-fluoro-benzoyl)-pyridin-3-yl]-3-trifluoromethyl-benzenesulfonamide (62 mg) as an off white solid in 67% yield. MS *m*/*z*: 527 (M+H), 549 (M+Na).

### Example 69: N-[2-(2-Amino-benzoyl)-5-chloro-pyridin-3-yl]-chloro-N-methoxymethyl-3-trifluoromethyl-benzenesulfonamide

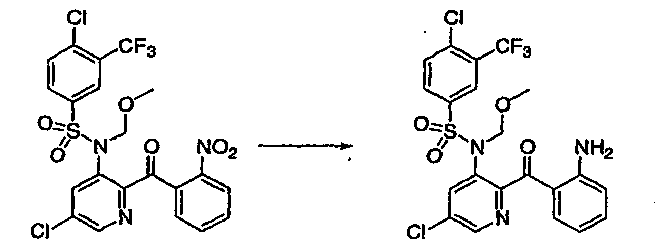

To a stirred suspension of Fe (376 mg, 6.73 mmol) in glacial AcOH (10 mL) at 80°C was added a solution 4-chloro-*N-*[5-chloro-2-(2-nitro-benzoyl)-pyridin-3-yl]-*N-*methoxymethyl-3-trifluoromethyl-benzenesulfonamide (700 mg, 1.34 mmol) in AcOH (5 mL) over 15 min. After complete addition, the mixture was stirred for 2 h and the progress of the reaction was followed by LCMS. Upon consumption of reactants, the reaction mixture was cooled to room temperature, diluted with EtOAc, and filtered through a pad of Celite. The filtrate was concentrated under reduced pressure and residue was partitioned with aqueous NaHCO₃ and EtOAc. The organic portion was separated and the aqueous portion was extracted with EtOAc. The combined extracts were dried (Na₂SO₄), filtered, and concentrated under reduced pressure. The residue was purified by flash column chromatography on silica gel to provide *N-*[2-(2-amino-benzoyl)-5-chloro-pyridin-3-yl]-4-chloro-*N-*methoxymethyl-3-trifluoromethyl-benzenesulfonamide. MS *m*/*z*: 556.0 (M+Na).

### Example 70: 4-Chloro-N-{5-chloro-2-[2-(methanesulfonyl-methyl-amino)-benzoyl]-pyridin-3-yl}-N-methoxymethyl-3-trifluoromethyl-benzenesulfonamide.

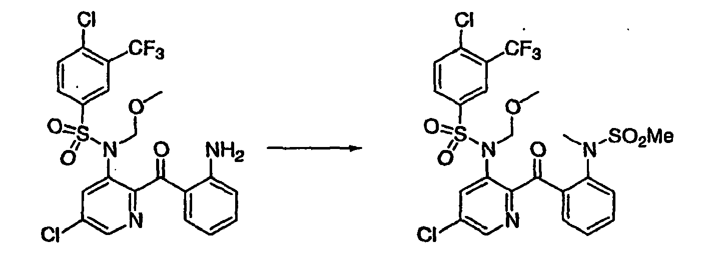

To a stirred suspension of *N-*[2-(2-amino-benzoyl)-5-chloro-pyridin-3-yl]-4-chloro-*N-*methoxymethyl-3-trifluoromethyl-benzenesulfonamide in pyridine (1 mL) was added methanesulfonyl chloride (26 mg, 0.226 mmol). After 2 h the reaction mixture was poured into 1 M HCl and the aqueous portions were subsequently extracted with EtOAc. The combined extracts were dried and concentrated under reduced pressure. The resulting residue was dissolved in THF (5 mL), treated with TBAF (1 M in THF, 200 µL, 0.200 mmol), and then stirred at room temperature for 3-4 h. The reaction mixture was subsequently poured into 1 M HCl and the aqueous layer was extracted with EtOAc. The combined organic extracts were dried (Na₂SO₄), filtered, and concentrated under reduced pressure to provide 4-chloro-*N-*[5-chloro-2-(2-methanesulfonylamino-benzoyl)-pyridin-3-yl]-*N-*methoxymethyl-3-trifluoromethyl-benzenesulfonamide. MS *m*/*z*: 634.0 (M+Na).

A mixture of this crude product, K₂CO₃ (31 mg, 0.226 mmol), and iodomethane (15 µL, 0.226 mmol) in anhydrous DMF (1 mL) were stirred at 50 °C overnight. The resulting mixture was poured into water and extracted with EtOAc. The extracts were concentrated to dryness under reduced pressure to provide 4-chloro-*N-*{5-chloro-2-[2-(methanesulfonyl-methyl-amino)-benzoyl]-pyridin-3-yl}-*N-*methoxymethyl-3-trifluoromethyl-benzenesulfonamide. MS *m*/*z*: 648.3 (M+Na).

### Example 71: 4-Chloro-N-{5-chloro-2-[2-(methanesulfonyl-methyl-amino)-benzoyl]-pyridin-3-yl}-3-trifluoromethyl-benzenesulfonamide.

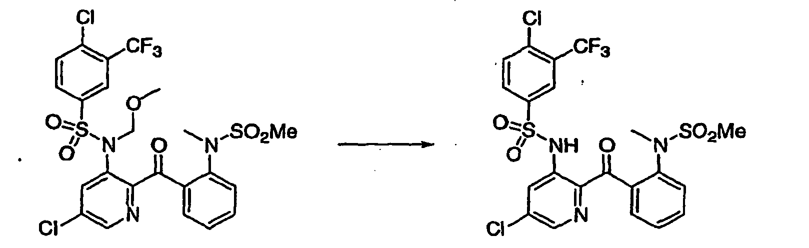

A mixture 4-chloro-*N-*{5-chloro-2-[2-(methanesulfonyl-methylamino)-benzoyl]-pyridin-3-yl}-*N-*methoxymethyl-3-trifluoromethyl-benzenesulfonamide (30 mg crude) in 4 M HCl in dioxane (12 mL) and water (4 mL) were heated at 100 °C for 18 h. The reaction mixture was concentrated to dryness under reduced pressure and subsequently neutralized aqueous NaHCO₃ to pH to 5-6. The aqueous layer was extracted with EtOAc, the combined extracts were dried (Na₂SO₄), filtered, and concentrated under reduced pressure. The residue was purified by preparative HPLC (20 → 80% gradient of MeCN-water) to provide 4-chloro-*N-*{5-chloro-2-[2-(methanesulfonyl-methyl-amino)-benzoyl]-pyridin-3-yl}-3-trifluoromethyl-benzenesulfonamide as a solid. MS *m*/*z*: 582.0 (M+H).

### Example 72: 5-Chloro-2-(2-fluoro-6-methoxy-phenoxy)-pyridin-3-ylamine

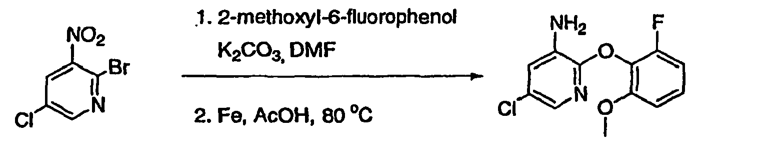

To a stirred solution of 2-bromo-5-chloro-3-nitropyridine (2.37 g, 10 mmol) was dissolved in 40 mL DMF was added of 2-methoxyl-6-fluorophenol (1.7 g 11.9 mmol) was added into the solution followed by potassium carbonate (2.76, 20 mmol). The mixture was stirred at room temperature for 5 h, then poured into ice water (200 mL). The precipitate was filtered and dried under high vacuum to provide 5-chloro-2-(2-fluoro-6-methoxy-phenoxy)-3-nitro-pyridine.

AcOH (30 mL) and iron powder (5 g) were charged into a round bottom flask equipped with a magnetic stirring bar and warmed to 80 °C. A solution of crude 5-chloro-2-(2-fluoro-6-methoxy-phenoxy)-3-nitro-pyridine in AcOH was added slowly into the mixture, keeping the temperature under 85 °C. The reaction mixture was then cooled to room temperature, diluted with EtOAc, and filtered through a pad of Celite. The filtrate was concentrated under reduced pressure and the residue was partitioned between NaHCO₃ and EtOAc. The organic portion was separated and the aqueous portion was extracted with EtOAc. The combined extracts were dried (Na₂SO₄), filtered, and concentrated under reduced pressure. The residue was purified by flash column chromatography on silica gel to provide 5-chloro-2-(2-fluoro-6-methoxyphenoxy)-pyridin-3-ylamine (3.56 g) as a colorless powder.

### General Procedure C: Synthesis of 3-Amino-5-chloro-pyridin-2-yl aryl ketones

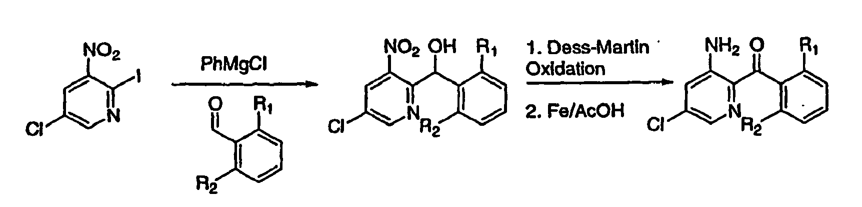

Step 1: To a stirred solution of 5-chloro-2-iodo-3-nitro-pyridine (2.85 g, 10.01 mmol) in anhydrous THF (20 mL) at -78 °C was added 2 M phenylmagnesiumchloride in THF (5.22 mL, 10.5 mmol). The reaction mixture stirred at the same temperature for 30 min and the appropriate aldehyde (15 mmol) was added in one portion (either via syringe if liquid or as solid). and the progress of the reaction was followed by LCMS. The reaction mixture was warmed to room temperature and stirred several hours (5-18 h). It was then quenched with saturated aqueous NH₄Cl (10 mL), and the aqueous layer was extracted with EtOAc. The combined extracts were washed with aqueous NaHCO₃ and brine, dried (Na₂SO₄), filtered, and concentrated under reduced pressure to afford the desired benzylic alcohol which was directly in the following reaction without further purification.

Step 2: A mixture of crude secondary alcohol and Dess-Martin periodinane (6.4 g, 15 mmol) in CH₂Cl₂ (30 mL) at room temperature was stirred for 5-10 h. A mixture of 10% aqueous Na₂S₂O₃ (20 mL) and saturated aqueous NaHCO₃ (20 mL) was then added and the biphasic mixture vigorously stirred for 30 min. The phases were then separated and the aqueous portion extracted with CH₂Cl₂. The combined organic extracts were washed with saturated aqueous NaHCO₃ and brine, dried (Na₂SO₄), and filtered. The filtrate was concentrated under reduced pressure and the residue purified by flash column chromatography over silica gel to provide the corresponding nitro ketone.

Step 3: To a stirred suspension of Fe (4-5 equiv.) in glacial AcOH at 80°C was added a solution of nitropyridyl ketone (1 equiv.) in AcOH (5 mL) over 15 minutes. After complete addition, the mixture was stirred at the same temperature for 1-2 h. The reaction mixture was cooled to room temperature, and diluted with EtOAc and filtered through a pad of Celite. The filtrate was concentrated under reduced pressure and residue was portioned between NaHCO₃ and EtOAc. The organic layer was separated and the aqueous portion was extracted with EtOAc. The combined extracts were dried (Na₂SO₄), filtered, and concentrated under reduced pressure. The residue was purified by flash column chromatography on silica gel to provide the corresponding aniline.

### Example 73: (3-Amino-5-chloro-pyridin-2-yl)-(2-chloro-ohenyl)-methanone

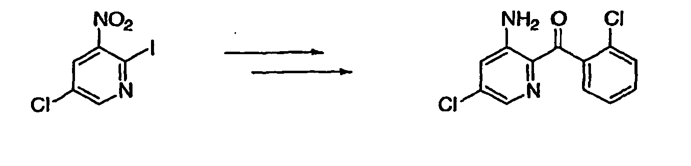

(3-Amino-5-chloro-pyridin-2-yl)-(2-chloro-phenyl)-methanone was prepared from 2-chlorobenzaldehyde and 5-chloro-2-iodo-3-nitro-pyridine in three steps according to the general procedure for synthesis of 3-Amino-5-chloro-pyridin-2-yl aryl ketones. MS *m*/*z*: 267.0 (M+H).

### Example 74: (3-Amino-5-chloro-pyridin-2-yl)-(2-methoxy-phenyl-methanone

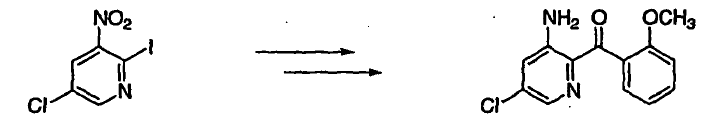

(3-Amino-5-chloro-pyridin-2-yl)-(2-methoxy-phenyl)-methanone was prepared from 2-methoxybenzaldehyde and 5-chloro-2-iodo-3-nitro-pyridine in three steps according to the General Procedure C. MS *m*/*z*: 263.1 (M+H).

### Example 75: (3-Amino-5-chloro-pyridin-2-yl)-(2-fluoro-6-methoxy-phenyl)-methanone

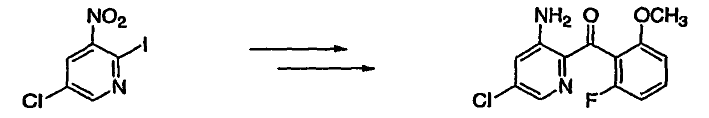

(3-Amino-5-chloro-pyridin-2-yl)-(2-fluoro-6-methoxy-phenyl)-methanone was prepared from 2-fluoro-6-methoxybenzaldehyde and 5-chloro-2-iodo-3-nitro-pyridine in three steps according to the General Procedure C. MS *m*/*z*: 281.0 (M+Na).

### Example 76: 3,4-Dichloro-N-[5-chloro-2-(2-chloro-benzoyl)-pyridin-3-yl]-benzenesulfonamide

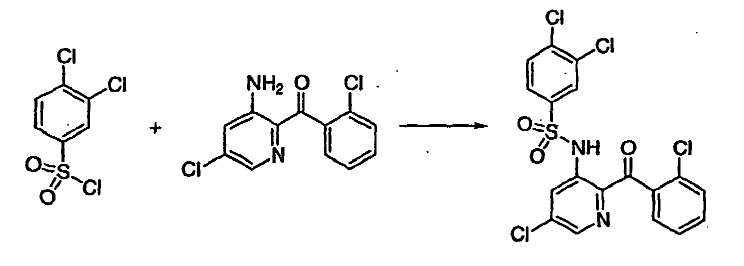

A mixture of (3-amino-5-chloro-pyridin-2-yl)-(2-chloro-phenyl)-methanone (26.8 mg, 0.10 mmol) and 3,4-dichloro-benzenesulfonyl chloride (24.5 mg, 0.10 mmol) in anhydrous pyridine (1 mL) were stirred overnight at 60 °C. Reaction mixture was concentrated and the residue was treated with 1 M NaOH (5 mL) and THF(5 mL) to hydrolyze bis-sulfonamide. The resultant solution was neutralized to pH 6 and the aqueous layer was extracted with EtOAc. The combined organic extracts were washed with brine, dried (Na₂SO₄) and concentrated. The was purified by preparative HPLC (20 → 90% gradient of MeCN-water) and pure product fractions were lyophilized to provide 3,4-dichloro-*N-*[5-chloro-2-(2-chloro-benzoyl)-pyridin-3-yl]-benzenesulfonamide as a solid. MS *m*/*z*: 498.0 (M+Na).

### Example 77: 3,4-Dichloro-N-[5-chloro-2-(2-methoxy-benzoyl)-pyridin-3-yl]-benzenesulfonamide

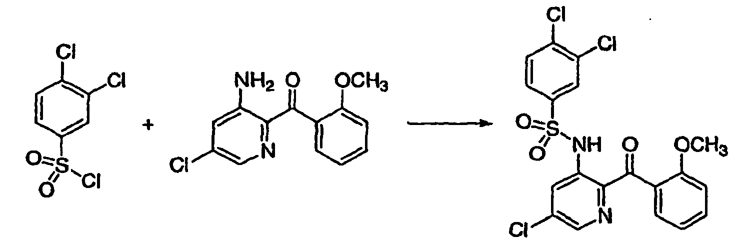

Following the procedure In example 76, 3,4-dichloro-*N-*[5-chloro-2-(2-methoxy-benzoyl)-pyridin-3-yl]-benzenesulfonamide was synthesized from (3-amino-5-chloro-pyridin-2-yl)-(2-methoxy-phenyl)-methanone (54 mg, 0.206 mmol) and 3,4-dichloro-benzenesulfonyl chloride (101 mg, 0.412 mmol). MS *m*/*z:* 471.0 (M+H).

### Example 78: 3,4-Dichloro-N-[5-chloro-2-(2-fluoro-6-methoxy-benzoyl)pyridin-3-yl]-benzenesulfonamide

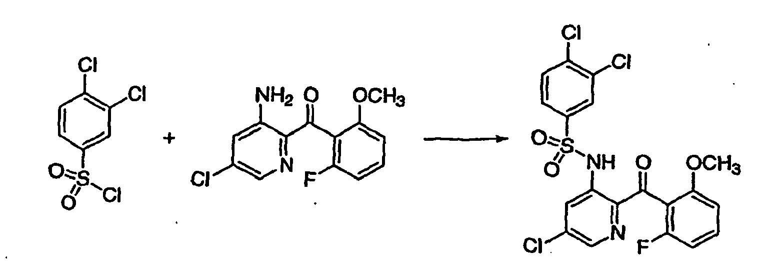

Following the procedure in example 76, 3,4-dichloro-*N-*[5-chloro-2-(2-fluoro-6-methoxy-benzoyl)-pyridin-3-yl]-3-benzenesulfonamide was synthesized from (3-amino-5-chloro-pyridin-2-yl)-(2-fluoro-6-methoxy-phenyl)-methanone (54 mg, 0.192 mmol) and 3,4-dichloro-benzenesulfonyl chloride (87 mg, 0.368 mmol). MS *m*/*z*: 510.9 (M+Na).

### Example 79: 4-Chloro-N-[5-chloro-2-(2-fluoro-6-methoxy-benzoyl)-pyridin-3-yl]-3-methyl-benzenesulfonamide

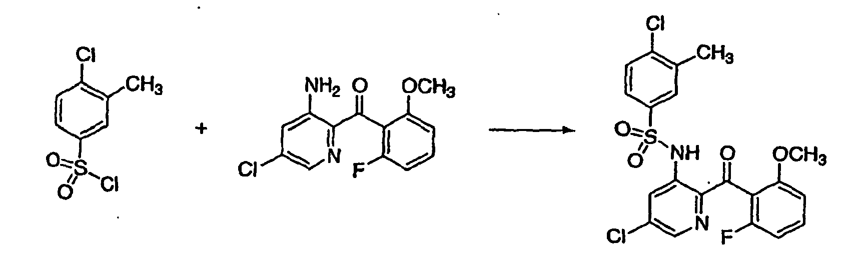

Following the procedure in example 76, 4-chloro-*N-*[5-chloro-2-(2-fluoro-6-methoxy-benzoyl)-pyridin-3-yl]-3-methyl-benzenesulfonamide was synthesized from (3-amino-5-chloro-pyridin-2-yl)-(2-fluoro-6-methoxy-phenyl)-methanone (56 mg, 0.20 mmol) and 4-Chloro-3-methyl-benzenesulfonyl chloride (87 mg, 0.368 mmol). MS *m*/*z*: 491.0 (M+Na).

### Example 80: 4-Chloro-N-[5-chloro-2-(2-fluoro-6-methoxy-benzoyl)-pyridin-3-yl]-3-methyl-benzenesulfonamide

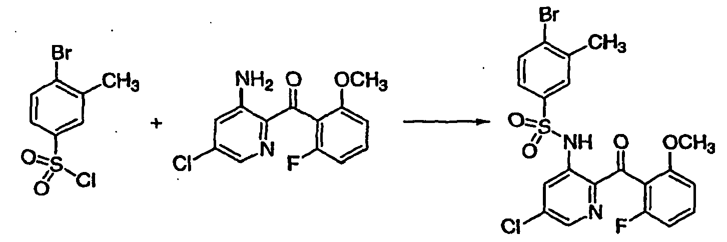

Following the procedure in example 76, 4-chloro-*N-*[5-chloro-2-(2-fluoro-6-methoxy-benzoyl)-pyridin-3-yl]-3-methyl-benzenesulfonamide was synthesized from (3-amino-5-chloro-pyridin-2-yl)-(2-fluoro-6-methoxy-phenyl)-methanone (66 mg, 0.20 mmol) and 4-bromo-3-methyl-benzenesulfonyl chloride (120 mg, 0.448 mmol). MS *m*/*z*: 491.0 (M+Na).

### Example 81: 2-Chloro-N-[5-chloro-2-(2-fluoro-6-methoxy-benzoyl)-pyridin-3-yl]-benzenesulfonamide

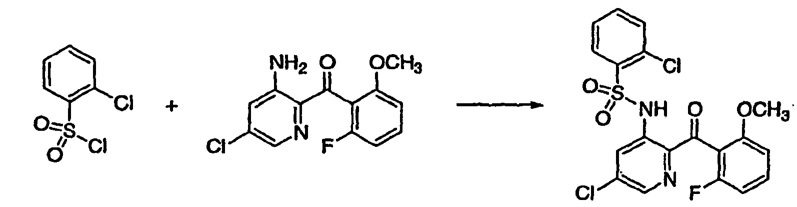

Following the procedure in example 76, 2-chloro-*N-*[5-chloro-2-(2-fluoro-6-methoxy-benzoyl)-pyridin-3-yl]-benzenesulfonamide was synthesized from (3-amino-5-chloro-pyridin-2-yl)-(2-fluoro-6-methoxy-phenyl)-methanone (50 mg, 0.178 mmol) and 2-chloro-benzenesulfonyl chloride (83 mg, 0.392 mmol). MS *m*/*z*: 455.0 (M+H).

### Example 82: 4-Chloro-N-[5-chloro-2-(2-fluoro-6-methoxy-phenoxy-pyridin-3-yl[-3-trifluoromethyl-benzenesulfonamide

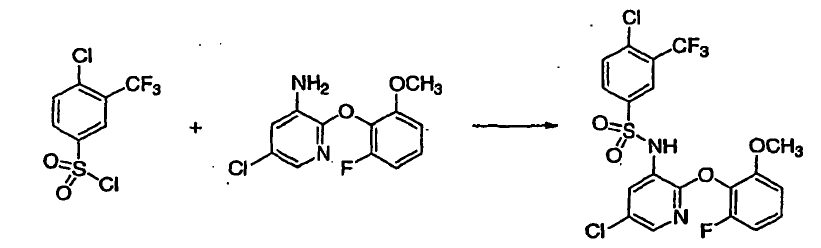

Following the procedure in example 76, 4-chloro-*N-*[5-chloro-2-(2-fluoro-6-methoxy-phenoxy)-pyridin-3-yl]-3-trifluoromethyl-benzenesulfonamide was synthesized from 5-chloro-2-(2-fluoro-6-methoxy-phenoxy)-pyridin-3-ylamine (100 mg), 4-chloro-3-trifluoromethyl-benzenesulfonyl chloride (100 mg). MS *m*/*z*: 511.1 (M+H).

### Example 83: 5-Chloro-3-[(4-chloro-3-trifluoromethyl-benzenesulfonyl)-methoxymethyl-amino]-pyridine-2-carboxylic acid

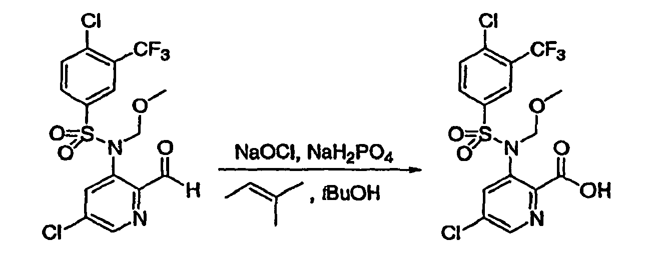

To a solution of 4-chloro-*N-*(5-chloro-2-formyl-pyridin-3-yl)-*N-*methoxymethyl-3-trifluoromethyl-benzenesulfonamide (590 mg, 1.33 mmol) in *tert-*butanol (26 mL) was added isobutylene (5 mL), sodium chlorite (751 mg, 6.66 mmol) sodium dihydrogen phosphate (919mg, 6.66 mmol) and water (26 mL). After the solution was stirred at room temperature for 3 h, EtOAc(10 mL) was added and the layers were separated. The organic layer was washed with saturated sodium bicarbonate (2 x 10 mL) and brine (1 x 10 mL), dried over magnesium sulfate, filtered, and concentrated in *vacuo* to yield 5-chloro-3-[(4-chloro-3-trifluoromethyl-benzenesulfonyl)-methoxymethyl-amino]-pyridine-2-carboxylic acid as a white solid.

### Example 84: 5-Chloro-3-(4-chloro-3-trifluoromethyl-benzenesulfonylamino)-pyridine-2-carboxylic acid isopropyl-phenyl-amide

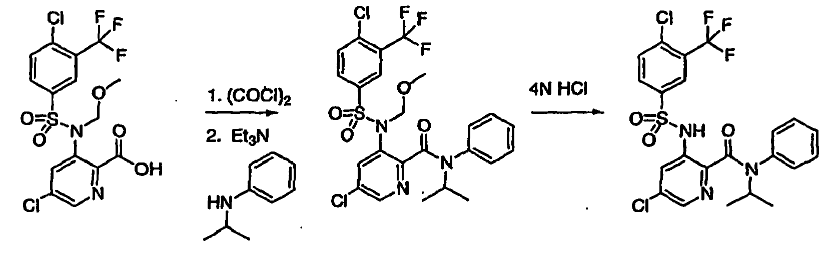

To a magnetically stirred solution of 5-chloro-3-[(4-chloro-3-trifluoromethyl-benzenesulfonyl)-methoxymethyl-amino]-pyridine-2-carboxylic acid (229 mg, 0.50 mmol) in anhydrous methylene chloride (5 mL) was added oxalyl chloride (1.0 mL) at room temperature. The reaction was heated at reflux for 2 h, then concentrated to dryness. The residue was dissolved in methylene chloride (4 mL) and this solution was added dropwise to a magnetically stirred solution of triethylamine (1.0 mL) and *N-*isopropylaniline (135 mg, 1.00 mmol) in methylene chloride (2 mL). The reaction mixture was stirred 1 h (the reaction was monitored by LCMS), then quenched with saturated aqueous NH₄Cl (50 mL), and the aqueous layer extracted with EtOAc (3 x 75 mL). The combined extracts were washed with 0.5 M HCl (50 mL), washed with water (50 mL), dried (MgSO₄), filtered, and concentrated. The residue was purified by chromatography on silica gel using a gradient of ethyl acetate-hexane (0:100 to 100:0) to afford pure 5-chloro-3-[(4-chloro-3-trifluoromethyl-benzenesulfonyl)-methoxymethyl-amino]-pyridine-2-carboxylic acid N-isopropyl-*N-*phenylamide. MS *m*/*z* 576.0 (M + H); 544.0 (M+H - MeOH).

The intermediate MOM derivative (180 mg, 0.313 mmol) was magnetically stirred in water (1.0 mL) and 4N HCl in dioxane (2.5 mL) at 85 °C (oil bath) for 7 h. Upon consumption of the reactant, the reaction mixture was concentrated, the residue neutralized (pH 7) with aqueous sodium bicarbonate, and the aqueous layer extracted with EtOAc (3 x 80 mL). The combined organic extracts were dried (MgSO₄), filtered, and chromatographed on silica gel using ethyl acetate-hexane gradient (0:100 to 50:50) to provide 5-chloro-3-(4-chloro-3-trifluoromethyl-benzenesulfonylamino)-pyridine-2-carboxylic acid isopropyl-phenyl-amide. MS *m*/*z* 532.0 (M + H).

### Example 85: 5-Chloro-3-(4-chloro-3-trifluoromethyl-benzenesulfinylamilno)-pyridine-2-carboxylic acid phenylamide

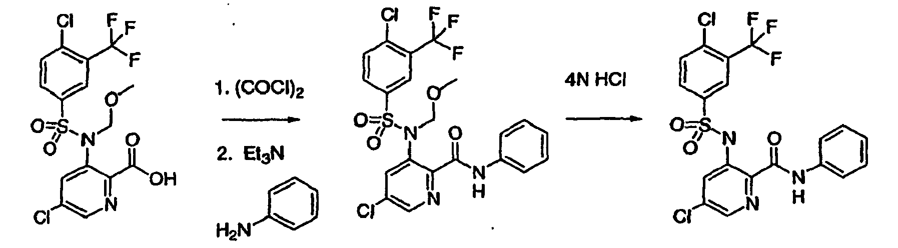

To a magnetically stirred solution of 5-chloro-3-[(4-chloro-3-trifluoromethyl-benzenesulfonyl)-methoxymethyl-amino]-pyridine-2-carboxylic acid (229 mg, 0.50 mmol) in anhydrous methylene chloride (5 mL) was added oxalyl chloride (0.5 mL) at room temperature. The reaction was heated at reflux for 1 h, then concentrated to dryness. The residue was dissolved in methylene chloride (4 mL) and this solution was added dropwise to a magnetically stirred solution of triethylamine (1.0 mL) and aniline (93 mg, 1.00 mmol) in methylene chloride (2 mL). The reaction mixture was stirred 1 h (the reaction was monitored by LCMS), quenched with saturated aqueous NH₄Cl (50 mL), and the aqueous layer was extracted with EtOAc (3 x 75 mL). The combined extracts were washed with 0.5 M HCl (50 mL) and water (50 mL), dried (MgSO₄), filtered, and concentrated. The residue was purified by chromatography on silica gel using a gradient of ethyl acetate-hexane (0:100 to 100:0) to afford pure 5-chloro-3-[(4-chloro-3-trifluoromethyl-benzenesulfonyl)-methoxymethyl-amino]-pyridine-2-carboxylic acid phenylamide. MS *m*/*z* 556.1 (M + Na); 502.1 (M+H-MeOH).

5-Chloro-3-[(4-chloro-3-trifluoromethyl-benzenesulfonyl)-methoxymethyl-amino]-pyridine-2-carboxylic acid phenylamide (120 mg, 0.224 mmol) was magnetically stirred in water (1.0 mL) and 4N HCl in dioxane (2.5 mL) at 85 °C (oil bath) for 4 h. Upon consumption of the reactant, the reaction mixture was concentrated, the residue neutralized (pH 7) with aqueous sodium bicarbonate, and the aqueous layer was extracted with EtOAc (3 x 80 mL). The combined organic extracts were dried (MgSO₄), filtered, and chromatographed on silica gel using ethyl acetate-hexane gradient (0:100 to 50:50) to provide pure final product.MS *m*/*z* 490.0 (M + H).

### Example 86: 5-Chloro-3-(4-chloro-3-trifluoromethyl-benzenesulfonylamino)-pyridine-2-carboxylic acid N-methyl-N-phenylamide

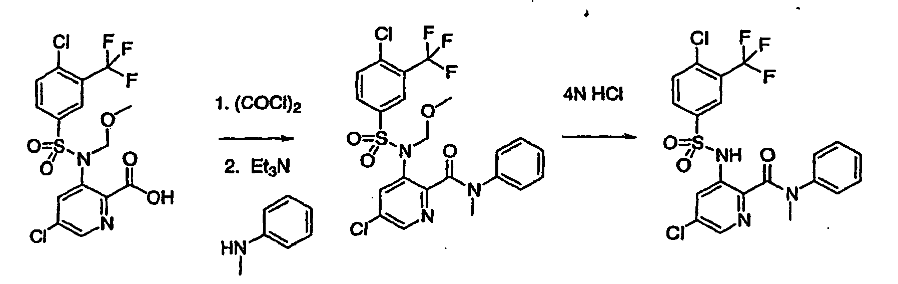

To a magnetically stirred solution of 5-chloro-3-[(4-chloro-3-trifluoromethyl-benzenesulfonyl)-methoxymethyl-amino]-pyridine-2-carboxylic acid (458mg, 1.00 mmol) in anhydrous methylene chloride (5 mL) was added oxalyl chloride (1.0 mL) at room temperature. The reaction was heated at reflux for 2 h, then concentrated to dryness. The residue was dissolved in methylene chloride (4 mL) and this solution was added dropwise to a magnetically stirred solution of triethylamine (1.0 mL) and N-methylaniline (108 mg, 1.00 mmol) in methylene chloride (2 mL). The reaction mixture was stirred 1 h (the reaction was monitored by LCMS), quenched with saturated aqueous NH₄Cl (50 mL), and the aqueous layer was extracted with EtOAc (3 x 75 mL). The combined extracts were washed with 0.5 M HCl (50 mL), washed with water (50 mL), dried (MgSO₄), filtered and concentrated. The residue was purified by chromatography on silica gel using a gradient of ethyl acetate-hexane (0:100 to 100:0) to afford pure 5-chloro-3-[(4-chloro-3-trifluoromethyl-benzenesulfonyl)-methoxymethylamino]-pyridine-2-carboxylic acid *N-*methyl-*N-*phenylamide. MS *m*/*z*:548.2 (M + H), 526.0 (M+H-MeOH).

5-Chloro-3-[(4-chloro-3-trifluoromethyl-benzenesulfonyl)-methoxymethyl-amino]-pyridine-2-carboxylic acid *N-*methyl-*N-*phenylamide (132 mg, 0.24mmol) was magnetically stirred in water (1.0 mL) and 4N HCl in dioxane (2.5 mL) and heated at 85°C (oil bath) for 7 h. LCMS indicated complete reaction; the reaction was concentrated and the residue was neutralized (pH 7) with aqueous sodium bicarbonate and the aqueous layer was extracted with EtOAc (3 x 80 mL). The extracts were dried (MgSO₄), filtered, and chromatographed on silica gel using ethyl acetate-hexane gradient (0:100 to 50:50) to provide 5-chloro-3-(4-chloro-3-trifluoromethyl-benzenesulfonylamino)-pyridine-2-carboxylic acid *N-*methyl-*N-*phenylamide. MS *m*/*z:* 504.1 (M + H).

### Example 87: 5-Chloro-3-(4-chloro-3-tifluoromethyl-benzenesulfonylamino)-pyridine-2-carboxylic acid N-methyl-N-(4-fluorophenyl)amide

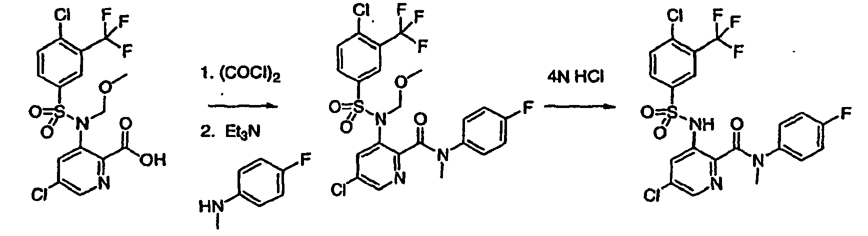

To a magnetically stirred solution of 5-chloro-3-[(4-chloro-3-trifluoromethyl-benzenesulfonyl)-methoxymethyl-amino]-pyridine-2-carboxylic acid (458 mg, 1.00 mmol) in anhydrous methylene chloride (5 mL) was added oxalyl chloride (1.0 mL) at room temperature. The reaction was heated at reflux for 1h then was concentrated to dryness. The residue was dissolved in methylene chloride (4 mL) and this solution was added dropwise to a magnetically stirred solution of triethylamine (1.0 mL) and N-methyl-*N-*(4-fluorophenyl)aniline (0.50 mL, 3.5 mmol) in methylene chloride (2 mL). The reaction mixture was stirred 1 h (the reaction was monitored by LCMS) then quenched with saturated aqueous NH₄Cl (50 mL), and extracted with EtOAc (3 x 75 mL). The combined extracts were washed with 0.5M HCl (50 mL), washed with water (50 mL), dried (MgSO₄), filtered and concentrated. The residue was purified by chromatography on silica gel using a gradient of ethyl acetate-hexane (0:100 to 100:0) to afford pure 5-chloro-3-[(4-chloro-3-trifluoromethyl-benzenesulfonyl)-methoxymethyl-amino]-pyridine-2-carboxylic acid *N-*methyl-*N-*(4-fluorophenyl)amide. MS *m*/*z* 570.1(M + Na); 526.0 (M+H-MeOH).

5-Chloro-3-[(4-chloro-3-trifluoromethyl-benzenesulfonyl)-methoxymethyl-amino]-pyridine-2-carboxylic acid N-methyl-*N-*(4-fluorophenyl)amide (141 mg, 0.258 mmol) was magnetically stirred in water (1.0 mL) and 4N HCl in dioxane (2.5 mL) and heated at 85 °C (oil bath) for 7 h. LCMS indicated complete reaction; the reaction was concentrated and the residue was neutralized (pH 7) with aqueous sodium bicarbonate and extracted with EtOAc (3 x 80 mL). The extracts were dried (MgSO₄), filtered, and chromatographed on silica gel using ethyl acetate-hexane gradient (0:100 to 50:50) to provide 5-chloro-3-(4-chloro-3-trifluoromethyl-benzenesulfonylamino)-pyridine-2-carboxylic acid *N-*methyl-*N-*(4-fluorophenyl)amide. MS *m*/*z* 522.1 (M + H).

### Example 88: 5-Chloro-3-(4-chloro-3-trifluoromethyl-benzenesulfonylamino)-pyridine-2-carboxylic acid 2-isopropylaminopyridin-2-yl amide

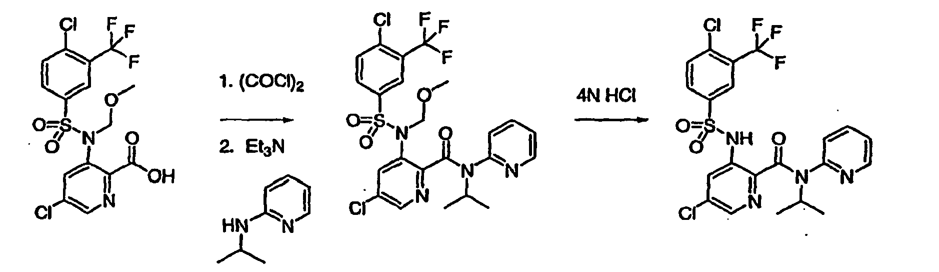

To a magnetically stirred solution of 5-chloro-3-[(4-chloro-3-trifluoromethyl-benzenesulfonyl)-methoxymethyl-amino]-pyridine-2-carboxylic acid (229 mg, 0.50 mmol) in anhydrous methylene chloride (5 mL) was added oxalyl chloride (0.5 mL) at room temperature. The reaction was heated at reflux for 1 h then was concentrated to dryness. The residue was dissolved in methylene chloride (4 mL) and this solution was added dropwise to a magnetically stirred solution of triethylamine (1.0 mL) and 2-(isopropylamino)pyridine (136 mg, 1.00 mmol) in methylene chloride (2 mL). The reaction mixture was stirred 1 h (the reaction was monitored by LCMS) then quenched with saturated aqueous NH₄Cl (50 mL), and extracted with EtOAc (3 x 75 mL). The combined extracts were washed with 0.5M HCl (50 mL), washed with water (50 mL), dried (MgSO₄) filtered and concentrated. The residue was purified by chromatography on silica gel using a gradient of ethyl acetate-hexane (0:100 to 100:0) to afford pure 5-chloro-3-[(4-chloro-3-trifluoromethyl-benzenesulfonyl)-methoxymethyl-amino]-pyridine-2-carboxylic acid 2-(isopropylamino)pyridylamide. MS *m*/*z* 577.1 (M + H).

5-Chloro-3-[(4-chloro-3-trifluoromethyl-benzenesulfonyl)-methoxymethyl-amino]-pyridine-2-carboxylic acid 2-(isopropylamino)pyridylamide (120 mg, 0.208 mmol) was magnetically stirred in water (1.0 mL) and 4N HCl in dioxane (2.5 mL) and heated at 85 °C (oil bath) for 9 h. LCMS indicated complete reaction; the reaction was concentrated and the residue was neutralized (pH 7) with aqueous sodium bicarbonate and extracted with EtOAc (3 x 80 mL). The extracts were dried (MgSO₄), filtered, and chromatographed on silica gel using ethyl acetate-hexane gradient (0:100 to 50:50) to provide 5-chloro-3-(4-chloro-3-trifluoromethyl-benzenesulfonylamino)-pyridine-2-carboxylic acid 2-isopropylaminopyridin-2-yl amide. MS *m*/*z*: 533.1 (M + H).

### Example 89: 4-Chloro-N-[5-chloro-2-(2,3-dihydro-benzo[1,4]oxazine-4-carbonyl)-pyridin-3-yl]-N-methoxymethyl-3-trifluoromethyl-benzenesulfonamide

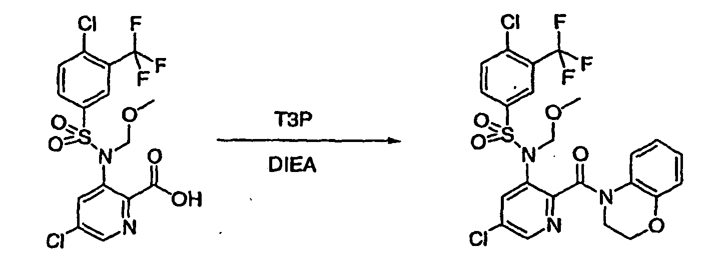

To a mixture of 5-chloro-3-[(4-chloro-3-trifluoromethyl-benzenesulfonyl)-methoxymethyl-amino]-pyridine-2-carboxylic acid (112 mg, 0.244 mmol), 100 mg (0.70 mmol) of 3,4-dihydro-2H-benzo[1,4]oxazine, and 0.174 mL (1.0 mmol) of DIEA in CH₂Cl₂ (2.5 mL) was added 0.19 mL 1-propanephosphonic acid cyclic anhydride (50% in ethyl acetate). After 3 h the reaction mixture was directly purified via flash column (50% EtOAc in hexane) to afford 79 mg of 4-chloro-*N-*[5-chloro-2-(2,3-dihydro-benzo[1,4]oxazine-4-carbonyl)-pyridin-3-yl]-*N-*methoxymethyl-3-trifluoromethyl-benzenesulfonamide as a white powder. MS *m*/*z*: 576.38 (M + H).

### Examele 90: 4-Chloro-N-[5-chloro-2-(2,3-dihydro-benzo[1,4]oxazine-4-carbonyl)-pyridin-3-yl]-3-trifluoromethyl-benzenesulfonamide

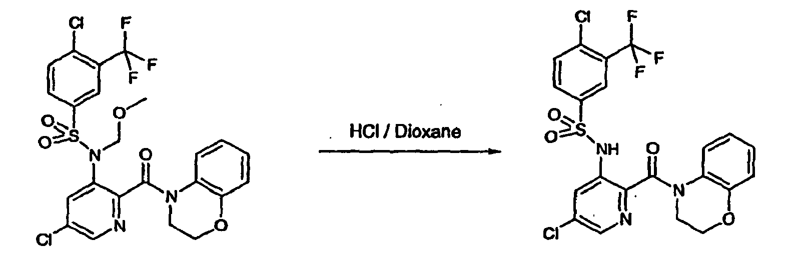

A solution of 4-chloro-*N-*[5-chloro-2-(2,3-dihydro-benzo[1,4]oxazine-4-carbonyl)-pyridin-3-yl]-*N-*methoxymethyl-3-trifluoromethyl-benzenesulfonamide (50 mg) in 3 mL HCl (4 M in dioxane) and water (1 mL) was refluxed for 2 h. Upon cooling to room temperature, the mixture was concentrated and the residue was purified via preparative TLC (50% EtOAc in hexane) to afford 4-chloro-*N-*[5-chloro-2-(2,3-dihydro-benzo[1,4]oxazine-4-carbonyl)-pyridin-3-yl]-3-trifluoromethyl-benzenesulfonamide (27.0 mg) as an off white solid. MS *m*/*z:* 532.2 (M + H).

### Example 91: 4-Chloro-N-[5-chloro-2-(3-methyl-morpholine-4-carbony)pyridin-3-yl]-N-methoxymethyl-3-trifluoromethyl-benzenesulfonamide

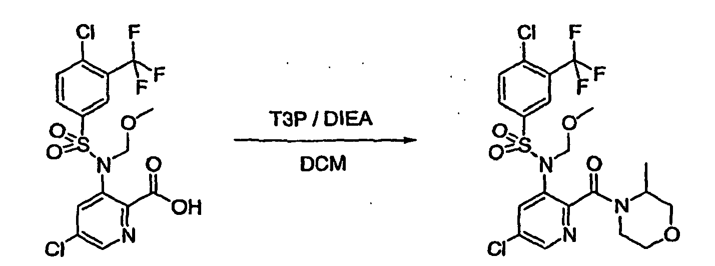

To a mixture of 112 mg (0.244 mmol) of 5-chloro-3-[(4-chloro-3-trifluoromethyl-benzenesulfonyl)-methoxymethyl-amino]-pyridine-2-carboxylic acid, 70 mg (0.70 mmol) of 2-methylmorpholine and 0.174 mL (1.0 mmol) of DIEA in CH₂Cl₂ (2.5 mL) was added 0.19 mL 1-propanephosphonic acid cyclic anhydride (50% in ethyl acetate). After 3 h the reaction mixture was directly purified via flash column (50% EtOAc in hexane) to afford 67 mg of 4-chloro-*N-*[5-chloro-2-(3-methyl-morpholine-4-carbonyl)-pyridin-3-yl]-*N-*methoxymethyl-3-trifluoromethyl-benzenesulfonamide as a white powder. MS *m*/*z*: 542.36 (M + H).

### Example 92: 4-Chloro-N-[5-chloro-2-(3-methyl-morpholine-4-carbonyl)-pyridin-3-yl]-3-trifluoromethyl-benzenesulfonamide

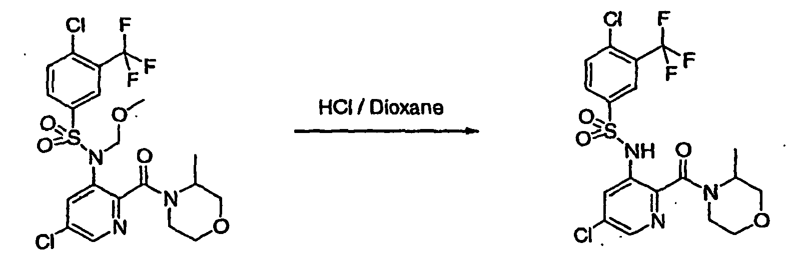

A solution of 4-chloro-*N-*[5-chloro-2-(3-methyl-morpholine-4-carbonyl)-pyridin-3-yl]-*N-*methoxymethyl-3-trifluoromethyl-benzenesulfonamide (40mg) in 3 mL HCl (4M in dioxane) and water (1 mL) was refluxed for 2 h. Upon Cooling to room temperature, the mixture was concentrated and the residue was purified via preparative TLC (50% EtOAc in hexane) to afford 21 mg of 4-chloro-*N-*[5-chloro-2-(3-methyl-morpholine-4-carbonyl)-pyridin-3-yl]-3-trifluoromethyl-benzenesulfonamide as an off white solid. MS *m*/*z*: 498.0 (M + H).

### Example 93: 5-Chloro-3-[(4-chloro-3-trifluoromethyl-benzenesulfonyl)-methoxymethyl-amino]-pyridine-2-carbonyl chloride

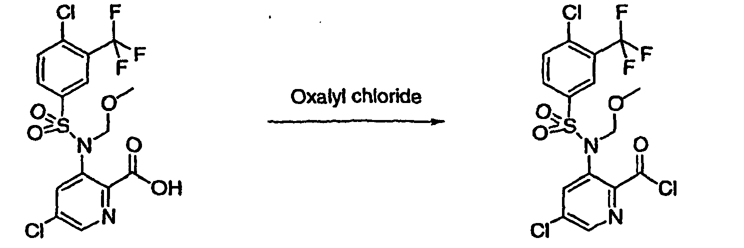

To a solution of 5-chloro-3-[(4-chloro-3-trifluoromethyl-benzenesulfonyl)-methoxymethyl-amino]-pyridine-2-carboxylic acid (450 mg, 1.0 mmol) in 3.0 mL CH₂Cl₂ and DMF (0.02 mL) was added oxalyl chloride (0.110 mL). The mixture was stirred for 2 h and concentrated to dryness provide 5-chloro-3-[(4-chloro-3-trifluoromethyl-benzenesulfonyl)-methoxymethyl-amino]-pyridine-2-carbonyl chloride.

### Example 94: 4-Chloro-N-[5-chloro-2-(3-methyl-2,3-dihydro-benzo[1,4]oxazine-4-carbonyl)-pyridin-3-yl]-N-methoxymethyl-3-trifluoromethyl-benzenesulfonamide

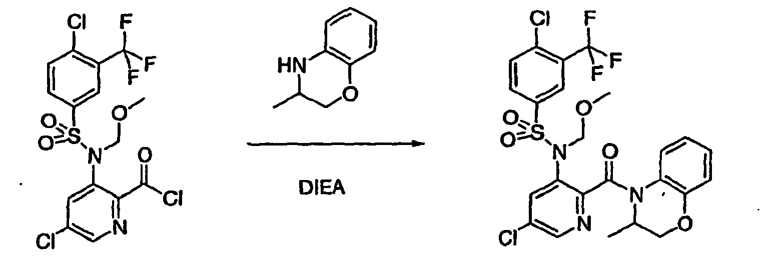

A mixture of 5-chloro-3-[(4-chloro-3-trifluoromethyl-benzenesulfonyl)-methoxymethyl-amino]-pyridine-2-carbonyl chloride (50 mg, 0.11 mmol), 3-sethyl-3,4-dihydro-2H-benzo[1,4]oxazine (60 mg, 0.40 mmol) and di-isopropylethylamine (DIEA, 0.07 mL, 0.40 mmol) in CH₂CL₂ (1.5 mL) was stirred at room temperature overnight. The mixture was concentrated to provide 4-chloro-*N-*[5-chloro-2-(3-methyl-2,3-dihydro-benzo[1,4]oxazine-4-carbonyl)-pyridin-3-yl]-*N-*methoxymethyl-3-trifluoromethyl-benzenesulfonamide, which was directly used in the next step without any purification.

### Example 95: 4-Chloro-N-[5-chloro-2-(2,6-dimethyl-morpholine-4-carbonyl)-pyridin-3-yl]-N-methoxymethyl-3-trifluoromethyl-benzenesulfonamide

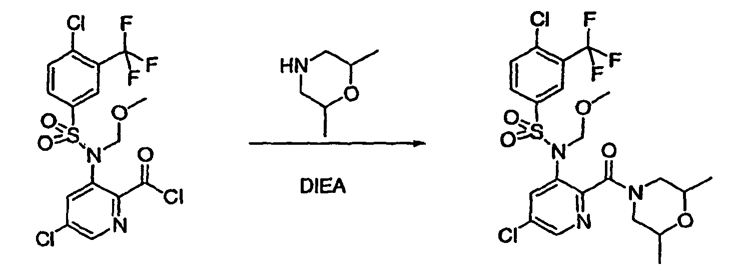

A mixture of 5-chloro-3-[(4-chloro-3-trifluoromethyl-benzenesulfonyl)-methoxymethyl-amino]-pyridine-2-carbonyl chloride (50mg, 0.11 mmol), cis-2,6-dimethylmorpholine (50 mg, 0.40 mmol) and di-isopropylethylamine (DIEA, 0.07 mL, 0.40 mmol) in CH₂Cl₂ (1.5 mL) was stirred at room temperature overnight. The mixture was concentrated to provide 4-chloro-*N-*[5-chloro-2-(*cis-*2,6-dimethyl-morpholine-4-carbonyl)-pyridin-3-yl]-*N-*methoxymethyl-3-trifluoromethyl-benzenesulfonamide, which was directly used in the next step without any purification.

### Example 96: Chloro-N-[5-chloro-2-(3-methyl-2,3-dihydro-benzo[1,4]oxazine-4-carbonyl)-pyridin-3-yl]-3-trifluoromethyl-benzenesulfonamide

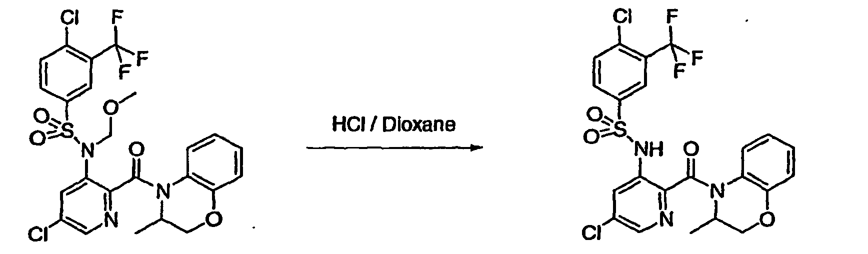

A solution of crude 4-chloro-*N-*[5-chloro-2-(3-methyl-2,3-dihydrobenzo[1,4]oxazine-4-carbonyl)-pyridin-3-yl]-*N-*methoxymethyl-3-trifluoromethyl-benzenesulfonamide in 3 mL HCl (4M in dioxane) and water (1 mL) was refluxed for 2 h. Upon cooling to room temperature, the mixture was concentrated and the residue was purified via preparative TLC (50% EtOAc in hexane) to afford 11 mg of 4-chloro-*N-*[5-chloro-2-(3-methyl-2,3-dihydro-benzo[1,4]oxazine-4-carbonyl)-pyridin-3-yl]-3-trifluoromethyl-benzenesulfonamide as an off white solid. MS *m*/*z*: 546.1 (M + H).

### Example 97: 4-Chloro-N-[5-chloro-2-(2,6-dimethyl-morpholine-4-carbonyl)-pyridin-3-yl]-3-trifluoromethyl-benzenesulfonamide

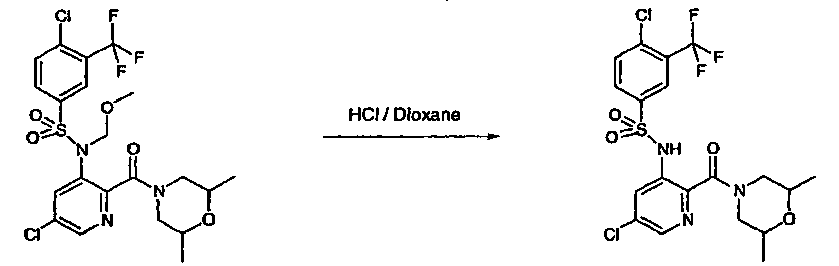

A solution of crude 4-chloro-*N-*[5-chloro-2-(2,6-dimethyl-morpholine-4-carbonyl)-pyridin-3-yl]-*N-*methoxymethyl-3-trifluoromethyl-benzenesulfonamide in 3 mL HCl (4M in dioxane) and water (1 mL) was refluxed for 2 h. Upon cooling to room temperature, the mixture was concentrated and the residue was purified via preparative TLC (50% EtOAc in hexane) to afford 28 mg of 4-chloro-*N-*[5-chloro-2-(2,6-dimethyl-morpholine-4-carbonyl)-pyridin-3-yl]-3-trifluoromethyl-benzenesulfonamide as an off white solid. MS *m*/*z:* 512.0 (M + H).

### Example 98: 5-Chloro-3-(4-chloro-3-(trifluoromethyl)phenylsulfonamido)picolinic acid

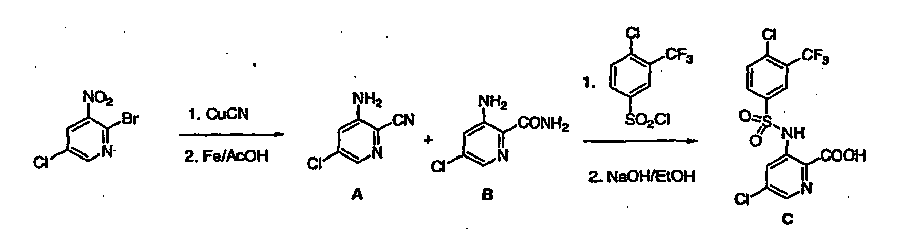

Step 1: A dry 250 mL flask was charged with 2-bromo-5-chloro-3-nitropyridine (24 g, 10 mmol), CuCN (19 g, 20 mmol) and DMF (100 mL). The resultant mixture was stirred at 110 °C for 2 h and then concentrated under reduced pressure. Water (100 mL) was added and the aqueous layer was extracted with EtOAc (250 mL X 3). The combined organic layer was washed with brine, dried (MgSO₄), and evaporated *in vacuo* to afford a light yellow solid (15 g) which was used directly for the next step.

Step 2: A 250 mL round-bottom flask was charged with the iron powder (15.6 g, 0.3 mol), AcOH (80 mL) and heated to 80 °C (oil bath) under N₂. To this mixture, was added a solution of nitrocyanopyridine (10 g, 0.055 mmol) in AcOH (80 mL) via addition funnel and stirred at 80 °C for an additional 30 min after the addition. The reaction was subsequently cooled to room temperature, diluted with EtOAc, filtered through a pad of Celite and concentrated under reduced pressure. The residue was dissolved in EtOAc and washed with 3 N NaOH and brine, dried over MgSO₄, and concentrated under reduced pressure and to afford the crude 3-amino-2-cyano-5-chloropyridine along with the 2-amide (7.7 g) which was used directly for the next step: MS 154.0 (M+H).

Step 3: A 500 mL round-bottom flask was charged with the above 3-amino-2-cyano-5-chloropyridine (7.7 g, 50 mmol), 4-Cl-3-trifluoromethyl-benzenesulfonyl chloride (28 g, 100 mmol), and pyridine (50 mL). The resultant solution was heated to 70 °C and stirred for 5 h. The pyridine was removed *in vacuo* and EtOH (80%, 260 mL) was added, followed by NaOH (30 g, 0.75 mol). The mixture was heated at reflux for 12 h. The solvent was subsequently removed *in vacuo* and ice (100 g) was added and the pH adjusted to 2-3 with *conc* HCl. The resultant aqueous solution was extracted with EtOAc, washed with brine, dried over MgSO₄, and concentrated under reduced pressure. The resultant light yellow solid was further crystallized from EtOAc/hexane (1:1) to afford 5-chloro-3-(4-chloro-3-(trifluoromethyl)phenylsulfonamido)picolinic acid as white needles (10 g, 44% overall): ¹H NMR (400 MHz, CDCl₃) δ 10.80 (s, 1H), 8.23 (m, 3H), 8.00 (d, 1H), 7.63 (d, 1H); MS (ES) 415.0 (M+H).

### General Procedure D: Synthesis of 5-chloro-3-(4-chloro-3-(trifluoromethyl)phenylsulfonamido)picolinic amides

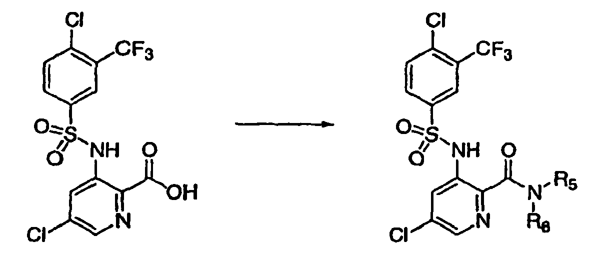

A 10 mL scintillation vial was charged with 5-chloro-3-(4-chloro-3-(trifluoromethyl)phenylsulfonamido)picolinic acid (1 equiv), amine (2-3 equiv.), coupling agent [HATU, BOP or T3P, 1.1-1.3 equiv), and base [DIEA (3-5 equiv.) or Et₃N (1-3 equiv.)] in anhydrous DMF (or CH₂Cl₂). The resultant solution was stirred at room temperature (or 70 °C) for several hours until the starting material was consumed. The reaction mixture was diluted with MeCN (1-2 mL) and purified by preparative HPLC (20 → 90% gradient of MeCN-water) and pure product fractions were lyophilized to provide pure product as a solid. Non-commercial amines were prepared according to procedures available in literature.

### Example 99: 4-chloro-N-(5-chloro-2-(1,2,3-4-tetrahydro-1,5-naphyridine-1-carbonyl-pyridine-3-yl)-3-(trifluoromethyl)benzenefulfonamide

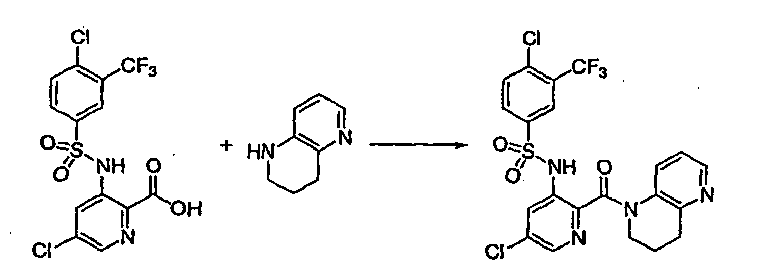

5-Chloro-3-(4-chloro-3-(trifluoromethyl)phenylsulfonamido)picolinic acid (164 mg, 0.4 mmol), 1,2,3,4-tetrahydro-1,5-napthyridine [(70 mg, 0.56 mmol), 1,2,3,4-tetrahydro-1,5-napthyridine prepared freshly from 1,5-napthyridine via hydrogenation over Pt₂O], HATU (200 mg, 0.5 mmol), DIEA (260 mg, 2 mmol) were reacted according to the procedure for the synthesis of 5-chloro-3-(4-chloro-3-(trifluoromethyl)phenylsulfonamido)-picolinic amides. HPLC purification (20 → 90% gradient of MeCN*-*water) provided 4-chloro-*N-*(5-chloro-2-(1,2,3-4-tetrahydro-1,5-naphyridine-1-carbonyl-pyridine-3-yl)-3-(trifluoromethyl)benzenefulfonamide: ¹H NMR (400 MHz, CDCl₃) δ 8.52 (d, 1H), 8.18 (m, 2H), 8.09 (d, 1H), 8.00 (m, 2H), 7.69 (d, 1H), 7.52 (m, 1H), 3.92 (m, 2H), 3.35 (t, 2H), 2.11 (m, 2H); MS *m*/*z* 531.0 (M+H).

### Example 100: 4-Chloro-N-[5-chloro-2-(3,4-dihydro-2H-[1,8]naphthyridine-1-carbonyl)-pyridin-3-yl]-3-trifluoromethyl-benzenesulfonamide

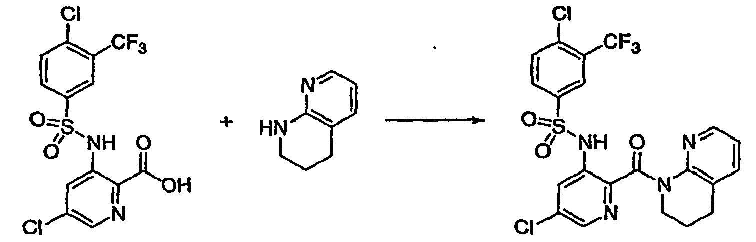

5-Chloro-3-(4-chloro-3-(trifluoromethyl)phenylsulfonamido)picolinic acid (208 mg, 0.50 mmol), 1,2,3,4-tetrahydro-[1,8]naphthyridine [(135 mg, 1.0 mmol) 1,2,3,4-tetrahydro-[1,8]naphthyridine was prepared freshly from 1,8-napthyridine via hydrogenation over Pt₂O], BOP (486 mg, 1.1 mmol), DIEA (185 mg, 1.4 mmol) were reacted according to the procedure for the synthesis of 5-chloro-3-(4-chloro-3-(trifluoromethyl)phenylsulfonamido)-picolinic amides. HPLC purification (20 → 90% gradient of MeCN-water) provided 4-chloro-*N-*[5-chloro-2-(3,4-dihydro-2H-[1,8]naphthyridine-1-carbonyl)-pyridin-3-yl]-3-trifluoromethyl-benzenesulfonamide: MS *m*/*z*: 531.0 (M+H).

### Example 101: 4-Chloro-N-[5-chloro-2-(8-fluoro-2,3-dihydro-benzo[1,4]oxazine-4-carbonyl)-pyridin-3-yl]-3-trifluoromethyl-benzenesulfonamide

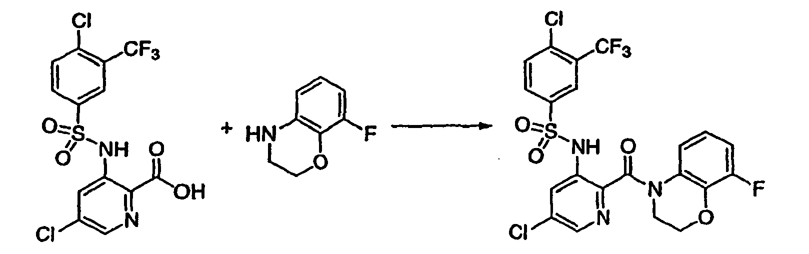

8-Fluoro-3,4-dihydro-2H-benzo[1,4]oxazine, 5-chloro-3-(4-chloro-3-(trifluoromethyl)phenylsulfonamido)picolinic acid, T3P, and Et₃N were reacted according to the procedure for the synthesis of 5-chloro-3-(4-chloro-3-(trifluoromethyl)phenylsulfonamido)-picolinic amides. HPLC purification provided 4-chloro-*N-*[5-chloro-2-(8-fluoro-2,3-dihydro-benzo[1,4]oxazine-4-carbonyl)-pyridin-3-yl]-3-trifluoromethyl-benzenesulfonamide. Reverse phase HPLC gradient acetonitrile 0.1% TFA 20-95% in 4 min: 2.986 min. MS *m*/*z* 550.1 (M+H).

### Example 102: 4-Chloro-N-[5-chloro-2-(6-chloro-2,3-dihdro-benzo[1,4]oxazine-4-carbonyl)-pyridin-3-yl]-3-trifluoromethyl-benzenesulfonamide

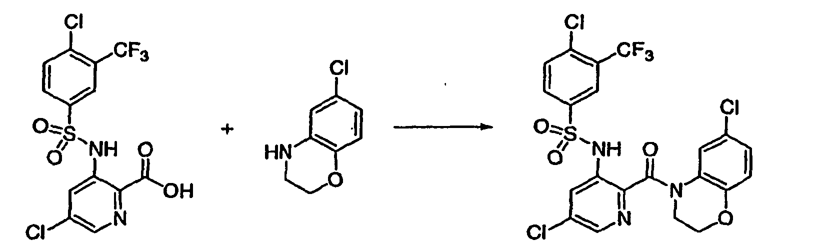

6-Chloro-3,4-dihydro-2H-benzo[1,4]oxazine 5-chloro-3-(4-chloro-3-(trifluoromethyl)phenylsulfonamido)picolinic acid, T3P, and Et₃N were reacted according to the procedure for the synthesis of 5-chloro-3-(4-chloro-3-(trifluoromethyl)phenylsulfonamido)-picolinic amides. HPLC purification provided 4-chloro-*N-*[5-chloro-2-(6-chloro-2,3-dihydro-benzo[1,4]oxazine-4-carbonyl)-pyridin-3-yl]-3-trifluoromethyl-benzenesulfonamide. Reverse phase HPLC gradient acetonitrile 0.1% TFA 20-95% in 4 min: 3.180 min MS *m*/*z*: 567.1 (M+H).

### Example 103: 4-Chloro-N-[5-chloro-2-(2,3-dihydro-pyrido[3,2-b][1,4]oxazine-4-carbonyl)-pyridin-3-yl]-3-trifluoromethyl-benzenesulfonamide

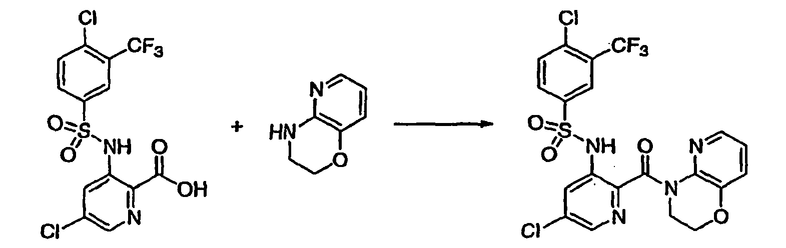

3,4-Dihydro-2H-pyrido[3,2-b][1,4]oxazine 5-chloro-3-(4-chloro-3-(trifluoromethyl)phenylsulfonamido)picolinic acid, T3P, and Et₃N were reacted according to the procedure for the synthesis of 5-chloro-3-(4-chloro-3-(trifluoromethyl)phenylsulfonamido)-picolinic amides. HPLC purification provided 4-chloro-N-[5-chloro-2-(2,3-dihydro-pyrido[3,2-b][1,4]oxazine-4-carbonyl)-pyridin-3-yl]-3-trifluoromethyl-benzenesulfonamide; Reverse phase HPLC gradient acetonitrile 0.1% TFA 20-95% in 4 min: 2.871 min; MS *m*/*z* 533.1 (M+H).

### Example 104: 4-Chloro-N-[5-chloro-2-(6-fluoro-2,3-dihydro-benzo[1,4]oxazine-4-carbonyl)-pyridin-3-yl]-3-trifluoromethyl-benzenesulfonamide

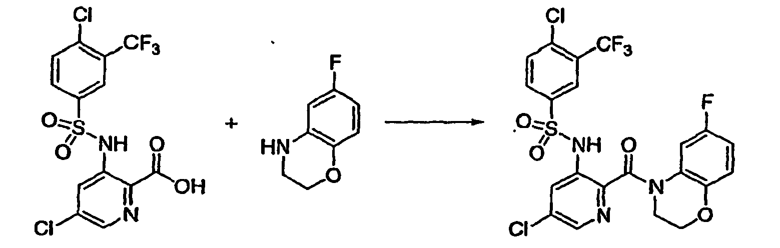

6-Fluoro-3,4-dihydro-2H-benzo[1,4]oxazine 5-chloro-3-(4-chloro-3-(trifluoromethyl)phenylsulfonamido)picolinic acid, T3P, and Et₃N were reacted according to the procedure for the synthesis of 5-chloro-3-(4-chloro-3-(trifluoromethyl)phenylsulfonamido)-picolinic amides. HPLC purification provided 4-chloro-N-[5-chloro-2-(6-fluoro-2,3-dihydro-benzo[1,4]oxazine-4-carbonyl)-pyridin-3-yl]-3-trifluoromethyl-benzenesulfonamide: Reverse phase HPLC gradient acetonitrile 0.1% TFA 20-95% in 4 min: 3.041 min MS *m*/*z* : 550.1 (M+H).

### Example 105: 4-Chloro-N-[5-chloro-2-(6-methyl-2,3-dihydro-benzo[1,4]oxazine-4-carbonyl)-pyridin-3-yl]-3-trifluoromethyl-benzenesulfonamide

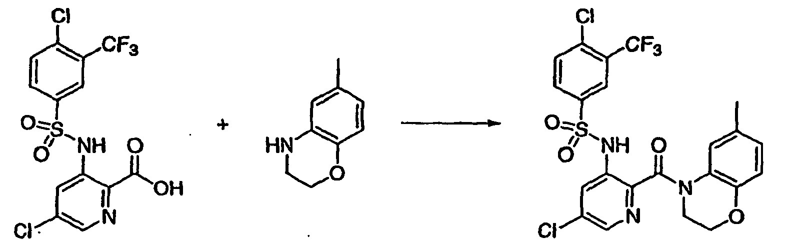

6-Methyl-3,4-dihydro-2H-benzo[1,4]oxazine 5-chloro-3-(4-chloro-3-(trifluoromethyl)phenylsulfonamido)picolinic acid, T3P, and Et₃N were reacted according to the procedure for the synthesis of 5-chloro-3-(4-chloro-3-(trifluoromethyl)phenylsulfonamido)-picolinic amides. HPLC purification provided 4-chloro-*N*-[5-chloro-2-(6-methyl-2,3-dihydro-benzo[1,4]oxazine-4-carbonyl)-pyridin-3-yl]-3-trifluoromethyl-benzenesulfonamide: Reverse phase HPLC gradient acetonitrile 0.1% TFA 20-95% in 4 min: 3.099 min; MS *m*/*z*: 546.1 (M+H).

### General Procedure E: Synthesis of 4-chlorobenzooxazin

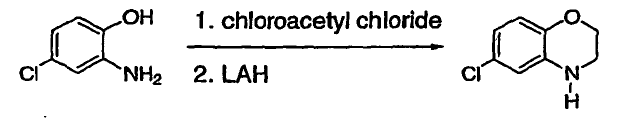

3-Chlorophenol (2.87 g, 20 mmol) was added into a magnetically stirred mixture of 50 mL saturated aqueous solution of sodium bicarbonate and 30 mL tetrahydrofuran. Chloroacetylchloride (2.82 g, 25 mmol) was added and the reaction mixture was stirred at room temperature for 2 h. The reaction was heated to 80 °C and stirred an additional 18 h. The mixture was poured into water (200 mL) and the aqueous layer was extracted with ethyl acetate (200 mL). The combined organic extracts were concentrated and dried under high vacuum.

The crude product was dissolved in tetrahydrofuran (30 mL) and stirred under nitrogen. 10 mL lithium aluminum hydride solution in THF (2.4 M, 24 mmol) was slowly added to the solution. The resultant mixture was stirred at room temperature for 2 h, quenched by adding saturated aqueous solution of sodium bicarbonate (20 mL). The aqueous layer was extracted with 50 mL chloroform twice and the combined organic extracts were concentrated under reduced pressure. The crude product was purified by flash chromatograph to yield 3.13 g of product as a colorless solid.

### Example 106: 5-Chloro-thiophene-2-sulfonic acid [5-chloro-2-(2-fluoro-6-methoxy-benzoyl)-pyridin-3-yl]-amide

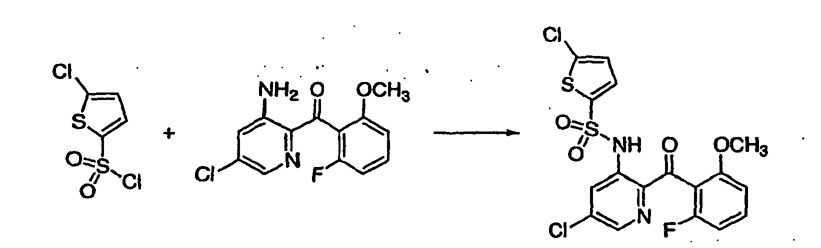

(3-Amino-5-chloro-pyridin-2-yl)-(2-fluoro-6-methoxy-phenyl)-methanone (50 mg, 0.178 mmol) and 5-chloro-thiophene-2-sulfonyl chloride (85 mg, 0.392 mmol) were reacted according to the procedure described in example 76. The crude product was purified by flash chromatography on silica gel using Combi-flash and pure product fractions were dried under reduced pressure to provide 5-chloro-thiophene-2-sulfonic acid [5-chloro-2-(2-fluoro-6-methoxybenzoyl)-pyridin-3-yl]-amide. MS *m*/*z*: 460.9 (M+H).

### Example 107: 4-Chloro-N-(5-chloro-2-formyl-pyridin-3-yl)-N-methoxymethyl-3-trifluoromethyl-benzenesulfonamide

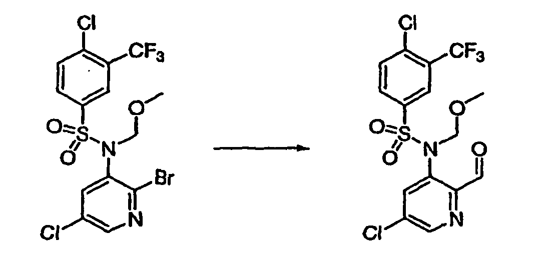

To a stirred solution of N-(2-Bromo-5-chloro-pyridin-3-yl)-4-chloro-N-methoxymethyl-3-trifluoromethyl-benzenesulfonamide (280 mg, 0.566 mmol) in anhydrous THF (3 mL) was added 2 M isopropylmagnesiumchloride in THF (700 µL, 1.36 mmol) at 0 °C. It was stirred at the same temperature for 20 minutes and then DMF (174 µL, 1.36 mmol) was added in dropwise and the progress of the reaction was followed by LCMS. The reaction mixture was warmed to room temperature and stirred for 6 h. It was then quenched with saturated aqueous NH₄Cl (2 mL), and extracted with EtOAc. The combined extracts were dried (Na₂SO₄), filtered and concentrated under reduced pressure. The residue was purified by flash column chromatography to provide 4-Chloro-N-(5-chloro-2-formyl-pyridin-3-yl)-N-methoxymethyl-3-trifluoromethyl-benzenesulfonamide Mass spectrum m/z : 443.0 (M+H).

### Example 108: 4-Chloro-N-{5-chloro-2-[hydroxy-(2-nitro-phenyl)-methyl]-pyridin-3-yl}-N-methoxymethyl-3-trifluoromethyl-benzenesulfonamide

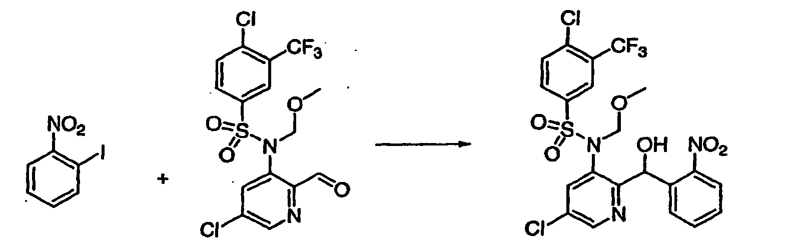

To a stirred solution of 1-lodo-2-nitro-benzene (769 mg, 3.08 mmol) in anhydrous THF (3 mL) was added 2 M phenylmagnesiumchloride in THF (1.62 mL, 3.24 mmol) at -40 °C under an atmosphere of nitrogen. It was stirred at the same temperature for 15 minutes and then 4-chloro-N-(5-chloro-2-formyl-pyridin-3-yl)-N-methoxymethyl-3-trifluoromethyl-benzenesulfonamide (1.04 g, 2.36 mmol) was added in one portion and the progress of the reaction was followed by LCMS. The reaction mixture was warmed to room temperature and stirred for 3 h. It was then quenched with saturated aqueous NH₄Cl (2 mL), and extracted with EtOAc. The combined extracts were dried (Na₂SO₄), filtered and concentrated under reduced pressure to provide crude 4-chloro-N-{5-chloro-2-[hydroxy-(2-nitro-phenyl)-methyl]-pyridin-3-yl}-N-methoxymethyl-3-trifluoromethyl-benzenesulfonamide, which was used in the following step without any purification. Mass spectrum m/z: 566.3 (M+H).

### Example 109: 4-Chloro-N-[5-chloro-2-(2-nitro-benzoyl)-pyridin-3-yl]-N-methoxvmethyl-3-trifluoromethyl-benzenesulfonamide

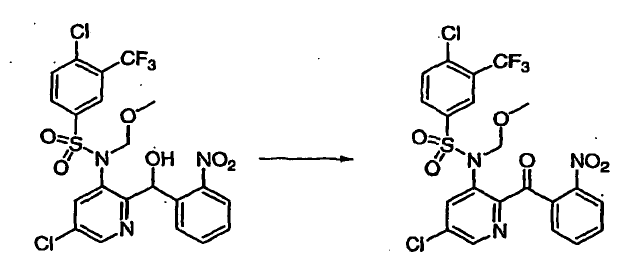

A mixture of 4-chloro-N-{5-chloro-2-[hydroxy-(2-nitro-phenyl)-methyl]-pyridin-3-yl}-N-methoxymethyl-3-trifluoromethyl-benzenesulfonamide (∼1.0 g) and Dess-Martin periodinane (1.50 g, 3.54 mmol) in CH₂Cl₂ (20 mL) at room temperature was stirred for 2-4 h. A mixture of 10% aqueous Na₂S₂O₃ (10 mL) and saturated aqueous NaHCO₃ (10 mL) was then added and the biphasic mixture vigorously stirred for 30 min. The phases were then separated and the aqueous portion extracted with CH₂Cl₂. The combined organic extracts were washed with saturated aqueous NaHCO₃, then brine, dried (Na₂SO₄) and filtered. The filtrate was concentrated under reduced pressure and the residue purified by flash column chromatography over silica gel (EtOAc/hexanes, 1:4, then 2:3) to provide 4-Chloro-N-[5-chloro-2-(2-nitro-benzoyl)-pyridin-3-yl]-N-methoxymethyl-3-trifluoromethyl-benzenesulfonamide. Mass spectrum m/z: 586.3 (M+Na).

### Example 110: 4-Chloro-N-[5-chloro-2-(2-nitro-benzoyl)-pyridin-3-yl]-3-trifluoromethyl-benzenesulfonamide

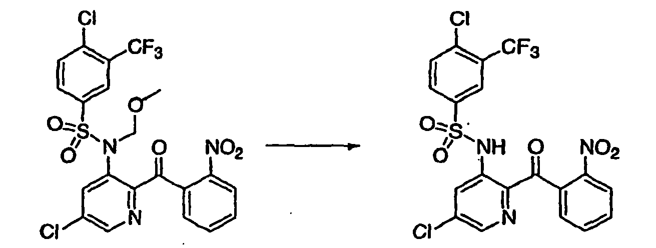

A mixture 4-chloro-N-[5-chloro-2-(2-nitro-benzoyl)-pyridin-3-yl]-N-methoxymethyl-3-trifluoromethyl-benzenesulfonamide (580 g, 1.02 mmol) in 4 M HCl in dioxane (15 mL), and water (5 mL) was heated at 100 °C for 8 h and then at room temperature for 10 h. The reaction mixture was concentrated to dryness under reduced pressure and it was treated with aqueous NaHCO₃ to adjust pH to 5-6 and extracted with EtOAc. The combined extracts were dried (Na₂SO₄), filtered and concentrated under reduced pressure. The residue was purified by flash column chromatography on silica gel to provide the desired product 4-Chloro-N-[5-chloro-2-(2-nitro-benzoyl)-pyridin-3-yl]-3-trifluoromethyl-benzenesulfonamide. ¹H NMR (400 MHz, CDCl₃) δ11.06 (s, 1 H), 8.23 (d, *J* = 2.4 Hz, 1 H), 8.18 (d, *J* = 2.4 Hz, 1 H), 8.14 (dd, *J* = 8.0,1.4 Hz, 1 H), 8.07 (d, *J* = 2.4 Hz, 1 H), 8.00 (dd, *J* = 8.4,2.0 Hz, 1 H), 7.80-7.77 (m, 1 H), 7.70-7.66 (m, 2 H), 7.45 (dd, *J* = 8.0, 1.6 Hz, 1 H); Mass spectrum m/z : 520.2 (M+H).

### Example 111: N-[2-(2-Amino-benzol)-5-chloro-pyridin-3-yl]-4-chloro-3-trifluoromethyl-benzenesulfonamide

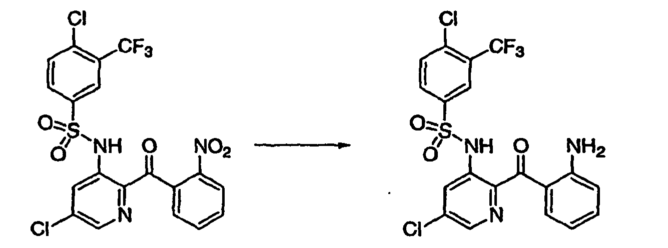

To a stirred suspension of Fe (176 mg, 3.15 mmol) in glacial acetic acid (5 mL) was added a solution 4-chloro-N-[5-chloro-2-(2-nitro-benzoyl)-pyridin-3-yl]-3-trifluoromethyl-benzenesulfonamide (410 mg, 0.788 mmol) in AcOH (5 mL) at 80 °C for 15 minutes. After complete addition the mixture was stirred at the same temperature for 30 minutes and the progress of the reaction was followed by LCMS. The reaction mixture was then cooled to room temperature and it was then diluted with EtOAc and filtered through a pad of Celite. The filtrate was concentrated under reduced pressure and residue was portioned between NaHCO₃ and EtOAc. The organic portion was separated and the aqueous portion was extracted with EtOAc. The combined extracts were dried (Na₂SO₄), filtered and concentrated under reduced pressure. The residue was purified by flash column chromatography on silica gel to provide N-[2-(2-aminobenzoyl)-5-chloro-pyridin-3-yl]-4-chloro-3-trifluoromethyl-benzenesulfonamide. ¹H NMR (400 MHz, CDCl₃) δ 8.98 (s, 1 H), 8.42 (d, *J* = 2.4 Hz, 1 H), 8.12 (d, *J* = 2.0 Hz, 1 H), 7.93 (d, *J* = 2.4 Hz, 1 H), 7.64-7.61 (m, 1 H),7.30-7.26 (m, 2 H), 7.03-7.00 (m, 1 H), 6.64 (d, *J* = 8.4 Hz, 1 H), 6.47-6.43 (m, 1 H), 6.26 (s, 2 H); Mass spectrum m/z: 490.0 (M+H).

### Example 112: 4-Chloro-N-[5-chloro-2-(2-methanesulfonylamino-benzoyl)-pyridin-3-yl]-3-trifluoromethyl-benzenesulfonamide

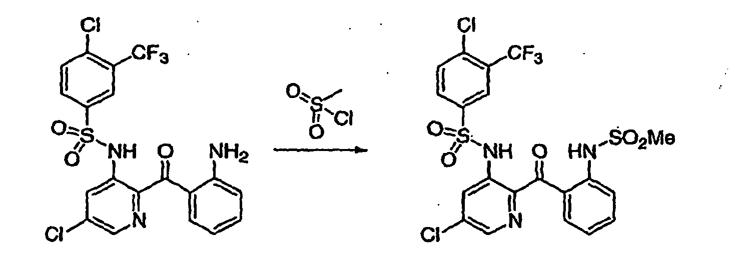

To a solution of N-[2-(2-amino-benzoyl)-5-chloro-pyridin-3-yl]-4-chloro-3-trifluoromethyl-benzenesulfonamide (24 mg, 0.048 mmol) in anhydrous pyridine (1 mL) was added methane sulfonyl chloride (9 mg, 0.075 mmol). The resulting mixture was stirred at room temperature for 2 h was poured into 1 M HCl and extracted with EtOAc. The extracts were concentrated under reduced pressure and the residues was purified by flash column chromatography to trissulfonamide Mass spectrum m/z: 515.0 (M+H). To this trisulfonamide (14 mg, 0.021 mmol) in THF was added TBAF (1 M in THF, 40 µL, 0.04 mmol) and the resulting mixture was stirred overnight. The reaction mixture was quenched with 1 M HCl and extracted with EtOAc. The combined extracts were dried (Na₂SO₄), filtered and concentrated under reduced pressure. The residue was purified by flash column chromatography on silica gel (combi-flash) to provide 4-Chloro-N-[5-chloro-2-(2-methanesulfonylamino-benzoyl)-pyridin-3-yl]-3-tifluoromethyl-benzenesulfonamide. ¹H NMR (400 MHz, CDCl₃) δ 9.60 (br, 2 H), 8.38 (d, *J* = 2.0 Hz, 1 H), 8.16 (d, *J* = 2.0 Hz, 1 H), 8.04 (d, *J* = 2.0 Hz, 1 H), 7.87 (dd, *J* = 8.4, 2.4 Hz, 1 H), 7.72-7.70 (m, 1 H), 7.62-7.57 (m, 1 H), 7.53 (d, *J* = 8.4 Hz, 1 H), 7.29 (dd, *J* = 8.0, 2.4 Hz, 1 H), 7.08-7.05 (m, 1 H), 3.07 (s, 3 H) ; Mass spectrum m/z: 515.0 (M+H).

### Example 113: 4-Chloro-N-[5-chloro-2-(2-methylsulfanyl-benzoyl)-pyridin-3-yl]-N-methoxymethyl-3-trifluoromethyl-benzenesulfonamide

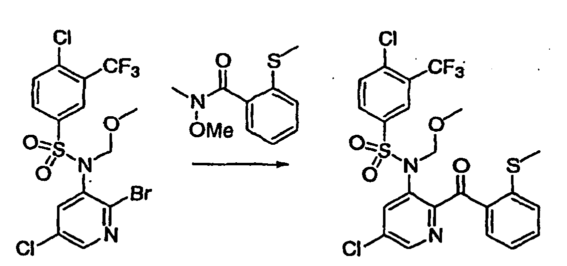

To a stirred solution of N-(2-bromo-5-chloro-pyridin-3-yl)-4-chloro-N-methoxymethyl-3-trifluoromethyl-benzenesulfonamide (8.26 g, 16.6 mmol) in anhydrous THF (50 mL) was added 2 M isopropylmagnesiumchloride in THF (18.4 mL, 36.8 mmol) at -40 °C. It was then warmed to 0 °C and stirred at the same temperature for 30 minutes and then N-Methoxy-N-methyl-2-methylsulfanyl-benzamide (10.5 g, 49.8 mmol) was added in and the progress of the reaction was followed by LCMS. The reaction mixture was warmed to room temperature and stirred overnight (18 h). It was then quenched with saturated aqueous NH₄Cl (2 mL), and extracted with EtOAc. The combined extracts were dried (Na₂SO₄), filtered and concentrated under reduced pressure. The residue was purified by flash column chromatography to provide 4-chloro-N-[5-chloro-2-(2-methylsulfanyl-benzoyl)-pyridin-3-yl]-N-methoxymethyl-3-trifluoromethyl-benzenesulfonamide. Mass spectrum m/z : 486.9 (M+Na).

### Example 114: 4-Chloro-N-[5-chloro-2-(2-methanesulfonyl-benzoyl)-pyridin-3-yl]-N-methoxymethyl-3-trifluoromethyl-benzenesulfonamide

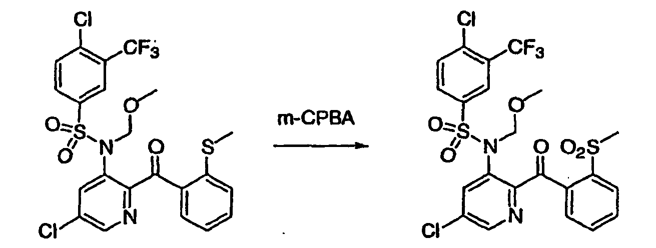

To a stirred solution of 4-chloro-N-15-chloro-2-(2-methylsulfanyl-benzoyl)-pyridin-3-yl]-N-methoxymethyl-3-trifluoromethyl-benzenesulfonamide (10.0 g, 17.7 mmol) in CH₂Cl₂ (100 mL) was added m-CPBA (8.9 g, 75% by wt, 38.9 mmol) in one portion and the progress of the reaction was monitored by LCMS. After overnight stirring at room temperature, the reaction mixture was quenched with pyridine (10 mL) and reaction mixture was stirred for 30 minutes. Then portioned between diethyl ether and water, the aqueous portion was separated and the organic portion was washed with NH₄Cl and NaHCO₃ respectively. The combined extracts were dried (Na₂SO₄), filtered and concentrated under reduced pressure. The residue was purified by flash column chromatography to provide 4-chloro-N-[5-chloro-2-(2-methanesulfonyl-benzoyl)-pyridin-3-yl]-N-methoxymethyl-3-trifluoromethyl-benzenesulfonamide. Mass spectrum m/z : 497.4 (M+H).

### Example 115: 4-Chloro-N-[5-chloro-2-(2-methanesulfonyl-benzoyl)-pyridin-3-yl]-3-trifluoromethyl-benzenesulfonamide

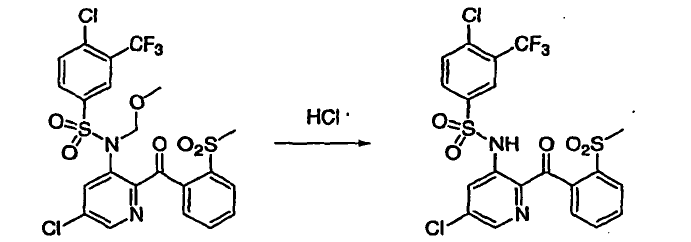

A mixture 4-chloro-N-[5-chloro-2-(2-methanesulfonyl-benzoyl)-pyndin-3-yl]-N-methoxymethyl-3-trifluoromethyl-benzenesulfonamide (9.6 g, 16.1 mmol) in 4 M HCl in dioxane (60 mL), and water (15 mL) was heated at 100 °C for 20 h. The reaction mixture was concentrated to dryness under reduced pressure and it was treated with aqueous NaHCO₃ to adjust pH to 5-6 and extracted with EtOAc. The combined extracts were dried (Na₂SO₄), filtered and concentrated under reduced pressure. The residue was purified by flash column chromatography on silica gel to provide the desired product. ¹H NMR (400 MHz, CDCl₃) δ 11.1 (s, 1 H), 8.22-8.14 (m, 2 H), 8.00-7.97 (m, 1 H), 7.72-7.65 (m, 3 H), 7.33 (d, *J* = 7.2 Hz, 1 H), 3.0.4 (s, 3 H); Mass spectrum m/z: 553.40 (M+H).

### Example 116: 4-Chloro-N-[5-chloro-6-methyl-(2-methylsulfanyl-benzoyl)-pyridin-3-yl]-N-methoxymethyl-3-trifluoromethyl-benzenesulfonamide

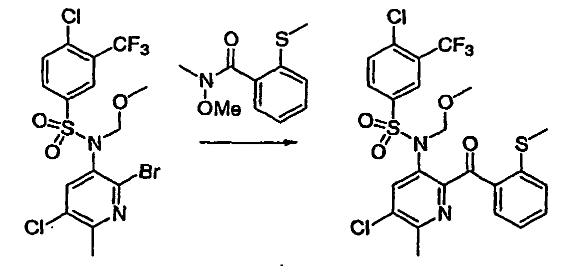

To a stirred solution of N-(2-bromo-5-chloro-6-methyl-pyridin-3-yl)-4-chloro-N-methoxymethyl-3-trifluoromethyl-benzenesulfonamide (255 mg, 0.5 mmol) in anhydrous THF (3 mL) was added 2 M isopropylmagnesiumchloride in THF (600 µL, 1.2 mmol) at -40 °C. It was then warmed to 0 °C and stirred at the same temperature for 1 h and then N-Methoxy-N-methyl-2-methylsulfanyl-benzamide (422 mg, 1.00 mmol) was added in and the progress of the reaction was followed by LCMS. The reaction mixture was warmed to room temperature and stirred overnight (18 h). It was then quenched with saturated aqueous NH₄Cl (2 mL), and extracted with EtOAc. The combined extracts were dried (Na₂SO₄), filtered and concentrated under reduced pressure. The residue was purified by flash column chromatography (ISCO) to provide 4-chloro-N-[5-chloro-6-methyl-2-(2-methylsuffanyl-benzoyl)-pyridin-3-yl]-N-methoxymethyl-3-trifluoromethyl-benzenesulfonamide. Mass spectrum m/z : 601.4 (M+Na).

### Example 117: 4-Chloro-N-5-chloro-2-(2-methanesulfonyl-benzoyl)-6-methyl-pyridin-3-yl]-N-methoxymethyl-3-trifluoromethyl-benzenesulfonamide

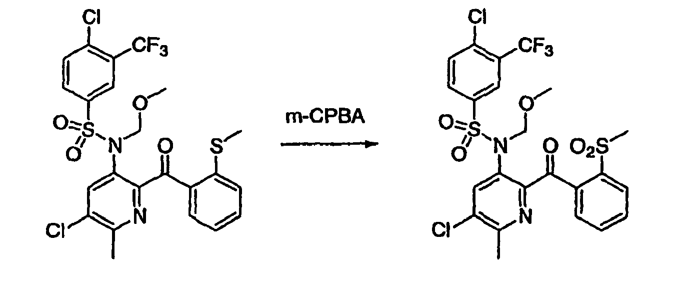

To a stirred solution of 4-chloro-N-[5-chloro-6-methyl-2-(2-methylsulfanyl-benzoyl)-pyridin-3-yl]-N-methoxymethyl-3-trifluoromethyl-benzenesulfonamide (100 mg, 0.17) in CH₂Cl₂ (5 mL) was added m-CPBA (125 g, 75% by wt, 0.54 mmol) in one portion and the progress of the reaction was monitored by LCMS. After overnight stirring at room temperature, the reaction mixture was quenched with pyridine (200 µL) and reaction mixture was purified by reverse phase HPLC to provide 4-chloro-N-[5-chloro-2-(2-methanesulfonyl-benzoyl)-6-methyl-pyridin-3-yl]-N-methoxymethyl-3-trifluoromethyl-benzenesulfonamide. Mass spectrum m/z: 633.4 (M+Na).

### Example 118: 4-Chloro-N-[5-chloro-2-(2-methanesulfonyl-benzoyl)-6-methyl-pyridin-3-yl]-3-trifluoromethyl-benzenesulfonamide

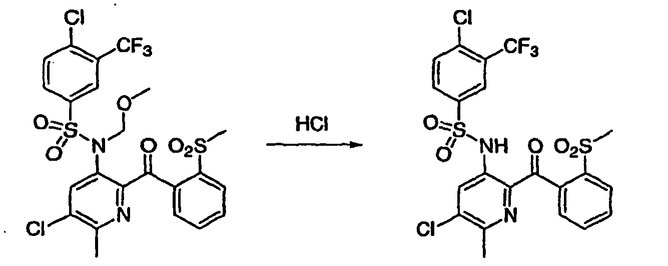

A mixture 4-chloro-N-[5-chloro-2-(2-methanesulfonyl-benzoyl)-6-methyl-pyridin-3-yl]-N-methoxymethyl-3-trifluoromethyl-benzenesulfonamide (40 mg, 0.07 mmol) in 4 M HCl in dioxane (2 mL), and water (1 mL) was heated at 100 °C for 4 h and then it was stirred at room temperature overnight. The reaction mixture was concentrated to dryness under reduced pressure and it was treated with aqueous NaHCO₃ to adjust pH to 5-6 and extracted with EtOAc. The combined extracts were dried (Na₂SO₄), filtered and concentrated under reduced pressure. The residue was purified by flash column chromatography on silica gel to provide the desired product. ¹H NMR (400 MHz, CDCl₃) δ 10.93 (s, 1 H), 8.21 (s, 1 H), 8.15 (s, 1 H), 7.98-7.96 (m, 2 H), 7.70-7.62 (m, 3 H), 7.33-7.31 (m, 1 H); Mass spectrum m/z: 567.4.0 (M+H).

### Example 119: 4-Chloro-N-[5-chloro-2-(2-methoxy-benzoyl)-pyridin-3-yl]-N-methoxymethyl-3-trifluoromethyl-benzenesulfonamide

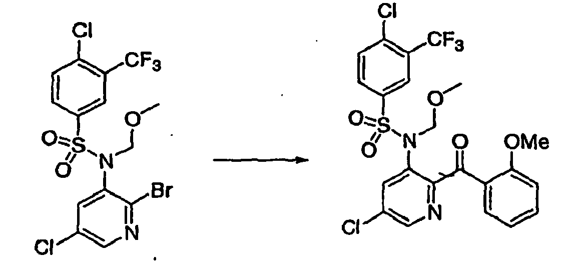

4-Chloro-N-[5-chloro-2-(2-methoxy-benzoyl)-pyridin-3-yl]-N-methoxymethyl-3-trifluoromethyl-benzenesulfonamide was prepared according to procedure described in example 24 from 2,N-dimethoxy-N-methyl-benzamide. Mass spectrum m/z: 571.0 (M+Na).

### Example 120: 4-Chloro-N-[5-chloro-2-(5-fluoro-2-methoxy-benzoyl)-pyridin-3-yl]-N-methoxymethyl-3-trifluoromethyl-benzenesulfonamide

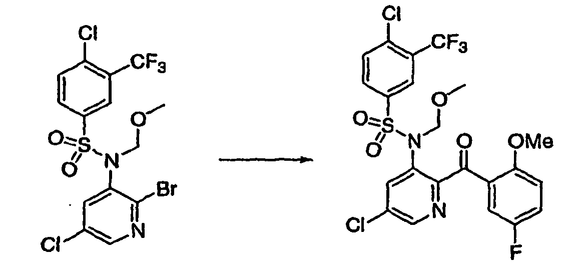

4-Chloro-N-[5-chloro-2-(5-fluoro-2-methoxy-benzoyl)-pyridin-3-yl]-N-methoxymethyl-3-trifluoromethyl-benzenesulfonamide was prepared according to procedure described in example 24. Mass spectrum m/z: 589.3 (M+Na).

### Example 121: 4-Chloro-N-[5-chloro-2-(2-fluoro-6-methoxy-benzoyl)-pyridin-3-yl]-N-methoxymethyl-3-trifluoromethyl-benzenesulfonamide

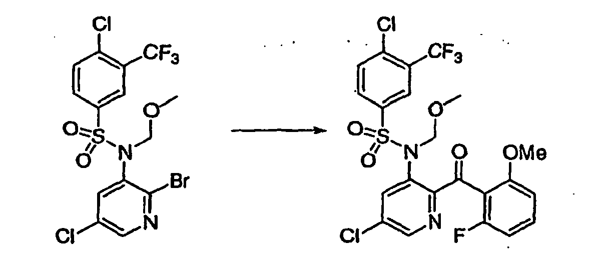

4-Chloro-N-[5-chloro-2-(2-fluoro-6-methoxy-benzoyl)-pyridin-3-yl]-N-methoxymethyl-3-triluoromethyl-benzenesulfonamide was prepared according to procedure described in example 24. Mass spectrum m/z : 589.0 (M+H).

### Example 122: 4-Chloro-N-[5-chloro-2-(2-methoxy-6-methyl-benzoyl)-pyridin-3-yl]-N-methoxymethyl-3-trifluoromethyl-benzenesulfonamide

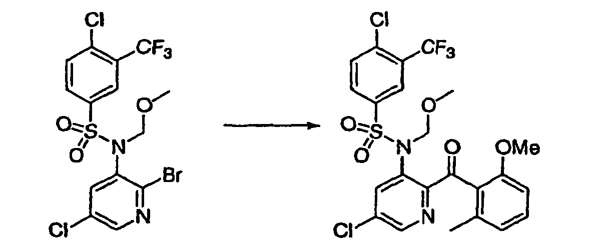

4-Chloro-N-[5-chloro-2-(2-methoxy-6-methyl-benzoyl)-pyridin-3-yl]-N-methoxymethyl-3-trifluoromethyl-benzenesulfonamide. was prepared according to procedure described in example 24. Mass spectrum m/z : 585.0 (M+H).

### Example 123: 4-Chloro-N-[5-chloro-2-(2-methoxy-benzoyl)-pyridin-3-yl]-3-trifluoromethyl-benzenesulfonamide

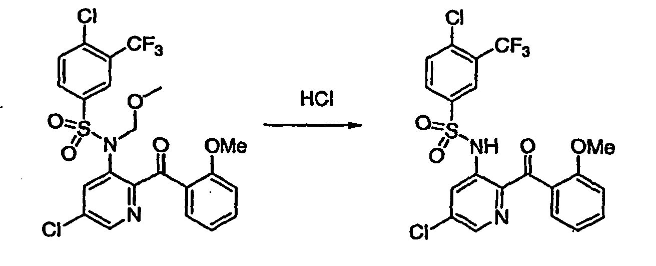

¹H NMR (400 MHz, CDCl₃) δ 10.81 (s, 1 H), 8.24 (d, *J* = 2.0 Hz, 1 H), 8.15-8.14 (m, 2 H), 7.95 (dd, *J* = 8.4, 2.0 Hz, 1 H), 7.60 (d, *J* = 8.4 Hz, 1 H), 7.51-7.47 (m, 1 H), 7.32-7.30 (m, 1 H), 7.05-7.01 (m, 1 H), 6.90 (d, *J* = 8.4 Hz, 1 H), 3.52 (s, 3 H); Mass spectrum mlz : 505.2 (M+H).

### Example 124: 4-Chloro-N-[5-chloro-2-(5-fluoro-2-methoxy-benzoyl)-pyridin-3-yl]-3-trifluoromethyl-benzenesulfonamide

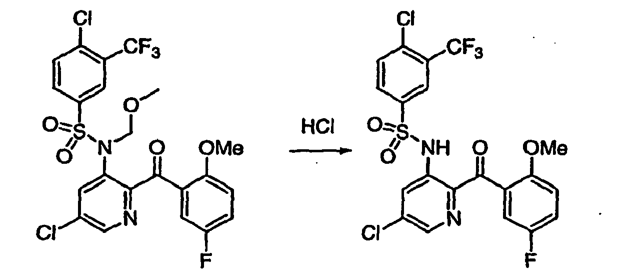

¹H NMR (400 MHz, CDCl₃) δ 10.76 (s, 1 H), 8.24 (d, *J* = 2.0 Hz, 1 H), 8.15-8.14 (m, 2 H), 7.96 (dd, *J* = 8.0, 2.0 Hz, 1 H), 7.62 (d, *J* = 8.4 Hz, 1 H), 7.25-7.16 (m, 1 H), 7.05-7.03 (m, 1 H), 6.87-6.84 (m, 1 H), 3.51 (s, 3 H); Mass spectrum m/z : 505.2 (M+H).

### Example 125: 4-Chloro-N-[5-chloro-2-(2-fluoro-6-methoxy-benzoyl)-pyridin-3-yl]-3-trifluoromethyl-benzenesulfonamide

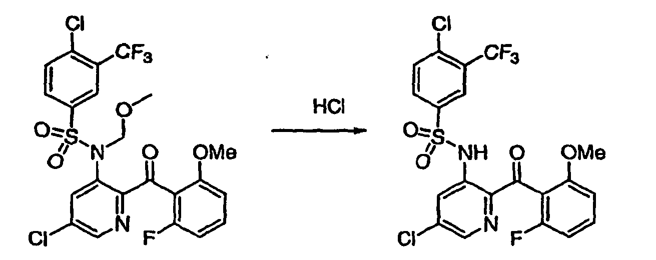

¹H NMR (400 MHz, CDCl₃) δ 11.10 (s, 1 H), 8.23 (d, *J* = 2.4 Hz, 1 H), 8.18 (d, *J* = 2.0 Hz, 1 H), 8.15 (d, *J* = 2.0 Hz, 1 H), 8.00 (dd, *J* = 8.4, 2.4 Hz, 1 H), 7.65 (d, *J* = 8.4 Hz, 1 H), 7.35-7.41 (m, 1 H), 6.70-6.75 (m, 2 H), 3.68 (s, 3 H); Mass spectrum m/z: 523.0 (M+H).

### Example 126: 4-Chloro-N-[5-chloro-2-(2-methoxy-6-methyl-benzol)-pyridin-3-yl]-N-methoxymethyl-3-trifluoromethyl-benzenesulfonamide

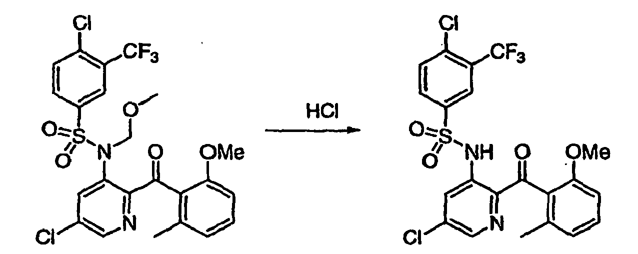

¹H NMR (400 MHz, CDCl₃) δ 11.32 (s, 1 H), 8.17-8.22 (m, 3 H), 8.13 (dd, *J* = 8.0, 2.0 Hz, 1 H), 7.65 (d, *J* = 8.0 Hz, 1 H), 7.25-7.32 (m, 1 H), 6.84 (d, *J* = 8.0 Hz, 1 H), 6.73 (d, *J* = 8.0 Hz, 1 H), 3.50 (s, 3 H), 2.06 (s, 3 H); Mass spectrum m/z: 519.0 (M+H).

### Example 127: 5-Chloro-2-iodo-3-nitro-pyridine

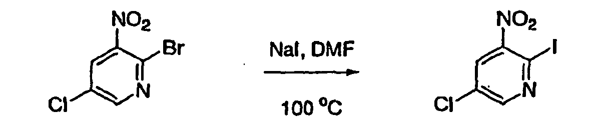

To a stirred mixture of 2-bromo-5-chloro-3-nitro-pyridine (2.5 g, 10.5 mmol) in anhydrous DMF (20 mL) was added Nal (12.0 g, 80 mmol) was added in one portion and the resulting mixture was heated at 95-100 °C for 2 days. The reaction mixture was cooled to roomtempearture and poured into water. It was then extracted with EtOAc and the combined extracts were washed with aqueous 10% Na₂S₂O₃. The combined extracts were dried (Na₂SO₄), filtered and concentrated under reduced pressure. The residue was purified by flash column chromatography on silica gel to provide 5-Chloro-2-iodo-3-nitro-pyridine. Mass spectrum m/z : 285.2 (M+H).

### Example 128: (5-Chloro-3-nitro-pyridin-2-yl)-(2,4-dimethyl-pyridin-3-yl)-methanol

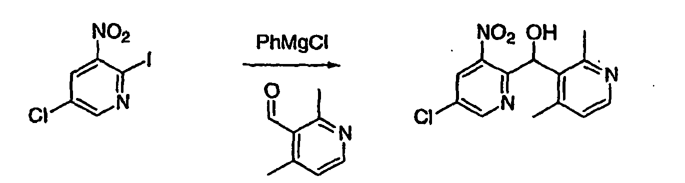

To a stirred solution of 5-Chloro-2-iodo-3-nitro-pyridine (2.5 g, 8.77 mmol) in anhydrous THF (20 mL) at -78 °C was added 2 M phenylmagnesiumchloride in THF (4.8 mL, 9.6 mmol) and stirred at the same temperature for 30 minutes and then 2,4-Dimethyl-pyridine-3-carbaldehyde (1.82 g, 13.15 mmol) was added in one portion and the progress of the reaction was followed by LCMS. The reaction mixture was warmed to room temperature and stirred overnight (18 h). It was then quenched with saturated aqueous NH₄Cl (10 mL), and extracted with EtOAc. The combined extracts were washed with aqueous NaHCO₃, brine, and then dried (Na₂SO₄), filtered and concentrated under reduced pressure. The residue was purified by flash column chromatography to (5-Chloro-3-nitro-pyridin-2-yl)-(2,4-dimethyl-pyridin-3-yl)-methanol. Mass spectrum m/z : 294.4 (M+H).

### Example 129: (5-Chloro-3-nitro-pyridin-2-yl)-(2,4-dimethyl-pyridin-3-yl)-methanone

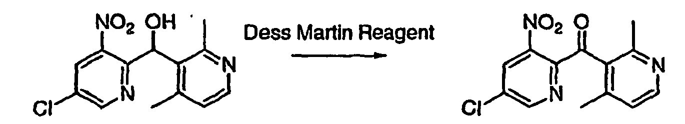

A mixture of (5-chloro-3-nitro-pyndin-2-yl)-(2,4-dimethyl-pyridin-3-yl)-methanol (1.46 g) and Dess-Martin periodinane (2.52 g, 5.91 mmol) in CH₂Cl₂ (15 mL) at room temperature was stirred for 5 h. A mixture of 10% aqueous Na₂S₂O₃ (10 mL) and saturated aqueous NaHCO₃ (10 mL) was then added and the biphasic mixture vigorously stirred for 30 min. The phases were then separated and the aqueous portion extracted with CH₂Cl₂. The combined organic extracts were washed with saturated aqueous NaHCO₃, then brine, dried (Na₂SO₄) and filtered. The filtrate was concentrated under reduced pressure and the residue purified by flash column chromatography over silica gel to provide (5-chloro-3-nitro-pyridin-2-yl)-(2,4-dimethyl-pyridin-3-yl)-methanone. Mass spectrum m/z : 292.3 (M+Na).

### Example 130: (3-Amino-5-chloro-pyridin-2-yl)-(2,4-dimethyl-pyridin-3-yl)-methanone

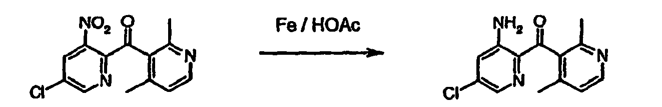

To a mixture of iron powder (220mg, 4.0 mmol) in 4ml of acetic acid at 80 °C was slowly added a solution of (3-Nitro-5-chloro-pyridin-2-yl)-(2,4-dimethyl-pyridin-3-yl)-methanone (260mg, 0.89mmol) in 2ml of acetic acid. After the completion of addition the mixture was stirred at 80 °C for half an hour and then cooled. The mixture was diluted with ethyl acetate, filtered through celite and the filtrate was concentrated. The residue was purified by flash column (50% ethyl acetate in hexane) to afford 140 mg of title compound as off-white solid. MS: (M+H) /z = 262.0.

### Example 131: 3,4-Dichloro-N-[5-chloro-2-(2,4-dimethyl-pyridine-3-carbonyl)-pyridin-3-yl]-benzenesulfonamide

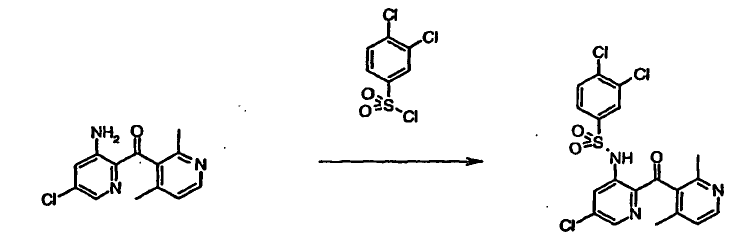

To a solution of (3-Amino-5-chloro-pyridin-2-yl)-(2,4-dimethyl-pyridin-3-yl)-methanone (43 mg, 0.16 mmol) and 10 mg of dimethylaminopyridine in 1.0 ml of pyridine was added 3,4-dichlorobenzenesulfonylchloride (70 mg, 0.28 mmol). The mixture was stirred at 80°C for 3 hours, cooled to room temperature and concentrated. The residue was directly purified by flash column (50% ethyl acetate in hexane) or preparative HPLC (20-80% acetonitrile in water) to afford 18 mg of title compound as an off-white solid. MS: (M+H) /z = 470.4.

### Example 132: 5-methyl-3-nitro-pyridin-2-ylamine

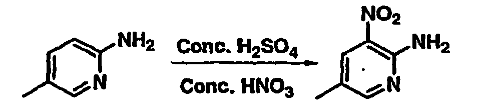

Sulfuric acid (97%, 100 mL) was placed in a -10 °C bath and when the internal temperature reached to 5 °C, 2-amino-picoline (25 g, 231.2 mmol) was added in small portions with stirring (in1 h). The suspension was stirred at ambient temperature to dissolve rest of the solid. The resulting solution was heated to 55 °C and 70% conc. HNO₃ (15.6 mL) was added dropwise while maintaining the internal temperature between 55-60 °C. The mixture was stirred further 30 min after the addition, poured into crushed ice (800 g), stirred to get solution and treated with 40% aqueous NaOH solution at 0 °C to reach pH 9 and extracted with CHCl₃ (3 x 250 mL). Combined organic layers were washed with brine (2 x 200 mL), dried (anhydrous Na₂SO₄) and concentrated under reduced pressure to afford 5-methyl-3-nitro-pyridin-2-ylamine (9.49 g) as yellow solid in 27% yield. ESMS *m*/z (relative intensity): 154 (M+H)⁺ (100).

### Example 133: 2-Bromo-5-methyl-3-nitro-pyridine:

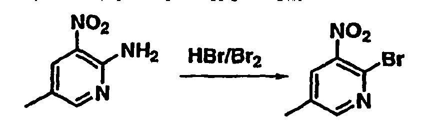

To concentrated HBr (48%, 28.6 mL) was added 5-methyl-3-nitro-pyridin-2-ylamine (5 g, 32.6 mmol) in portions at 0 °C temperature with stirring. The mixture was stirred until the internal temperature reached to -10 °C, then bromine was added drop wise. A solution of NaNO₂ (7.6 g, 110.84 mmol) in water (11 mL) was added slowly to maintain the reaction mixture temperature below 0 °C. The dark mixture (gas evolution was observed) was stirred for 1 h at 0 °C then was carefully treated (slow addition) with a solution of NaOH (12 g, 300 mmol) in water (17 mL) while maintaining the internal temperature below 20 °C. The mixture was stirred for an additional 1 h, then was filtered, dried under vacuum for 6 h and purified by recrystalization using 95% EtOH to get pure *2-bromo-5-methyl-3-nitro-pyridine* (1.96 g) as yellow solid in 28% first crop yield. ESMS *m*/*z* (relative intensity): 217 (M+H)⁺ (100).

### Example 134: 2-Bromo-5-methyl-pyridin-3-ylamine

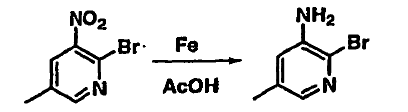

To Fe power (2.18 g, 39.0 mmol), AcOH (10 mL) was added drop wise and heated to 80 °C. To it, a solution of 2-bromo-5-methyl-3-nitro-pyridine (1.96 g, 9.07 mmol) in AcOH (10 mL) was added slowly. After 30 min, reaction mixture was allowed to cool to room temperature and diluted with EtOAc (25 mL), filtered through a pad of celite. The filter cake was washed with EtOAc (25 mL) and filterate was concentrated. The residual liquid was slowly treated with saturated aqueous NaHCO₃ solution (70 mL), followed by small portions of solid NaHCO₃ to neutralize the AcOH, extracted with EtOAc (2 x 50 mL) and the extracts were dried (anhydrous Na₂SO₄) and concentrated under reduced pressure to get *2-bromo-5-mehtyl-pyridin-3-ylamine* (1.45 g) as a brown solid in 86.3% yield. ESMS *m*/*z* (relative intensity): 187 (M+H)⁺ (100).

### Example 135: N-(2-Bromo-5-methyl-pyridin-3-yl)-4-chloro-3-trifluoromehtyl benzenesulfonamide

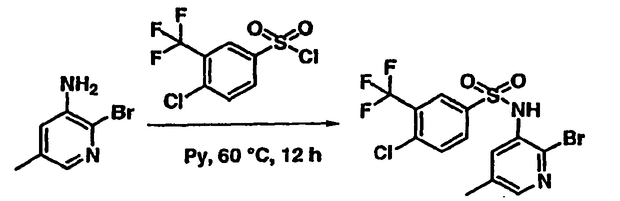

A solution of 2-bromo-5-mehtyl-pyridin-3-ylamine (1 g, 5.38 mmol) and 4-chloro-3-trifluoromethylbenzenesulfonylchloride (1.8 g, 6.46 mmol) in pyridine (5 mL) was stirred at 60 °C for 12 h. The mixture was concentrated under reduced pressure and to it, was added 1:1 EtOAc-10% aqueous HCl (50 mL). Aqueous layer was separated and extracted with EtOAc (2 x 50 mL). Combined EtOAc layers were washed with 10% aqueous HCl (50 mL), dried (anhydrous Na₂SO₄), concentrated and column purified (SiO₂, 50% EtOAc-hexanes) to obtain N-(2-bromo-5-methyl-pyridin-3-yl)-4-chloro-3-trifluoromehtyl-benzenesulfonamide (1.42 g) in 84% yield as a half white solid. ESMS *m*/*z* (relative intensity): 429 (M+H)⁺(100).

### Example 136: N-(2-bromo-5-methy-pyridin-3 yl)-4-chloro-N methoxy mehtyl-3-trifluoromethyl-benzenesulfonamide

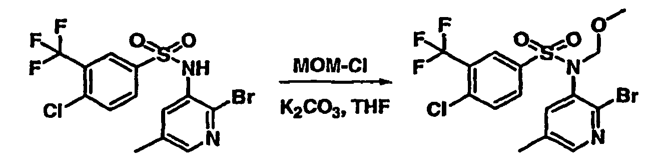

A mixture of *N-*(2-bromo-5-methyl-pyridin-3-yl)-4-chloro-3-trifluoromehtyl-benzenesulfonamide (1.4 g, 3.27 mmol), chloromethyl methyl ether (0.4 mL, 5.23 mmol) and K₂CO₃ (1.35 g, 9.81 mmol) in THF (20 mL) was stirred at room temperature for 3 h. The mixture was filtered and the filtrate was concentrated and purified by flash chromatography (SiO₂, 20% EtOAc-hexanes) to afford N-(2-bromo-5-methyl-pyridin-3-yl)-4-chloro-*N*-methoxymehtyl-3-trifluoromethyl-benz-enesulfonamide (1.3 g) as a white solid in 84% yield. ESMS *m*/*z* (relative intensity): 473 (M+H)⁺ (100).

### Example 137: 4-Chloro-N-{2-[(2,4-dimethyl-pyridin-3-yl)-hydroxy-methyl]-5-methyl]-5-methyl-pyridin-3-yl}-N-methoxymethyl-3-trifluoromethyl-benzenesulfonamide

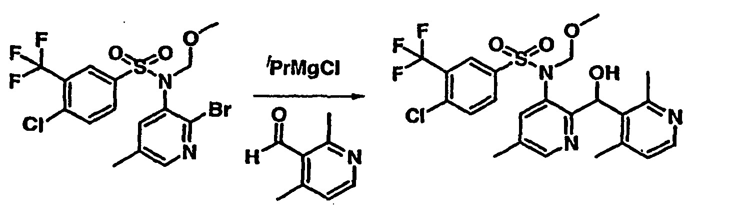

To a solution of *N-*(2-bromo-5-methyl-pyridin-3-yl)-4-chloro-*N-*methoxymehtyl-3-trifluoromethyl-benzenesulfonamide (500 mg, 1.06 mmol) in THF (3 mL) under nitrogen atmosphere at -30 °C was added drop wise isopropylmagnesium chloride (2 M solution in THF, 1.27 mL, 2.54 mmol). The mixture was then stirred for 30 min at 0 °C. followed by the addition of a solution of 2,4-dimethyl-pyridine-3-carbaldehyde (271 mg, 2.01 mmol) at -30 °C. The mixture was stirred at room temperature for 3 hours, quenched with saturated aqueous NH₄Cl solution (5 mL) and extracted with EtOAc (2 x 25 mL). Combined organic layers were washed with saturated aqueous NH₄Cl solution (25 mL), brine (25 mL), dried (anhydrous Na₂SO₄) and concentrated under reduced pressure. Obtained residue was column purified (SiO₂, 50% EtOAc-hexanes) to obtain 4-chloro-N-{2-[(2,4-dimethyl-pyridin-3-yl)-hydroxy-methyl]-5-methyl]-5-methyl-pyridin-3-yl}-N-methoxymethyl-3-trifluoromethyl-benzene sulfonamide (288 mg) as yellow foam in 51% yield. ESMS *m*/*z* (relative intensity): 530 (M+H)⁺ (100).

### Example 138: 4-Chloro-N-[2-(2,4-dimethyl-pyridine-3-carbonyl)-5-methyl-pyridin-3-yl]-N-methoxymethyl-3-trifluoromethyl-benzenesulfonamide

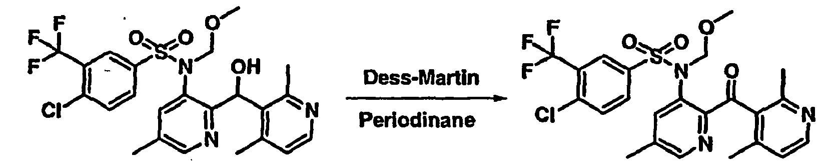

To a solution of 4-chloro-*N-*{2-[(2,4-dimethyl-pyridin-3-yl)-hydroxymethyl]-5-methyl]-5-methyl-pyridin-3-yl}-*N-*mehtoxymethyl-3-trifluoromethylbenzene sulfonamide (280 mg, 0.53 mmol) in CH₂Cl₂ (5 mL) was added Dess-Martin periodinane (403 mg, 0.95 mmol) and stirred for 3 h at room temperature. 10% Na₂S₂O₃ (5 mL) and saturated aqueous NaHCO₃ solution (5 mL) was added and stirred for 30 min. Aqueous layer was separated and extracted with EtOAc (2 x 25 mL). Combined organic layers were washed with saturated aqueous NaHCO₃ solution (20 mL), brine (20 mL), dried (anhydrous Na₂SO₄), concentrated to obtain 4-chloro-N-[2-(2,4-dimethyl-pyridine-3-carbonyl)-5-methyl-pyridin-3-yl]-N-methoxymethyl-3-trifluoromethyl-benzenesulfonamide (277 mg) as thick yellow syrup in quantitative crude yield which was used for further transformation without purification. ESMS *m*/*z* (relative intensity): 528 (M+H)⁺ (100).

### Example 139: 4-Chloro-N-[2-(2,4-dimethyl-pyridine-3-carbonyl)-5-methyl-pyridin-3-yl]-3-trifluoromethyl-benzenesulfonamide

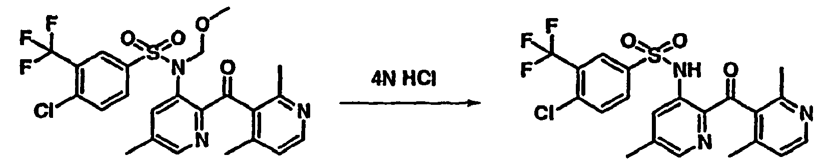

A mixture of 4-chloro-N-[2-(2,4-dimethyl-pyridine-3-carbonyl)-5-methyl-pyridin-3-yl]-N-methoxymethyl-3-trifluoromethyl-benzenesulfonamide (260 mg, 0.49 mmol) in 4M HCl in dioxane (5 mL) and water (1 mL) was refluxed for 3 h. Reaction mixture was cooled to room temperature, evaporated to dryness and treated with saturated aqueous NaHCO₃ Solution till pH 7-8. The mixture was extracted with EtOAc (2 x 25 mL), dried (anhydrous Na₂SO₄) and concentrated. The obtained residue was purified by flash chromatography (SiO2, 70% EtOAc-hexanes) to afford 4-chloro-N-[2-(2,4-dimethyl-pyridine-3-carbonyl)-5-methyl-pyridin-3-yl]-3-trifluoromethyl-benzenesulfonamide (78 mg) as an off white solid (after lyophilization) in 33% yield. ESMS *m*/*z* (relative intensity): 484 (M+H)⁺ (100).

### Example 140: -4-Chloro-N-{2-[(2-chlorophenyl)-hydroxy-methyl]-5-methyl]-5-methyl-pyridin-3-yl}-N-methoxymethyl-3-trifluoromethyl-benzenesulfonamide

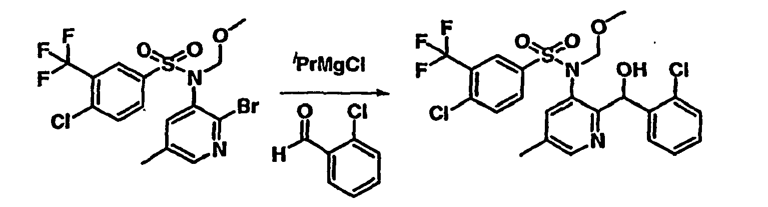

To a solution of *N-*(2-bromo-5-methyl-pyridin-3-yl)-4-chloro-*N-*methoxymethyl-3-trifluoromethyl-benzenesulfonamide (500 mg, 1.06 mmol) in THF (3 mL) under nitrogen atmosphere at -30 °C was added drop wise isopropylmagnesium chloride (2 M solution in THF, 1.27 mL, 2.54 mmol). The mixture was then stirred for 30 min at 0 °C followed by the addition of a solution of 2-chlorobenzaldehyde (271 µL, 2.01 mmol) at -30 °C. The mixture was stirred at room temperature for 3 hours, quenched with saturated aqueous NH₄Cl solution (5 mL) and extracted with EtOAc (2 x 25 mL). Combined organic layers were washed with saturated aqueous NH₄Cl solution (25 mL), brine (25 mL), dried (anhydrous Na₂SO₄) and concentrated under reduced pressure. Obtained residue was column purified (SiO₂, 50% EtOAc-hexanes) to obtain 4-chloro-N-12-[(2-chlorophenyl)-hydroxy-methyl]-5-methyl]-5-methyl-pyridin-3-yl}-N-methoxymethyl-3-trifluoromethyl-benzenesulfonamide (274 mg) as yellow foam in 48% yield. ESMS *m*/*z* (relative intensity): 535 (M+H)⁺ (100).

### Example 141: 4-Chloro-N-[2-(2-chloro-benzoyl)-5-methyl-pyridin-3-yl]-N-methoxymethyl-3-trifluoromethyl-benzenesulfonamide

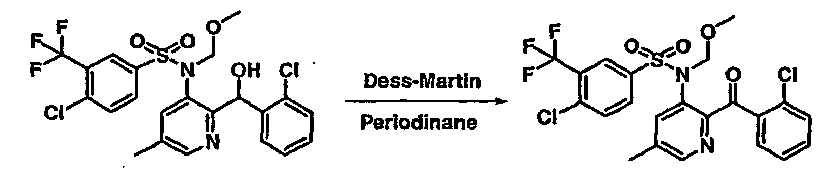

To a solution of 4-chloro-N-{2-[(2-chlorophenyl)-hydroxy-methyl]-5-methyl]-5-methyl-pyridin-3-yl}-N-mehtoxymethyl-3-trifluoromethyl-benzene sulfonamide (260 mg, 0.49 mmol) in CH₂Cl₂ (5 mL) was added Dess-Martin periodinane (373 mg, 0.88 mmol) and stirred for 3 h at room temperature. 10% Na₂S₂O₃ (5 mL) and saturated aqueous NaHCO₃ solution (5 mL) was added and stirred for 30 min. Aqueous layer was separated and extracted with EtOAc (2 x 25 mL). Combined organic layers were washed with saturated aqueous NaHCO₃ solution (20 mL), brine (20 mL), dried (anhydrous Na₂SOₐ), concentrated to obtain 4-chloro-N-[2-(2-chloro-benzoyl)-5-methyl-pyridin-3-yl]-N-methoxymethyl-3-trifluoromethyl-benzenesulfonamide (260 mg) as thick yellow syrup in quantitative crude yield which was used for further transformation without purification. ESMS *m*/*z* (relative intensity): 501 (M-MeOH)⁺ (100).

### Example 142: 4-Chloro-N-[2-(2-chloro-benzoyl)-5-methyl-pyridin-3-yl]-3-trifluoromethyl benzenesulfonamide

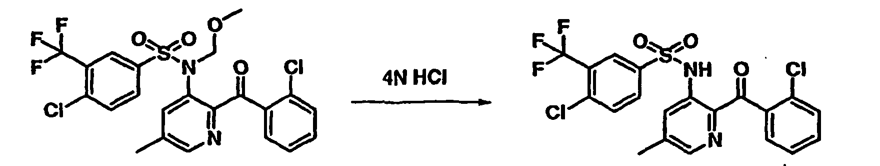

A mixture of 4-chloro-N-[2-(2-chloro-benzoyl)-5-methyl-pyridin-3-yl]-N-methoxymethyl-3-trifluoromethyl-benzenesulfonamide (260 mg, 0.49 mmol) in 4M HCl in dioxane (5 mL) and water (1 mL) was refluxed for 3 h. Reaction mixture was cooled to room temperature, evaporated to dryness and treated with saturated aqueous NaHCO₃ solution till pH 7-8. The mixture was extracted with EtOAc (2 x 25 mL), dried (anhydrous Na₂SO₄) and concentrated. The obtained residue was purified by flash chromatography (SiO2, 70% EtOAc-hexanes) to afford 4-chloro-N-[2-(2-chloro-benzoyl)-5-methyl-pyridin-3-yl]-3-trifluoromethyl-benzenesulfonamide (106 mg) as a white solid (after lyophilization) in 45% yield. ESMS *m*/*z* (relative intensity): 489 (M+H)⁺(100).

### Example 143: 2-{5-Chloro-3-[(4-chloro-3-trifluoromethyl-benzenesulfonyl)-methoxymethyl-amino]-pyridine-2-carbonyl}-benzoic acid

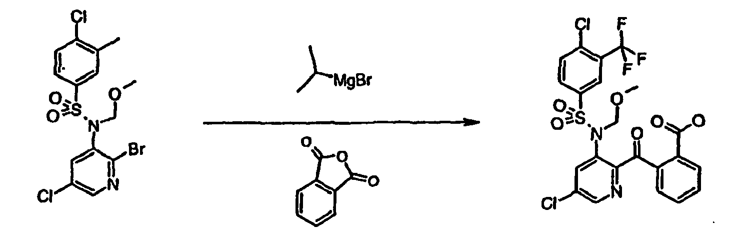

To a solution of N-(2-Bromo-5-chloro-pyridin-3-yl)-4-chloro-N-methoxymethyl-3-trifluoromethyl-benzenesulfonamide (250 mg, 0.50 mmol) in 4.0 ml of THF under nitrogen atmosphere at 0 °C was added drop-wise 0.6 ml (1.2 mmol) of isopropylmagnesium chloride. The mixture was then stirred for 20 min at 0 °C followed by addition of a solution of phthalic anhydride (148mg, 1.0 mmol) in 1ml of DCM. The mixture was stirred at room temperature overnight, quenched with saturated ammonium chloride and extracted with ethyl acetate. After concentration the residue was purified by flash chromatograph (70% ethyl acetate in hexane) to afford 80 mg of title compound as an off white solid. MS: (M+H) /z = 564.0, (M-32)/z = 532.0.

### Example 144: 2-[5-Chloro-3-(4-chloro-3-trifluoromethyl-benzenesulfonylamino)-pyridine-2-carbonyl]-benzoic acid

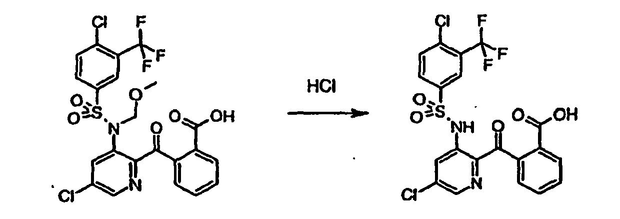

A mixture of 2-{5-Chloro-3-[(4-chloro-3-trifluoromethyl-benzenesulfonyl)-methoxymethyl-amino]-pyridine-2-carbonyl}-benzoic acid (150 mg, 0.27 mmol) in 3 ml of 4M HCl in dioxane and 1 ml of water was refluxed for 2 hours. After cooling to room temperature the mixture was concentrated and then diluted with water. Sodium bicarbonate was added until PH was 6. The mixture was extracted with ethyl acetate, dried and concentrated. The residue was further purified via flash column (70% ethyl acetate in hexane) to afford 60 mg of title compound as an off white solid. MS: (M+H) /z = 519.4.

### Example 145: 2-Oxazol-2-yl-benzoic acid methyl ester

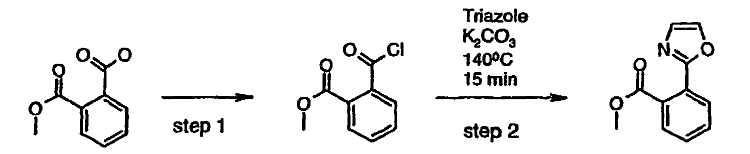

Step 1: To a solution of phthalic acid monomethyl ester (1.8g, 10mmol)) and 0.020 ml of DMF in 10 ml of DCM was added oxalyl chloride (1.52 g, 12mmol)). The mixture was stirred at room temperature for 2 hours and then concentrated. The residue was used directly for the step 2.

Step 2: A mixture of 2-Chlorocarbonyl-benzoic acid methyl ester (1.3 gram, 6.56 mmol), 1H-triazole (0.42 ml, 7.22 mmol) and potassium carbonate (2.78g, 20 mmol) in 12 ml of tetramethylene sulfone was heated at 140°C for half an hour. The mixture was cooled, diluted with ethyl acetate, washed with water 5 times, and dried. After concentration the crude was purified by flash column (50% ethyl acetate in hexane) to afford 250 mg of 2-Oxazol-2-yl-benzoic acid methyl ester as a light yellow liquid. MS: (M+H) /z = 204.1.

### Example 146: N-Methoxy-N-methyl-2-oxazol-2-yl-benzamide

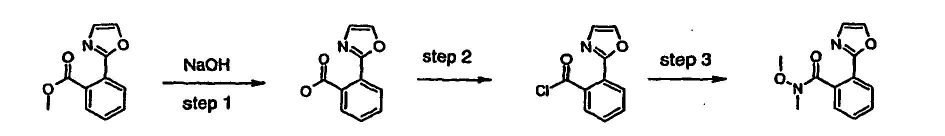

Step 1: A mixture of 2-Oxazol-2-yl-benzoic acid methyl ester (200mg) in a mixture of 5ml of methanol and 10 ml of 1 M NaOH was stirred for 3 hours at room temperature. The mixture was neutralized with 2N HCl until pH was 4. The mixture was concentrated and 30 ml of ethyl acetate was added and the resulting mixture was filtrated, the filtrate was concentrated and the residue was used directly at step 2.

Step 2: 2-Oxazol-2-yl-benzoyl chloride was made by according to the previously described procedure.

Step 3: To a mixture of 2-Oxazol-2-yl-benzoyl chloride (104 mg, 0.5 mmol), DIEA (0.26 mL, 1.5 mmol) in 2 ml of DCM was added N,O-dimethylhydroxylamine hydrochloride (59 mg, 0.6 mmol). The mixture was stirred at room temperature for 2 hours, washed with dilute HCl solution followed by brine. Concentration of the organic layer afforded 100 mg of N-Methoxy-N-methyl-2-oxazol-2-yl-benzamide as a sticky light yellow liquid. MS: (M+H) /z = 233.1.

### Example 147: 4-Chloro-N-[5-chloro-2-(2-oxazol-2-yl-benzoyl)-pyridin-3-yl]-N-methoxymethyl-3-trifluoromethyl-benzenesulfonamide

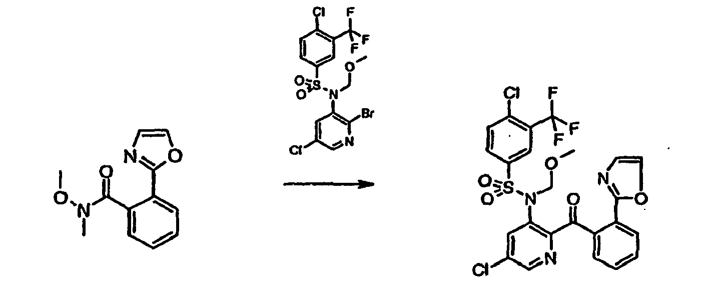

To a solution of N-(2-Bromo-5-chloro-pyridin-3-yl)-4-chloro-N-methoxymethyl-3-trifluoromethyl-benzenesulfonamide (125 mg, 0.25 mmol) in 2.0 mL of THF under nitrogen atmosphere at 0°C was added drop-wise 0.3 ml (0.6 mmol) of isopropylmagnesium chloride. The mixture was then stirred for 20 min at 0 °C followed by addition of N-Methoxy-N-methyl-2-oxazol-2-yl-benzamide (100mg, 0.43 mmol). The mixture was stirred at room temperature overnight, quenched with saturated ammonium chloride and extracted with ethyl acetate. After concentration the residue was purified by flash chromatograph (30% ethyl acetate in hexane) to afford 30 mg of title compound as an off white solid. MS: (M+H) /z = 586.1.

### Example 148: 4-Chloro-N-[5-chloro-2-(2-oxazol-2-benzoyl)-pyridin-3-yl]-3-trifluoromethyl-benzenesulfonamide

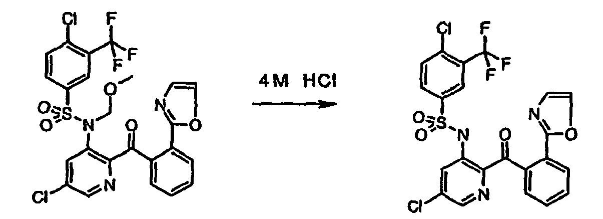

A mixture of 4-Chloro-N-[5-chloro-2-(2-oxazol-2-yl-benzoyl)-pyridin-3-yl]-N-methoxymethyl-3-trifluoromethyl-benzenesulfonamide(15 mg) in 2 mL of 4M HCl in dioxane and 1 mL of water was refluxed for 2 hours. After cooling to rt the mixture was concentrated, diluted with water and then sodium bicarbonate was added until pH was 6. The mixture was extracted with ethyl acetate, dried and concentrated. The residue was purified via flash column (35% ethyl acetate in hexane) to afford 8.0 mg of title compound as an off white solid. MS: (M+H) /z = 542.3.

### Example 149: N-[2-(2-Bromo-benzoyl)-5-chloro-pvridin-3-yl]-4-chloro-N-methoxymethyl-3-trifluoromethyl-benzenesulfonamide

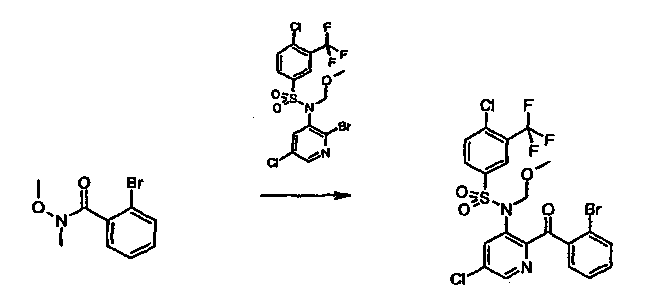

To a solution of N-(2-Bromo-5-chloro-pyridin-3-yl)-4-chloro-N-methoxymethyl-3-trifluoromethyl-benzenesulfonamide (250 mg, 0.50 mmol) in 4.0 ml of THF under nitrogen atmosphere at 0°C was added drop-wise 0.6 mLl (1.2 mmol) of isopropylmagnesium chloride. The mixture was then stirred for 20 min at 0 °C followed by addition of a solution of 2-Bromo-N-methoxy-N-methyl-benzamide(244mg, 1.0 mmol) in 1mL of THF. The mixture was stirred at room temperature overnight, quenched with saturated ammonium chloride and extracted with ethyl acetate. After concentration of the solvent the residue was purified by flash chromatograph (20% ethyl acetate in hexane) to afford 180 mg of title compound as an off white solid. MS: (M+Na) /z = 621.2, (M-32)/z = 567.0.

### Example 150: N-[2-(2-Bromo-benzoyl)-5-chloro-pyridin-3-yl]-4-chloro-3-trifluoromethyl-benzenesulfonamide

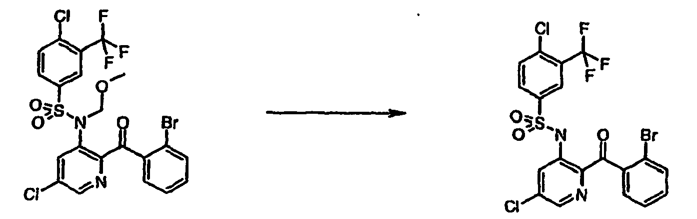

A mixture of N-[2-(2-Bromo-benzoyl)-5-chloro-pyridin-3-yl]-4-chloro-N-methoxymethyl-3-trifluoromethyl-benzenesulfonamide (100 mg, 0.167 mmol) in 3 ml of 4M HCl in dioxane and 1 mL of water was refluxed for 3 hours. After cooling to rt the mixture was concentrated, diluted with water and then sodium bicarbonate was added until pH was 6. The mixture was extracted with ethyl acetate, dried and concentrated. The residue was further purified via flash column (30% ethyl acetate in hexane) to afford 50 mg of title compound as an off white solid. MS: (M+H) /z = 555.3, (M+Na)/z = 575.3.

### Example 151: 4-Chloro-N-[5-chloro-2-(1-hydroxy-2,4-dimethyl-pyridine-3-carbonyl)-pyridin-3-yl]-N-methoxymethyl-3-trifluoromethyl-benzenesulfonamide

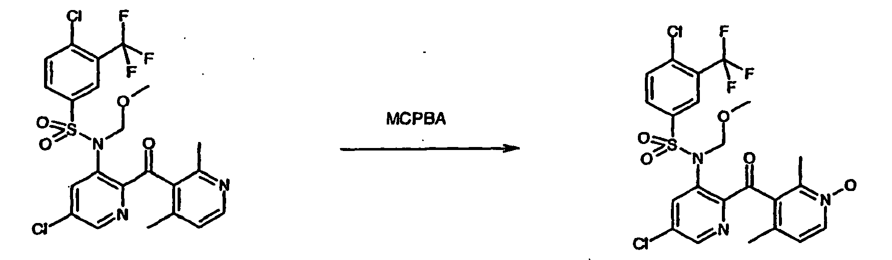

To a solution of 4-Chloro-N-[5-chloro-2-(2,4-dimethyl-pyridine-3-carbonyl)-pyridin-3-yl]-N-methoxymethyl-3-trifluoromethyl-benzenesulfonamide (100mg, 0.182 mmol) in 3,5 mL of DCM was added meta-chloroperoxybenzoic acid (77%, 95mg, 0.424 mmol). After thirty hours the crude mixture was added 1.0 ml of pyridine and the mixture was concentrated. The mixture was diluted with ethyl acetate, washed with water, dried over sodium sulfate and concentrated. The crude was used at next step without any further purification. MS: (M+H) /z = 564.0.

### Example 152: 4-Chloro-N-[5-chloro-2-(1-hydroxy-2,4-dimethyl-pyridine-3-carbonyl)-pyridin-3-yl]-3-trifluoromethyl-benzenesulfonamide

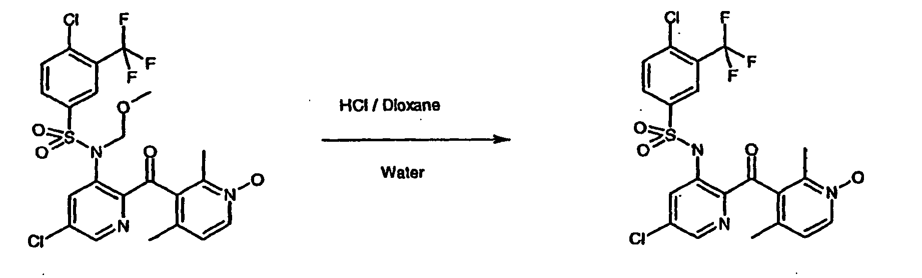

A solution of 4-Chloro-N-[5-chloro-2-(1-hydroxy-2,4-dimethylpyridine-3-carbonyl)-pyridin-3-yl]-N-methoxymethyl-3-trifluoromethyl-benzenesulfonamide (21 mg) in 6 ml of HCl (4M in dioxane) and 2 mL of water was refluxed for 2 hours. After cooling to rt the mixture was concentrated and was added 3 mL of water. Sodium bicarbonate was added until the final pH was 6. The mixture was extracted with ethyl acetate, dried and concentrated. The residue was further purified via short flash column (8% methanol in DCM) to afford 7.9 mg of title compound as an off white solid. MS: (M+H) /z = 520.0.

### Example 153: 4-Chloro-N-[5-chloro-2-(2-chloro-benzoyl)-pyridin-3-yl]-3-trifluoromethyl-benzenesulfonamide

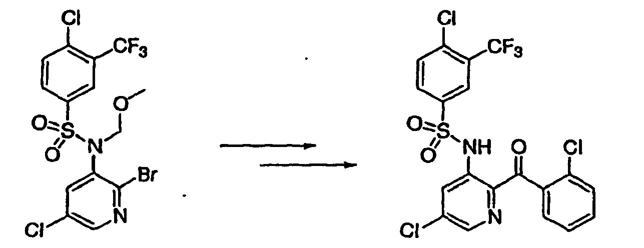

To a magnetically stirred solution of N-(2-bromo-5-chloro-pyridin-3-yl)-4-chloro-N-methoxymethyl-3-(trifluoromethyl)-benzenesulfonamide (1.5 g, 3.03 mmol) in anhydrous THF (15 mL) was added 2 M isopropylmagnesium chloride in THF (3.3mL, 6.6 mmol) at -20 °C. The temperature was allowed to slowly rise to 10° and then 2-chloro-N-methoxy-N-methylbenzamide (1.2 g, 6.0 mmol) was added and the progress of the reaction was followed by LCMS. The reaction mixture was warmed to room temperature and stirred for 16 h. It was then quenched with saturated aqueous NH₄Cl (50 mL), and extracted with ethyl acetate (3 x 100mL). The combined extracts were dried (MgSO₄) filtered and concentrated. The residue was purified by chromatography on silica gel using ethyl acetate-hexane mixtures (2:98, 5:95) to provide 4-chloro-N-[5-chloro-2-(2-chloro-benzoyl)-pyridin-3-yl]-N-methoxymethyl-3-trifluoromethyl-benzenesulfonamide mass spectrum m/z 574.8 (M + Na); 521.0 (M - 31).

The intermediate MOM derivative (1.1 g, 1.99 mmol) was stirred in water (2.5 mL) and 4N HCl in dioxane (12 mL) and heated at 100 °C (oil bath) for 2.5 h. LCMS indicated complete reaction; the reaction was concentrated and the residue was neutralized with saturated aqueous sodium bicarbonate and extracted with ethyl acetate (3 x 80 mL). The extracts were dried (MgSO₄), filtered, and chromatographed on silica gel using EtOAc-hexane mixtures (2:98, 5:95) to provide pure product. mass spectrum m/z 510.9 (M + H); 532.9 (M + Na).

### Example 154: 5-Chloro-2-(2,6-dimethyl-phenoxy)-pyridin-3-ylamine

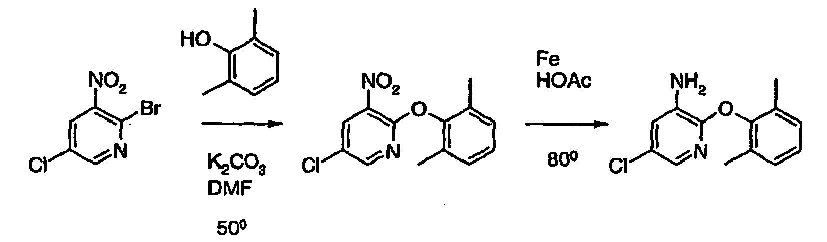

2-Bromo-3-nitro-5-chloropyridine (4.8g, 20mmol) and 2,6-dimethylphenol (5.0g, 41 mmol) were magnetically stirred in dry DMF (65mL) and potassium carbonate () was added. The mixture was heated at 50 °C for 4 days, allowed to cool to room temperature and added to ice; the product was extracted with ethyl acetate (3 x 100 mL). The extracts were washed with saturated aqueous NaHCO3 and dried (MgSO₄), filtered and concentrated to provide the desired product.

The nitro compound (4.5g, 16 mmol) was dissolved in glacial acetic acid (80 mL) and this solution was added dropwise to a well-stirred suspension of iron powder (4.5 g, 80 mmol) in glacial acetic acid (40 mL) heated in an oil bath at 80 °C under nitrogen. The progress of the reaction was checked by LCMS. After 20 min, the reaction was allowed to cool and was diluted with ethyl acetate (120 mL). The resulting mixture was vacuum filtered through a pad of Celite, the filter cake was washed with ethyl acetate (100 mL) and the filtrate was concentrated. The residue was slowly treated with saturated aqueous sodium bicarbonate, followed by the addition of small portions of solid sodium bicarbonate to neutralize the acetic acid. The mixture was extracted using ethyl acetate (3 x 150 mL) and the extracts were dried (MgSO₄), filtered and concentrated (rotovap). The product was isolated as a crystalline solid after drying (vacuum). mass spectrum m/z 251.3 (M + H).

### Example 155: 4-Chloro-N-[5-chloro-2-(2-hydroxymethyl-4-methyl-pyridine-3-carbonyl)-pyridin-3-yl]-3-trifluoromethyl-benzenesulfonamide and 4-Chloro-N-[5-chloro-2-(4-hydroxymethyl-2-methyl-pyridine-3-carbonyl)-pyridin-3-yl]-3-trifluoromethyl-benzenesulfonamide

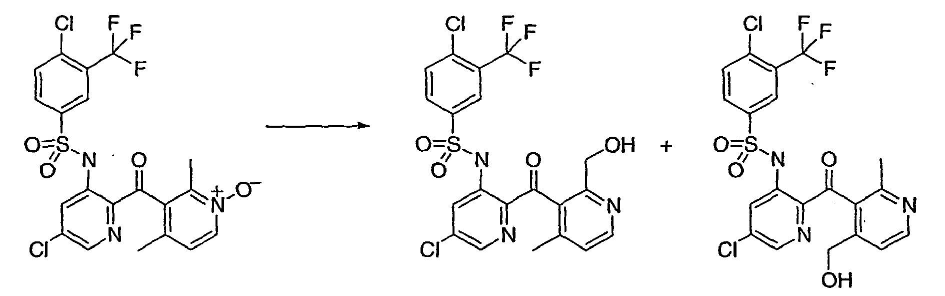

The starting N-Oxide (1.0 g, 1.92 mmol) was dissolved in methylene chloride (35mL) and cooled in an ice bath. Trifluoroacetic anhydride (0.7 mL, 1.05 g) was added dropwise to the magnetically stirred solution, and more (0.7 mL) was added after 5 min. The deep red reaction mixture was stirred 16h at room temperature; no starting material was detected by LCMS. The reaction was cooled to 0 °C and water (20 mL) was added dropwise, then the mixture was allowed to come to room temperature. The layers were separated and the water layer was extracted with methylene chloride (2 x 25 mL). The combined organic layer was dried (MgSO₄), filtered and concentrated to give a red oil. Chromatography on silica gel (ethyl acetate - hexane) using a 5:95 to 95:5 gradient gave several mixed fractions and a few fractions each of the two products. Major Isomer: 4-Chloro-N-[5-chloro-2-(2-hydroxymethyl-4-methylpyridine-3-carbonyl)-pyridin-3-yl]-3-trifluoromethyl-benzenesulfonamide mass spectrum m/z 520.4 (M +1). Minor Isomer: 4-Chloro-N-[5-chloro-2-(4-hydroxymethyl-2-methyl-pyridine-3-carbonyl)-pyridin-3-yl]-3-trifluoromethyl-benzenesulfonamide. mass spectrum m/z 520.4 (M +1).

### Measuring Efficacy of Chemokine Modulators

### In Vitro Assays

A variety of assays can be used to evaluate the compounds provided herein, including signaling assays, migration assays, ligand binding assays, and other assays of cellular response. Chemokine receptor signaling assays can be used to measure the ability of a compound, such as a potential CCR2 antagonist, to block CCR2 ligand- (e.g. MCP-1)-induced signaling or a potential CCR9 antagonist, to block CCR9 ligand- (e.g. TECK)-induced signaling. A migration assay can be used to measure the ability of a compound of interest, such as a possible chemokine antagonist, to block chemokine-mediated cell migration in vitro. The latter is believed to resemble chemokine-induced cell migration in vivo. A ligand binding assay can be used to measure the ability of a compound, such as a potential CCR2 antagonist, to block the interaction of MCP-1 with its receptor or a potential CCR9 antagonist, to block the interaction of TECK with its receptor.

In a suitable assay, a chemokine protein (whether isolated or recombinant) is used which has at least one property, activity, or functional characteristic of a mammalian chemokine protein. The property can be a binding property (to, for example, a ligand or inhibitor), a signaling activity (e.g., activation of a mammalian G protein, induction of rapid and transient increase in the concentration of cytosolic free calcium), cellular response function (e.g., stimulation of chemotaxis or inflammatory mediator release by leukocytes), and the like.

The assay can be a cell-based assay that utilizes cells stably or transiently transfected with a vector or expression cassette having a nucleic acid sequence that encodes the chemokine receptor. Cell lines naturally expressing the chemokine can also be used. The cells are maintained under conditions appropriate for expression of the receptor and are contacted with a putative agent under conditions appropriate for binding to occur. Binding can be detected using standard techniques. For example, the extent of binding can be determined relative to a suitable control (for example, relative to background in the absence of a putative agent, or relative to a known ligand). Optionally, a cellular fraction, such as a membrane fraction, containing the receptor can be used in lieu of whole cells.

Detection of binding or complex formation can be detected directly or indirectly. For example, the putative agent can be labeled with a suitable label (e.g., fluorescent label, chemiluminescent label, isotope label, enzyme label, and the like) and binding can be determined by detection of the label. Specific and/or competitive binding can be assessed by competition or displacement studies, using unlabeled agent or a ligand (e.g., MCP-1 or TECK) as a competitor.

Binding inhibition assays can be used to evaluate the present compounds. In these assays, the compounds are evaluated as inhibitors of ligand binding using, for example, MCP-1 or TECK. In one embodiment, the CCR2 receptor is contacted with a ligand such as MCP-1 and a measure of ligand binding is made. The receptor is then contacted with a test agent in the presence of a ligand (e.g., MCP-1) and a second measurement of binding is made. In another embodiment, the CCR9 receptor is contacted with a ligand such as TECK and a measure of ligand binding is made. The receptor is then contacted with a test agent in the presence of a ligand (e.g., TECK) and a second measurement of binding is made. A reduction in the extent of ligand binding is indicative of inhibition of binding by the test agent. The binding inhibition assays can be carried out using whole cells which express the chemokine, or a membrane fraction from cells which express the chemokine.

The binding of a G protein coupled receptor by, for example, an agonist, can result in a signaling event by the receptor. Accordingly, signaling assays can also be used to evaluate the compounds of the present invention and induction of signaling function by an agent can be monitored using any suitable method. For example, G protein activity, such as hydrolysis of GTP to GDP, or later signaling events triggered by receptor binding can be assayed by known methods (see, for example, PCT/US97/15915; Neote et al., Cell, 72:415425 (1993); Van Riper et al., J. Exp. Med., 177:851-856 (1993) and Dahinden et al., J. Exp. Med., 179:751-756 (1994)).

Chemotaxis assays can also be used to assess receptor function and evaluate the compounds provided herein. These assays are based on the functional migration of cells in vitro or in vivo induced by an agent, and can be used to assess the binding and/or effect on chemotaxis of ligands, inhibitors, or agonists. A variety of chemotaxis assays are known in the art, and any suitable assay can be used to evaluate the compounds of the present invention. Examples of suitable assays include those described in PCT/US97/15915; Springer et al., WO 94/20142; Berman et al., Immunol. Invest., 17:625-677 (1988); and Kavanaugh et al., J. Immunol., 146:4149-4156 (1991)).

Calcium signaling assays measure calcium concentration over time, preferably before and after receptor binding. These assays can be used to quantify the generation of a receptor-signaling mediator, Ca⁺⁺, following receptor binding (or absence thereof). These assays are useful in determining the ability of a compound, such as those of the present invention, to generate the receptor signaling mediator by binding to a receptor of interest. Also, these assays are useful in determining the ability of a compound, such as those of the present invention, to inhibit generation of the receptor signaling mediator by interfering with binding between a receptor of interest and a ligand.

In calcium signaling assays used to determine the ability of a compound to interfere with binding between a chemokine and a known chemokine ligand, chemokineCCR2-expressing cells (CCR2-expressing cells such as THP-1 cells or CCR9-expressing cells such as T cell line MOLT-4 cells) are first incubated with a compound of interest, such as a potential chemokine antagonist, at increasing concentrations. The cell number can be from 10⁵ to 5 x 10⁵ cells per well in a 96-well microtiter plate. The concentration of the compound being tested may range from 0 to 100 µM. After a period of incubation (which can range from 5 to 60 minutes), the treated cells are placed in a Fluorometric Imaging Plate Reader (FLIPR^{®}) (available from Molecular Devices Corp., Sunnyvale, CA) according to the manufacturer's instruction. The FLIPR^{®} system is well known to those skilled in the art as a standard method of performing assays. The cells are then stimulated with an appropriate amount of the chemokine ligand (MCP-1 for CCR2 or TECK for CCR9) at 5-100 nM final concentration, and the signal of intracellular calcium increase (also called calcium flux) is recorded. The efficacy of a compound as an inhibitor of binding between the chemokine and the ligand can be calculated as an IC₅₀ (the concentration needed to cause 50% inhibition in signaling) or IC₉₀ (at 90% inhibition).

In vitro cell migration assays can be performed (but are not limited to this format) using the 96-well microchamber (called ChemoTX^{™}). The ChemoTX^{™} system is well known to those skilled in the art as a type of chemotactic/cell migration instrument. In this assay, CCR2-expressing cells (such as THP-1) or CCR9-expressing cells (such as MOLT-4) are first incubated with a compound of interest, such as a possible CCR2 or CCR9 antagonist, at increasing concentrations. Typically, fifty thousand cells per well are used, but the amount can range from 10³-10⁶ cells per well. The chemokine ligand (for example, CCR2 ligand MCP-1, typically at 0.1 nM (but can range from 5-100 nM ); or CCR9 ligand TECK, typically at 50 nM (but can range from 5-100 nM)), is placed at the lower chamber and the migration apparatus is assembled. Twenty microliters of test compound-treated cells are then placed onto the membrane. Migration is allowed to take place at 37°C for a period of time, typically 1.5 hours for CCR2 or 2.5 hours for CCR9. At the end of the incubation, the number of cells that migrated across the membrane into the lower chamber is then quantified. The efficacy of a compound as an inhibitor of chemokine-mediated cell migration is calculated as an IC₅₀ (the concentration needed to reduce cell migration by 50%) or IC₉₀ (for 90% inhibition).

### In vivo efficacy models for human IBD

T cell infiltration into the small intestine and colon have been linked to the pathogenesis of human inflammatory bowel diseases which include Coeliac disease, Crohn's disease and ulcerative colitis. Blocking trafficking of relevant T cell populations to the intestine is believed to be an effective approach to treat human IBD. CCR9 is expressed on gut-homing T cells in peripheral blood, elevated in patients with small bowel inflammation such as Crohn's disease and Coeliac disease. CCR9 ligand TECK is expressed in the small intestine. It is thus believed that this ligand-receptor pair plays a role in IBD development by mediating migration of T cells to the intestine. Several animal models exist and can be used for evaluating compounds of interest, such as potential CCR9 antagonists, for an ability to affect such T cell migration and/or condition or disease, which might allow efficacy predictions of antagonists in humans.

### Animal models with pathology similar to human ulcerative colitis

A murine model described by Panwala and coworkers (Panwala, et al., J Immunol., 161 (10):5733-44 (1998)) involves genetic deletion of the murine multi-drug resistant gene (MDR). MDR knockout mice (MDR-/-) are susceptible to developing a severe, spontaneous intestinal inflammation when maintained under specific pathogen-free facility conditions. The intestinal inflammation seen in MDR-/- mice has a pathology similar to that of human inflammatory bowel disease (IBD) and is defined by Th1 type T cells infiltration into the lamina propria of the large intestine.

Another murine model was described by Davidson et al., J Exp Med., 184(1):241-51(1986). In this model, the murine IL-10 gene was deleted and mice rendered deficient in the production of interleukin 10 (IL-10-/-). These mice develop a chronic inflammatory bowel disease (IBD) that predominates in the colon and shares histopathological features with human IBD.

Another murine model for IBD has been described by Powrie et al., Int Immunol., 5(11):1461-71 (1993), in which a subset of CD4+ T cells (called CD45RB(high)) from immunocompetent mice are purified and adoptively transferred into immunodeficient mice (such as C.B-17 scid mice). The animal restored with the CD45RBhighCD4+ T cell population developed a lethal wasting disease with severe mononuclear cell infiltrates in the colon, pathologically similar with human IBD.

### Murine models with pathology similar to human Crohn's disease

The TNF ARE(-/-) model. The role of TNF in Crohn's disease in human has been demonstrated more recently by success of treatment using anti-TNF alpha antibody by Targan et al., N Engl J Med., 337(15):1029-35 (1997). Mice with aberrant production of TNF-alpha due to genetic alteration in the TNF gene (ARE-/-) develop Crohn's-like inflammatory bowel diseases (*see* Kontoyiannis et al., Immunity, 10(3):387-98 (1999)).

The SAMP/yit model. This is model described by Kosiewicz et al., J Clin Invest., 107(6):695-702 (2001). The mouse strain, SAMP/Yit, spontaneously develops a chronic inflammation localized to the terminal ileum. The resulting ileitis is characterized by massive infiltration of activated T lymphocytes into the lamina propria, and bears a remarkable resemblance to human Crohn's disease.

### Examples of in vitro assays

### Reagents

THP-1 cells and MOLT-4 cells were obtained from the American Type Culture Collection (Manassas, VA) and cultured in RPMI tissue culture medium supplemented with 10% fetal calf serum (FCS) in a humidified 5% CO₂ incubator at 37 °C. Recombinant human chemokine proteins MCP-1 and TECK were obtained from R&D Systems (Minneapolis, MN). ¹²⁵I-labeled MCP-1 protein was obtained from Amersham (Piscataway, NJ). ChemoTX^{®} chemotaxis microchambers were purchased from Neuro Probe (Gaithersburg, MD). CyQUANT^{®} cell proliferation kits were purchased from Molecular Probes (Eugene, Oregon). Calcium indicator dye Fluo-4 AM was purchased from Molecular Devices (Mountain View, CA).

### Conventional migration assay

Conventional migration assay was used to determine the efficacy of potential receptor antagonists in blocking migration mediated through chemokines (such as CCR2 or CCR9). This assay was routinely performed using the ChemoTX^{®} microchamber system with a 5-µm pore-sized polycarbonate membrane. To begin such an assay, chemokine expressing cells (such as THP-1 cells for CCR2 assay or MOLT-4 cells for CCR9 assay) were harvested by centrifugation of cell suspension at 1000 PRM on a GS-6R Beckman centrifuge. The cell pellet was resuspended in chemotaxis buffer (HBSS with 0.1% BSA) at 10x10⁶ cells/mL for CCR2 assay (5x10⁶ cells/mL for CCR9 assay). Test compounds at desired concentrations were prepared from 10 mM stock solutions by serial dilutions in chemotaxis buffer. An equal volume of cells and compounds were mixed and incubated at room temperature for 15 minutes. Afterwards, 20 µL of the mixture was transferred onto the porous membrane of a migration microchamber, with 29 µL of chemokine ligand (0.1 nM chemokine MCP-1 protein for CCR2 assay or 50 nm chemokine TECK protein for CCR9 assay) placed at the lower chamber. Following an incubation at 37 °C (90-minute for CCR2; 150-minute for CCR9), during which cells migrated against the chemokine gradient, the assay was terminated by removing the cell drops from atop the filter. To quantify cells migrated across the membrane, 5 µL of 7X CyQUANT^{®} solution was added to each well in the lower chamber, and the fluorescence signal measured on a Spectrafluor Plus fluorescence plate reader (TECAN, Durham, NC). The degree of inhibition was determined by comparing migration signals between compound-treated and untreated cells. IC₅₀ calculation was further performed by non-linear squares regression analysis using Graphpad Prism (Graphpad Software, San Diego, CA).

### BiRAM Assay

The primary screen to identify CCR2 antagonists was carried out using BiRAM assay (WO 02101350, US2004023286), which detects potential hits by their ability to activate cell migration under inhibitory chemokine concentration. To begin such an assay, chemokine expressing cells (such as THP-1 cells for CCR2 assay or MOLT-4 cells for CCR9 assay) were harvested by centrifugation of cell suspension at 1000 RPM on a GS-6R Beckman centrifuge. The cell pellet was resuspended in chemotaxis buffer (HBSS/0.1 % BSA) at 10 x 10⁶ cells/mL for CCR2 assay (5x10⁶ cells/mL for CCR9 assay). Twenty-five microliters of cells was mixed with an equal volume of a test compound diluted to 20 µm in the same buffer. Twenty microliters of the mixture was transferred onto the filter in the upper chemotaxis chamber, with 29 µl of chemokine solution containing chemokine ligand (100 nM chemokine MCP-1 and MIP-1α protein for CCR2 assay or 500 nm chemokine TECK protein for CCR9 assay) was placed in the lower chamber. Following an incubation at 37°C (90-minute for CCR2; 150-minute for CCR9), the assay was terminated by removing the cell drops from atop the filter. To quantify cells migrated across the membrane, 5 µL of 7X CyQUANT^{®} solution was added to each well in the lower chamber, and the fluorescence signal measured on a Spectrafluor Plus fluorescence plate reader (TECAN, Durham, NC).

For selection of potential hits, the level of migration activation was calculated as a RAM index-the ratio between the signal of a particular well and the median signal of the whole plate. Compounds with a RAM index of greater than 1.5 for CCR2 assay (1.8 for CCR9 assay) were regarded as RAM positive, and were selected for IC₅₀ determinations in conventional functional assays.

### Calcium flux assay

Calcium flux assay measures an increase in intracellular calcium following ligand-induced receptor activation. In the screen of chemokine antagonists, it was used as a secondary assay carried out on a FLIPR^{®} machine (Molecular Devices, Mountain View, CA). To begin an assay, chemokine expressing cells (such as THP-1 cells for CCR2 assay or MOLT-4 cells for CCR9 assay) were harvested by centrifugation of cell suspension, and resuspended to 1.5 x 10⁶ cells/mL in HBSS (with 1 % fetal calf serum). Cells were then labeled with a calcium indicator dye Fluo-4 AM for 45 minutes at 37 °C with gentle shaking. Following incubation, cells were pelletted, washed once with HBSS and resuspended in the same buffer at a density of 1.6 x 10⁶ cells/mL. One hundred microliters of labeled cells were mixed with 10 µL of test compound at the appropriate concentrations on an assay plate. Chemokine protein (MCP-1 at a final concentration of 0.1 nM for CCR2 assay or TECK at a final concentration of 25 nM for CCR9 assay) to activate the receptor. The degree of inhibition was determined by comparing calcium signals between compound-treated and untreated cells. IC₅₀ calculations were further performed by non-linear squares regression analysis using Graphpad Prism (Graphpad Software, San Diego, CA).

### Ligand binding assay

Ligand binding assay was used to determine the ability of potential CCR2 antagonists to block the interaction between CCR2 and its ligand MCP-1. CCR2 expressing THP-1 cells were centrifuged and resuspended in assay buffer (20 mM HEPES pH 7.1, 140 mM NaCl, 1 mM CaCl₂, 5 mM MgCl₂, and with 0.2% bovine serum albumin) to a concentration of 2.2 x 10⁵ cells/mL. Binding assays were set up as follows. First, 0.09 mL of cells (1x10⁵ THP-1 cells/well) was added to the assay plates containing the compounds, giving a final concentration of ~2-10 µM each compound for screening (or part of a dose response for compound IC₅₀ determinations). Then 0.09 mL of ¹²⁵I labeled MCP-1 (obtained from Amersham; Piscataway, NJ) diluted in assay buffer to a final concentration of -50 pM, yielding -30,000 cpm per well, was added, the plates sealed and incubated for approximately 3 hours at 4 °C on a shaker platform. Reactions were aspirated onto GF/B glass filters pre-soaked in 0.3% polyethyleneimine (PEI) solution, on a vacuum cell harvester (Packard Instruments; Meriden, CT). Scintillation fluid (50 µL; Microscint 20, Packard Instruments) was added to each well, the plates were sealed and radioactivity measured in a Top Count scintillation counter (Packard Instruments). Control wells containing either diluent only (for total counts) or excess MCP-1 (1 µg/mL, for non-specific binding) were used to calculate the percent of total inhibition for compound. The computer program Prism from GraphPad, Inc. (San Diego, Ca) was used to calculate IC₅₀ values. IC₅₀ values are those concentrations required to reduce the binding of labeled MCP-1 to the receptor by 50%.

### Discovery of chemokine antagonists

The discovery of chemokine antagonists was carried out in two steps: First, BiRAM assay was used to screen a compound library in a high-throughput manner. The assay detected compounds by their ability to cause a positive migration signal under RAM condition. Secondly, BiRAM positive compounds were tested to determine their IC₅₀ values using the conventional migration, calcium flux assays and ligand binding assays.

For instance, in a screen of approximately 100,000 compounds, 2000 individual wells representing approximately 2% of total compounds showed a desired RAM index (greater than 1.5 for CCR2; greater than 1.8 for CCR9). These compounds were cherry-picked and retested in duplicate wells by RAM assay. A total of 156 compounds were confirmed BiRAM positives.

Since a BiRAM positive signal indicates only the presence of a receptor antagonist and not how strongly it blocks receptor functions, the BiRAM positive compounds were further tested for potency in conventional migration, calcium flux and ligand binding assays. IC₅₀ determinations on this subset discovered several compounds with an IC₅₀ less than 1 µM and that did not inhibit other chemokine receptors examined at significant levels.

### In Vivo Efficacy

### Evaluation of the compound as shown in comparative Example 1 in a rat model of collagen-induced arthritis

A 17-day study of type II collagen-induced arthritis was conducted to evaluate the effects of the compound as shown in comparative Example 1 on arthritis-induced clinical ankle swelling. Rat collagen-induced arthritis is an experimental model of polyarthritis that has been widely used for preclinical testing of numerous antiarthritic agents (see Trentham et al., J. Exp. Med 146(3):857-868 (1977), Bendele et al., Toxicologic Pathol. 27:134-142 (1999), Bendele et al., Arthritis Rheum. 42:498-506 (1999)). The hallmarks of this model are reliable onset and progression of robust, easily measurable polyarticular inflammation, marked cartilage destruction in association with pannus formation and mild to moderate bone resorption and periosteal bone proliferation.

Female Lewis rats (approximately 0.2 kilograms) were anesthetized with isoflurane and injected with Freund's Incomplete Adjuvant containing 2 mg/mL bovine type II collagen at the base of the tail and two sites on the back on days 0 and 6 of this 17-day study. Compound comparative Example 1" was dosed daily by sub-cutaneous injection from day 9 to day 17 at a dose of 100 mg/kg and a volume of 1 mL/kg in the following vehicle (24.5 % Cremaphore EL, 24.5% common oil, 1% Benzylalcohol and 50% Distilled water). Caliper measurements of the ankle joint diameter were taken daily, and reducing joint swelling was taken as a measure of efficacy. As shown in Figure 1, the test compound significantly and specifically inhibited the arthritis-induced ankle swelling.

The MDR1 α-knockout mice, which lack the P-glycoprotein gene, spontaneously develop colitis under specific pathogen-free condition. The pathology in these animals has been characterized as Th1-type T cell-mediated inflammation similar to ulcerative colitis in humans. Disease normally begins to develop at around 8-10 weeks after birth. However the ages at which disease emerges and the ultimate penetrance level often vary considerably among different animal facilities.

In a study using the MDR1a-knockout mice, a CCR9 antagonist is evaluated by prophylactic administration for its ability to delay disease onset. Female mice (n=34) are dosed with 50 mg/kg twice a day by subcutaneous injections for 14 consecutive weeks starting at age 10 weeks. The study is evaluated for IBD-associated growth retardation.

### Evaluation of 4-chloro-3-trifluoromethyl-N-[5-chloro-2-(2-methanesulfonyl-benzoyl)-pyridin-3-yl]-benzenesulfonamide in a rat model of thioglycollate-induced peritoneal inflammation

A 2-day study of thioglycollate-induced inflammation was conducted to evaluate the effects of the test compound, 4-chloro-3-trifluoromethyl-N-[5-chloro-2-(2-methanesulfonyl-benzoyl)-pyridin-3-yl]-benzenesulfonamide. The hallmarks of this model are reliable onset and progression of robust, easily measurable inflammatory cellular infiltrate. For the induction of inflammatory peritonitis in Lewis rats, Brewer-Thioglycollate (1.0 mL, 4 % solution in distilled water) was injected intra peritoneal (i.p.). Before this injection, the treatment group received test compound, 4-chloro-3-trifluoromethyl-N-[5-chloro-2-(2-methanesulfonyl-benzoyl)-pyridin-3-yl]-benzenesulfonamide, or vehicle and the control group received the same volume of PBS as i.p. injection. After 2 days, a peritoneal lavage was performed with ice-cold PBS containing 1 mM EDTA. The recovered cells were counted with a cell counter (Coulter Counter; Coulter Pharmaceutical, Palo Alto, CA) and monocytes/macrophages were identified by flow cytometry using light-scatter properties.

The test compound significantly and specifically inhibited the number of inflammatory macrophages elicited following tioglycollate injection.

### Evaluation of 4-chloro-3-trifluoromethyl-N-[5-chloro-2-(2-methanesulfonyl-benzoyl)-pyridin-3-yl]-benzenesulfonamide in a mouse model of bacterial infection

A 1-day study of streptococcus *pneumoniae* infection was conducted to evaluate the effects of the test compound, 4-chloro-3-trifluoromethyl-N-[5-chloro-2-(2-methanesulfonyl-benzoyl)-pyridin-3-yl]-benzenesulfonamide. The model measures bacterial infection and spread in an animal following pulmonary infection with live bacterial cultures, measured by inflammatory cellular infiltrate, and assessment of bacterial burden. C57/B6 mice were inoculated intra nasally with LD50 400 CFU at day 0. Group were either compound or vehicle control treated 1 day prior to bacterial inoculation and twice daily throughout the study. Bacterial burden was measured at 24 hours by plating serial dilutions of homogenized lung tissue on agar plates and counting colonies.

The bacterial burden in the lung was significantly reduced by the test compound after 24 hours compared to vehicle control.

### Pharmacologies to be used in conjunction with CCR2 compounds

Pharmacological agents that can be used in conjunction with the CCR2 antagonists of the current invention include those used for the treatments of atherosclerosis, restenosis., multiple sclerosis, pulmonary fibrosis, inflammatory bowel disease, rheumatoid arthritis, graft-versus-host disease, renal fibrosis, psoriasis, transplantation rejection, obesity, diabetes, hypercholesterolemia and cancer.

In the tables below, structures and activity are provided for representative compounds described herein. Activity is provided as follows for either or both of the chemotaxis assay and/or calcium mobilization assays, described above: +, IC₅₀ > 1000 nM; and ++, IC₅₀ < 1000 nM.

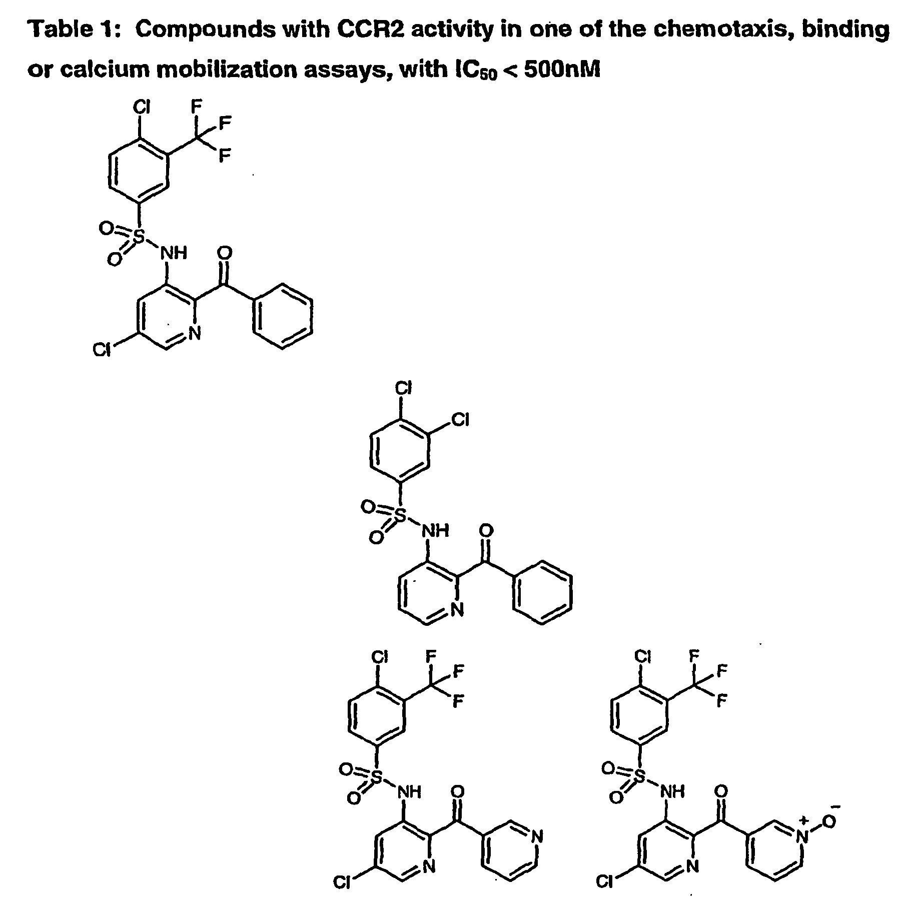

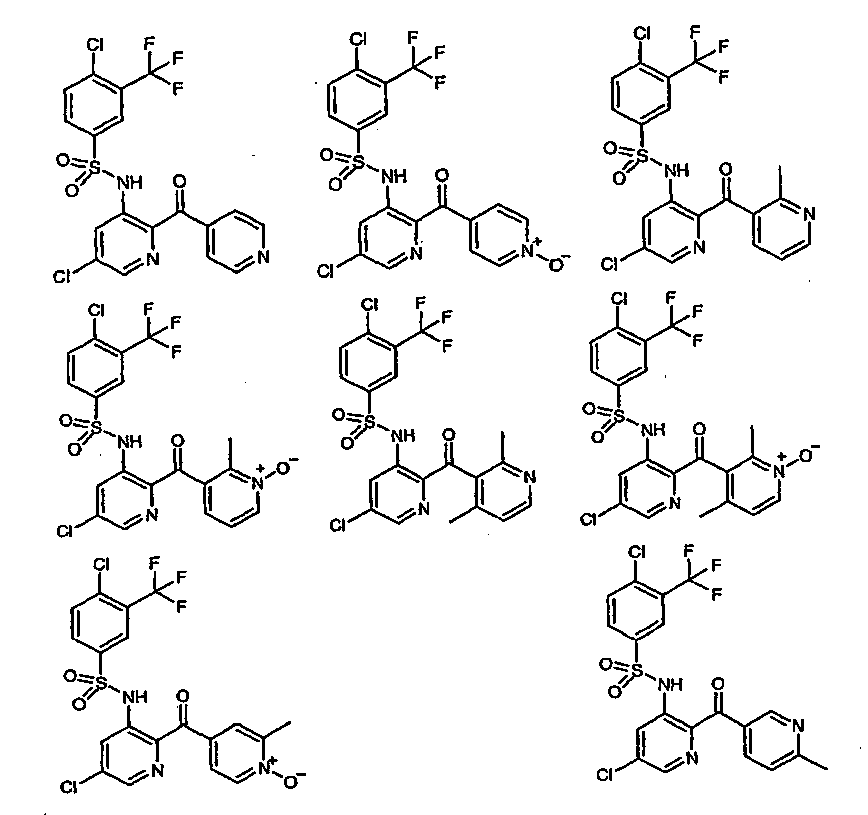

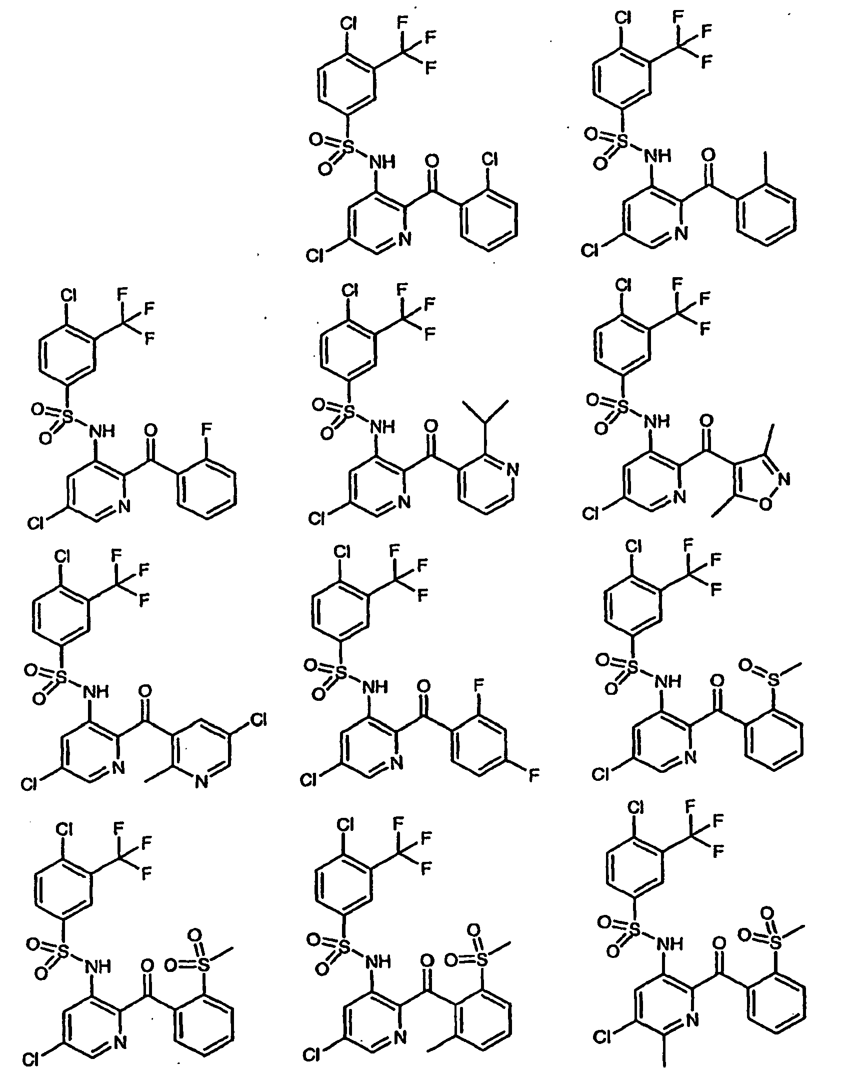

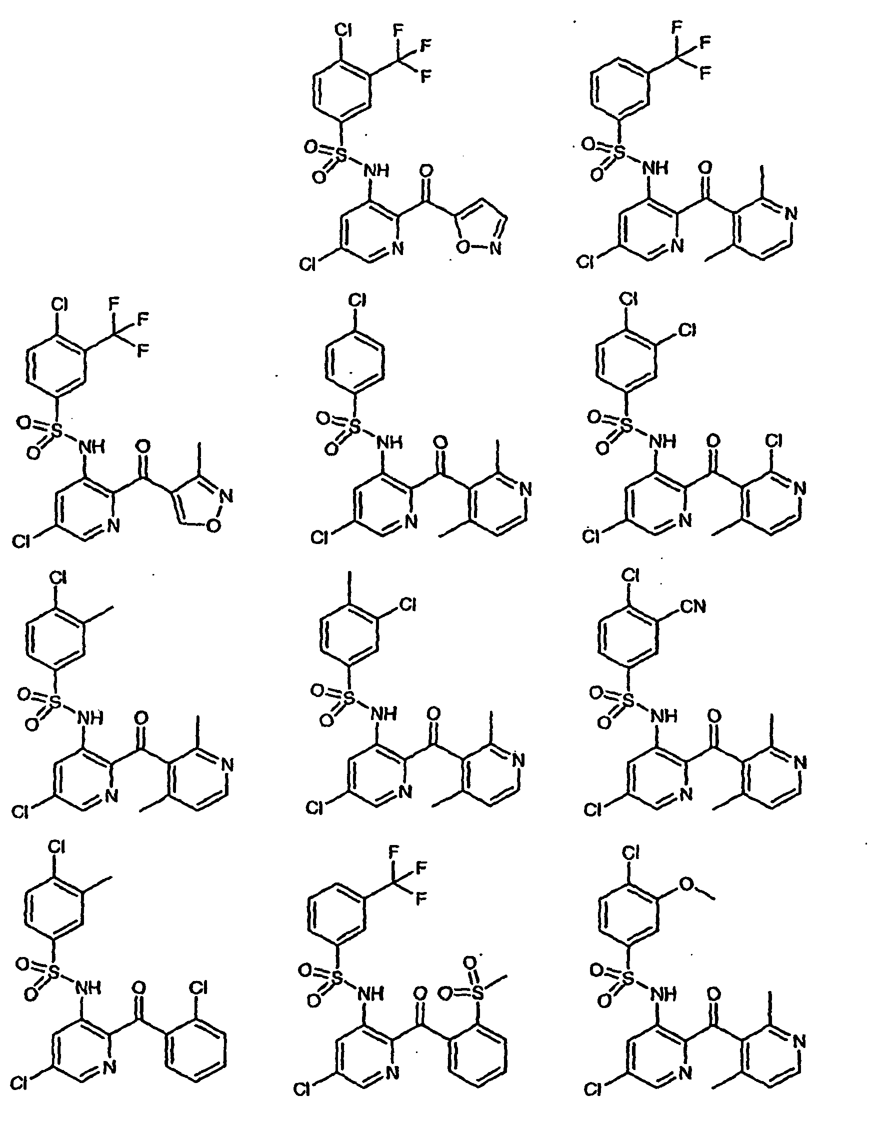

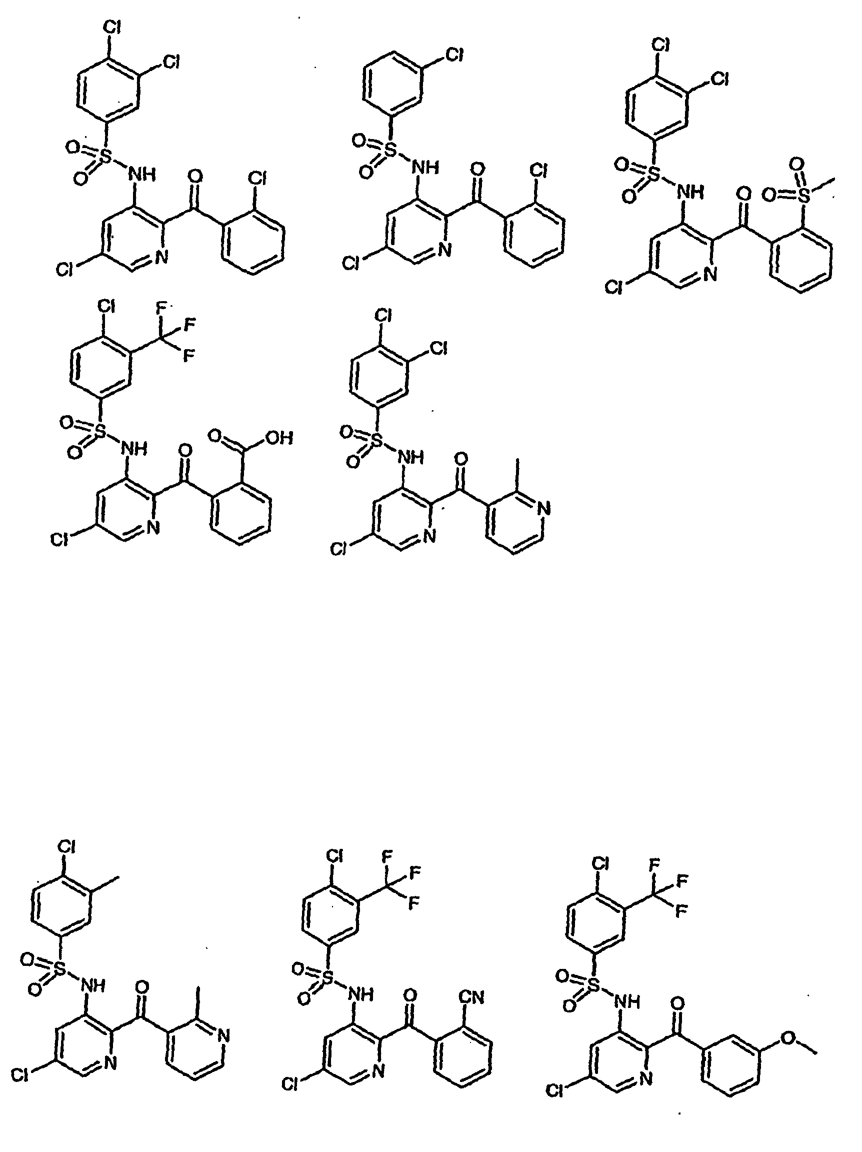

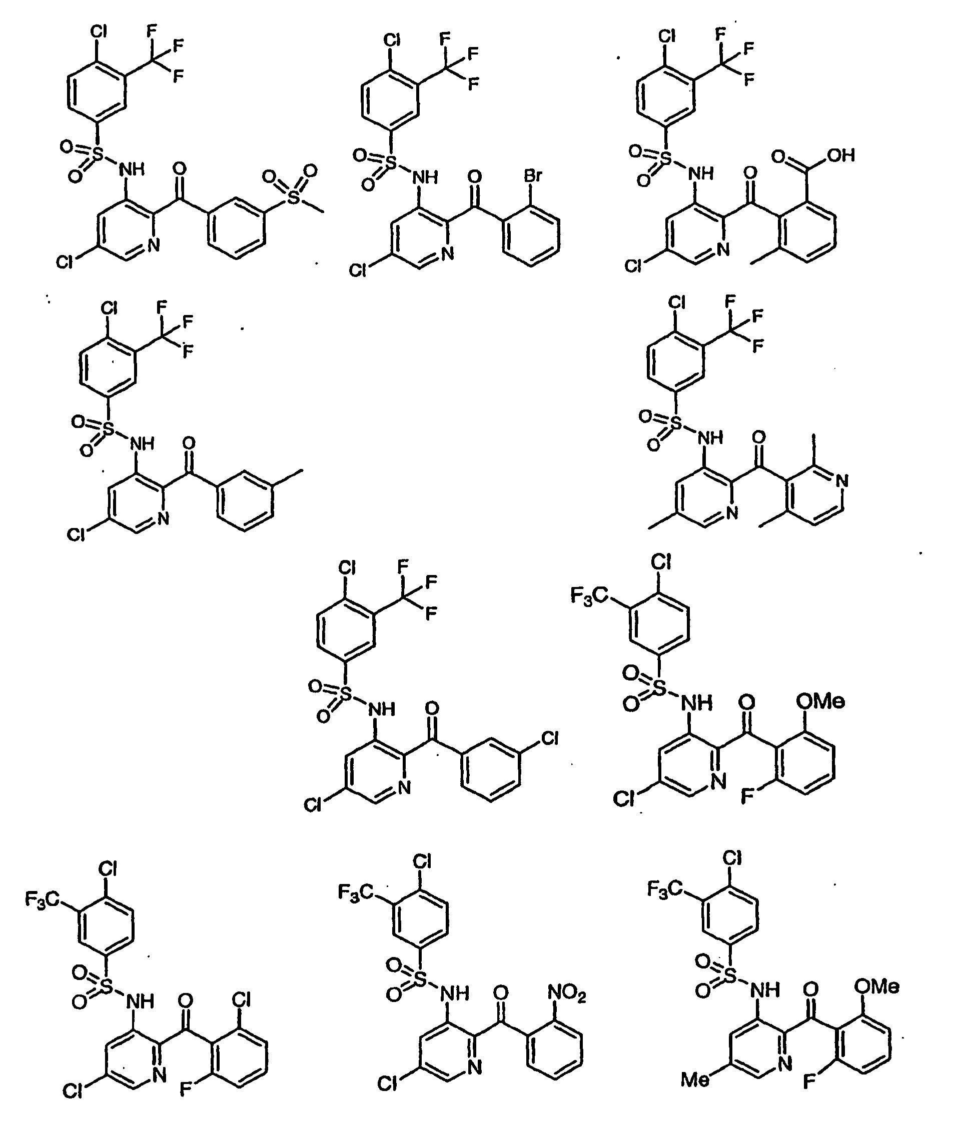

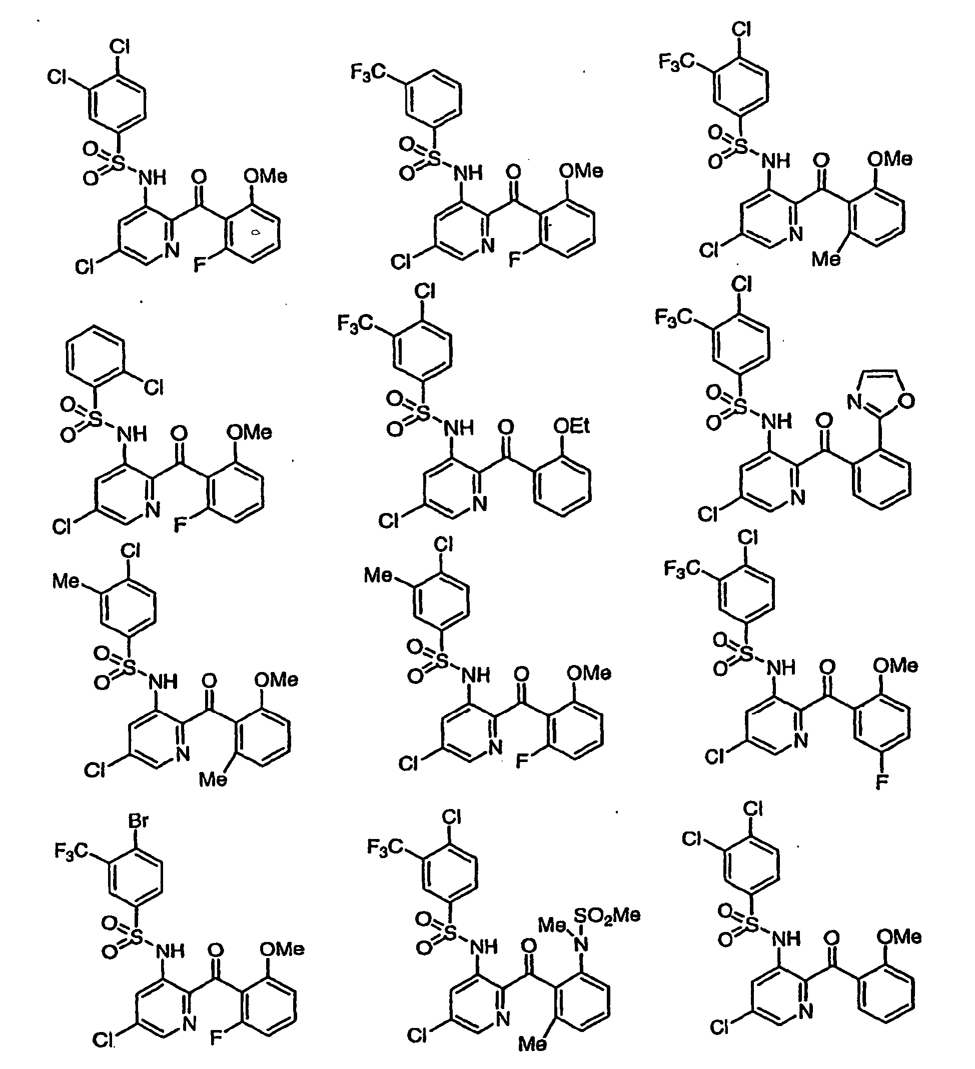

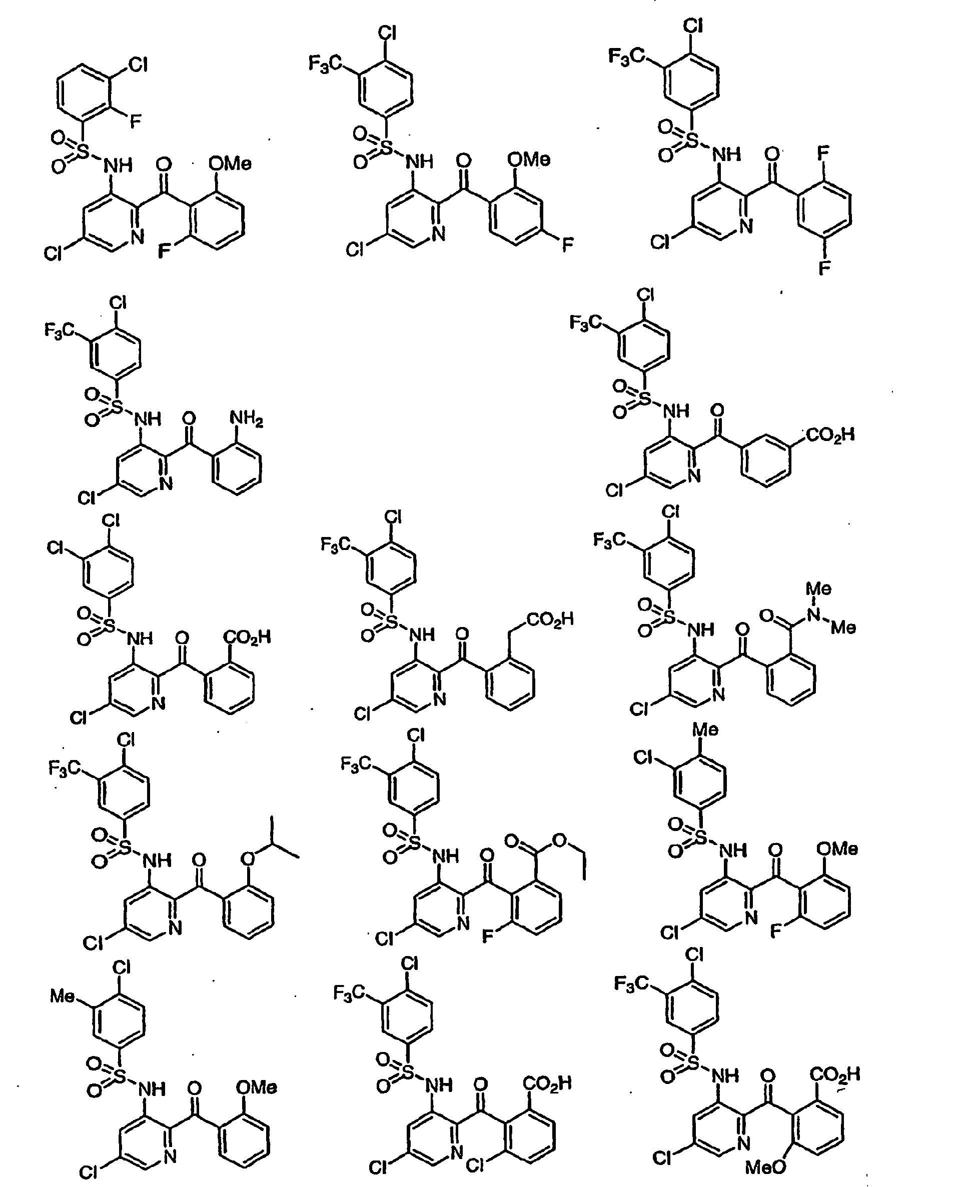

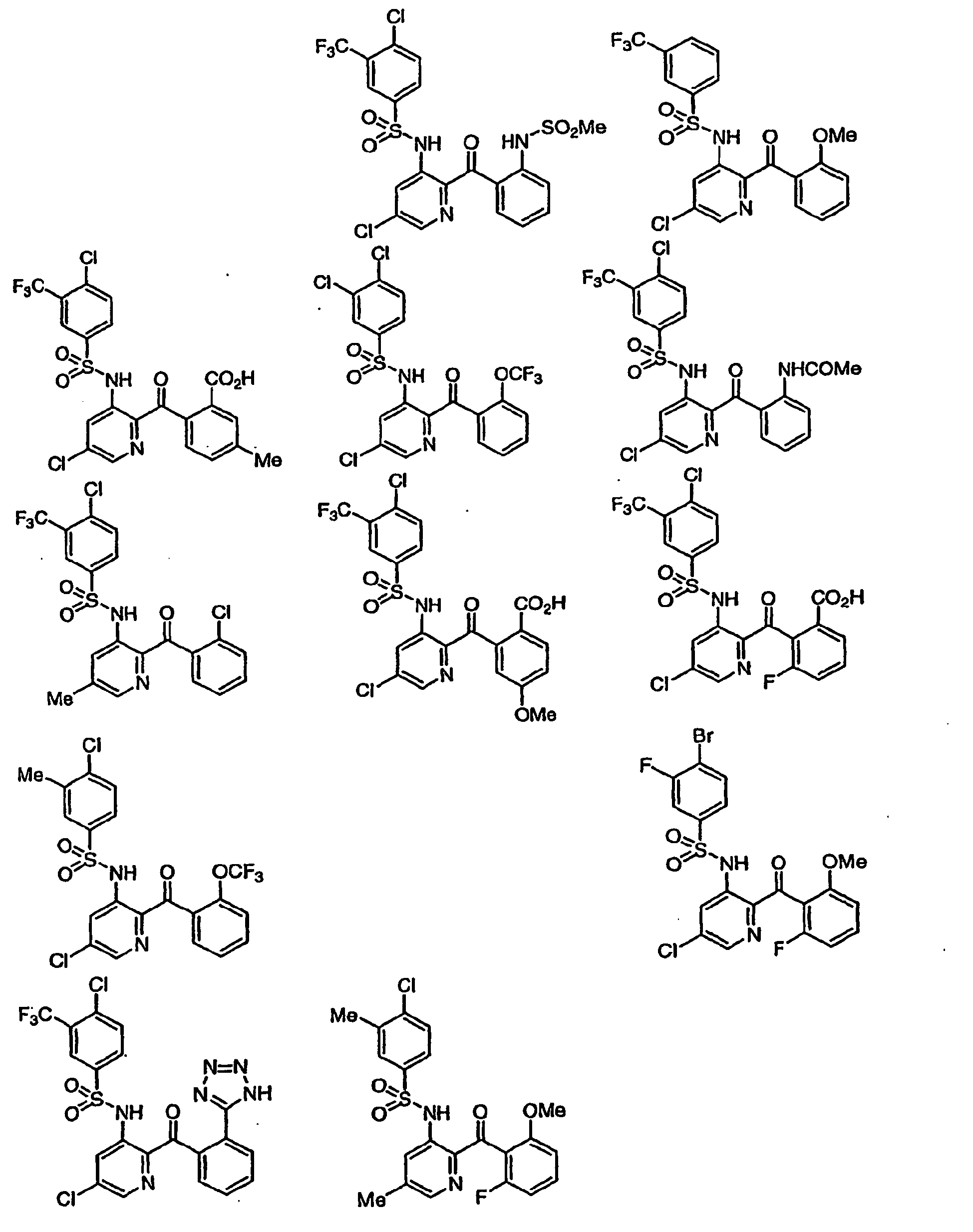

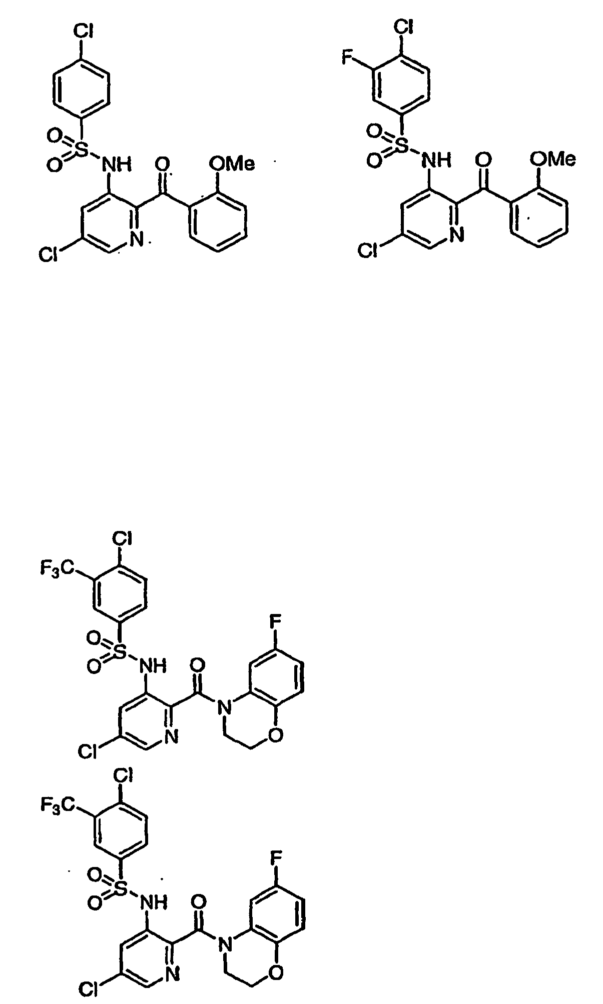

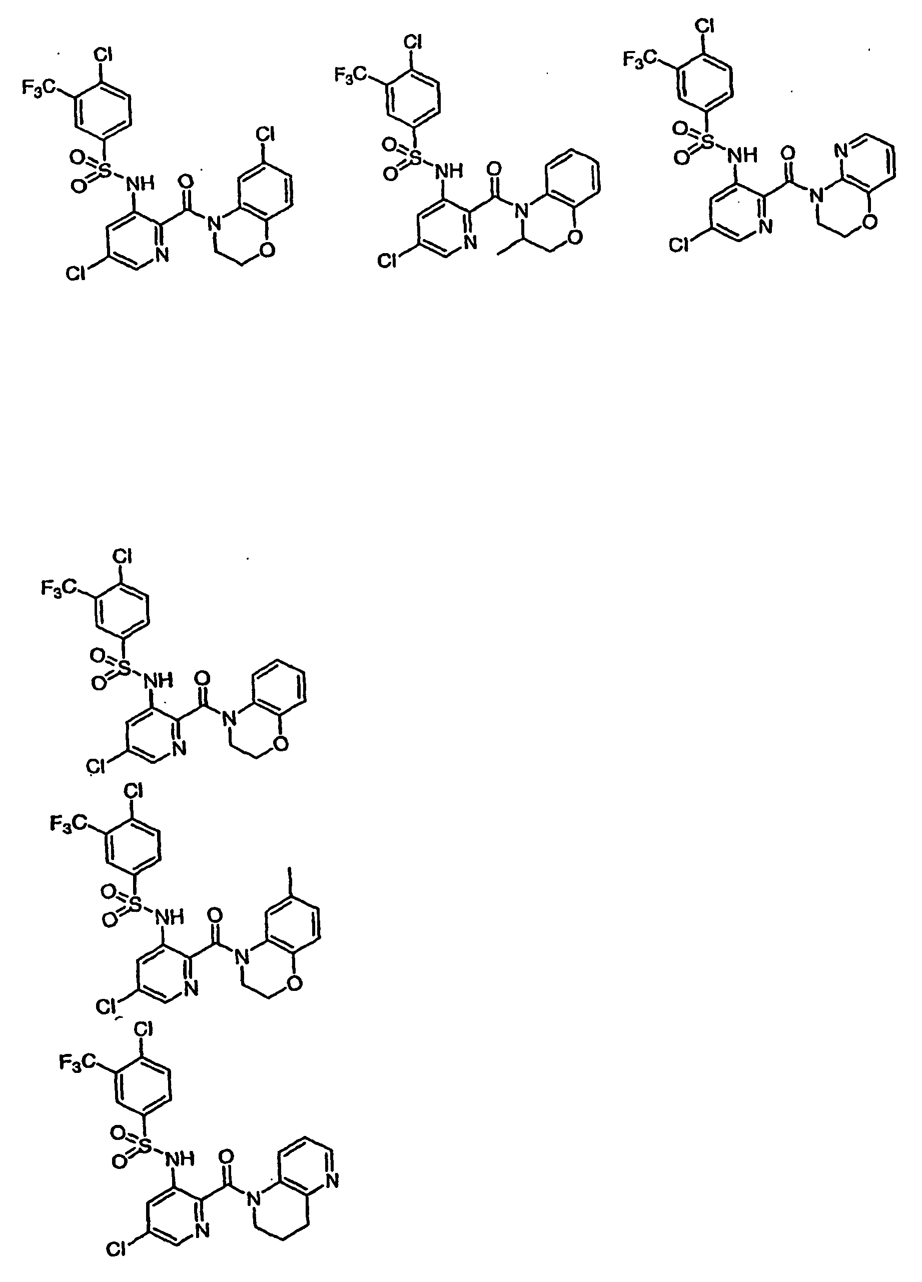

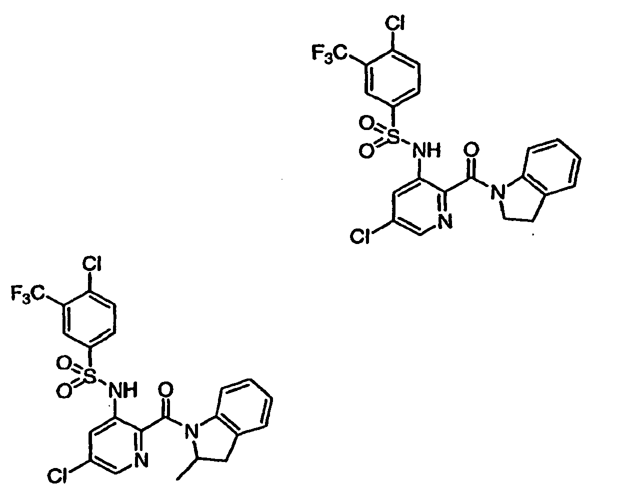

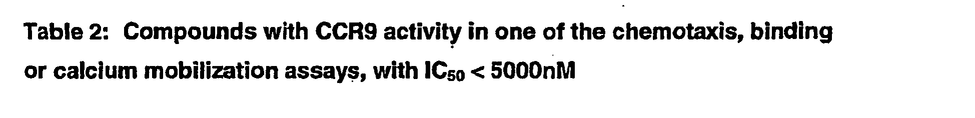

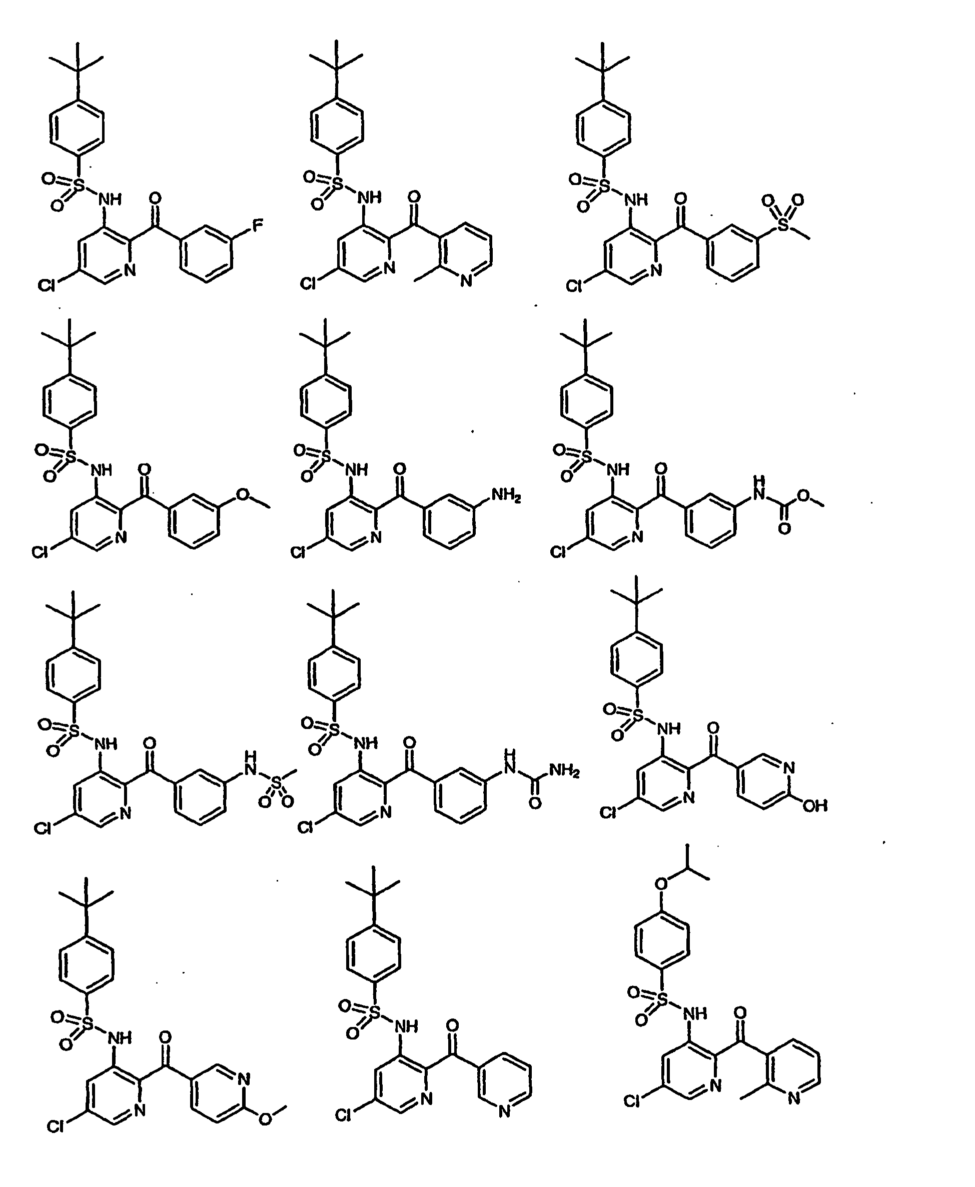

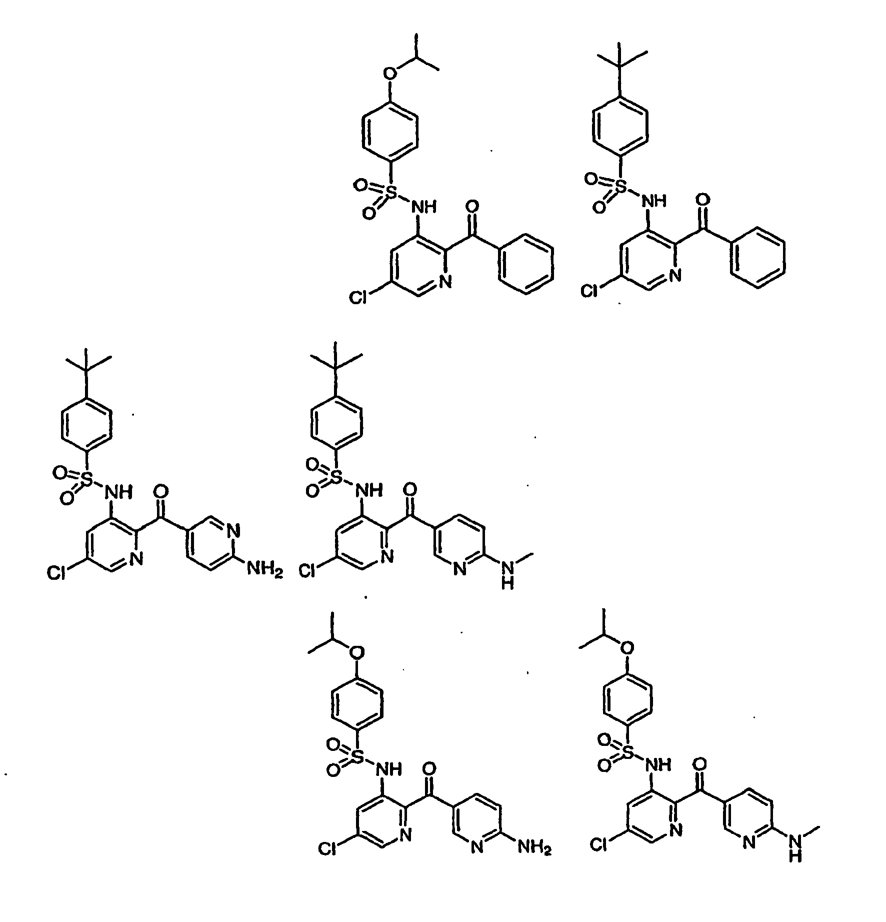

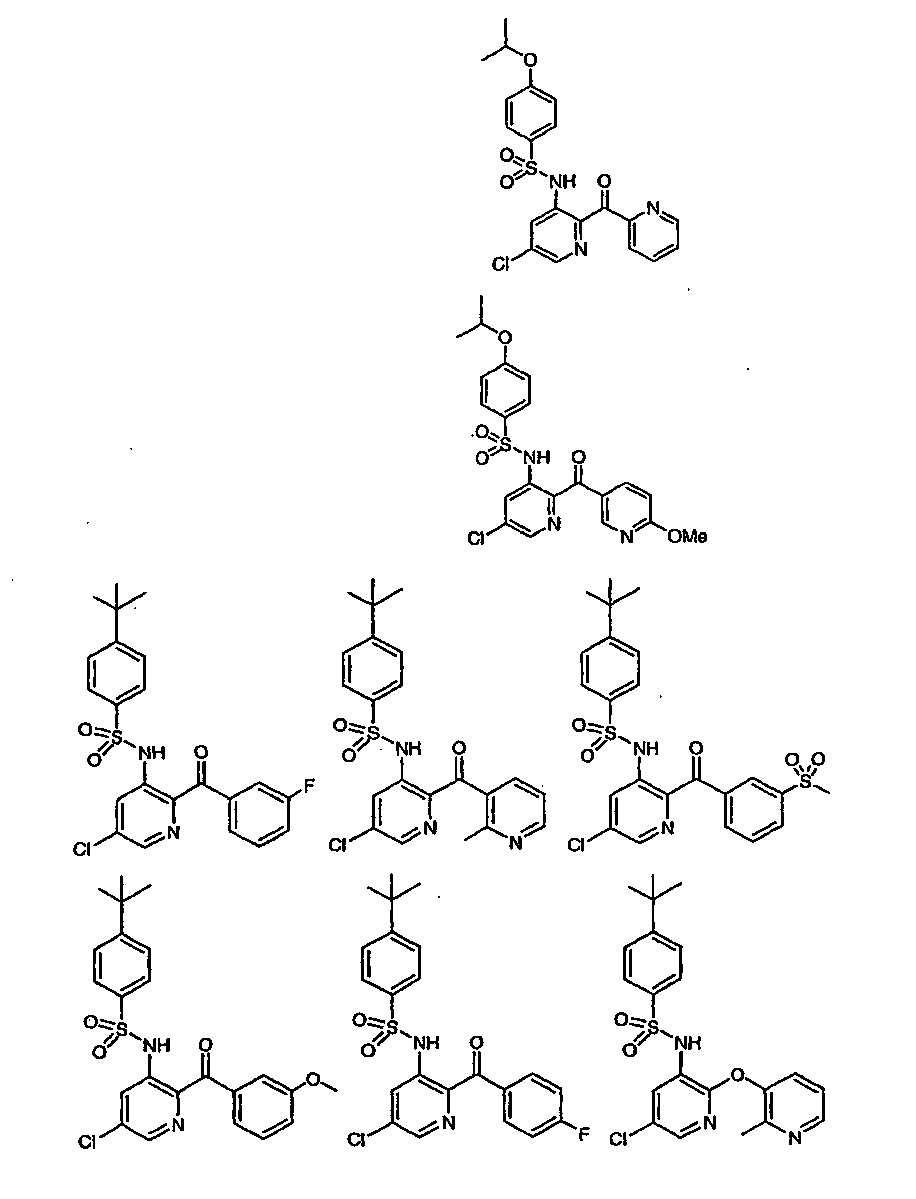

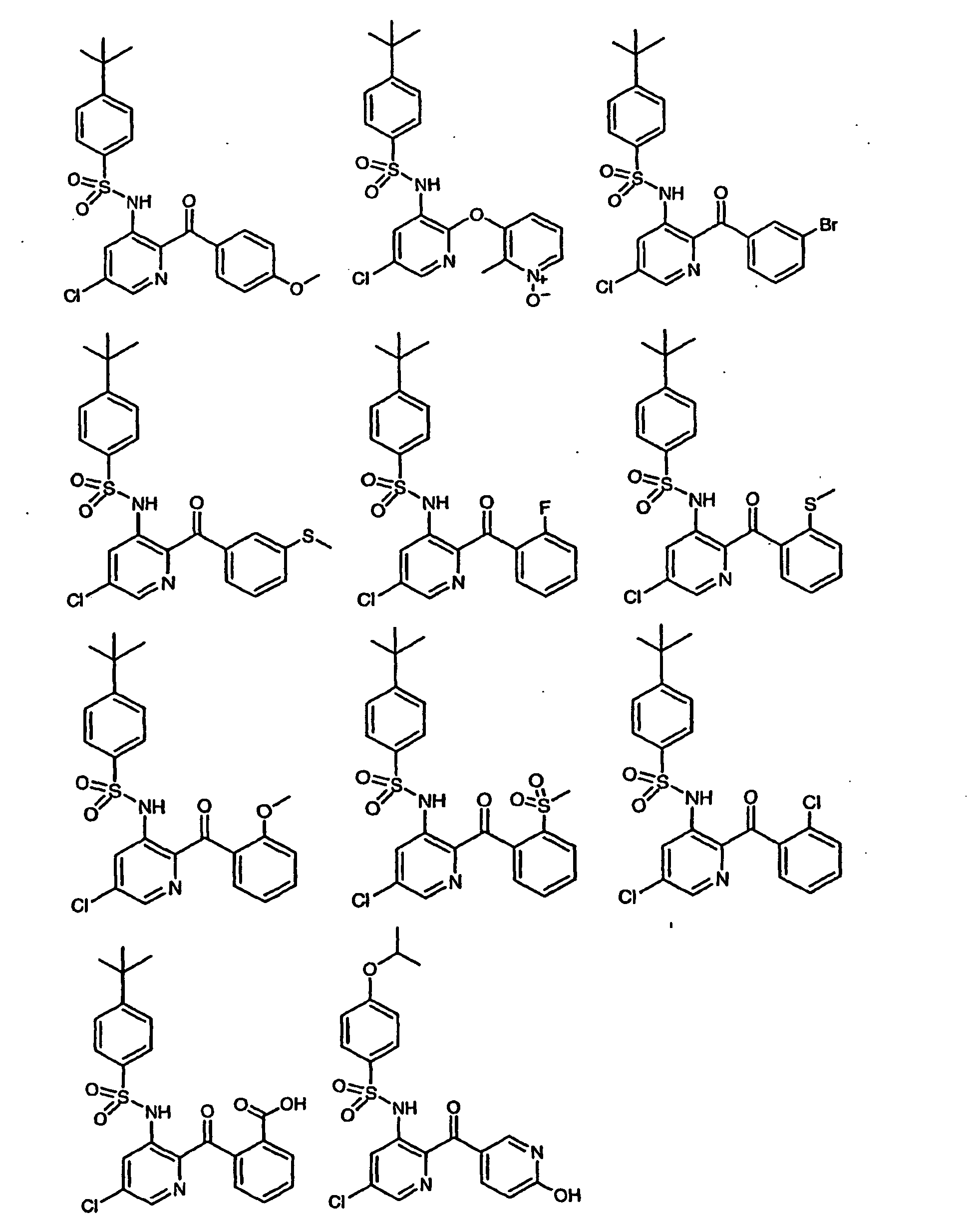

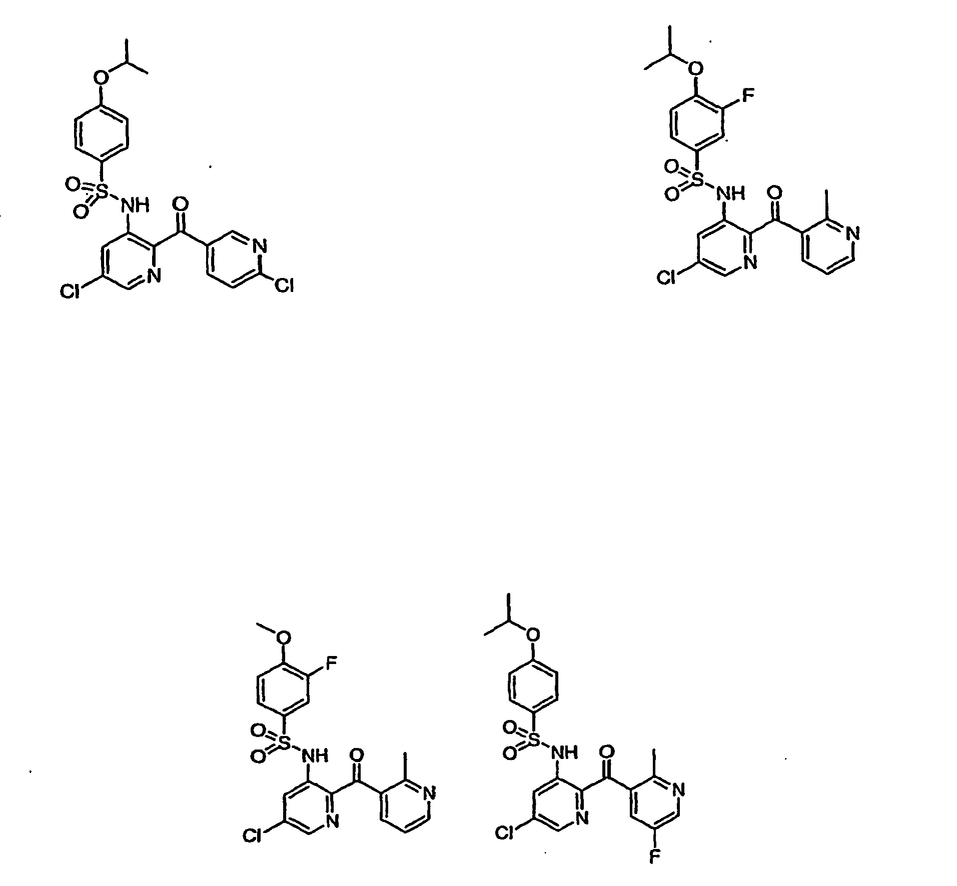

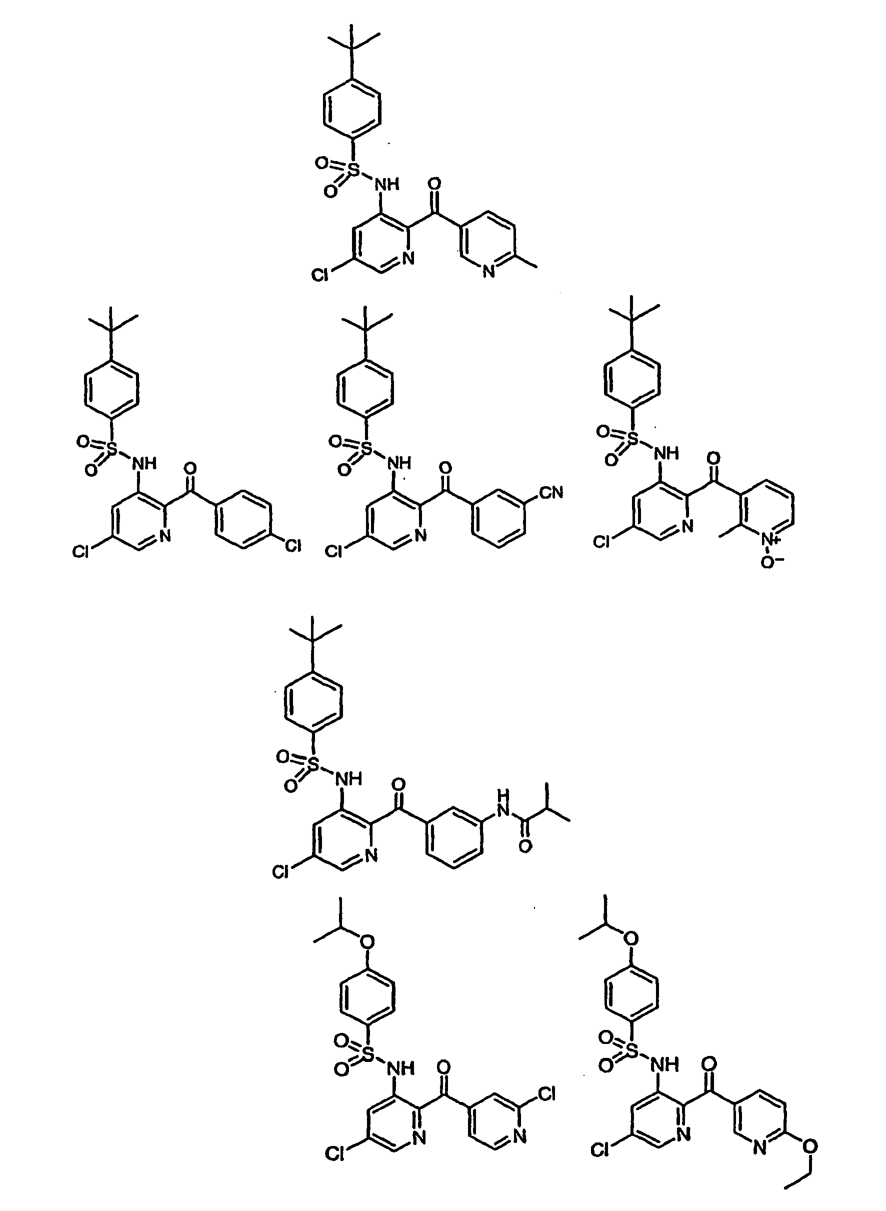

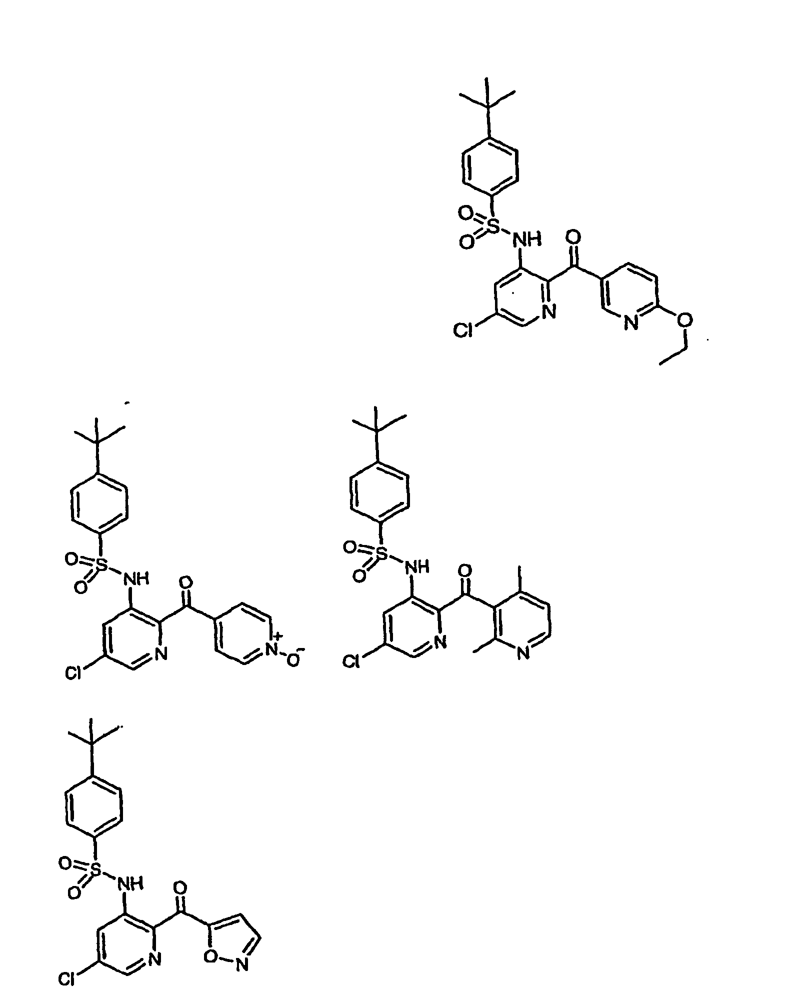

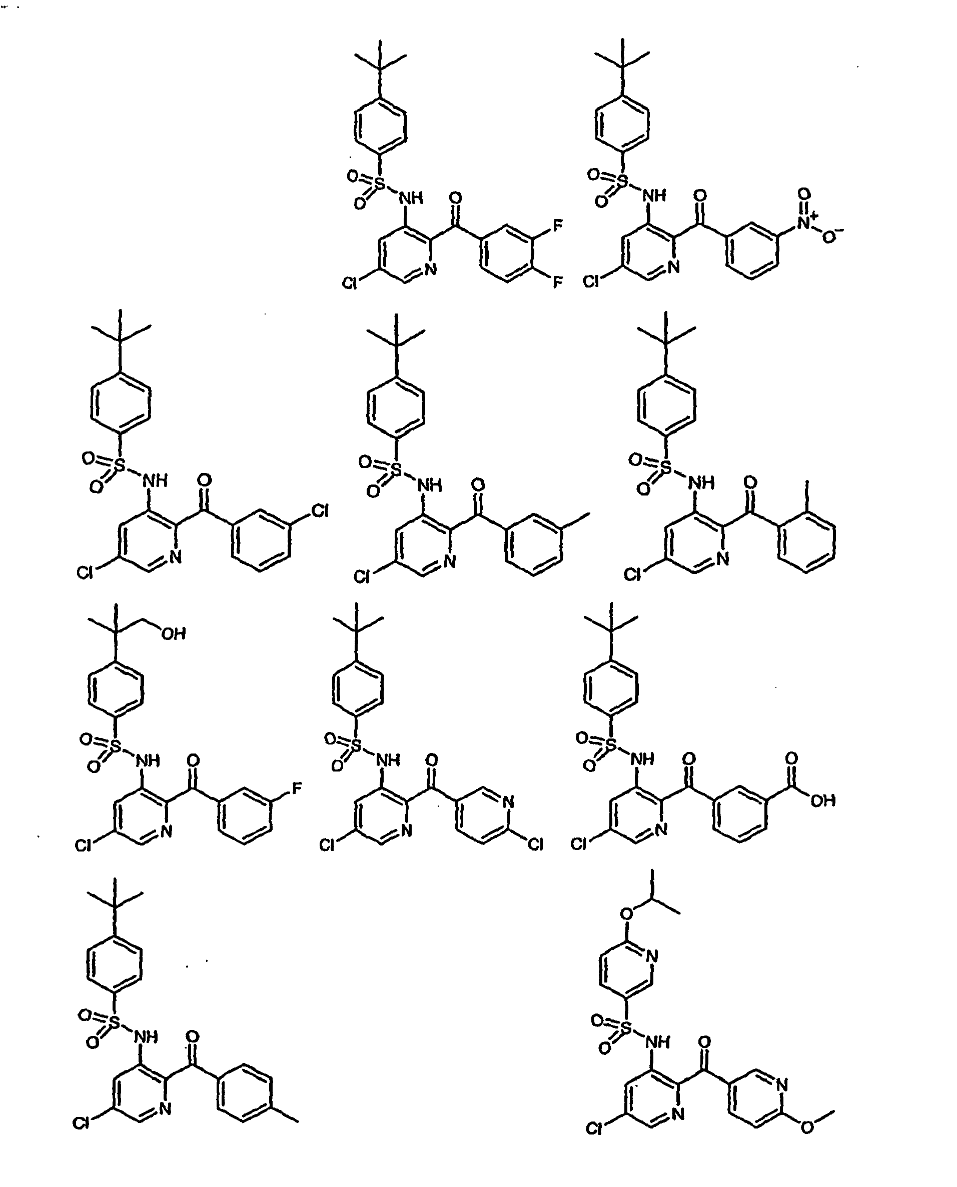

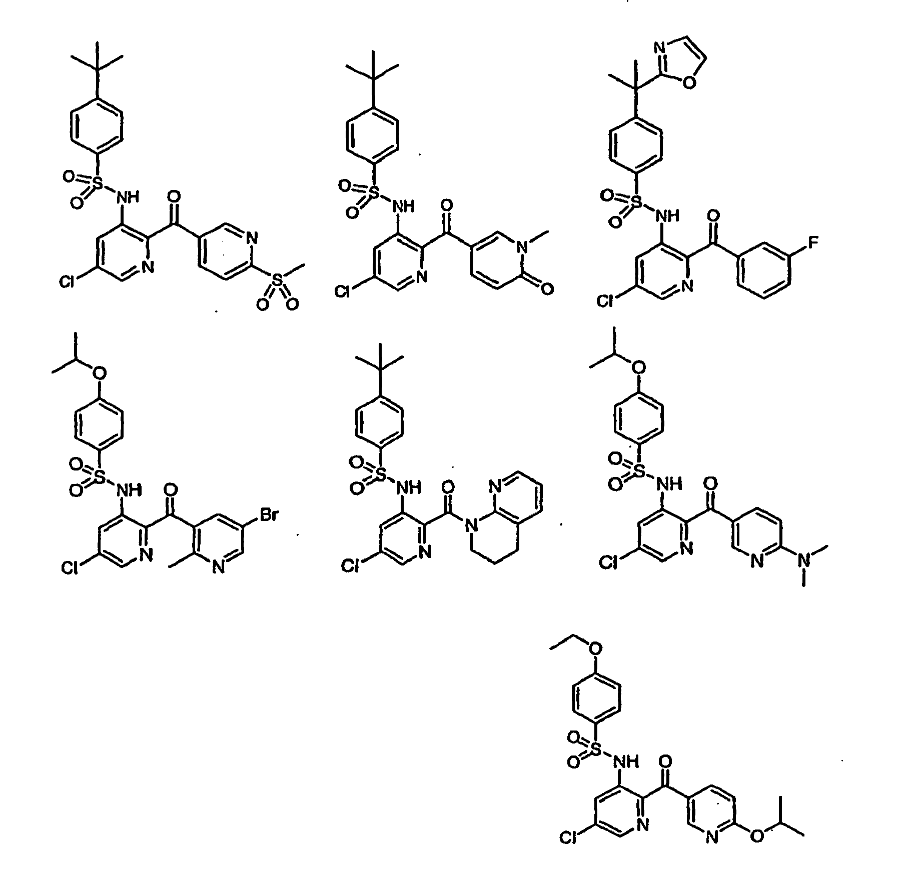

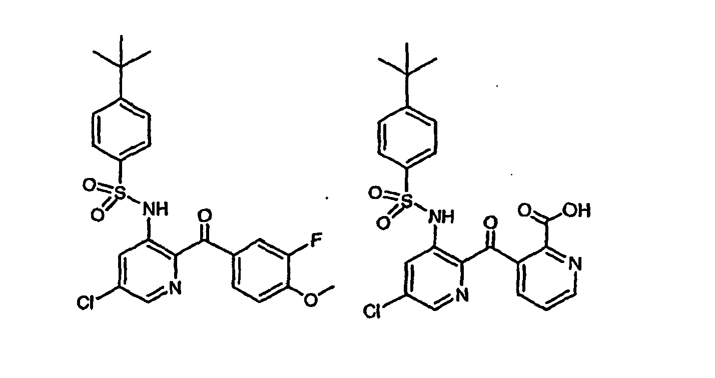

## Claims

1. A compound of formula (I), or a salt thereof: 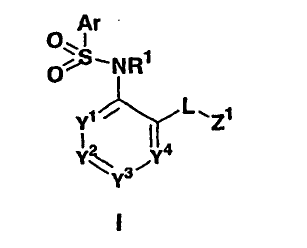 where
Ar is unsubstituted phenyl or phenyl substituted with one or more of halogen, substituted or unsubstituted C₁₋₈ alkyl, substituted or unsubstituted C₂₋₈ alkenyl, substituted or unsubstituted C₂₋₈ alkynyl, -CN, -C(O)R¹⁸, -CO₂R¹⁸, -C(O)NR¹⁸R¹⁹, -OC(O)R¹⁹, -OC(O)NR¹⁸R¹⁹, -NO₂, -NR¹⁸C(O)R¹⁹, -NR₁₈C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸_{,} -S(O)₂NR¹⁸R¹⁹, substituted or unsubstituted C₆₋₁₀ aryl, substituted or unsubstituted 5- to 10-membered heteroaryl, and substituted or unsubstituted 3- to 10-membered heterocyclyl;
R¹⁸, R¹⁹ and R²⁰ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted aryl, substituted or unsubstituted heteroaryl, and substituted or unsubstituted heterocyclyl;
R¹ is selected from the group consisting of hydrogen, unsubstituted C₁ alkyl, and unsubstituted C₃₋₈ alkyl;
Y¹ is -CR^{2a}-;
Y² is -CR^{2b}-;
Y³ is -CR^{2C}-;
R^{2a}, R^{2b} , and R^{2c} are each independently selected from the group consisting of hydrogen, halogen, and unsubstituted C₁₋₈ alkyl;
Y⁴ is -N-;
L is -C(O)-; and
Z¹ is substituted or unsubstituted phenyl;
wherein
substituents for substituted alkyl, substituted alkenyl, and substituted alkynyl are selected from halogen, -CN, -CO₂R', -C(O)R', -C(O)NR'R", oxo (=O or -O⁻) , -R', -OC(O)R', -OC(O) NR'R", -NO₂, -NR'C(O) R", -NR"'C(O)NR'R", -NR'R", -NR'CO₂R", -NR'S(O)R", .-NR'S(O)₂R"', -NR"'S(O)NR'R", -NR'"S(O)₂NR'R", -SR', -S(O)R', -S(O)₂R', -S(O)₂NR'R", -NR'-C(NHR")=NR'", -SiR'R"R'", -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)₂NR'R", -NR'-C(NHR")=NR'" , -SiR'R''R'", -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R"' are selected from hydrogen, C₁₋₈
alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R"' may together with the atom(s) to which they are attached, form a 5-, 6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NMR""-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH2-, -O-, -NMR"'-, -S-, -S(O)-, -S(O)₂-, -S(O)₂NR'"-or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula-(CH₂)ₛ-X-(CH₂)ₜ-, where s and t are independently integers of from 0 to 3, and X is -O-, -NMR""-, -S-, -S(O)-, -S(O)₂-, or -S(O)₂NR'-, wherein R"" is selected from hydrogen or unsubstituted C₁₋₈ alkyl.

2. The compound of claim 1, which is represented by formula (II) or a salt thereof: 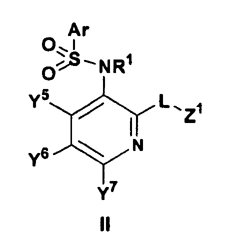 where
Y⁵, Y⁶ and Y⁷ are each independently selected from the group consisting of hydrogen, halogen, and unsubstituted C₁₋₈ alkyl.

3. The compound of claim 1, which is represented by formula (CLIII) or a salt thereof: 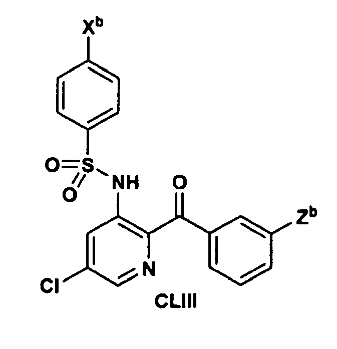 where
X^{b} is selected from the group consisting of CF₃, and unsubstituted C₁₋₈ alkyl; and
Z^{b} is halogen.

4. A compound of formula III, or a salt thereof: 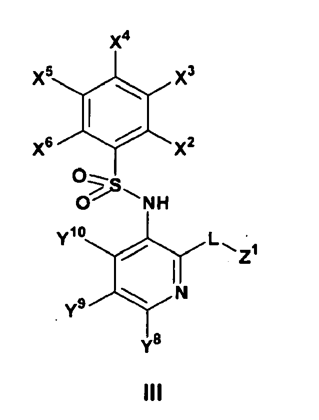 where
L is -C(O)-;
Z¹ is selected from the group consisting of substituted or unsubstituted 5- to 10-membered heteroaryl;
X², X³, X⁴, X⁵, and X⁶ are each independently selected from the group consisting of hydrogen, halogen, substituted or unsubstituted C₁₋₈ alkyl, substituted or unsubstituted C₂₋₈ alkenyl, substituted or unsubstituted C₂₋₈ alkynyl, -CN, -NO₂, -C(O)R¹⁸, -CO₂R¹⁸, -C(O)NR¹⁸R¹⁹, -OR¹⁸, -OC(O)R¹⁹, -OC(O)NR¹⁸R¹⁹ -NR¹⁸C(O)R¹⁹, -NR¹⁸C(O)NR¹⁹R²⁰, -NR¹⁸R¹⁹, -NR¹⁸CO₂R¹⁹, -NR¹⁸S (O)₂R¹⁹, -SR¹⁸, -S (O) R¹⁸_{,} -S (O)₂R¹⁸, -S(O)₂NR¹⁸R¹⁹, substituted or unsubstituted C₆₋₁₀ aryl, substituted or unsubstituted 5- to 10-membered heteroaryl, and substituted or unsubstituted 3- to 10-membered heterocyclyl;
R¹⁸, R¹⁹ and R²⁰ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl;
Y⁸, Y⁹ and Y¹⁰ are each independently selected from the group consisting of hydrogen, halogen, -CN, -NO₂, -OR²¹, -CO₂R²¹, -OC(O)R²¹, -OC(O)NR²¹R²², -C(O)NR²¹R²², -C(O)R²¹, -SR²¹ ,-S(O)R²¹, -S(O)₂R²¹, NR²¹R²², -NR²¹C(O)R²², -NR²¹C(O)₂R²², -NR²¹S(O)₂R²², -NR²¹C(O)NR²²R²³, substituted or unsubstituted C₁₋₈ alkyl and substituted or unsubstituted 3- to 10-membered heterocyclyl,
R²¹, R²² and R²³ are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl;
wherein
substituents for substituted alkyl, substituted alkenyl, and substituted alkynyl are selected from halogen, -CN, -CO₂R', -C(O)R', -C(O)NR'R", oxo (=O or -O⁻) , -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R"', -NR"'S(O)NR'R", -NR'"S(O)₂NR'R", -SR' , -S(O)R', -S(O)₂R_{'}, -S(O)₂NR'R", -NR'-C(NHR")=NR"', -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻) , -OR', -OC(O)R', -OC(O)NR'R", -NO₂, -NR'C(O)R", -NR'"(O)NR'R", -NR'R", -NR'CO₂R", -NR'S(O)R", -NR'S (O)₂R", -NR"'S(O)NR'R", -NR"'S(O)₂NR'R", -SR', -S(O)R', -S(O)₂R', -S(O)₂NR'R", -NR'-C(NHR")=NR'", -SiR'R"R"', -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R'" are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5-6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NMR""-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NMR"'-, -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR""-, -S-, -S(O)-, -S(O)₂-, or -5(O)₂NR'-, wherein R"" is selected from hydrogen or unsubstituted C₁₋₈ alkyl.

5. The compound of claim 4, or a salt thereof, wherein Z¹ is selected from the group consisting of substituted or unsubstituted 9- or 10-membered heteroaryl with 1 to 2 nitrogen atoms and with 0 or 2 substituents each independently selected from the group consisting of halogen, substituted or unsubstituted C₁₋₈ alkyl, substituted or unsubstituted C₂₋₈ alkenyl, substituted or unsubstituted C₂₋₈ alkynyl, -CN, oxo (=O or -O⁻) , -NO₂, -OR⁴⁴, -OC(O)R⁴⁴, -CO₂R ⁴⁴, -C(O)R⁴⁴, C(O)NR⁴⁴R⁴⁵, -OC(O) NR⁴⁴R⁴⁵, -NR⁴⁴C(O)R⁴⁵, -NR⁴⁴C(O)NR⁴⁵R⁴⁶, -NR⁴⁴R⁴⁵, -NR⁴⁴CO₂R⁴⁵, -SR⁴⁴, -S(O)R⁴⁴, -S(O)₂R⁴⁴, -S(O)₂NR ⁴⁴R⁴⁵, -NR⁴⁴S(O)₂R⁴⁵, substituted or unsubstituted C₆₋₁₀ aryl, substituted or unsubstituted 5- to 10-membered heteroaryl and substituted or unsubstituted 3-to 10-membered heterocyclyl;
R⁴⁴, R⁴⁵ and _{R}⁴⁶ are each independently selected from the group consisting of hydrogen, substituted or unsubstituted C₁₋₈ alkyl, substituted or unsubstituted 3- to 10-membered heterocyclyl, substituted or unsubstituted C₂₋₆ alkenyl, substituted or unsubstituted C₂₋₆ alkynyl, substituted or unsubstituted C₆₋₁₀ aryl, and substituted or unsubstituted 5-to 10-membered heteroaryl.

6. The compound of claim 5, or a salt thereof, wherein Z¹ is 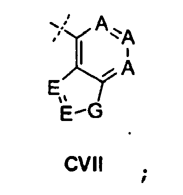 wherein
A is selected from the group consisting of -CH- and -N-;
E is -CH-;
G is -NR⁴⁴-; and
R⁴⁷ is hydrogen.

7. The compound of claim 4, or a salt thereof, wherein Z¹ is 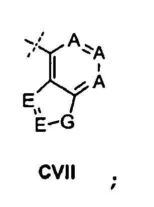 wherein
A is selected from the group consisting of -CH-, -CZ¹⁶- and -N⁺(O)⁻-;
each occurrence of Z¹⁶ is independently selected from the group consisting of unsubstituted C₁₋₈ alkyl;
E is -CH-;
G is -NR⁴⁷-_{;}
R⁴⁷ is hydrogen.

8. The compound of claims 6 or 7, or a salt thereof, wherein X⁴ is selected from the group consisting of halogen and -CH₃, and X³ is selected from the group consisting of halogen, -CH₃, -CF₃, -OCH₃, and -OCF₃.

9. The compound of claim 8, or a salt thereof, wherein X⁴ is chloro and X³ is -CF₃.

10. The compound of claim 9, or a salt thereof, wherein Y⁸, Y⁹, and Y¹⁰ are each independently selected from the group consisting of hydrogen, halogen, -CO₂R³, -OR³, and substituted or unsubstituted C₁₋₈ alkyl, where 1 to 2 of Y⁸, Y⁹, and Y¹⁰ are other than hydrogen.

11. The compound of claim 9, or a salt thereof, wherein Y⁸ and Y¹⁰ are hydrogen and Y⁹ is halogen.

12. The compound of claim 11, or a salt thereof, wherein Y⁸ and Y¹⁰ are hydrogen and Y⁹ is chloro.

13. A compound of formula (CLI) or a salt thereof: 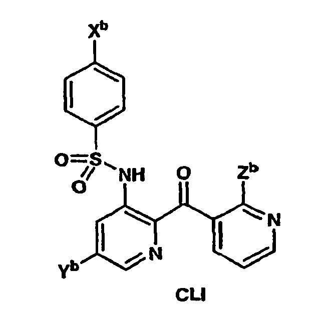 where
X^{b} is selected from the group consisting of -OR^{24a}, and substituted or unsubstituted C₁₋₈ alkyl;
R^{24a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
Y^{b} is selected from the group consisting of hydrogen, halogen, -CN, -C(O)R^{27a}, -CO₂R^{27a}, -C(O)NR^{27a}R^{28a}, -OR^{27a}, -OC(O)R^{27a}, -OC(O)NR^{27a}R^{28a}, -SR^{27a}, -S(O)R^{27a}, -S(O)₂R^{27a} -S(O)₂NR^{27a}R^{28a} -NO₂, -NR^{27a}R^{28a}, -NR^{27a}C (O)R^{28a}, -NR^{27a}C (O)₂R^{28a} -NR^{27a}S (O)₂R^{28a}, -NR^{27a}C(O)NR^{28a}R^{29a}, substituted or unsubstituted C₁₋₈ alkyl, substituted or unsubstituted C₂₋₈ alkenyl, substituted or unsubstituted C₂₋₈ alkynyl, substituted or unsubstituted 3- to 10-membered heterocyclyl, substituted or unsubstituted C₆₋₁₀ aryl, and substituted or unsubstituted 5- to 10-membered heteroaryl;
R^{27a}, R^{28a} and R^{29a} are each independently selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10- membered heterocyclyl; and
R^{27a} and R^{28a}, R^{28a} and R^{29a} or R^{27a} and R^{29a} may, together with the atoms to which they are attached, form a substituted or unsubstituted 5-, 6-, or 7-membered ring;
Z^{b} is selected from the group consisting of substituted or unsubstituted C₁₋₈ alkyl;
wherein
substituents for substituted alkyl, substituted alkenyl, and substituted alkynyl are selected from halogen, -CN, -CO₂R', -C(O)R', -C(O)NR'R", oxo (=O or -O⁻), -R', -OC(O)R', -OC(O)NR'R", -NO₂ , -NR'C(O)R'', -NR'"C(O)NR'R", -NR'R", -NR'CO₂R'', -NR'S(O)R", -NR'S(O)₂R"', -NR'"S(O)NR'R", -NR"'S(O)₂NR'R", -SR', -S(O)R', -S(O)₂R', -S(O)₂NR'R", -NR'-C(NHR")=NR"', -SiR'R"R"', -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted
heteroaryl and substituted heterocyclyl are selected from halogen, -CN, -CO₂R', -C(O)R', -C(O)NR'R", oxo (=O or -O⁻), -R', -OC(O)R', -OC(O)NR'R", -NO₂, -NR'C(O)R", -NR'"C(O)NR'R", -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)2NR'R", -NR'-C(NHR")=NR"', -SiR'R"R"', -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R"' are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5-6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NR""-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR"'-, -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR""-, -S-, -S(O)-, -S(O)₂-, or -S(O)₂NR'-, wherein R"" is selected from hydrogen or unsubstituted C₁₋₈ alkyl.

14. A compound of formula (CLII) or a salt thereof: 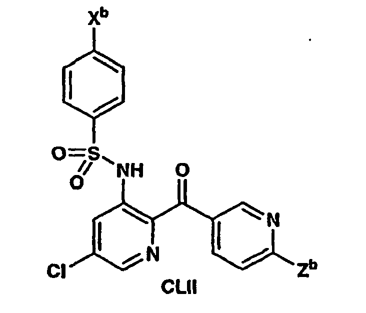 where
X^{b} is selected from the group consisting of -OR^{24a,} and substituted or unsubstituted C₁₋₈ alkyl;
R^{29a} is selected from the group consisting of
hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
Z^{b} is OR^{30a};
R^{30a} is selected from the group consisting of
hydrogen and substituted or unsubstituted C₁₋₈ alkyl;
wherein
substituents for substituted alkyl, substituted
alkenyl, and substituted alkynyl are selected from halogen, -CN, -CO₂R' -C(O)R', -C(O)NR'R", oxo (=O or -O⁻) , -R', -OC(O)R', -OC(O)NR'R", -NO₂, -NR'C(O)R", -NR"'C(O)NR'R", -NR'R", -NR'CO₂R", -NR'S(O)R", -NR'S(O)₂R"', -NR'"S(O)NR'R", -NR'"S(O)₂NR'R", -SR', -S(O)R', -S(O)₂R', -S(O)₂NR'R", -NR'-C(NHR")=NR'", -SiR'R"R"', -N₃, C₆-₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻), -OR', -OC(O)R', -OC(O)NR'R", -NO₂, -NR'C(O)R", -NR'"C(O)NR'R", -NR'R", -NR'CO₂R", -NR'S (O) R", -NR'S (O)₂R", -NR"'S(O)NR'R", -NR"'S(O)₂NR'R", -SR', -S(O)R', -S(O)₂R', -S(O)NR'R", -NR'-C(NHR")=NR"', -SiR'R"R"', -N₃, C₁₋₈ alkyl, C₂₋₈ alkenyl, C₂₋₈ alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R'" are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5-6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NMR""-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR'"-, -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR""-, -S-, -S(O)-, -S(O)₂-, or -S(O)₂NR'-, wherein R"" is selected from hydrogen or unsubstituted C₁₋₈ alkyl.

15. A compound of formula (CLIII) or a salt thereof: 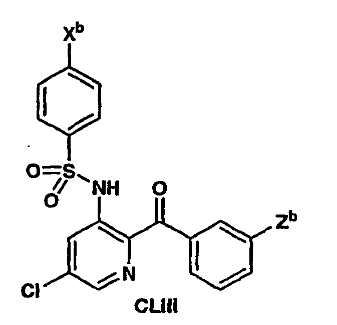 where
X^{b} is selected from the group consisting of -OR^{24a,} and substituted or unsubstituted C₁₋₈ alkyl;
R^{29a} is selected from the group consisting of hydrogen, substituted or unsubstituted C₁₋₈ alkyl, substituted or unsubstituted C₂₋₈ alkenyl, substituted or unsubstituted C₂₋₈ alkynyl, substituted or unsubstituted C₆₋₁₀ aryl, substituted or unsubstituted 5- to 10-membered heteroaryl, and substituted or unsubstituted 3- to 10-membered heterocyclyl; and
Z^{b} is halogen;
wherein
substituents for substituted alkyl, substituted alkenyl, and substituted alkynyl are selected from halogen, -CN, -CO₂R', -C(O)R', -C(O)NR'R", oxo (=O or -O⁻) , -R', -OC(O)R', -OC(O)NR'R", -NO₂, -NR'C(O)R", -NR'"C(O)NR'R", -NR'R", -NR'CO₂R", -NR'S(O)R", -NR'S(O)₂R"', -NR"'S(O)NR'R'', -NR'"S(O)₂NR'R", -SR', -S(O)R', -S(O)₂R', -S(O)₂NR'R", -NR'-C(NHR")=NR''', -SiR'R"R'", -N₃, C₆₋₁₀ aryl, 5- to 10-membered heteroaryl, and 3- to 10-membered heterocyclyl; the number of possible substituents range from zero to (2m'+1), where m' is the total number of carbon atoms in such radical;
substituents for substituted aryl, substituted heteroaryl and substituted heterocyclyl are selected from halogen, -CN, -CO₂R', -C(O)R', -C(O)NR'R", oxo (=O or -O⁻), -OR', -OC(O)R', -OC(O)NR'R", -NO₂, -NR'C(O)R", -NR"'C(O)NR'R", -NR'R", -NR'CO₂R", -NR'S (O) R", -NR'S (O)₂R", -NR'''S(O)NR'R'', -NR"'S(O)₂NR'R", -SR', -S(O)R', -S(O)₂R', -S(O)₂NR'R", -NR'-C(NHR")=NR'", -SiR'R"R"', -N₃, C₁₋₈ alkyl, C₂₋₈ alkenyl, C₂₋₈. alkynyl, C₆₋₁₀ aryl, 5- to 10-membered heteroaryl, and 3- to 10-membered heterocyclyl; the number of possible substituents range from zero to the total number of open valences on the aromatic ring system;
wherein
R', R" and R'" are selected from hydrogen, C₁₋₈ alkyl, C₂₋₈ alkenyl, C₂₋₈ alkynyl, aryl, heteroaryl, heterocyclyl, arylalkyl, and aryloxyalkyl; when R' and R" are attached to the same nitrogen atom, they can be combined with the nitrogen atom to form a 3-, 4-, 5-, 6-, or 7-membered ring; R' and R", R" and R"', or R' and R'" may together with the atom(s) to which they are attached, form a 5-6-, or 7-membered ring;
two of the substituents on adjacent atoms of an aryl or heteroaryl ring may optionally be replaced with a substituent of the formula -T-C(O)-(CH₂)_{q}-U-, wherein T and U are independently -NR""-, -O-, -CH₂- or a single bond, and q is an integer of from 0 to 2; two of the substituents on adjacent atoms of the aryl or heteroaryl ring may optionally be replaced with a substituent of the formula -A'-(CH₂)ᵣ-B'-, wherein A' and B' are independently -CH₂-, -O-, -NR"'-, -S-, -S(O)-, -S(O)₂-, -S(O)₂NR""- or a single bond, and r is an integer of from 1 to 3; one of the single bonds of the new ring so formed may optionally be replaced with a double bond; two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR""-, -S-, -S(O)-, -S(O)₂-, or -S(O)₂NR'- wherein R"" is selected from hydrogen or unsubstituted C₁₋₈ alkyl.

16. A composition comprising a pharmaceutically acceptable carrier and a compound of any of the preceding claims.

17. A composition comprising a pharmaceutically acceptable carrier and a compound of claim 6 or 7, or a salt thereof.

18. A compound of any of claims 1 to 15 for use in treating a CCR2-mediated condition or disease, where the CCR2-mediated condition or disease is selected from atherosclerosis, restenosis, multiple sclerosis, inflammatory bowel disease, renal fibrosis, rheumatoid arthritis, obesity, diabetes, chronic obstructive pulmonary disease, idiopathic pulmonary fibrosis, idiopathic pneumonia syndrome, pulmonary fibrosis, transplantation rejection, graft-versus-host disease, cancer, and neuropathic pain.

19. The compound of claim 18, wherein said compound is a compound of claim 6 or 7, or a salt thereof.

20. The compound of claim 18, where the CCR2-mediated disease or condition is atherosclerosis.

21. The compound of claim 18, where the CCR2-mediated disease or condition is restenosis.

22. The compound of claim 18, where the CCR2-mediated condition or disease is multiple sclerosis.

23. The compound of claim 18, where the CCR2-mediated condition or disease is selected from the group consisting of inflammatory bowel disease, renal fibrosis, rheumatoid arthritis, obesity and diabetes.

24. The compound of claim 18, where the CCR2-mediated condition or disease is selected from the group consisting of chronic obstructive pulmonary disease, idiopathic pulmonary fibrosis and idiopathic pneumonia syndrome.

25. The compound of claim 18, where the CCR2-mediated condition or disease is selected from the group consisting of pulmonary fibrosis, transplantation rejection, graft-versus-host disease and cancer.

26. The compound of claim 18, where the CCR2-mediated condition or disease is neuropathic pain.

27. The compound of claim 18, where the condition or disease is treated by administering the compound by the oral, parenteral, rectal, transdermal, sublingual, nasal or topical route.

28. The compound of claim 18, where the compound is administered in combination with an anti-inflammatory or analgesic agent.

29. An in vitro method of modulating CCR2 function in a cell, comprising contacting the cell with a CCR2 modulating amount of the compound of any of claims 1 to 15.

30. An in vitro method of modulating CCR9 function in a cell, comprising contacting the cell with a CCR9 modulating amount of the compound of any of claims 1 to 15.

## Patentansprüche

1. Verbindung der Formel (I) oder ein Salz davon: 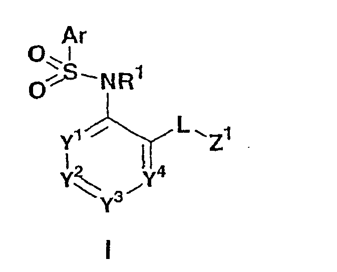 wobei
Ar unsubstituiertes Phenyl oder Phenyl substituiert mit einem oder mehreren Halogen(en), substituiertes oder unsubstituiertes C₁₋₈-Alkyl, substituiertes oder unsubstituiertes C₂₋₈-Alkenyl, substituiertes oder unsubstituiertes C₂₋₈-Alkinyl, -CN, -C(O)R¹⁸, -CO₂R¹⁸, -C(O)NR¹⁸R¹⁹, -OC(O)R¹⁹, -OC(O)NR¹⁸R¹⁹, -NO₂, -NR¹⁸C(O)R¹⁹, -NR¹⁸C(O)NR¹⁹R²⁰, -NR¹⁸R¹⁹, -NR¹⁸CO₂R¹⁹, -NR¹⁸S(O)R¹⁹, -SR¹⁸, -S(O)R¹⁸, -S(O)₂R¹⁸, -S(O)2NR¹⁸R¹⁹, substituiertes oder unsubstituiertes C₆₋₁₀-Aryl, substituiertes oder unsubstituiertes 5- bis 10-gliedriges Heteroaryl und substituiertes oder unsubstituiertes 3- bis 10-gliedriges Heterocyclyl ist;
R¹⁸, R¹⁹ und R²⁰ jeweils unabhängig voneinander ausgewählt sind aus der Gruppe bestehend aus Wasserstoff, substituiertem oder unsubstituiertem C₁₋₈-Alkyl, substituiertem oder unsubstituiertem ₂₋₈-Alkeny), substituiertem oder unsubstituiertem C₂₋₈-Alkinyl, substituiertem oder unsubstituiertem Aryl, substituiertem oder unsubstituiertem Heteroaryl und substituiertem oder unsubstituiertem Heterocyclyl;
R¹ ausgewählt ist aus der Gruppe bestehend aus Wasserstoff, unsubstituiertem C₁-Alkyl und unsubstituiertem C₃₋₈-Alkyl;
Y¹ -CR²⁸-ist;
Y² -CR^{2b}- ist;
Y³ -CR^{2c}- ist,
R^{2a}, R^{2b} und R^{2c} jeweils unabhängig voneinander ausgewählt sind aus der Gruppe bestehend aus Wasserstoff, Halogen und unsubstituiertem C₁₋₈-Alkyl;
Y⁴ -N- ist;
L -C(O)- ist; und
Z¹ substituiertes oder unsubstituiertes Phenyl ist;
wobei
Substituenten für substituiertes Alkyl, substituiertes Alkenyl und substituiertes Alkinyl ausgewählt sind aus Halogen, -CN, -CO₂R', -C(O)R', -C(O)NR'R", oxo (=O oder -O⁻), -OR', -OC(O)R', -OC(O)NR'R", -NO₂, -NR'C(O)R", -NR"'C(O)NR'R", -NR'R", -NR'CO2R", -NR'S(O)R", -NR'S(O)₂R"', -NR"'S(O)NR'R", -NR"'S(O)₂NR'R" -SR', -S(O)R', -S(O)₂R', -S(O)₂NR'R", -NR', -C(NHR")=NR"', -SiR'R"R"', -N₃, C₆₋₁₀-Aryl, 5- bis 10-gliedrigem Heteroaryl und 3- bis 10- gliedrigem Heterocyclyl; wobei die Anzahl an möglichen Substituenten von Null bis (2m'+1) reicht, wobei m' die Gesamtanzahl an Kohlenstoffantomen in solch einem Radikal ist;
Substituenten für substituiertes Aryl, substituiertes Heteroaryl und substituiertes Heterocyclyl ausgewählt sind aus Halogen, -CN, -CO₂R' -C(O)R', -C(O)NR'R", oxo (=O oder -O⁻), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR"'S(O)NR'R", -NR"'S(O)₂NR'R" -SR', -S(O)R', -S(O)₂R', -S(O)₂NR'R", -NR', -C(NHR")=NR"', -SiR'R"R"', -N₃, C₁₋₈-Alkyl, C₂₋₈-Alkenyl, C₂₋₈-Alkinyl, C₆₋₁₀-Aryl, 5- bis 10-gliedrigem Heteroaryl und 3- bis 10-gliedrigem Heterocyclyl; wobei die Anzahl an möglichen Substituenten von Null bis zur Gesamtanzahl an offenen Valenzen an dem aromatischen Ringsystem reicht;
wobei
R', R" und R'" ausgewählt sind aus Wasserstoff, C₁₋₈-Alkyl, C₂₋₈-Alkenyl, C₂₋₈-Alkinyl, Aryl, Heteroaryl, Heterocyclyl, Arylakyl und Aryloxyalkyl; wenn R' und R" an das selbe Stickstoffatom gebunden sind, können sie mit dem Stickstoffatom kombiniert werden, um einen 3-, 4-, 5-, 6- oder 7-gliedrigen Ring zu bilden; wobei R' und R", R" und R'" oder R' und R'" zusammen mit dem/den Atom/Atomen an das/die sie gebunden sind einen 5-, 6- oder 7-gliedrigen Ring bilden können;
zwei der Substituenten an benachbarten Atomen eines Aryl- oder Heteroarylrings gegebenenfalls durch einen Substituenten der Formel -T-C(O)-(CH₂)_{q}-U- ersetzt werden können , wobei T und U unabhängig voneinander -NR""-, -O-, -CH₂-oder eine Einfachbindung sind und q eine ganze Zahl von 0 bis 2 ist; zwei der Substituenten an benachbarten Atomen des Aryl- oder Heteroarylrings gegebenenfalls durch einen Substituenten der Formel -A'-(CH₂)ᵣ-B'- ersetzt werden können, wobei A' und B' unabhängig voneinander -CH₂-,-O-, -NR"'-, -S-, -S(O)-, -S(O)₂-, -S(O)₂NR""- oder eine Einfachbindung sind und r eine ganze Zahl von 1 bis 3 ist; wobei eine der Einfachbindungen des neuen Rings, der so gebildet wird, gegebenenfalls durch eine Doppelbindung ersetzt werden kann; zwei der Substituenten an benachbarten Atomen des Aryl- oder Heteroarylrings gegebenenfalls durch einen Substituenten der Formel -(CH₂)ₛ-X-(CH₂)ₜ- ersetzt werden können, wobei s und t unabhängig voneinander ganze Zahlen von 0 bis 3 sind und X -O-, -NR""-, -S-, -S(O)-, -S(O)₂-oder -S(O)₂NR'- ist, wobei R"" ausgewählt ist aus Wasserstoff oder unsubstituiertem C₁₋₈-Alkyl.

2. Verbindung nach Anspruch 1, die durch die Formel (II) dargestellt wird oder ein Salz davon: 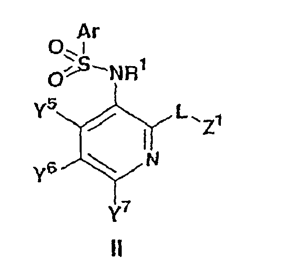 wobei
Y⁵, Y⁶ und Y⁷ jeweils unabhängig voneinander ausgewählt sind aus der Gruppe bestehend aus Wasserstoff, Halogen und unsubstituiertem C₁₋₈-Alkyl.

3. Verbindung nach Anspruch 1, die durch die Formel (CLIII) dargestellt wird oder ein Salz davon: 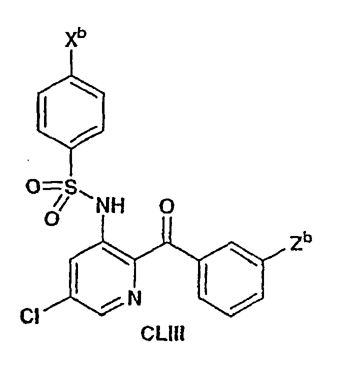 wobei
X^{b} ausgewählt ist aus der Gruppe bestehend aus CF₃ und unsubstituiertem C₁₋₈-Alkyl; und
Z^{b} Halogen ist.

4. Verbindung der Formel III oder ein Salz davon: 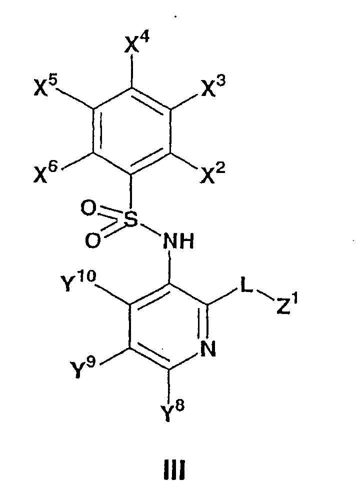 wobei
L -C(O)- ist;
Z¹ ausgewählt ist aus der Gruppe bestehend aus substituiertem oder unsubstituiertem 5- bis 10-gliedrigem Heteroaryl;
X², X³, X⁴, X⁵ und X⁶ jeweils unabhängig voneinander ausgewählt sind aus der Gruppe bestehend aus Wasserstoff, Halogen, substituiertem oder unsubstituiertem C₁₋₈-Alkyl, substituiertem oder unsubstituiertem C₂₋₈-Alkenyl, substituiertem oder unsubstituiertem C₂-₈-Alkinyl, -CN, -NO₂, -C(O)R¹⁸, -CO₂R¹⁸, -C(O)NR¹⁸R¹⁹, -OR¹⁸, -OC(O)R¹⁹, -OC(O)NR¹⁸R¹⁹, -NR¹⁸C(O)R¹⁹, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
R¹⁸, R¹⁹ und R²⁰ jeweils unabhängig voneinander ausgewählt sind aus der Gruppe bestehend aus Wasserstoff, substituiertem oder unsubstituiertem C₁₋₈-Alkyl, substituiertem oder unsubstituiertem C₂-₈-Alkenyl, substituiertem oder unsubstituiertem C₂-₈-Alkinyl, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
Y⁸, Y⁹ und Y¹⁰ jeweils unabhängig voneinander ausgewählt sind aus der Gruppe bestehend aus Wasserstoff, Halogen, -CN, -NO₂, -OR²¹, -CO₂R²¹, -OC(O)R²¹, -OC(O)NR²¹R²², -C(O)NR²¹R²², -C(O)R²¹, -SR²¹, -S(O)R²¹, -S(O)₂R²¹, -NR²¹R²², -NR²¹C(O)R²², -NR²¹C(O)R²², -NR²¹S(O)₂R²², -NR²¹C(O)NR²²R²³, substituiertem oder unsubstituiertem C₁₋₈-Alkyl und substituiertem oder unsubstituiertem 3-bis 10-gliedrigem Heterocyclyl,
R²¹ , R²² und R²³ unabhängig voneinander ausgewählt sind aus der
Gruppe bestehend aus Wasserstoff,
substituiertem oder unsubstituiertem C₁₋₈-Alkyl, substituiertem oder unsubstituiertem C₂₋₈-Alkenyl, substituiertem oder unsubstituiertem C₂-₈-Alkinyl, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
wobei
Substituenten für substituiertes Alkyl, substituiertes Alkenyl und substituiertes Alkinyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NRS(O)₂R-"', -NR"'S(O)NR'R", -NR'"S(O)₂NR'R" -SR', -S(O)R', -S(O)₂R', -S(O)₂NR'-R", -NR', -C(NHR")=NR"', -SiR'R"R"', -N₃, C₆₋₁₀-Aryl, 5- bis 10-gliedrigem Heteroaryl und 3- bis 10-gliedrigem Heterocyclyl; wobei die Anzahl an möglichen Substituenten von Null bis (2m'+1) reicht, wobei m' die Gesamtanzahl an Kohlenstoffantomen in solch einem Radikal ist;
Substituenten für substituiertes Aryl, substituiertes Heteroaryl und substituiertes Heterocyclyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR"'S(O)NR'R", -NR"'S(O)₂NR'R" -SR', -S(O)R', -S(O)₂R', -S(O)₂NR'R", -NR', -C(NHR")=NR"', -SiR'R"R"', -N₃, C₁₋₈-Alkyl, C₂₋₈-Alkenyl, C₂₋₈-Alkinyl, C₆₋₁₀-Aryl, 5- bis 10-gliedrigem Heteroaryl und 3- bis 10-gliedrigem Heterocyclyl; wobei die Anzahl an möglichen Substituenten von Null bis zur Gesamtanzahl an offenen Valenzen an dem aromatischen Ringsystem reicht;
wobei
R', R" und R"' ausgewählt sind aus Wasserstoff, C₁₋₈-Alkyl, C₂₋₈-Alkenyl, C₂₋₈-Alkinyl, Aryl, Heteroaryl, Heterocyclyl, Arylakyl und Aryloxyalkyl; wenn R' und R" an das selbe Stickstoffatom gebunden sind, können sie mit dem Stickstoffatom kombiniert werden, um einen 3-, 4-, 5-, 6- oder 7-gliedrigen Ring zu bilden; wobei R' und R", R" und R'" oder R' und R"' zusammen mit dem/den Atom/Atomen an das/die sie gebunden sind einen 5-, 6- oder 7-gliedrigen Ring bilden können;
zwei der Substituenten an benachbarten Atomen eines Aryl- oder Heteroarylrings gegebenenfalls durch einen Substituenten der Formel -T-C(O)-(CH₂)_{q}-U- ersetzt werden können, wobei T und U unabhängig voneinander -NR""-, -O-, -CH₂- oder eine Einfachbindung sind und q eine ganze Zahl von 0 bis 2 ist; zwei der Substituenten an benachbarten Atomen des Aryl- oder Heteroarylrings gegebenenfalls durch einen Substituenten der Formel -A'-(CH₂)ᵣ-B'-, ersetzt werden können, wobei A' und B' unabhängig voneinander -CH₂-, -O-, -NR"'-, -S-, -S(O)-, -S(O)₂-, -S(O)₂NR""- oder eine Einfachbindung sind und r eine ganze Zahl von 1 bis 3 ist; wobei eine der Einfachbindungen des neuen Rings, der so gebildet wird, gegebenenfalls durch eine Doppelbindung ersetzt werden kann; zwei der Substituenten an benachbarten Atomen des Aryl- oder Heteroarylrings gegebenenfalls durch einen Substituenten der Formel -(CH₂)₂-X-(CH₂)ₜ- ersetzt werden können, wobei s und t unabhängig voneinander ganze Zahlen von 0 bis 3 sind und X -O-, -NR""-, -S-, -S(O)-, -S(O)₂- oder -S(O)₂NR'- ist, wobei R"" ausgewählt ist aus Wasserstoff oder unsubstituiertem C₁₋₈-Alkyl.

5. Verbindung nach Anspruch 4 oder ein Salz davon, wobei Z¹ ausgewählt ist aus der Gruppe bestehend aus substituiertem oder unsubstituiertem 9- oder 10-gliedrigem Heteroaryl mit 1 bis 2 Stickstoffatomen und mit 0 oder 2 Substituenten, die jeweils unabhängig voneinander ausgewählt sind aus der Gruppe bestehend aus Halogen, substituiertem oder unsubstituiertem C₁₋₈-Alkyl, substituiertem oder unsubstituiertem C₂₋₈-Alkenyl, substituiertem oder unsubstituiertem C₂₋₈-Alkinyl, -CN, oxo (=O oder -O-), -NO₂, -OR⁴⁴, -OC(O)R⁴⁴, -C(O)₂R⁴⁴, -C(O)R⁴⁴, -C(O)NR⁴⁴R⁴⁵, -OC(O)NR⁴⁴R⁴⁵, -NR⁴⁴C(O)R⁴⁵, -NR⁴⁴C(O)NR⁴⁵R⁴⁶, -NR⁴⁴R⁴⁵, -NR⁴⁴CO₂R⁴⁵, -SR⁴⁴, -S(O)R⁴⁴, -S(O)₂R⁴⁴, -S(O)NR⁴⁴R⁴⁵, -NR⁴⁴S(O)₂R⁴⁵, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
R⁴⁴, R⁴⁵ und R⁴⁶ jeweils unabhängig voneinander ausgewählt sind aus der Gruppe bestehend aus Wasserstoff, substituiertem oder unsubstituiertem C₁₋₈-Alkyl, substituiertem oder unsubstituiertem 3- bis 10-gliedrigem Heterocyclyl, substituiertem oder unsubstituiertem C₂₋₆-Alkenyl, substituiertem oder unsubstituiertem C₂₋₆-Alkinyl, substituiertem oder unsubstituiertem C₆₋₁₀-Aryl und substituiertem oder unsubstituiertem 5- bis 10-gliedrigem Heteroaryl.

6. Verbindung nach Anspruch 5 oder ein Salz davon, wobei Z¹ 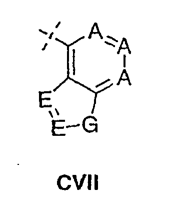 ist,
wobei
A ausgewählt ist aus der Gruppe bestehend aus -CH- und -N-; E -CH- ist;
G -NR⁴⁷- ist; und
R⁴⁷ Wasserstoff ist.

7. Verbindung nach Anspruch 4 oder ein Salz davon, wobei Z¹ 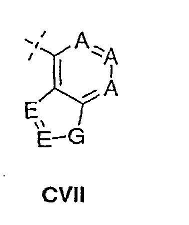 ist,
wobei
A ausgewählt ist aus der Gruppe bestehend aus -CH-, -CZ¹⁶- und -N⁺ (O)⁻-;
wobei jedes Auftreten von Z¹⁶ unabhängig voneinander ausgewählt ist aus der Gruppe bestehend aus unsubstituiertem C₁₋₈-Alkyl;
E -CH- ist;
G -NR⁴⁷- ist; und
R⁴⁷ Wasserstoff ist.

8. Verbindung nach Ansprüchen 6 oder 7 oder ein Salz davon, wobei X⁴ ausgewählt ist aus der Gruppe bestehend aus Halogen und -CH₃, und X³ ausgewählt ist aus der Gruppe bestehend aus Halogen, -CH₃, -CF₃, -OCH₃ und -OCF₃.

9. Verbindung nach Anspruch 8 oder ein Salz davon, wobei X⁴ Chlor und X³ -CF₃ ist.

10. Verbindung nach Anspruch 9 oder ein Salz davon, wobei Y⁸, Y⁹ und Y¹⁰ jeweils unabhängig voneinander ausgewählt sind aus der Gruppe bestehend aus Wasserstoff, Halogen, -CO₂R³, -OR³ und substituiertem oder unsubstituiertem C₁₋₈-Alkyl, wobei 1 bis 2 von Y⁸, Y⁹ und Y¹⁰ anders als Wasserstoff sind.

11. Verbindung nach Anspruch 9 oder ein Salz davon, wobei Y⁸ und Y¹⁰ Wasserstoff sind und Y⁹ Halogen ist.

12. Verbindung nach Anspruch 11 oder ein Salz davon, wobei Y⁸ und Y¹⁰ Wasserstoff sind und Y⁹ Chlor ist.

13. Verbindung der Formel (CLI) oder ein Salz davon: 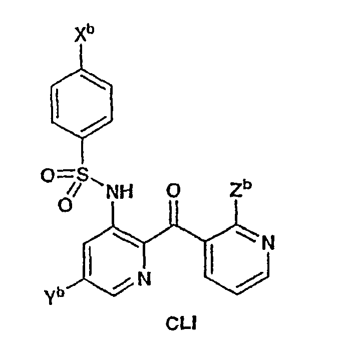 wobei
X^{b} ausgewählt ist aus der Gruppe bestehend aus -OR^{24a} und
substituiertem oder unsubstituiertem C₁₋₈-Alkyl,
R^{24a} ausgewählt ist aus der Gruppe bestehend aus Wasserstoff,
substituiertem oder unsubstituiertem C₁₋₈-Alkyl, substituiertem oder unsubstituiertem C₂₋₈-Alkenyl, substituiertem oder unsubstituiertem C₂₋₈-Alkinyl, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
und
Y^{b} ausgewählt ist aus der Gruppe bestehend aus Wasserstoff,
Halogen, -CN, -C(O)R^{27a}, -CO₂R^{27a}, -C(O)NR^{27a}R^{28a}, -OR^{27a}, -OC(O)R^{27a}, -OC(O)NR^{27a}R^{28a}, -SR^{27a}, -S(O)R^{27a}, -S(O)₂R^{27a}, -S(O)NR^{27a}R^{2Ba}, -NO₂, -NR^{27a}R^{28a}, -NR^{27a}C(O)R^{28a}, -NR^{27a}C(O)R^{28a}, -NR^{27a}S(O)R^{28a}, -NR^{27a}C(O)NR^{28a}R^{29a}, substituiertem oder unsubstituiertem C₁₋₈-Alkyl, substituiertem oder unsubstituiertem C₂₋₈-Alkenyl, substituiertem oder
unsubstituiertem C₂₋₈-Alkinyl, substituiertem oder unsubstituiertem 3- bis 10-gliedrigem Heterocyclyl, substituiertem oder unsubstituiertem C₆₋₁₀-Aryl und substituiertem oder unsubstituiertem 5- bis 10-gliedrigem Heteroaryl; R^{27a} , R^{28a} und R^{29a} jeweils unabhängig voneinander ausgewählt
aus der Gruppe bestehend aus Wasserstoff, substituiertem oder unsubstituiertem C₁₋₈-Alkyl, substituiertem oder unsubstituiertem C₂₋₈-Alkenyl, substituiertem oder unsubstituiertem C₂₋₈-Alkinyl, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl; und
R^{27a} und R^{28a}, R^{28a} und R^{29a} oder R^{27a} und R^{29a} zusammen mit den Atomen, an die sie gebunden sind, einen substituierten oder unsubstituierten 5-, 6- oder 7-gliedrigen Ring bilden können;
Z^{b} ausgewählt ist aus der Gruppe bestehend aus substituiertem oder
unsubstituiertem C₁₋₈-Alkyl;
wobei
Substituenten für substituiertes Alkyl, substituiertes Alkenyl und substituiertes Alkinyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R-", -NR"'S(O)NR'R", -NR"'S(O)₂NR'R" -SR', -S(O)R', -S(O)₂R', -S(O)₂NR'R", -NR', -C(NHR")=NR"', -SiR'R"R"', -N₃, C₆₋₁₀Aryl, 5- bis 10-gliedrigem Heteroaryl und 3- bis 10- gliedrigem Heterocyclyl; wobei die Anzahl an möglichen Substituenten von Null bis (2m'+1) reicht, wobei m' die Gesamtanzahl an Kohlenstoffantomen in solch einem Radikal ist;
Substituenten für substituiertes Aryl, substituiertes Heteroaryl und substituiertes Heterocyclyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR"'S(O)NR'R", -NR"'S(O)2NR'R" -SR', -S(O)R', -S(O)₂R -S(O)₂NR'R", -NR', -C(NHR")=NR"', -SiR'R"R"', -N₃, C₁₋₈-Alkyl, C₂₋₈-Alkenyl, C₂₋₈-Alkinyl, C₆-₁₀-Aryl, 5- bis 10-gliedrigem Heteroaryl und 3- bis 10-gliedrigem Heterocyclyl; wobei die Anzahl an möglichen Substituenten von Null bis zur Gesamtanzahl an offenen Valenzen an dem aromatischen Ringsystem reicht;
wobei
R', R" und R"' ausgewählt sind aus Wasserstoff, C₁₋₈-Alkyl, C₂₋₈-Alkenyl, C₂₋₈-Alkinyl, Aryl, Heteroaryl, Heterocyclyl, Arylakyl und Aryloxyalkyl; wenn R' und R" an das selbe Stickstoffatom gebunden sind, können sie mit dem Stickstoffatom kombiniert werden, um einen 3-, 4-, 5-, 6- oder 7-gliedrigen Ring zu bilden; wobei R' und R", R" und R'" oder R' und R'" zusammen mit dem/den Atom/Atomen an das/die sie gebunden sind einen 5-, 6- oder 7-gliedrigen Ring bilden können;
zwei der Substituenten an benachbarten Atomen eines Aryl- oder Heteroarylrings gegebenenfalls durch einen Substituenten der Formel -T-C(O)-(CH₂)q-U- ersetzt werden können, wobei T und U unabhängig voneinander -NR""-, -O-, -CH₂- oder eine Einfachbindung sind und q eine ganze Zahl von 0 bis 2 ist; zwei der Substituenten an benachbarten Atomen des Aryl- oder Heteroarylrings gegebenenfalls durch einen Substituenten der Formel -A'-(CH₂)ᵣ-B'-, ersetzt werden können, wobei A' und B' unabhängig voneinander -CH₂-, -O-, -NR"'-, -S-, -S(O)-, -S(O)₂-, -S(O)₂NR""- oder eine Einfachbindung sind und r eine ganze Zahl von 1 bis 3 ist; wobei eine der Einfachbindungen des neuen Rings, der so gebildet wird, gegebenenfalls durch eine Doppelbindung ersetzt werden kann; zwei der Substituenten an benachbarten Atomen des Aryl- oder Heteroarylrings gegebenenfalls durch einen Substituenten der Formel -(CH₂)ₛ-X-(CH₂)ₜ- ersetzt werden können, wobei s und t unabhängig voneinander ganze Zahlen von 0 bis 3 sind und X -O-, -NR""-, -S-, -S(O)-, -S(O)₂- oder -S(O)₂NR'- ist, wobei R"" ausgewählt ist aus Wasserstoff oder unsubstituiertem C₁₋₈-Alkyl.

14. Verbindung der Formel (CLII) oder ein Salz davon: 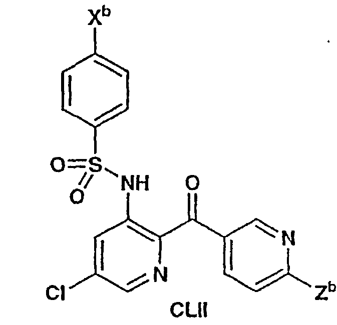 wobei
X^{b} ausgewählt ist aus der Gruppe bestehend aus -OR^{24a} und substituiertem oder unsubstituiertem C₁₋₈-Alkyl;
R^{24a} ausgewählt ist aus der Gruppe bestehend aus Wasserstoff, substituiertem oder unsubstituiertem C₁₋₈-Alkyl, substituiertem oder unsubstituiertem C₂₋ₐ-Alkenyl, substituiertem oder unsubstituiertem C₂₋₈-Alkinyl, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
und
Z^{b} -OR^{30a} ist;
wobei R^{31a} ausgewählt ist aus der Gruppe bestehend aus Wasserstoff und substituiertem oder unsubstituiertem C₁₋₈-Alkyl;
wobei
Substituenten für substituiertes Alkyl, substituiertes Alkenyl und substituiertes Alkinyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R"', -NR"'S(O)NR'R", -NR"'S(O)2NR'R" -SR', -S(O)R', -S(O)₂R', -S(O)₂NR'R", -NR', -C(NHR")=NR"', -SiR'R"R"', -N₃, C₆₋₁₀-Aryl, 5- bis 10-gliedrigem Heteroaryl und 3- bis 10- gliedrigem Heterocyclyl; wobei die Anzahl an möglichen Substituenten von Null bis (2m'+1) reicht, wobei m' die Gesamtanzahl an Kohlenstoffantomen in solch einem Radikal ist;
Substituenten für substituiertes Aryl, substituiertes Heteroaryl und substituiertes Heterocyclyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR"'S(O)NR'R", -NR"'S(O)2NR'R" -SR', -S(O)R', -S(O)₂R', -S(O)₂NR'R", -NR', -C(NHR")=NR"', -SiR'R"R"', -N₃, C₁₋₈-Alkyl, C₂₋₈-Alkenyl, C₂₋₈-Alkinyl, C₆₋₁₀-Aryl, 5- bis 10-gliedrigem Heteroaryl und 3- bis 10-gliedrigem Heterocyclyl; wobei die Anzahl an möglichen Substituenten von Null bis zur Gesamtanzahl an offenen Valenzen an dem aromatischen Ringsystem reicht;
wobei
R', R" und R"' ausgewählt sind aus Wasserstoff, C₁₋₈-Alkyl, C₂₋₈-Alkenyl, C₂₋₈-Alkinyl, Aryl, Heteroaryl, Heterocyclyl, Arylakyl und Aryloxyalkyl; wenn R' und R" an das selbe Stickstoffatom gebunden sind, können sie mit dem Stickstoffatom kombiniert werden, um einen 3-, 4-, 5-, 6- oder 7-gliedrigen Ring zu bilden; wobei R' und R", R" und R'" oder R' und R'" zusammen mit dem/den Atom/Atomen an das/die sie gebunden sind einen 5-, 6- oder 7-gliedrigen Ring bilden können;
zwei der Substituenten an benachbarten Atomen eines Aryl- oder
Heteroarylrings gegebenenfalls durch einen Substituenten der Formel -T-C(O)-(CH₂)q-U- ersetzt werden können, wobei T und U unabhängig voneinander -NR""-, -O-, -CH₂- oder eine Einfachbindung sind und q eine ganze Zahl von 0 bis 2 ist; zwei der Substituenten an benachbarten Atomen des Aryl- oder Heteroarylrings gegebenenfalls durch einen Substituenten der Formel -A'-(CH₂)ᵣ-B'-, ersetzt werden können, wobei A' und B' unabhängig voneinander -CH₂-, -O-, -NR"'-, -S-, -S(O)-, -S(O)₂-, -S(O)₂NR""- oder eine Einfachbindung sind und r eine ganze Zahl von 1 bis 3 ist; wobei eine der Einfachbindungen des neuen Rings, der so gebildet wird, gegebenenfalls durch eine Doppelbindung ersetzt werden kann; zwei der Substituenten an benachbarten Atomen des Aryl- oder Heteroarylrings gegebenenfalls durch einen Substituenten der Formel -(CH₂)ₛ-X-(CH₂)ₜ- ersetzt werden können, wobei s und t unabhängig voneinander ganze Zahlen von 0 bis 3 sind und X -O-, -NR""-, -S-, -S(O)-, -S(O)₂- oder -S(O)₂NR'- ist, wobei R"" ausgewählt ist aus Wasserstoff oder unsubstituiertem C₁₋₈-Alkyl.

15. Verbindung der Formel (CLIII) oder ein Salz davon: 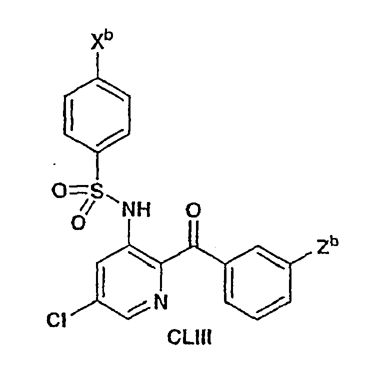 wobei
X^{b} ausgewählt ist aus der Gruppe bestehend aus -OR^{24a} und
substituiertem oder unsubstituiertem C₁₋₈-Alkyl;
R^{24a} ausgewählt ist aus der Gruppe bestehend aus Wasserstoff, substituiertem oder unsubstituiertem C₁₋₈-Alkyl, substituiertem oder unsubstituiertem C₂₋₈-Alkenyl, substituiertem oder unsubstituiertem C₂₋₈-Alkinyl, substituiertem oder unsubstituiertem C₆₋₁₀-Aryl, substituiertem oder unsubstituiertem 5- bis 10-gliedrigem Heteroaryl und substituiertem oder unsubstituiertem 3- bis 10-gliedrigem Heterocyclyl;
und
Z^{b} Halogen ist;
wobei Substituenten für substituiertes Alkyl, substituiertes Alkenyl und substituiertes Alkinyl ausgewählt sind aus Halogen, -CN, -CO₂R' -C(O)R', -C(O)NR'R", oxo (=O oder -O-), -OR', -OC(O)R', -OC(O)NR'R", -NO₂ -NR'C(O)R", -NR"'C(O)NR'R", -NR'R", -NR'CO₂R", -NR'S(O)R", -NR'S(O)₂R"', -NR"'S(O)NR'R", -NR"'S(O)₂NR'R" -SR', -S(O)R', -S(O)₂R', -S(O)₂NR'R", -NR', -C(NHR")=NR"', -SiR'R"R"', -N₃, C₆₋₁₀-Aryl, 5- bis 10-gliedrigem Heteroaryl und 3- bis 10-gliedrigem Heterocyclyl; wobei die Anzahl an möglichen Substituenten von Null bis (2m'+1) reicht, wobei m' die Gesamtanzahl an Kohlenstoffantomen in solch einem Radikal ist;
Substituenten für substituiertes Aryl, substituiertes Heteroaryl und substituiertes Heterocyclyl ausgewählt sind aus Halogen, -CN, -CO₂R', -C(O)R', -C(O)NR'R", oxo (=O oder -O-), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R", -NR"'S(O)NR'R", -NR'"S(O)₂NR'R" -SR', -S(O)R', -S(O)₂R', -S(O)₂NR'R", -NR', -C(NHR")=NR"', -SiR'R"R"', -N₃, C₁₋₈-Alkyl, C₂₋₈-Alkenyl, C₂₋₈-Alkinyl, C₆₋₁₀-Aryl, 5- bis 10-gliedrigem Heteroaryl und 3- bis 10-gliedrigem Heterocyclyl; wobei die Anzahl an möglichen Substituenten von Null bis zur Gesamtanzahl an offenen Valenzen an dem aromatischen Ringsystem reicht;
wobei
R', R" und R'" ausgewählt sind aus Wasserstoff, C₁₋₈-Alkyl, C₂₋₈-Alkenyl, C₂₋₈-Alkinyl, Aryl, Heteroaryl, Heterocyclyl, Arylakyl und Aryloxyalkyl; wenn R' und R" an das selbe Stickstoffatom gebunden sind, können sie mit dem Stickstoffatom kombiniert werden, um einen 3-, 4-, 5-, 6- oder 7-gliedrigen Ring zu bilden; wobei R' und R", R" und R'" oder R' und R"' zusammen mit dem/den Atom/Atomen an das/die sie gebunden sind einen 5-, 6- oder 7-gliedrigen Ring bilden können;
zwei der Substituenten an benachbarten Atomen eines Aryl- oder Heteroarylrings gegebenenfalls durch einen Substituenten der Formel -T-C(O)-(CH₂)q-U- ersetzt werden können, wobei T und U unabhängig voneinander -NR""-, -O-, -CH₂- oder eine Einfachbindung sind und q eine ganze Zahl von 0 bis 2 ist; zwei der Substituenten an benachbarten Atomen des Aryl- oder Heteroarylrings gegebenenfalls durch einen Substituenten der Formel -A'-(CH₂)ᵣ-B'-, ersetzt werden können, wobei A' und B' unabhängig voneinander -CH₂-, -O-, -NR"'-, -S-, -S(O)-, -S(O)₂-, -S(O)₂NR""- oder eine Einfachbindung sind und r eine ganze Zahl von 1 bis 3 ist; wobei eine der Einfachbindungen des neuen Rings, der so gebildet wird, gegebenenfalls durch eine Doppelbindung ersetzt werden kann; zwei der Substituenten an benachbarten Atomen des Aryl- oder Heteroarylrings gegebenenfalls durch einen Substituenten der Formel -(CH₂),-X-(CH₂)ₜ- ersetzt werden können, wobei s und t unabhängig voneinander ganze Zahlen von 0 bis 3 sind und X -O-, -NR""-, -S-, -S(O)-, -S(O)₂- oder -S(O)₂NR'- ist, wobei R"" ausgewählt ist aus Wasserstoff oder unsubstituiertem C₁₋₈-Alkyl.

16. Zusammensetzung, umfassend einen pharmazeutisch akzeptablen Träger und eine Verbindung nach einem der vorhergehenden Ansprüchen.

17. Zusammensetzung, umfassend einen pharmazeutisch akzeptablen Träger und eine Verbindung nach Anspruch 6 oder 7 oder ein Salz davon.

18. Verbindung nach einem der Ansprüche 1 bis 15 zur Verwendung bei der Behandlung eines/einer CCR2-vermittelten Zustands oder Krankheit, wobei der/die CCR2-vermittelte Zustand oder Krankheit ausgewählt ist aus Atherosklerose, Restenose, Multipler Sklerose, Chronisch-Entzündlicher Darmerkrankung, Nierenfibrose, Rheumatoider Arthritis, Adipositas, Diabetes, chronisch obstruktiver Lungenerkrankung, idiopathischer pulmonale Fibrose, idiopathischem Pneumoniesyndrom (idiopathic pneumonia syndrome), Lungenfibrose, Transplantat-Abstoßung, Graft-versus-Host-Reaktion, Krebs und neuropathischem Schmerz.

19. Verbindung nach Anspruch 18, wobei die Verbindung eine Verbindung nach Anspruch 6 oder 7 oder ein Salz davon ist.

20. Verbindung nach Anspruch 18, wobei die/der CCR2-vermittelte Krankheit oder Zustand Atherosklerose ist.

21. Verbindung nach Anspruch 18, wobei die/der CCR2-vermittelte Krankheit oder Zustand Restenose ist.

22. Verbindung nach Anspruch 18, wobei der/die CCR2-vermittelte Zustand oder Krankheit Multiple Sklerose ist.

23. Verbindung nach Anspruch 18, wobei der/die CCR2-vermittelte Zustand oder Krankheit ausgewählt ist aus der Gruppe bestehend aus Chronisch-Entzündlicher Darmerkrankung, Nierenfibrose, Rheumatoider Arthritis, Adipositas und Diabetes.

24. Verbindung nach Anspruch 18, wobei der/die CCR2-vermittelte Zustand oder Krankheit ausgewählt ist aus der Gruppe bestehend aus chronisch obstruktiver Lungenerkrankung, idiopathischer pulmonaler Fibrose und idiopathischem Pneumoniesyndrom.

25. Verbindung nach Anspruch 18, wobei der/die CCR2-vermittelte Zustand oder Krankheit ausgewählt ist aus der Gruppe bestehend aus Lungenfibrose, Transplantat-Abstoßung, Graft-versus-Host-Reaktion und Krebs.

26. Verbindung nach Anspruch 18, wobei der/die CCR2-vermittelte Zustand oder Krankheit neuropathischer Schmerz ist.

27. Verbindung nach Anspruch 18, wobei der Zustand oder die Krankheit durch die Verabreichung der Verbindung auf oralem, parenteralem, rectalem, transdermalem, sublingualem, nasalem oder topischem Weg behandelt wird.

28. Verbindung nach Anspruch 18, wobei die Verbindung in Kombination mit einem entzündungshemmenden Mittel oder Analgetikum verabreicht wird.

29. In vitro Verfahren zur Modulation der CCR2-Funktion in einer Zelle, umfassend in Kontakt bringen der Zelle mit einer CCR2-modulierenden Menge der Verbindung nach einem der Ansprüche 1 bis 15.

30. In vitro Verfahren zur Modulation der CCR9-Funktion in einer Zelle, umfassend in Kontakt bringen der Zelle mit einer CCR9-modulierenden Menge der Verbindung nach einem der Ansprüche 1 bis 15.

## Revendications

1. Composé de formule (I), ou un de ses sels: 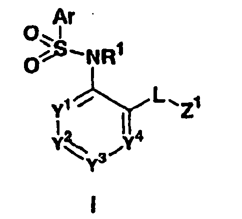 formule dans laquelle
Ar représente un groupe phényle non substitué ou phényle substitué avec un ou plusieurs substituants halogéno, alkyle en C₁ à C₈ substitué ou non substitué, alcényle en C₂ à C₈ substitué ou non substitué, alcynyle en C₂ à C₈ substitué ou non substitué, -CN, -C(O)R¹⁸, -CO₂R¹⁸, -C(O)NR¹⁸R¹⁹, -OC(O)R¹⁹, OC-(O)NR¹⁸R¹⁹, -NO₂, -NR¹⁸C(O)R¹⁹, -NR¹⁸C(O)NR¹⁹R²⁰, -NR¹⁸R¹⁹, -NR¹⁸CO₂R¹⁹, -NR¹⁸S(O)₂R¹⁹, -SR¹⁸, -S(O)R¹⁸, -S(O)₂R¹⁸, -S(O)₂NR¹⁸R¹⁹, aryle en C₆ à C₁₀ substitué ou non substitué, hétéroaryle penta- ou décagonal substitué ou non substitué, et hétérocyclyle tri- à décagonal substitué ou non substitué ;
R¹⁸, R¹⁹ et R²⁰ sont choisis chacun indépendamment dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle substitué ou non substitué, hétéroaryle substitué ou non substitué, et hétérocyclyle substitué ou non substitué ;
R¹ est choisi dans le groupe consistant en un atome d'hydrogène, des groupes alkyle en C₁ non substitué et alkyle en C₃ à C₈ non substitué ;
Y¹ représente un groupe -CR^{2a}- ;
Y² représente un groupe -CR^{2b}- _{;}
Y³ représente un groupe -CR^{2c}- ;
R^{2a}, R^{2b} et R^{2c}, sont choisis chacun indépendamment dans le groupe consistant en un atome d'hydrogène, des groupes halogéno et alkyle en C₁ à C₈ non substitué ;
Y⁴ représente un groupe -N- ;
L représente un groupe -C(O)- ; et
Z¹ représente un groupe phényle substitué ou non substitué ;
où
les substituants pour les groupes alkyle substitué, alcényle substitué et alcynyle substitué sont choisis entre des substituants halogéno, -CN, -CO₂R', -C(O)R', -C(O)NR'R'', oxo (=O ou -O⁻) , -OR', -OC(O)R', -OC(O)NR'R" , -NO₂, -NR'C(O)R", -NR"'C(O)NR'R", -NR'R", -NR'CO₂R", -NR'S(O)R", -NR'S(O)₂R"', -NR'''S(O)NR'R'', -NR"'S(O)₂NR'R", -SR', -S(O)R', -S(O)₂R', -S(O)₂NR'R", -NR'-C(NHR'')=NR''', -SiR'R"R'", -N₃, aryle en C₆ à C₁₀, hétéroaryle penta- à décagonal et hétérocyclyle tri- à décagonal ; le nombre de substituants possibles va de zéro à (2m' + 1), où m' représente le nombre total d'atomes de carbone dans un tel radical ;
les substituants pour les groupes aryle substitué, hétéroaryle substitué et hétérocyclyle substitué sont choisis entre des substituants halogéno, -CN, -CO₂R', -C(O)R', -C(O)NR'R'', oxo (=O ou -O⁻) , -OR', -C)C(O)R', -OC(O)NR'R", -NO₂, -NR'C(O)R", -NR"'C(O)NR'R", -NR'R", -NR'CO₂R", -NR'S(O)R'', -NR'S(O)₂R", -NR"'S(O)NR'R", -NR"'S(O)₂NR'R", -SR', -S(O)R', -S(O)₂R', -S(O)₂NR'R", -NR'-C(NHR")=NR'", -SiR'R"R"', -N₃, alkyle en C₁ à C₈, alcényle en C₂ à C₈, alcynyle en C₂ à C₈, aryle en C₆ à C₁₀, hétéroaryle penta- à décagonal et hétérocyclyle tri- à décagonal ; le nombre de substituants possibles va de zéro au nombre total de valences libres sur le système cyclique aromatique ;
où
R', R" et R"' sont choisis entre un atome d'hydrogène, des groupes alkyle en C₁ à C₈, alcényle en C₂ à C₈, alcynyle en C₂ à C₈, aryle, hétéroaryle, hétérocyclyle, arylalkyle et aryloxyalkyle ; lorsque R' et R" sont fixés au même atome d'azote, ils peuvent être combinés avec l'atome d'azote pour former un noyau tri-, tétra-, penta, hexa- ou heptagonal ; R' et R", R" et R"' ou bien R' et R"' peuvent, conjointement avec le ou les atomes auxquels ils sont fixés, former un noyau penta-, hexa- ou heptagonal ;
deux des substituants sur des atomes adjacents d'un noyau aryle ou hétéroaryle peuvent être facultativement remplacés par un substituant de formule -T-C(O)-(CH₂)_{q}-U-, dans laquelle T et U représentent indépendamment un groupe -NR"'-, -O- , -CH₂- ou une liaison simple, et q représente un nombre entier de 0 à 2 ; deux des substituants sur des atomes adjacents du noyau aryle ou hétéroaryle peuvent être facultativement remplacés par un substituant de formule -A'-(CH₂)ᵣ-B'-, dans laquelle A' et B' représentent indépendamment un groupe -CH₂-, -O-, -NR'''-, -S-, -S(O)-, -S(O)₂-, -S(O)₂NR""- ou une liaison simple, et r représente un nombre entier de 1 à 3 ; une des liaisons simples du noyau nouveau ainsi formé peut être facultativement remplacée par une double liaison ; deux des substituants sur des atomes adjacents du noyau aryle ou hétéroaryle peuvent être facultativement remplacés par un substituant de formule -(CH₂)ₛ-X-(CH₂)ₜ-, dans laquelle s et t représentent indépendamment des nombres entiers de 0 à 3, et X représente un groupe -O-, -NR""-, -S-, -S(O)-, -S(O)₂- ou -S(O)₂NR'-, dans lequel R"" est choisi entre un atome d'hydrogène et un groupe alkyle en C₁ à C₈ non substitué.

2. Composé suivant la revendication 1, qui est représenté par la formule (II), ou un de ses sels : 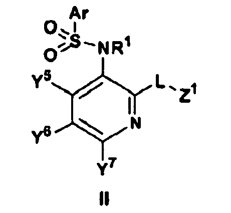 formule dans laquelle
Y⁵, Y⁶ et Y⁷ sont choisis indépendamment dans le groupe consistant en un atome d'hydrogène, un atome d'halogène et un groupe alkyle en C₁ à C₈ non substitué

3. Composé suivant la revendication 1, qui est représenté par la formule (CLIII), ou un de ses sels : 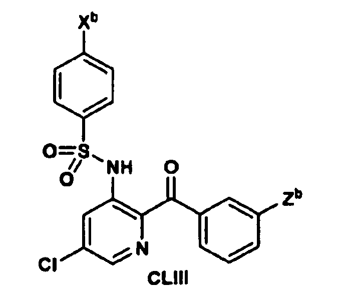 formule dans laquelle
X^{b} est choisi dans le groupe consistant en des groupes CF₃ et alkyle en C₁ à C₈ non substitué ;
Z^{b} représente un atome d'halogène.

4. Composé de formule III, ou un de ses sels : 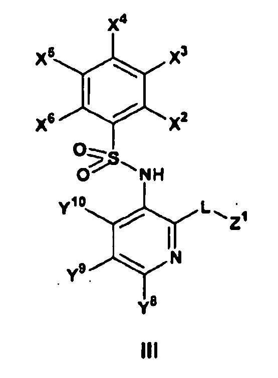 formule dans laquelle
L représente un groupe -C(O)- ;
Z est choisi dans le groupe consistant en des groupes hétéroaryle penta- à décagonaux substitués ou non substitués ;
X², X³, X⁴, X⁵ et X⁶ sont choisis chacun indépendamment dans le groupe consistant en un atome d'hydrogène, un atome d'halogène, des groupes alkyle en C₁ à C₈ substitué ou non substitué, alcényle en C₂ à C₈ substitué ou non substitué, alcynyle en C₂ à C₈ substitué ou non substitué, -CN, -NO₂, -C(O)R¹⁸, -CO₂R¹⁸, -C(O)NR¹⁸R¹⁹, -OR¹⁸, -OC(O)R¹⁹, -OC(O)NR¹⁸R¹⁹, -NR¹⁸C(O)R¹⁹, -NR¹⁸C(O)NR¹⁹R²⁰, -NR¹⁸R¹⁹, -NR¹⁸CO²R¹⁹, -NR¹⁸S(O)₂R¹⁹, -SR¹⁸, -S(O)R¹⁸, -S(O)₂R¹⁸, -S(O)₂NR¹⁸R¹⁹, aryle en C₆ à C₁₀ substitué ou non substitué, hétéroaryle penta- à décagonal substitué ou non substitué et hétérocyclyle tri- à décagonal substitué ou non substitué ;
R¹⁸, R¹⁹ et R²⁰ sont choisis chacun indépendamment dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle en C₆ à C₁₀ substitué ou non substitué, hétéroaryle penta- à décagonal substitué ou non substitué, et hétérocyclyle tri- à décagonal substitué ou non substitué ;
Y⁸, Y⁹ et Y¹⁰ sont choisis chacun indépendamment dans le groupe consistant en un atome d'hydrogène, un atome d'halogène, des groupes -CN, -NO₂, -OR²¹, -CO₂R²¹, -OC(O)R²¹R²², -OC(O)NR²¹R²2, -C(O)NR²¹R²², -C(O)NR²¹R²², -C(O)R²¹, -SR²¹, -S(O)R²¹, -S(O)₂R²¹, NR²¹R²², -NR²¹C(O)R²², -NR²¹C(O)₂R²², -NR²¹S (O)₂R²², -NR²¹C(O)NR²²R²³, alkyle en C₁ à C₈ substitué ou non substitué et hétérocyclyle tri- à décagonal substitué ou non substitué,
R²¹, R²² et R²³ sont choisis chacun indépendamment dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle en C₆ à C₁₀ substitué ou non substitué, hétéroaryle penta- à décagonal substitué ou non substitué, et hétérocyclyle tri- à décagonal substitué ou non substitué ;
où
les substituants pour les groupes alkyle substitué, alcényle substitué et alcynyle substitué sont choisis entre des substituants halogéno, -CN, -CO₂R', -C(O)R', -C(O)NR'R'', oxo (=O ou -O⁻) , -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R"', -NR"'S(O)NR'R", -NR"'S(O)₂NR'R", -SR', -S(O)R', -S(O)₂R', -S(O)₂NR'R", -NR'-C(NHR")=NR"', -SiR'R"R"', -N₃, aryle en C₆ à C₁₀, hétéroaryle penta- à décagonal et hétérocyclyle tri- à décagonal ; le nombre de substituants possibles va de zéro à (2m' + 1), où m' représente le nombre total d'atomes de carbone dans un tel radical ;
les substituants pour les groupes aryle substitué, hétéroaryle substitué et hétérocyclyle substitué sont choisis entre des substituants halogéno, -CN, -CO₂R', -C(O)R', -C(O)NR'R", oxo (=O ou -O⁻), -OR', -OC(O)R', -OC(O)NR'R", -NO₂, -NR'C(O)R", -NR''C(O)NR'R", -NR'R", -NR'CO₂R", -NR'S(O)R", -NR'S(O)₂R", -NR"'S(O)NR'R", -NR"'S(O)₂NR'R", -SR', -S(O)R', -S(O)₂R', -S(O)₂NR'R", -NR'-C(NHR")=NR"', -SiR'R"R'", -N₃, alkyle en C₁ à C₈, alcényle en C₂ à C₈, alcynyle en C₂ à C₈, aryle en C₆ à C₁₀, hétéroaryle penta- à décagonal et hétérocyclyle tri- à décagonal ; le nombre de substituants possibles va de zéro au nombre total de valences libres sur le système cyclique aromatique ;
où
R', R" et R"' sont choisis entre un atome d'hydrogène, des groupes alkyle en C₁ à C₈, alcényle en C₂ à C₈, alcynyle en C₂ à C₈, aryle, hétéroaryle, hétérocyclyle, arylalkyle et aryloxyalkyle ; lorsque R' et R" sont fixés au même atome d'azote, ils peuvent être combinés avec l'atome d'azote pour former un noyau tri-, tétra-, penta, hexa- ou heptagonal ; R' et R", R" et R"' ou bien R' et R"' peuvent, conjointement avec le ou les atomes auxquels ils sont fixés, former un noyau penta-, hexa- ou heptagonal ;
deux des substituants sur des atomes adjacents d'un noyau aryle ou hétéroaryle peuvent être facultativement remplacés par un substituant de formule -T-C(O)-(CH₂)_{q}-U-, dans laquelle T. et U représentent indépendamment un groupe -NR"'-, -O-, -CH₂- ou une liaison simple, et q représente un nombre entier de 0 à 2 ; deux des substituants sur des atomes adjacents du noyau aryle ou hétéroaryle peuvent être facultativement remplacés par un substituant de formule -A'-(CH₂)ᵣ-B'-, dans laquelle A' et B' représentent indépendamment un groupe -CH₂-, -O-, -N"'-, -S-, -S(O)-, -S(O)₂-, -S(O)₂NR"", - ou une liaison simple, et r représente un nombre entier de 1 à 3 ; une des liaisons simples du noyau nouveau ainsi formé peut être facultativement remplacée par une double liaison ; deux des substituants sur des atomes adjacents du noyau aryle ou hétéroaryle peuvent être facultativement remplacés par un substituant de formule -(CH₂)ₛ-X-(CH₂)ₜ-, dans laquelle s et t représentent indépendamment des nombres entiers de 0 à 3, et X représente un groupe -O-, -NR""-, -S-, -S (O) -, -S(O)₂- ou -S(O)₂NR'-, dans lequel R"" est choisi entre un atome d'hydrogène et un groupe alkyle en C₁ à C₈ non substitué.

5. Composé suivant la revendication 4, ou un de ses sels, dans lequel Z¹ est choisi dans le groupe consistant en des groupes hétéroaryle nona- ou décagonaux substitués ou non substitués avec 1 ou 2 atomes d'azote et avec 0 ou 2 substituants choisis chacun indépendamment dans le groupe consistant en des substituants halogéno, alkyle en C₁ à C₈ substitué ou non substitué, alcényle en C₂ à C₈ substitué ou non substitué, alcynyle en C₂ à C₈ substitué ou non substitué, -CN, oxo (=O ou -O⁻), -NO₂ -OR⁴⁴, -OC(O)R⁴⁴, -CO₂R⁴⁴, -C(O)R⁴⁴, -C(O)NR⁴⁴R⁴⁵, -OC(O)NR⁴⁴R⁴⁵, -NR⁴⁴C(O)R⁴⁵, -NR⁴⁴C(O)NR⁴⁵R⁴⁶, -NR⁴⁴R⁴⁵, -NR⁴⁴CO₂R⁴⁵ -SR⁴⁴, -S(O)R⁴⁴ -S(O)₂R⁴⁴, -S(O)₂NR⁴⁴R⁴⁵, -NR⁴⁴S(O)₂R⁴⁵, aryle en C₆ à C₁₀ substitué ou non substitué, hétéroaryle penta- à décagonal substitué ou non substitué et hétérocyclyle tri- à décagonal substitué ou non substitué ;
R⁴⁴, R⁴⁵ et R⁴⁶ sont choisis chacun indépendamment dans le groupe consistant en un atome d'hydrogène, des groupes alkyle en C₁ à C₈ substitué ou non substitué, hétérocyclyle tri- à décagonal substitué ou non substitué, alcényle en C₂ à C₆ substitué ou non substitué, alcynyle en C₂ à C₆ substitué ou non substitué, aryle en C₆ à C₁₀ substitué ou non substitué et hétéroaryle penta- à décagonal substitué ou non substitué.

6. Composé suivant la revendication 5, ou un de ses sels, dans lequel Z¹ représente un groupe 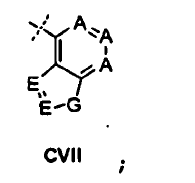 dans lequel
A est choisi dans le groupe consistant en des groupes - CH- et -N- ;
E représente un groupe -CH- ;
G représente un groupe -NR⁴⁷- ; et
R⁴⁷ représente un atome d'hydrogène.

7. Composé suivant la revendication 4, ou un de ses sels, dans lequel Z¹ représente un groupe 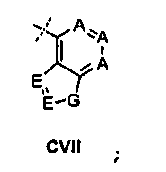 dans lequel
A est choisi dans le groupe consistant en des groupes -CH-, -CZ¹⁶ et -N+(O)⁻- ; chaque occurrence de Z¹⁶ est choisie indépendamment dans le groupe consistant en des groupes alkyle en C₁ à C₈ non substitués ;
E représente un groupe -CH- ;
G représente un groupe -NR⁴⁷- _{;}
R⁴⁷ représente un atome d'hydrogène.

8. Composé suivant la revendication 6 ou 7, ou un de ses sels, dans lequel X⁴ est choisi dans le groupe consistant des groupes halogéno et -CH₃, et X³ est choisi dans le groupe consistant des groupes halogéno, -CH₃, -OCH₃ et -OCF₃.

9. Composé suivant la revendication 8, ou un de ses sels, dans lequel X⁴ représente un groupe chloro et X³ représente un groupe -CF₃.

10. Composé suivant la revendication 9, ou un de ses sels, dans lequel Y⁸, Y⁹ et Y¹⁰ sont choisis chacun indépendamment dans le groupe consistant en un atome d'hydrogène, un atome d'halogène, des groupes -CO₂R³, -OR³, et alkyle en C₁ à C₈ substitué ou non substitué, où 1 ou 2 de Y⁸, Y⁹ et Y¹⁰ sont autres qu'un atome d'hydrogène.

11. Composé suivant la revendication 9, ou un de ses sels, dans lequel Y⁸ et Y¹⁰ représentent des atomes d'hydrogène et Y⁹ représente un atome d'halogène.

12. Composé suivant la revendication 11, ou un de ses sels, dans lequel Y⁸ et Y¹⁰ représentent des atomes d'hydrogène et Y⁹ représente un groupe chloro.

13. Composé de formule (CLI) ou un de ses sels : 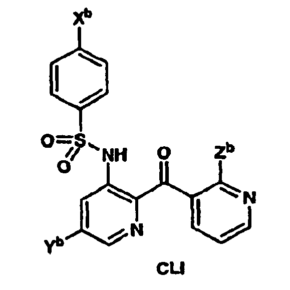 formule dans laquelle
X^{b} est choisi dans le groupe consistant en des groupes -OR^{24a}, et alkyle en C₁ à C₈ substitué ou non substitué
R^{24a} est choisi dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle en C₆ à C₁₀ substitué ou non substitué, hétéroaryle penta- à décagonal substitué ou non substitué, et hétérocyclyle tri- à décagonal substitué ou non substitué ; et
Y^{b} est choisi dans le groupe consistant en un atome d'hydrogène, un atome d'halogène, des groupes -CN, -C(O)R^{27a}, -CO₂R^{27a}, -C(O)NR27^{a}R^{28a}, -OR^{27a}, -OC(O)R^{27a}, -OC(O)NR^{27a}R^{28a}, -SR^{27a}, -S(O)R^{27a}, -S(O)₂R^{27a}, -S(O)₂NR^{27a}R^{28a}, -NO₂, -NR^{27a}R^{28a}, -NR^{27a}C(O)R^{28a}, -NR^{27a}C(O)₂R^{28a}, -NR^{27a}S(O)₂R^{28a}, -NR^{27a}C(O)NR^{28a}R^{29a}, alkyle en C₁ à C₈ substitué ou non substitué, alcényle en C₂ à C₈ substitué ou non substitué, alcynyle en C₂ à C₈ substitué ou non substitué, hétérocyclyle tri à décagonal substitué ou non substitué, aryle en C₆ à C₁₀ substitué ou non substitué, et hétéroaryle penta- à décagonal substitué ou non substitué,
R^{27a}, R^{28a} et R^{29a} sont choisis chacun indépendamment dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle en C₆ à C₁₀ substitué ou non substitué, hétéroaryle penta- à décagonal substitué ou non substitué, et hétérocyclyle tri- à décagonal substitué ou non substitué ; et
R^{27a} et R^{28a}, R^{28a} et R^{29a} ou bien R^{27a} et R^{29a} peuvent, conjointement avec les atomes auxquels ils sont fixés, former un noyau penta-, hexa- ou heptagonal substitué ou non substitué ;
Z^{b} est choisi dans le groupe consistant en des groupes alkyle en C₁ à C₈ substitués ou non substitués ;
où
les substituants pour les groupes alkyle substitué, alcényle substitué et alcynyle substitué sont choisis entre des substituants halogéno, -CN, -CO₂R', -C(O)R', -C(O)NR'R", oxo (=O ou -O⁻), -OR', -OC(O)R', -OC(O)NR'R", -NO₂, -NR'C(O)R", -NR"'C(O)NR'R", -NR'R", -NR'CO₂R", -NR'S(O)R", -NR'S(O)₂R"', -NR"'S(O)NR'R", -NR'"S(O)₂NR'R", -SR', -S(O)R', -S(O)₂R', -S(O)₂NR'R", -NR'-C(NHR")=NR"', -SiR'R"R"', -N₃, aryle en C₆ à C₁₀, hétéroaryle penta- à décagonal et hétérocyclyle tri- à décagonal ; le nombre de substituants possibles va de zéro à (2m' + 1), où m' représente le nombre total d'atomes de carbone dans un tel radical ;
les substituants pour les groupes aryle substitué, hétéroaryle substitué et hétérocyclyle substitué sont choisis entre des substituants halogéno, -CN, -CO₂R', -C(O)R', -C(O)NR'R", oxo (=O ou -O⁻), -R', -OC(O)R', -OC(O)NR'R", -NO₂, -NR'C(O)R", -NR'"C(O)NR'R", -NR'R", -NR'CO₂R", -NR'S(O)R", -NR'S(O)₂R", -NR'"S(O)NR'R", -NR'"S(O)₂NR'R", -SR', -S(O)R', -S(O)₂R', -S(O)₂NR'R", -NR'-C(NHR")=NR"', -SiR'R''R"', -N₃, alkyle en C₁ à C₈, alcényle en C₂ à C₈, alcynyle en C₂ à C₈, aryle en C₆ à C₁₀, hétéroaryle penta- à décagonal et hétérocyclyle tri- à décagonal ; le nombre de substituants possibles va de zéro au nombre total de valences libres sur le système cyclique aromatique ;
où
R', R" et R"' sont choisis entre un atome d'hydrogène, des groupes alkyle en C₁ à C₈, alcényle en C₂ à C₈, alcynyle en C₂ à C₈, aryle, hétéroaryle, hétérocyclyle, arylalkyle et aryloxyalkyle ; lorsque R' et R'' sont fixés au même atome d'azote, ils peuvent être combinés avec l'atome d'azote pour former un noyau tri-, tétra-, penta, hexa- ou heptagonal ; R' et R", R" et R"' ou bien R' et R'" peuvent, conjointement avec le ou les atomes auxquels ils sont fixés, former un noyau penta-, hexa- ou heptagonal ;
deux des substituants sur des atomes adjacents d'un noyau aryle ou hétéroaryle peuvent être facultativement remplacés par un substituant de formule -T-C(O)-(CH₂)_{q}-U-, dans laquelle T et U représentent indépendamment un groupe -NR''''-, -O-, -CH₂- ou une liaison simple, et q représente un nombre entier de 0 à 2 ; deux des substituants sur des atomes adjacents du noyau aryle ou hétéroaryle peuvent être facultativement remplacés par un substituant de formule -A'-(CH₂)ᵣ-B'-, dans laquelle A' et B' représentent indépendamment un groupe -CH₂-, -O-, -NR'"-, -S-, -S(O)-, -S(O)₂-, -S(O)₂NR""- ou une liaison simple, et r représente un nombre entier de 1 à 3 ; une des liaisons simples du noyau nouveau ainsi formé peut être facultativement remplacée par une double liaison ; deux des substituants sur des atomes adjacents du noyau aryle ou hétéroaryle peuvent être facultativement remplacés par un substituant de formule -(CH₂)ₛ-X-(CH₂)ₜ-, dans laquelle s et t représentent indépendamment des nombres entiers de 0 à 3, et X représente un groupe -O-, -NR""-, -S-, -S(O)-, -S(O)₂- ou -S(O)₂NR'-, dans lequel R"" est choisi entre un atome d'hydrogène et un groupe alkyle en C₁ à C₈ non substitué.

14. Composé de formule (CLII) ou un de ses sels : 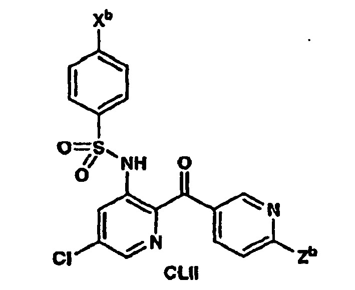 formule dans laquelle
X^{b} est choisi dans le groupe consistant en des groupes -OR^{24a} et alkyle en C₁ à C₈ substitué ou non substitué ;
R^{24a} est choisi dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle en C₆ à C₁₀ substitué ou non substitué, hétéroaryle penta- à décagonal substitué ou non substitué, et hétérocyclyle tri- à décagonal substitué ou non substitué ; et
Z^{b} représente un groupe -OR^{30a} ;
R^{30a} est choisi dans le groupe consistant en un atome d'hydrogène et un groupe alkyle en C₁ à C₈ substitué ou non substitué ;
où
les substituants pour les groupes alkyle substitué, alcényle substitué et alcynyle substitué sont choisis entre des substituants halogéno, -CN, -CO₂R', -C(O)R', -C(O)NR'R'', oxo (=O ou -O⁻), -OR', -OC(O)R', -OC(O)NR'R", -NO₂, -NR'C(O)R", -NR"'C(O)NR'R", -NR'R", -NR'CD₂R", -NR'S(O)R", -NR'S(O)₂R"', -NR"'S(O)NR'R", -NR"'S(O)₂NR'R'",-SR', -S(O)R', -S(O)₂R', -S(O)₂NR'R", -NR'-C(NHR")=NR"', -SiR'R"R"', -N₃, aryle en C₆ à C₁₀, hétéroaryle penta- à décagonal et hétérocyclyle tri- à décagonal ; le nombre de substituants possibles va de zéro à (2m' + 1), où m' représente le nombre total d'atomes de carbone dans un tel radical ;
les substituants pour les groupes aryle substitué, hétéroaryle substitué et hétérocyclyle substitué sont choisis entre des substituants halogéno, -CN, -CO₂R', -C(O)R', -C(O)NR'R", oxo (=O ou -O-), -R', -OC(O)R', -OC(O)NR'R", -NO₂, -NR'C(O)R'', -NR"'C(O)NR'R", NR'R", -NR'CO₂R", -NR'S(O)R", -NR'S(O)₂R", -NR"'S(O)NR'R", -NR'S(O)₂NR'R", -SR', -S(O)R', -S(O)₂R', -S(O)₂NR'R", -NR'-C(NHR")=NR"', -SiR'R''R''', -N₃, alkyle en C₁ à C₈, alcényle en C₂ à C₈, alcynyle en C₂ à C₈, aryle en C₆ à C₁₀, hétéroaryle penta- à décagonal et hétérocyclyle tri- à décagonal ; le nombre de substituants possibles va de zéro au nombre total de valences libres sur le système cyclique aromatique ;
où
R', R'' et R''' sont choisis entre un atome d'hydrogène, des groupes alkyle en C₁ à C₈, alcênyle en C₂ à C₈, alcynyle en C₂ à C₈, aryle, hétéroaryle, hétérocyclyle, arylalkyle et aryloxyalkyle ; lorsque R' et R'' sont fixés au même atome d'azote, ils peuvent être combinés avec l'atome d'azote pour former un noyau tri-, tétra-, penta, hexa- ou heptagonal ; R' et R", R" et R"' ou bien R' et R"' peuvent, conjointement avec le ou les atomes auxquels ils sont fixés, former un noyau penta-, hexa- ou heptagonal ;
deux des substituants sur des atomes adjacents d'un noyau aryle ou hétéroaryle peuvent être facultativement remplacés par un substituant de formule -T-C(O)-(CH₂)_{q}-U-, dans laquelle T et U représentent indépendamment un groupe -NR""-, -O-, -CH₂- ou une liaison simple, et q représente un nombre entier de 0 à 2 ; deux des substituants sur des atomes adjacents du noyau aryle ou hétéroaryle peuvent être facultativement remplacés par un substituant de formule -A'-(CH₂)ᵣ-B'-, dans laquelle A' et B' représentent indépendamment un groupe -CH₂-, -O-, NR"'-, -S-, -S(O)-, -S(O)₂-, -S(O)₂NR""- ou une liaison simple, et r représente un nombre entier de 1 à 3 ; une des liaisons simples du noyau nouveau ainsi formé peut être facultativement remplacée par une double liaison ; deux des substituants sur des atomes adjacents du noyau aryle ou hétéroaryle peuvent être facultativement remplacés par un substituant de formule -(CH₂)ₛ-X-(CH₂)ₜ-, dans laquelle s et t représentent indépendamment des nombres entiers de 0 à 3, et X représente un groupe -O-, -NR""-, -S-, -S(O)-, -S(O)₂- ou -S(O)₂NR'-, dans lequel R"" est choisi entre un atome d'hydrogène et un groupe alkyle en C₁ à C₈ non substitué.

15. Composé de formule (CLIII) ou un de ses sels : 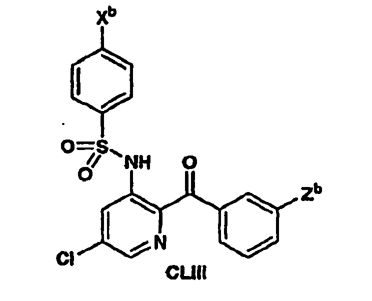 formule dans laquelle
X^{b} est choisi dans le groupe consistant en des groupes -OR^{24a} et alkyle en C₁ à C₈ substitué ou non substitué ;
R^{24a} est choisi dans le groupe consistant en un atome d'hydrogène, des groupes alkyle en C₁ à C₈ substitué ou non substitué, alcényle en C₂ à C₈ substitué ou non substitué, alcynyle en C₂ à C₈ substitué ou non substitué, aryle en C₆ à C₁₀ substitué ou non substitué, hétéroaryle penta- à décagonal substitué ou non substitué, et hétérocyclyle tri- à décagonal substitué ou non substitué ; et
Z^{b} représente un atome d'halogène ;
où
les substituants pour les groupes alkyle substitué, alcényle substitué et alcynyle substitué sont choisis entre des substituants halogéno, -CN, -CO₂R', -C(O)R', -C(O)NR'R'', oxo (=O ou -O⁻), -R', -OC(O)R', -OC(O)NR'R", -NO₂, -NR'C(O)R'', -NR'''C(O)NR'R'', -NR'R", -NR'CO₂R", -NR'S(O)R'', -NR'S(O)₂R"', -NR"'S(O)NR'R", -NR"'S(O)₂NR'R", -SR', -S(O)R', -S(O)₂R', -S(O)₂NR'R", -NR'-C(NHR")=NR"', -SiR'R"R"', -N₃, aryle en C₆ à C₁₀, hétéroaryle penta- à décagonal et hétérocyclyle tri- à décagonal ; le nombre de substituants possibles va de zéro à (2m' + 1), où m' représente le nombre total d'atomes de carbone dans un tel radical ; les substituants pour les groupes aryle substitué, hétéroaryle substitué et hétérocyclyle substitué sont choisis entre des substituants halogéno, -CN, -CO₂R', -C(O)R', -C(O)NR'R", oxo (=O ou -O⁻), -OR', -OC(O)R', -OC(O)NR'R", -NO₂, -NR'C(O)R", -NR'''C(O)NR'R'', -NR'R", -NR'CO₂R", -NR'S(O)R'', -NR'S(O)₂R", -NR"'S(O)NR'R'', -NR"'S(O)₂NR'R", -SR', -S(O)R', -S(O)₂R', -S(O)₂NR'R", -NR'-C(NHR")=NR"', -SiR'R"R"', -N₃, alkyle en C₁ à C₈, alcényle en C₂ à C₈, alcynyle en C₂ à C₈, aryle en C₆ à C₁₀, hétéroaryle penta- à décagonal et hétérocyclyle tri- à décagonal ; le nombre de substituants possibles va de zéro au nombre total de valences libres sur le système cyclique aromatique ; où
R', R'' et R"' sont choisis entre un atome d'hydrogène, des groupes alkyle en C₁ à C₈, alcényle en C₂ à C₈, alcynyle en C₂ à C₈, aryle, hétéroaryle, hétérocyclyle, arylalkyle et aryloxyalkyle ; lorsque R' et R" sont fixés au même atome d'azote, ils peuvent être combinés avec l'atome d'azote pour former un noyau tri-, tétra-, penta, hexa- ou heptagonal ; R' et R", R'' et R"' ou bien R' et R"' peuvent, conjointement avec le ou les atomes auxquels ils sont fixés, former un noyau penta-, hexa- ou heptagonal ;
deux des substituants sur des atomes adjacents d'un noyau aryle ou hétéroaryle peuvent être facultativement remplacés par un substituant de formule -T-C(O)-(CH₂)_{q}-U-, dans laquelle T et U représentent indépendamment un groupe -NR"'-, -O-, -CH₂- ou une liaison simple, et q représente un nombre entier de 0 à 2 ; deux des substituants sur des atomes adjacents du noyau aryle ou hétéroaryle peuvent être facultativement remplacés par un substituant de formule -A'-(CH₂)ᵣ-B'-, dans laquelle A' et B' représentent indépendamment un groupe -CH₂-, -O-, -NR"'-, -S-, -S(O)-, -S(O)₂-, -S(O)₂NR""- ou une liaison simple, et r représente un nombre entier de 1 à 3 ; une des liaisons simples du noyau nouveau ainsi formé peut être facultativement remplacée par une double liaison ; deux des substituants sur des atomes adjacents du noyau aryle ou hétéroaryle peuvent être facultativement remplacés par un substituant de formule -(CH₂)ₛ-X-(CH₂)ₜ-, dans laquelle s et t représentent indépendamment des nombres entiers de 0 à 3, et X représente un groupe -O-, -NR""-, -S-, -S(O)-, -S(O)₂- ou -S(O)₂NR'-, dans lequel R"" est choisi entre un atome d'hydrogène et un groupe alkyle en C₁ à C₈ non substitué.

16. Composition comprenant un support pharmaceutiquement acceptable et un composé de l'une quelconque des revendications précédentes.

17. Composition comprenant un support pharmaceutiquement acceptable et un composé de la revendication 6 ou 7, ou un de ses sels.

18. Composé suivant l'une quelconque des revendications 1 à 15, destiné à être utilisé dans le traitement d'une affection ou maladie à médiation par CCR2, ladite affection ou maladie à médiation par CCR2 étant choisie entre l'athérosclérose, une resténose, la sclérose en plaques, une maladie intestinale inflammatoire, la fibrose rénale, la polyarthrite rhumatoïde, l'obésité, le diabète, une maladie pulmonaire obstructive chronique, la fibrose pulmonaire idiopathique, le syndrome de pneumonie idiopathique, la fibrose pulmonaire, le rejet d'un transplant, la réaction du greffon contre l'hôte, un cancer et une douleur neuropathique.

19. Composé suivant la revendication 18, ledit composé étant un composé de la revendication 6 ou 7 ou un de ses sels.

20. Composé suivant la revendication 18, ladite maladie ou affection à médiation par CCR2 étant l'athérosclérose.

21. Composé suivant la revendication 18, ladite maladie ou affection à médiation par CCR2 étant une resténose.

22. Composé suivant la revendication 18, ladite affection ou maladie à médiation par CCR2 étant la sclérose en plaques.

23. Composé suivant la revendication 18, ladite affection ou maladie à médiation par CCR2 étant choisie dans le groupe consistant en une maladie intestinale inflammatoire, la fibrose rénale, la polyarthrite rhumatoïde, l'obésité et le diabète.

24. Composé suivant la revendication 18, ladite affection ou maladie à médiation par CCR2 étant choisie dans le groupe consistant en une maladie pulmonaire obstructive chronique, la fibrose pulmonaire idiopathique et le syndrome de pneumonie idiopathique.

25. Composé suivant la revendication 18, ladite affection ou maladie à médiation par CCR2 étant choisie dans le groupe consistant en la fibrose pulmonaire, le rejet d'un transplant, la réaction du greffon contre l'hôte et un cancer.

26. Composé suivant la revendication 18, ladite affection ou maladie à médiation par CCR2 étant la douleur neuropathique.

27. Composé suivant la revendication 18, ladite affection ou maladie étant traitée en administrant le composé par la voie orale, parentérale, rectale, transdermique, sublinguale, nasale ou topique.

28. Composé suivant la revendication 18, ledit composé étant administré en association avec un agent anti-inflammatoire ou analgésique.

29. Méthode in vitro pour moduler la fonction de CCR2 dans une cellule, comprenant la mise en contact de la cellule avec une quantité modulatrice de CCR2 du composé de l'une quelconque des revendications 1 à 15.

30. Méthode in vitro pour moduler la fonction de CCR9 dans une cellule, comprenant la mise en contact de la cellule avec une quantité modulatrice de CCR9 du composé de l'une quelconque des revendications 1 à 15.
